# EUROPEAN PATENT APPLICATION

(11) **EP 3 611 259 A1**
(43) Date of publication of application: **19.02.2020**
(21) Application number: 19186620.1
(22) Date of filing: 14.03.2016
(51) Int. Cl.: C12N 9/54, C11D 3/386, C12N 9/50

(54) **COMPOSITIONS AND METHODS COMPRISING LG12-CLADE PROTEASE VARIANTS**

(30) Priority: 12.03.2015 US 201562131834 P
(62) Divisional of application: 16713207.5
(71) Applicant: Danisco US Inc., Palo Alto, CA 94304 (US)
(72) Inventor: AUGUSTYN, Katherine, Palo Alto, CA 94304 (US); BABE, Lilia Maria, Palo Alto, CA 94304 (US); BOTT, Richard R., Palo Alto, CA 94304 (US); SCOTCHER, Miles Christopher, Palo Alto, CA 94304 (US); MULDER, Harm, Palo Alto, CA 94304 (US); MILLER, Jeffrey Veach, Palo Alto, CA 94304 (US); GOEDEGEBUUR, Frits, Palo Alto, CA 94304 (US); ESTELL, David A, Palo Alto, CA 94304 (US); YAO, Jian, Palo Alto, CA 94304 (US)
(74) Representative: Mewburn Ellis LLP

(57) **Abstract**

The present disclosure provides LG12-clade enzyme variants, compositions comprising these enzyme variants, and method of using these enzymes and compositions, as well as the polynucleotides encoding these enzymes, the vectors comprising these polynucleotides, and the host cells transformed with such vectors.

SEQUENCE LISTING

<110>  Danisco US, Inc.

<120>  COMPOSITIONS AND METHODS COMPRISING LG12-CLADE PROTEASE VARIANTS

<130>  007505845

<140>  Divisional of EPA 16713207.5
<141>  2016-03-14

<150>  62/131834
<151>  2015-03-12

<160>  272

<170>  PatentIn version 3.5

<210>  1
<211>  275
<212>  PRT
<213>  artificial sequence

<220>
<223>  LG12-clade protease variant

<400>  1

Thr Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Ser Ile Gly
65                  70                  75                  80

Val Leu Gly Val Ala Pro Asn Ala Glu Leu Tyr Ala Val Lys Val Leu
                85                  90                  95

Gly Ala Ser Gly Ser Gly Ser Val Ser Ser Ile Ala Gln Gly Leu Glu
            100                 105                 110

Trp Ala Gly Asn Asn Gly Met His Val Ala Asn Leu Ser Leu Gly Ser
        115                 120                 125

Pro Ser Pro Ser Ala Thr Leu Glu Gln Ala Val Asn Ser Ala Thr Ser
    130                 135                 140

Arg Gly Val Leu Val Val Ala Ala Ala Gly Asn Ser Gly Thr Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  2
<211>  4
<212>  PRT
<213>  artificial sequence

<220>
<223>  placeholder

<400>  2

Gln Gln Gln Gln
1

<210>  3
<211>  275
<212>  PRT
<213>  artificial sequence

<220>
<223>  LG12-clade protease variant

<400>  3

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
                20                  25                  30

Thr Gly Ile Asp Ala Asn His Ala Asp Leu Asn Val Lys Gly Gly Ala
            35                  40                  45

Ser Phe Val Ala Gly Glu Pro Asn Ala Leu Gln Asp Gly Asn Gly His
    50                  55                  60

Gly Thr His Val Ala Gly Thr Val Ala Ala Leu Asp Asn Ser Ile Gly
65                  70                  75                  80

Val Leu Gly Val Ala Pro Asn Ala Glu Leu Tyr Ala Val Lys Val Leu
                85                  90                  95

Gly Ala Ser Gly Ser Gly Ser Val Ser Ser Ile Ala Gln Gly Leu Glu
            100                 105                 110

Trp Ala Gly Asn Asn Gly Met His Val Ala Asn Leu Ser Leu Gly Ser
        115                 120                 125

Pro Ser Pro Ser Val Thr Leu Glu Gln Ala Val Asn Ser Ala Thr Ser
    130                 135                 140

Arg Gly Val Leu Val Val Ala Ala Ala Gly Asn Ser Gly Thr Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Leu Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser

260                 265                 270

Ala Leu Gln
        275

<210>  4
<211>  275
<212>  PRT
<213>  artificial sequence

<220>
<223>  LG12-clade protease variant

<400>  4

Thr Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Ser Ile Gly
65                  70                  75                  80

Val Leu Gly Val Ala Pro Asn Ala Glu Leu Tyr Ala Val Lys Val Leu
                85                  90                  95

Gly Ala Ser Gly Ser Gly Ser Val Ser Ser Ile Ala Gln Gly Leu Glu
            100                 105                 110

Trp Ala Gly Asn Asn Gly Met His Val Ala Asn Leu Ser Leu Gly Ser
        115                 120                 125

Pro Ser Pro Ser Val Thr Leu Glu Gln Ala Val Asn Ser Ala Thr Ser
    130                 135                 140

Arg Gly Val Leu Val Val Ala Ala Ala Gly Asn Ser Gly Thr Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Tyr Pro Gly Ser Thr Tyr Ala Thr Leu Asn Gly Thr Ser Met Ala Thr
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Ser Ser Thr Ala Thr Tyr
                245                 250                 255

Leu Gly Asp Pro Phe Phe Tyr Gly Lys Gly Val Ile Asn Val Glu Ser
            260                 265                 270

Ala Leu Gln
        275

<210> 5
<211> 275
<212> PRT
<213> artificial sequence

<220>
<223> LG12-clade protease variant

<400> 5

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Ser Ile Gly
65                  70                  75                  80

Val Leu Gly Val Ala Pro Asn Ala Glu Leu Tyr Ala Val Lys Val Leu
                85                  90                  95

Gly Ala Ser Gly Ser Gly Ser Val Ser Ser Ile Ala Gln Gly Leu Glu
            100                 105                 110

Trp Ala Gly Asn Asn Gly Met His Val Ala Asn Leu Ser Leu Gly Ser
        115                 120                 125

Pro Ser Pro Ser Ala Thr Leu Glu Gln Ala Val Asn Ser Ala Thr Ser
    130                 135                 140

Arg Gly Val Leu Val Val Ala Ala Ala Gly Asn Ser Gly Thr Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Tyr Pro Gly Ser Thr Tyr Ala Thr Leu Asn Gly Thr Ser Met Ala Thr
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  6
<211>  275
<212>  PRT
<213>  artificial sequence

<220>
<223>  LG12-clade protease variant

<400>  6

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Ser Ile Gly
65                  70                  75                  80

Val Leu Gly Val Ala Pro Ser Val Ser Leu Tyr Ala Val Lys Val Leu
                85                  90                  95

Gly Ala Ser Gly Ser Gly Ser Val Ser Ser Ile Ala Gln Gly Leu Glu
            100                 105                 110

Trp Ala Gly Asn Asn Gly Met His Val Ala Asn Leu Ser Leu Gly Ser
        115                 120                 125

Pro Ser Pro Ser Ala Thr Leu Glu Gln Ala Val Asn Ser Ala Thr Ser
    130                 135                 140

Arg Gly Val Leu Val Val Ala Ala Ala Gly Asn Ser Gly Thr Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Leu Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  7
<211>  275
<212>  PRT
<213>  artificial sequence

<220>
<223>  LG12-clade protease variant

<400>  7

Thr Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Ser Ile Gly
65                  70                  75                  80

Val Leu Gly Val Ala Pro Asn Ala Glu Leu Tyr Ala Val Lys Val Leu
                85                  90                  95

Gly Ala Ser Gly Ser Gly Ser Val Ser Ser Ile Ala Gln Gly Leu Glu
            100                 105                 110

Trp Ala Gly Asn Asn Gly Met His Val Ala Asn Leu Ser Leu Gly Ser
        115                 120                 125

Pro Ser Pro Ser Ala Thr Leu Glu Gln Ala Val Asn Ser Ala Thr Ser
    130                 135                 140

Arg Gly Val Leu Val Val Ala Ala Ala Gly Asn Ser Gly Thr Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Tyr Pro Gly Ser Thr Tyr Ala Thr Leu Asn Gly Thr Ser Met Ala Thr
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  8
<211>  275
<212>  PRT
<213>  artificial sequence

<220>
<223>  LG12-clade protease variant

<400>  8

Thr Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Ser Ile Gly
65                  70                  75                  80

Val Leu Gly Val Ala Pro Ser Val Ser Leu Tyr Ala Val Lys Val Leu
                85                  90                  95

Gly Ala Ser Gly Ser Gly Ser Val Ser Ser Ile Ala Gln Gly Leu Glu
            100                 105                 110

Trp Ala Gly Asn Asn Gly Met His Val Ala Asn Leu Ser Leu Gly Ser
        115                 120                 125

Pro Ser Pro Ser Ala Thr Leu Glu Gln Ala Val Asn Ser Ala Thr Ser

130                 135                 140

Arg Gly Val Leu Val Val Ala Ala Ala Gly Asn Ser Gly Thr Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Tyr Pro Gly Ser Thr Tyr Ala Thr Leu Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  9
<211>  828
<212>  DNA
<213>  artificial sequence

<220>
<223>  LG12-clade protease variant

<400>  9
acgcaaacag tgccatgggg aattcctcat atcaaagccg acaaagccca tgctgctgga      60

gtgacaggtt ctggtgtaaa agttgctatc ctcgatacag gtattgacgc aaaccatgcc     120

gacttaaatg taaaaggtgg cgctagcttt gtatctgggg aaccaaatgc tcttcaagat     180

gggaatgggc atggcacgca tgtggctggg acggtagctg ctttagataa ttcgattggc     240

gttcttggcg tagcaccgaa cgcggaacta tacgctgtta aagtattagg ggcgagcggt     300

tcaggttcgg tcagctcgat tgcccaagga ttggaatggg cagggaacaa tggcatgcac     360

gttgctaatt tgagtttagg aagcccttcg ccaagtgcca cacttgagca agctgttaat     420

agcgcgactt ctagaggcgt tcttgttgta gcggcagctg ggaattcagg tacatcaggc     480

aatcggaata cgatggatta tccggcccgt tatagctctg taatcgcagt cggagctgtt 540

agtagcaaca acactcgcgc cagcttttca agtgtaggca gtgaacttga agtaatggca 600

ccaggtgtaa acattttgag cacaactcct ggtaacaatt atgccagctt caacggtaca 660

tcgatggctg ctcctcatgt tgcaggtgca gcagccctta tcaaagcgaa gtacccatct 720

atgacaaatg tacaaatccg caatcgtcta aagaatacgg caacgaattt aggagatcct 780

ttcttctatg gaaaaggagt catcaatgtg gaaagtgcat tacaataa 828

<210> 10
<211> 275
<212> PRT
<213> Bacillus sp.

<400> 10

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  11
<211>  10
<212>  DNA
<213>  artificial sequence

<220>
<223>  placeholder

<400>  11
aaaaaaaaaa                                                              10

<210>  12
<211>  828
<212>  DNA
<213>  artificial sequence

<220>
<223>  LG12-clade protease variant

<400>  12
gcgcaaacag tgccatgggg aattcctcat atcaaagccg acaaagccca tgctgctgga      60

gtgacaggtt ctggtgtaaa agttgctatc ctcgatacag gtattgacgc aaaccatgcc     120

gacttaaatg taaaaggtgg cgctagcttt gtagctgggg aaccaaatgc tcttcaagat     180

gggaatgggc atggcacgca tgtggctggg acggtagctg ctttagataa ttcgattggc     240

gttcttggcg tagcaccgaa cgcggaacta tacgctgtta aagtattagg ggcgagcggt     300

tcaggttcgg tcagctcgat tgcccaagga ttggaatggg cagggaacaa tggcatgcac     360

gttgctaatt tgagtttagg aagcccttcg ccaagtgtca cacttgagca agctgttaat 420

agcgcgactt ctagaggcgt tcttgttgta gcggcagctg ggaattcagg tacatcaggc 480

aatcggaata cgatggatta tccggcccgt tatagctctg taatcgcagt cggagctgtt 540

agtagcaaca acactcgcgc cagcttttca agtgtaggca gtgaacttga agtaatggca 600

ccaggtgtaa acattttgag cacaactcct ggtaacaatt atgccagctt aaacggtaca 660

tcgatggctg ctcctcatgt tgcaggtgca gcagccctta tcaaagcgaa gtacccatct 720

atgacaaatg tacaaatccg caatcgtcta aagaatacgg caacgaattt aggagatcct 780

ttcttctatg gaaaaggagt catcaatgtg gaaagtgcat tacaataa 828

<210> 13
<211> 825
<212> DNA
<213> artificial sequence

<220>
<223> LG12-clade protease variant

<400> 13
acgcaaacag tgccatgggg aattcctcat atcaaagccg acaaagccca tgctgctgga 60

gtgacaggtt ctggtgtaaa agttgctatc ctcgatacag gtattgacgc aaaccatgcc 120

gacttaaatg taaaaggtgg cgctagcttt gtatctgggg aaccaaatgc tcttcaagat 180

gggaatgggc atggcacgca tgtggctggg acggtagctg ctttagataa ttcgattggc 240

gttcttggcg tagcaccgaa cgcggaacta tacgctgtta aagtattagg ggcgagcggt 300

tcaggttcgg tcagctcgat tgcccaagga ttggaatggg cagggaacaa tggcatgcac 360

gttgctaatt tgagtttagg aagcccttcg ccaagtgtca cacttgagca agctgttaat 420

agcgcgactt ctagaggcgt tcttgttgta gcggcagctg ggaattcagg tacatcaggc 480

aatcggaata cgatggatta tccggcccgt tatagctctg taatcgcagt cggagctgtt 540

agtagcaaca acactcgcgc cagcttttca agtgtaggca gtgaacttga agtaatggca 600

ccaggtgtaa acattttgag cacataccca ggttcaacgt atgccacatt aaacggtaca 660

tcgatggcta ctcctcatgt tgcaggtgca gcagccctta tcaaagcgaa gtacccatct 720

atgacaaatg tacaaatccg caatcgtcta tccagcacgg caacgtattt aggagatcct 780

ttcttctatg gaaaaggagt catcaatgtg gaaagtgcat tacaa 825

<210> 14
<211> 825
<212> DNA
<213> artificial sequence

<220>
<223> LG12-clade protease variant

<400>  14
gcgcaaacag tgccatgggg aattcctcat atcaaagccg acaaagccca tgctgctgga      60

gtgacaggtt ctggtgtaaa agttgctatc ctcgatacag gtattgacgc aaaccatgcc     120

gacttaaatg taaaaggtgg cgctagcttt gtatctgggg aaccaaatgc tcttcaagat     180

gggaatgggc atggcacgca tgtggctggg acggtagctg ctttagataa ttcgattggc     240

gttcttggcg tagcaccgaa cgcggaacta tacgctgtta aagtattagg ggcgagcggt     300

tcaggttcgg tcagctcgat tgcccaagga ttggaatggg cagggaacaa tggcatgcac     360

gttgctaatt tgagtttagg aagcccttcg ccaagtgcca cacttgagca agctgttaat     420

agcgcgactt ctagaggcgt tcttgttgta gcggcagctg ggaattcagg tacatcaggc     480

aatcggaata cgatggatta tccggcccgt tatagctctg taatcgcagt cggagctgtt     540

agtagcaaca acactcgcgc cagcttttca agtgtaggca gtgaacttga agtaatggca     600

ccaggtgtaa acattttgag cacataccca ggttcaacgt atgccacatt aaacggtaca     660

tcgatggcta ctcctcatgt tgcaggtgca gcagccctta tcaaagcgaa gtacccatct     720

atgacaaatg tacaaatccg caatcgtcta aagaatacgg caacgaattt aggagatcct     780

ttcttctatg gaaaaggagt catcaatgtg gaaagtgcat tacaa                     825

<210>  15
<211>  825
<212>  DNA
<213>  artificial sequence

<220>
<223>  LG12-clade protease variant

<400>  15
gcgcaaacag tgccatgggg aattcctcat atcaaagccg acaaagccca tgctgctgga      60

gtgacaggtt ctggtgtaaa agttgctatc ctcgatacag gtattgacgc aaaccatgcc     120

gacttaaatg taaaaggtgg cgctagcttt gtatctgggg aaccaaatgc tcttcaagat     180

gggaatgggc atggcacgca tgtggctggg acggtagctg ctttagataa ttcgattggc     240

gttcttggcg tagcaccgag cgtatcccta tacgctgtta aagtattagg ggcgagcggt     300

tcaggttcgg tcagctcgat tgcccaagga ttggaatggg cagggaacaa tggcatgcac     360

gttgctaatt tgagtttagg aagcccttcg ccaagtgcca cacttgagca agctgttaat     420

agcgcgactt ctagaggcgt tcttgttgta gcggcagctg ggaattcagg tacatcaggc     480

aatcggaata cgatggatta tccggcccgt tatagctctg taatcgcagt cggagctgtt     540

agtagcaaca acactcgcgc cagcttttca agtgtaggca gtgaacttga agtaatggca     600

ccaggtgtaa acattttgag cacaactcct ggtaacaatt atgccagctt aaacggtaca     660

tcgatggctg ctcctcatgt tgcaggtgca gcagccctta tcaaagcgaa gtacccatct     720

atgacaaatg tacaaatccg caatcgtcta aagaatacgg caacgaattt aggagatcct 780

ttcttctatg gaaaaggagt catcaatgtg gaaagtgcat tacaa 825

<210> 16
<211> 825
<212> DNA
<213> artificial sequence

<220>
<223> LG12-clade protease variant

<400> 16
acgcaaacag tgccatgggg aattcctcat atcaaagccg acaaagccca tgctgctgga 60

gtgacaggtt ctggtgtaaa agttgctatc ctcgatacag gtattgacgc aaaccatgcc 120

gacttaaatg taaaaggtgg cgctagcttt gtatctgggg aaccaaatgc tcttcaagat 180

gggaatgggc atggcacgca tgtggctggg acggtagctg ctttagataa ttcgattggc 240

gttcttggcg tagcaccgaa cgcggaacta tacgctgtta aagtattagg ggcgagcggt 300

tcaggttcgg tcagctcgat tgcccaagga ttggaatggg cagggaacaa tggcatgcac 360

gttgctaatt tgagtttagg aagcccttcg ccaagtgcca cacttgagca agctgttaat 420

agcgcgactt ctagaggcgt tcttgttgta gcggcagctg ggaattcagg tacatcaggc 480

aatcggaata cgatggatta tccggcccgt tatagctctg taatcgcagt cggagctgtt 540

agtagcaaca acactcgcgc cagcttttca agtgtaggca gtgaacttga agtaatggca 600

ccaggtgtaa acattttgag cacataccca ggttcaacgt atgccacatt aaacggtaca 660

tcgatggcta ctcctcatgt tgcaggtgca gcagccctta tcaaagcgaa gtacccatct 720

atgacaaatg tacaaatccg caatcgtcta aagaatacgg caacgaattt aggagatcct 780

ttcttctatg gaaaaggagt catcaatgtg gaaagtgcat tacaa 825

<210> 17
<211> 825
<212> DNA
<213> artificial sequence

<220>
<223> LG12-clade protease variant

<400> 17
acgcaaacag tgccatgggg aattcctcat atcaaagccg acaaagccca tgctgctgga 60

gtgacaggtt ctggtgtaaa agttgctatc ctcgatacag gtattgacgc aaaccatgcc 120

gacttaaatg taaaaggtgg cgctagcttt gtatctgggg aaccaaatgc tcttcaagat 180

gggaatgggc atggcacgca tgtggctggg acggtagctg ctttagataa ttcgattggc 240

gttcttggcg tagcaccgag cgtatcccta tacgctgtta aagtattagg ggcgagcggt 300

tcaggttcgg tcagctcgat tgcccaagga ttggaatggg cagggaacaa tggcatgcac 360

gttgctaatt tgagtttagg aagcccttcg ccaagtgcca cacttgagca agctgttaat 420

agcgcgactt ctagaggcgt tcttgttgta gcggcagctg ggaattcagg tacatcaggc 480

aatcggaata cgatggatta tccggcccgt tatagctctg taatcgcagt cggagctgtt 540

agtagcaaca acactcgcgc cagcttttca agtgtaggca gtgaacttga agtaatggca 600

ccaggtgtaa acattttgag cacgtaccca ggttcaacgt atgccacatt aaacggtaca 660

tcgatggctg ctcctcatgt tgcaggtgca gcagccctta tcaaagcgaa gtacccatct 720

atgacaaatg tacaaatccg caatcgtcta aagaatacgg caacgaattt aggagatcct 780

ttcttctatg gaaaaggagt catcaatgtg gaaagtgcat tacaa 825

<210> 18
<211> 275
<212> PRT
<213> Bacillus sp.

<400> 18

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Arg Gly Gly Ala
        35                  40                  45

Ser Phe Val Ala Gly Glu Pro Asn Ala Leu Gln Asp Gly Asn Gly His
    50                  55                  60

Gly Thr His Val Ala Gly Thr Val Ala Ala Leu Asn Asn Ser Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ala Asn Gly Met Asp Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Ile Gly Tyr Pro Ala Arg Tyr Ser Ser Val Ile Ala
                165                 170                 175

Val Gly Ala Val Asp Ser Asn Asn Asn Arg Ala Ser Phe Ser Ser Val
            180                 185                 190

Gly Asn Glu Leu Glu Val Met Ala Pro Gly Ala Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asp Arg Leu Arg Asn Thr Ala Thr Asn
                245                 250                 255

Leu Gly Asp Pro Phe Phe Tyr Gly Asn Gly Val Ile Asn Val Glu Ser
            260                 265                 270

Ala Leu Gln
        275

<210>  19
<211>  275
<212>  PRT
<213>  Bacillus horikoshii

<400>  19

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Asn Ala Ser Gly Ser Gly Thr Leu Ser Gly Ile Ala Gln Gly Ile Glu
            100                 105                 110

Trp Ser Ile Ala Asn Gly Met Asp Val Ile Asn Met Ser Leu Gly Gly
        115                 120                 125

Ser Thr Gly Ser Thr Ala Leu Gln Gln Ala Ser Asn Asn Ala Tyr Asn
    130                 135                 140

Arg Gly Ile Val Val Ile Ala Ala Ala Gly Asn Ser Gly Ser Leu Gly
145                 150                 155                 160

Asn Arg Asn Thr Ile Gly Tyr Pro Ala Arg Tyr Ser Ser Val Ile Ala
                165                 170                 175

Val Gly Ala Val Asp Ser Asn Asn Asn Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Asn
225                 230                 235                 240

Met Thr Asn Val Gln Ile Arg Asp Arg Leu Arg Asn Thr Ala Thr Asn
                245                 250                 255

Leu Gly Asp Pro Phe Phe Tyr Gly Arg Gly Val Ile Asn Val Glu Ser
            260                 265                 270

Ala Leu Gln
        275

<210> 20
<211> 4
<212> PRT
<213> artificial sequence

<220>
<223> placeholder

<400> 20

Gln Gln Gln Gln
1

<210>  21
<211>  275
<212>  PRT
<213>  Bacillus amyloliquefaciens

<400>  21

<210>  22
<211>  269
<212>  PRT
<213>  Bacillus lentus

<400>  22

Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Ser Ala Glu Leu Tyr Ala Val Lys Val Leu Gly Ala
                85                  90                  95

Ser Gly Ser Gly Ser Val Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Gly Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
    210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Leu
                245                 250                 255

Tyr Gly Ser Gly Leu Val Asn Ala Glu Ala Ala Thr Arg
            260                 265

<210>  23
<211>  274
<212>  PRT
<213>  Bacillus licheniformis

<400>  23

Ala Gln Thr Val Pro Tyr Gly Ile Pro Leu Ile Lys Ala Asp Lys Val
1               5                   10                  15

Gln Ala Gln Gly Phe Lys Gly Ala Asn Val Lys Val Ala Val Leu Asp
            20                  25                  30

Thr Gly Ile Gln Ala Ser His Pro Asp Leu Asn Val Val Gly Gly Ala
        35                  40                  45

Ser Phe Val Ala Gly Glu Ala Tyr Asn Thr Asp Gly Asn Gly His Gly
    50                  55                  60

Thr His Val Ala Gly Thr Val Ala Ala Leu Asp Asn Thr Thr Gly Val
65                  70                  75                  80

Leu Gly Val Ala Pro Ser Val Ser Leu Tyr Ala Val Lys Val Leu Asn
                85                  90                  95

Ser Ser Gly Ser Gly Ser Tyr Ser Gly Ile Val Ser Gly Ile Glu Trp
            100                 105                 110

Ala Thr Thr Asn Gly Met Asp Val Ile Asn Met Ser Leu Gly Gly Ala
        115                 120                 125

Ser Gly Ser Thr Ala Met Lys Gln Ala Val Asp Asn Ala Tyr Ala Arg
    130                 135                 140

Gly Val Val Val Val Ala Ala Ala Gly Asn Ser Gly Ser Ser Gly Asn
145                 150                 155                 160

Thr Asn Thr Ile Gly Tyr Pro Ala Lys Tyr Asp Ser Val Ile Ala Val
                165                 170                 175

Gly Ala Val Asp Ser Asn Ser Asn Arg Ala Ser Phe Ser Ser Val Gly
            180                 185                 190

Ala Glu Leu Glu Val Met Ala Pro Gly Ala Gly Val Tyr Ser Thr Tyr
        195                 200                 205

Pro Thr Asn Thr Tyr Ala Thr Leu Asn Gly Thr Ser Met Ala Ser Pro
    210                 215                 220

His Val Ala Gly Ala Ala Ala Leu Ile Leu Ser Lys His Pro Asn Leu
225                 230                 235                 240

Ser Ala Ser Gln Val Arg Asn Arg Leu Ser Ser Thr Ala Thr Tyr Leu
                245                 250                 255

Gly Ser Ser Phe Tyr Tyr Gly Lys Gly Leu Ile Asn Val Glu Ala Ala
            260                 265                 270

Ala Gln

<210>  24
<211>  269
<212>  PRT
<213>  Bacillus gibsonii

<400>  24

Gln Gln Thr Val Pro Trp Gly Ile Thr Arg Val Gln Ala Pro Ala Val
1               5                   10                  15

His Asn Arg Gly Ile Thr Gly Ser Gly Val Arg Val Ala Ile Leu Asp
            20                  25                  30

Ser Gly Ile Ser Ala His Ser Asp Leu Asn Ile Arg Gly Gly Ala Ser
        35                  40                  45

Phe Val Pro Gly Glu Pro Thr Thr Ala Asp Leu Asn Gly His Gly Thr
    50                  55                  60

His Val Ala Gly Thr Val Ala Ala Leu Asn Asn Ser Ile Gly Val Ile
65                  70                  75                  80

Gly Val Ala Pro Asn Ala Glu Leu Tyr Ala Val Lys Val Leu Gly Ala
                85                  90                  95

Asn Gly Ser Gly Ser Val Ser Gly Ile Ala Gln Gly Leu Glu Trp Ala
            100                 105                 110

Ala Thr Asn Asn Met His Ile Ala Asn Met Ser Leu Gly Ser Asp Phe
        115                 120                 125

Pro Ser Ser Thr Leu Glu Arg Ala Val Asn Tyr Ala Thr Ser Arg Asp
    130                 135                 140

Val Leu Val Ile Ala Ala Thr Gly Asn Asn Gly Ser Gly Ser Val Gly
145                 150                 155                 160

Tyr Pro Ala Arg Tyr Ala Asn Ala Met Ala Val Gly Ala Thr Asp Gln
                165                 170                 175

Asn Asn Arg Arg Ala Asn Phe Ser Gln Tyr Gly Thr Gly Ile Asp Ile
            180                 185                 190

Val Ala Pro Gly Val Asn Val Gln Ser Thr Tyr Pro Gly Asn Arg Tyr
        195                 200                 205

Val Ser Met Asn Gly Thr Ser Met Ala Thr Pro His Val Ala Gly Ala
    210                 215                 220

Ala Ala Leu Val Lys Gln Arg Tyr Pro Ser Trp Asn Ala Thr Gln Ile
225                 230                 235                 240

Arg Asn His Leu Lys Asn Thr Ala Thr Asn Leu Gly Asn Ser Ser Gln
                245                 250                 255

Phe Gly Ser Gly Leu Val Asn Ala Glu Ala Ala Thr Arg
            260                 265

<210>  25
<211>  277
<212>  PRT
<213>  Bacillus agaradhaerens

<400>  25

Gln Gln Gln Thr Pro Trp Gly Ile Thr Arg Val Gln Gly Ile Ala Ala
1               5                   10                  15

Gln Ser Gln Gly Tyr Thr Gly Asn Asn Val Lys Val Ala Val Leu Asp
            20                  25                  30

Ser Gly Ile Asp Arg Ser His Pro Asp Leu Ser Ala Asn Val Arg Gly
        35                  40                  45

Gly Tyr Ser Val Phe Gly Asp Ser Pro Tyr Asn Asp Gly Asn Gly His
    50                  55                  60

Gly Thr His Val Ala Gly Thr Val Gly Ala Val Asn Asn Asn Ile Gly
65                  70                  75                  80

Val Ile Gly Val Ala Pro Gln Ala Asp Leu Tyr Ala Val Lys Val Leu
                85                  90                  95

Asn Asn Ser Gly Ser Gly Ser Tyr Ala Gly Ile Ala Gln Gly Ile Glu
            100                 105                 110

Trp Ser Ile Asn Asn Gly Met Asp Ile Ile Asn Met Ser Leu Gly Gly
        115                 120                 125

Ser Ser Ser Ser Ser Ile Leu Glu Gln Tyr Cys Asn Leu Ala Tyr Asn
    130                 135                 140

Arg Gly Leu Leu Val Val Ala Ala Ala Gly Asn Ser Gly Thr Ala Ala
145                 150                 155                 160

Gly Asn Thr Asn Thr Val Gly Tyr Pro Ala Arg Tyr Asn Ser Val Ile
                165                 170                 175

Ala Val Ala Ala Thr Asn Ser Asn Asn Val Arg Ala Asn Phe Ser Ser
            180                 185                 190

Thr Gly Pro Thr Val Glu Leu Ser Ala Pro Gly Val Ser Val Leu Ser
        195                 200                 205

Thr Thr Pro Gly Gly Asn Tyr Ala Ser Tyr Asn Gly Thr Ser Met Ala
    210                 215                 220

Ser Pro His Val Ala Gly Val Ala Ala Gln Val Trp Gln Ala Arg Pro
225                 230                 235                 240

Asn Leu Ser Asn Ala Gln Leu Arg Gln Ile Leu Asn Ala Ser Ala Gln
                245                 250                 255

Asn Leu Gly Ser Ser Tyr Gln Tyr Gly Asn Gly Leu Val Arg Ser Leu
            260                 265                 270

Asn Ala Ile Gln Tyr
        275

<210> 26
<211> 276
<212> PRT
<213> Bacillus sp.

<400> 26

Met His Pro Asn Gln Gln Trp His Tyr Asn Met Ile Asn Ala Pro Gln
1               5                   10                  15

Ala Trp Gly Thr Thr Thr Gly Ser Ser Ser Val Ile Gln Ala Val Leu
            20                  25                  30

Asp Thr Gly Ile Asp His Asn His Gln Ser Leu Ala Asn Leu Val Asn
        35                  40                  45

Thr Ser Leu Gly Gln Ser Phe Val Gly Gly Ser Thr Met Asp Val Gln
    50                  55                  60

Gly His Gly Thr His Val Ala Gly Thr Ile Ala Ser Tyr Gly Ser Val
65                  70                  75                  80

Ser Gly Val Met His Asn Ala Thr Leu Val Pro Val Lys Val Leu Asn
                85                  90                  95

Asp Ser Gly Ser Gly Ser Leu Phe Gly Ile Thr Gln Gly Ile Leu Tyr
            100                 105                 110

Ser Ala Asp Ile Gly Ala Asp Val Ile Asn Met Ser Leu Gly Gly Gly
        115                 120                 125

Gly Tyr Asn Gln Ser Met Ala Glu Ala Ala Gln Thr Ala Val Asn Ala
    130                 135                 140

Gly Ser Ile Val Ile Ala Ala Ser Gly Asn Asp Gly Ala Gly Ser Ile
145                 150                 155                 160

Ser Tyr Pro Ala Ala Tyr Ser Ser Val Ile Ala Val Gly Ser Val Thr
                165                 170                 175

Ser Thr Gly Ala Arg Ser Asn Phe Ser Asn Tyr Gly Ser Gly Leu Glu
            180                 185                 190

Leu Met Ala Pro Gly Ser Asn Ile Tyr Ser Thr Val Pro Asn Asn Gly
        195                 200                 205

Tyr Ala Thr Phe Ser Gly Thr Ser Met Ala Ser Pro His Ala Ala Gly
    210                 215                 220

Val Ala Gly Leu Met Arg Ala Val Asn Pro Asn Leu Ser Val Ser Asn
225                 230                 235                 240

Ala Arg Ser Ile Met Gln Asn Thr Ala Gln Tyr Ala Gly Ser Pro Thr
                245                 250                 255

Phe Tyr Gly Tyr Gly Ile Val Asp Ala Asn Ala Ala Val Gln Gln Ala
            260                 265                 270

Ser Gly Gly Ser
        275

<210>  27
<211>  269
<212>  PRT
<213>  Bacillus sp.

<400>  27

Ala Gln Ala Ile Pro Trp Gly Ile Ser Gln Ile Ser Ala Pro Glu Ala
1               5                   10                  15

Gln Ile Ala Gly Phe Thr Gly Glu Gly Val Asn Val Ala Val Leu Asp
            20                  25                  30

Thr Gly Ile Glu Asp His Pro Asp Leu Asn Val Gln Gly Gly Val Ser
        35                  40                  45

Phe Val Gln Gly Glu Pro Asp Tyr Gln Asp Gly Asn Gly His Gly Thr
    50                  55                  60

His Val Ala Gly Thr Ile Ala Ala Leu Asp Asn Asp Glu Gly Val Ile
65                  70                  75                  80

Gly Val Ala Pro Asn Ala Asp Leu Tyr Ala Val Lys Val Leu Gly Ala
                85                  90                  95

Asn Gly Ser Gly Ser Val Ser Ser Ile Ala Gln Gly Leu Glu Trp Ala
            100                 105                 110

Gly Glu Asn Gly Met Asp Ile Ala Asn Leu Ser Leu Gly Ser Ser Ala
        115                 120                 125

Pro Ser Ala Thr Leu Glu Gln Ala Val Asp Glu Ala Thr Ala Asn Gly
    130                 135                 140

Val Leu Val Val Ala Ala Ser Gly Asn Ser Gly Ala Ser Ser Ile Gly
145                 150                 155                 160

Tyr Pro Ala Arg Tyr Asp Asn Ala Met Ala Val Gly Ala Thr Asp Gln
                165                 170                 175

Ser Asp Gly Leu Ala Ser Phe Ser Gln Tyr Gly Asp Gly Leu Asp Ile
            180                 185                 190

Val Ala Pro Gly Val Gly Ile Asp Ser Thr Tyr Pro Gly Ser Ser Tyr
        195                 200                 205

Asp Ser Leu Ser Gly Thr Ser Met Ala Thr Pro His Val Ala Gly Ala
    210                 215                 220

Ala Ala Leu Val Lys Glu Lys Asn Pro Leu Trp Ser Asn Glu Gln Ile
225                 230                 235                 240

Arg Ala His Leu Asn Glu Thr Ala Thr Asp Leu Gly Asp Met Tyr Arg
                245                 250                 255

Phe Gly Asn Gly Leu Leu Asn Ala His Ala Ala Val Glu
            260                 265

<210>  28
<211>  276
<212>  PRT
<213>  Bacillus sp.

<400>  28

Ser Gln Thr Val Pro Tyr Gly Ile Glu Asn Ile Gly Ala Val Asp Val
1               5                   10                  15

Gln Gln Asn Asp Gly Asn Thr Gly Asp Gly Val Ser Val Ala Val Leu
            20                  25                  30

Asp Thr Gly Ile Gln Pro His Glu Asp Leu Asn Val Leu Gly Gly Ala
        35                  40                  45

Ser Phe Ile Ser Asp Glu Pro Thr Trp Glu Asp Phe Asn Gly His Gly
    50                  55                  60

Thr His Val Ala Gly Thr Val Ala Ala Leu Asp Asn Asp Leu Gly Val
65                  70                  75                  80

Leu Gly Val Ser Pro Asp Val Asp Leu Tyr Ala Val Lys Val Leu Gly
                85                  90                  95

Ala Ser Gly Gly Gly Ser His Ala Ser Ile Ala Gln Gly Ile Glu Trp
            100                 105                 110

Ala Val Gln Asn Asp Met Asp Ile Val Asn Met Ser Leu Gly Ser Ser
        115                 120                 125

Thr Gly Ser Thr Thr Leu Arg Gln Ala Val Asp Tyr Ala His Gly Glu
    130                 135                 140

Gly Val Thr Leu Val Ala Ala Ala Gly Asn Ser Gly Ser Phe Leu Phe
145                 150                 155                 160

Leu Asn Thr Ile Gly Tyr Pro Ala Lys Tyr Asp Asn Val Ile Ala Val
                165                 170                 175

Gly Ala Val Asp Glu Asn Asn Ser Arg Ala Ser Phe Ser Ser Val Gly
            180                 185                 190

Asn Gln Leu Asp Val Met Ala Pro Gly Val Ala Thr His Ser Thr Tyr
        195                 200                 205

Leu Asp Asn Ser Tyr Ala Ser Leu Ser Gly Thr Ser Met Ala Ser Pro
    210                 215                 220

His Val Ala Gly Ala Ala Ala Leu Leu Leu Ala His Asn Pro Val Val
225                 230                 235                 240

Thr Asn Glu Gly Ile Arg Glu Ala Leu Asn Ser Thr Ala Ile Pro Leu
                245                 250                 255

Gly Ser His Phe Tyr Tyr Gly Asn Gly Val Ile Asp Val Arg Ala Ala
            260                 265                 270

Leu Glu Ala Gln
        275

<210>  29
<211>  275
<212>  PRT
<213>  artificial sequence

<220>
<223>  LG12-clade protease variant

<400>  29

Gly Gln Thr Val Pro Trp Gly Ile Pro His Ile Lys Ala Asp Lys Ala
1               5                   10                  15

His Ala Ala Gly Val Thr Gly Ser Gly Val Lys Val Ala Val Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Asp Thr Gly
65                  70                  75                  80

Val Leu Gly Val Ala Tyr Asn Ala Asp Leu Tyr Ala Thr Lys Val Leu
                85                  90                  95

Ser Ala Ser Gly Ser Gly Glu Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Asp Gly
145                 150                 155                 160

Asn Arg Asn Thr Met Ser Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Pro Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Asn Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  30
<211>  275
<212>  PRT
<213>  artificial sequence

<220>
<223>  LG12-clade protease variant

<400>  30

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Gly Ile Ala Ala Ala Gly Asn Asn Gly Ser Ser Gly
145                 150                 155                 160

Asn Arg Asn Thr Met His Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210> 31
<211> 275
<212> PRT
<213> artificial sequence

<220>
<223> LG12-clade protease variant

<400> 31

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Gly Ile Ala Ala Ala Gly Asn Asn Gly Ser Asp Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  32
<211>  275
<212>  PRT
<213>  artificial sequence

<220>
<223>  LG12-clade protease variant

<400>  32

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp

20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Gly Ile Ala Ala Ala Gly Asn Asn Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  33
<211>  275
<212>  PRT
<213>  artificial sequence

<220>
<223>  LG12-clade protease variant

<400>  33

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Gly Ile Ala Ala Ala Gly Asn Asn Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Glu Asn Asn Thr Arg Ala Ser Phe Ser Ser Val
            180                 185                 190

Gly Ser Glu Leu Glu Val Met Ala Pro Gly Val Asn Ile Leu Ser Thr

195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  34
<211>  275
<212>  PRT
<213>  artificial sequence

<220>
<223>  LG12-clade protease variant

<400>  34

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Leu Ile Ala Ala Ala Gly Asn His Gly Ser Asp Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Gln Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Gln
            260                 265                 270

Ala Leu Gln
        275

<210>  35
<211>  275
<212>  PRT
<213>  artificial sequence

<220>
<223>  LG12-clade protease variant

<400>  35

Gly Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Gln Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Gly Ile Ala Ala Ala Gly Asn Asn Gly Ser Asn Gly
145                 150                 155                 160

Asn Arg Asn Thr Met Gly Tyr Pro Ala Arg Tyr Ser Ser Val Ile Ala
                165                 170                 175

Val Gly Ala Val Ser Glu Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Phe Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Gln
            260                 265                 270

Ala Leu Gln
        275

<210>  36
<211>  275

<212>  PRT
<213>  artificial sequence

<220>
<223>  LG12-clade protease variant

<400>  36

Gly Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Gln Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Thr Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Asn Gly
145                 150                 155                 160

Asn Arg Asn Thr Met Gly Tyr Pro Ala Arg Tyr Ser Ser Val Ile Ala
                165                 170                 175

Val Gly Ala Val Ser Glu Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Phe Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Gln
            260                 265                 270

Ala Leu Gln
        275

<210>  37
<211>  275
<212>  PRT
<213>  artificial sequence

<220>
<223>  LG12-clade protease variant

<400>  37

Gly Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Leu Asp Leu Asn Val Lys Gly Gly Ala
        35                  40                  45

Ser Phe Val Ser Glu Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Lys Ser Gly Thr Leu Ser Gly Ile Ala Gln Gly Ile Glu
            100                 105                 110

Trp Ser Ile Ser Glu Gly Met Asn Val Ile Asn Met Ser Leu Gly Asn
        115                 120                 125

Ser Ser Gly Ser Thr Ala Leu Gln Gln Ala Cys Asn Asn Ala Tyr Asn
    130                 135                 140

Asn Gly Ile Val Val Ile Ala Ala Ala Gly Asn Ser Gly Ser Asp Gly
145                 150                 155                 160

Asn Arg Asn Thr Met Asn Tyr Pro Ala Arg Tyr Ser Ser Val Ile Ala
                165                 170                 175

Val Gly Ala Val Ser Ala Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Leu Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Arg Asn Thr Ala Thr Arg
                245                 250                 255

Leu Gly Asp Pro Phe Phe Tyr Gly Lys Gly Val Ile Asn Val Phe Ser
            260                 265                 270

Ala Leu Gln
        275

<210>  38
<211>  275
<212>  PRT
<213>  artificial sequence

<220>
<223>  LG12-clade protease variant

<400>  38

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Gln His Ala Asp Leu Asn Val Lys Gly Gly Ala
        35                  40                  45

Ser Phe Val Ser Glu Glu Pro Asn Ala Leu Gln Asp Gly Asn Gly His
    50                  55                  60

Gly Thr His Val Ala Gly Thr Val Ala Ala Leu Asn Asn Thr Thr Gly

65                  70                  75                  80

Val Leu Gly Val Ala Tyr Asn Ala Asp Leu Tyr Ala Val Lys Val Leu
                85                  90                  95

Ser Ala Ser Ala Ser Gly Thr Leu Ser Gly Ile Ala Gln Gly Ile Glu
            100                 105                 110

Trp Ser Ile Ser Ser Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ala Val Val Ile Ala Ala Ala Gly Asn Ser Gly Thr Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Pro
                245                 250                 255

Leu Gly Asp Pro Phe Phe Tyr Gly Lys Gly Val Ile Asn Val Glu Lys
            260                 265                 270

Ala Leu Gln
        275

<210>  39
<211>  275
<212>  PRT
<213>  artificial sequence

<220>
<223>  LG12-clade protease variant

<400>  39

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Gln His Ala Asp Leu Asn Val Lys Gly Gly Ala
        35                  40                  45

Ser Phe Val Ser Glu Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Ala Ser Gly Thr Leu Ser Gly Ile Ala Gln Gly Ile Glu
            100                 105                 110

Trp Ser Ile Ser Ser Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ala Val Val Ile Ala Ala Ala Gly Asn Ser Gly Thr Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn

245                 250                 255

Leu Gly Asp Pro Phe Phe Tyr Gly Lys Gly Val Ile Asn Val Glu Lys
            260                 265                 270

Ala Leu Gln
        275

<210>  40
<211>  275
<212>  PRT
<213>  artificial sequence

<220>
<223>  LG12-clade protease variant

<400>  40

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Gln His Ala Asp Leu Asn Val Lys Gly Gly Ala
        35                  40                  45

Ser Phe Val Ser Glu Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Ser Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ala Val Val Ile Ala Ala Ala Gly Asn Ser Gly Thr Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Pro
                245                 250                 255

Leu Gly Asp Pro Phe Phe Tyr Gly Lys Gly Val Ile Asn Val Glu Lys
            260                 265                 270

Ala Leu Gln
        275

<210> 41
<211> 275
<212> PRT
<213> artificial sequence

<220>
<223> LG12-clade protease variant

<400> 41

Ala Gln Thr Val Pro Trp Gly Ile Pro His Ile Lys Ala Asp Lys Ala
1               5                   10                  15

His Ala Ala Gly Val Thr Gly Thr Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Glu Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Leu Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Pro
                245                 250                 255

Leu Gly Asp Pro Phe Phe Tyr Gly Lys Gly Val Ile Asn Val Glu Ser
            260                 265                 270

Ala Leu Gln
        275

<210> 42
<211> 275
<212> PRT
<213> artificial sequence

<220>
<223> LG12-clade protease variant

<400> 42

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Asp Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Arg
                245                 250                 255

Leu Gly Asp Pro Phe Phe Tyr Gly Lys Gly Val Ile Asn Val Glu Ser
            260                 265                 270

Ala Leu Gln
        275

<210>  43
<211>  275
<212>  PRT
<213>  artificial sequence

<220>
<223>  LG12-clade protease variant

<400>  43

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Asp Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Thr Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ala Val Val Ile Ala Ala Ala Gly Asn Ser Gly Ser Asn Gly
145                 150                 155                 160

Asn Arg Asn Thr Met Asp Tyr Pro Ala Arg Tyr Ser Ser Val Ile Ala
                165                 170                 175

Val Gly Ala Val Ser Glu Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  44
<211>  14
<212>  PRT
<213>  artificial sequence

<220>
<223>  LG12-clade sequence motif

<400>  44

Glu Pro Asn Ala Leu Gln Asp Gly Asn Gly His Gly Thr His
1               5                   10

<210>  45
<211>  275
<212>  PRT
<213>  Bacillus horikoshii

<400>  45

Ala Gln Thr Val Pro Trp Gly Ile Pro His Ile Lys Ala Asp Lys Ala
1               5                   10                  15

His Ala Ser Gly Val Thr Gly Ser Gly Val Lys Val Ala Ile Leu Asp
            20                  25                  30

Thr Gly Ile Asp Ala Asn His Thr Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Ser Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ala Asn Gly Met Asn Val Ile Asn Met Ser Leu Gly Gly
        115                 120                 125

Ser Thr Gly Ser Thr Ala Leu Gln Gln Ala Cys Asp Asn Ala Tyr Ala
    130                 135                 140

Arg Gly Ile Val Val Ile Ala Ala Ala Gly Asn Ser Gly Ser Arg Gly
145                 150                 155                 160

Lys Gln Asn Thr Met Gly Tyr Pro Ala Arg Tyr Ser Ser Val Ile Ala
                165                 170                 175

Val Gly Ala Val Asp Ser Ser Asn Asn Arg Ala Ser Phe Ser Ser Val
            180                 185                 190

Gly Asn Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ser
    210                 215                 220

Pro His Val Ala Gly Ala Ala Ala Leu Ile Lys Ala Lys Tyr Pro Ser
225                 230                 235                 240

Met Thr Asn Val Gln Ile Arg Asp Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  46
<211>  275
<212>  PRT
<213>  Bacillus horikoshii

<400>  46

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Val Ala Asn Gly Met Asp Val Ile Asn Met Ser Leu Gly Gly
        115                 120                 125

Ser Ser Gly Ser Ser Ala Leu Gln Gln Ala Cys Asp Asn Ala Tyr Ala
    130                 135                 140

Arg Gly Val Val Val Ile Ala Ala Ala Gly Asn Ser Gly Ser Arg Gly
145                 150                 155                 160

Lys Gln Asn Thr Met Gly Tyr Pro Ala Arg Tyr Ser Ser Val Ile Ala
                165                 170                 175

Val Gly Ala Val Asp Ser Ser Asn Asn Arg Ala Ser Phe Ser Ser Val
            180                 185                 190

Gly Asn Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ser
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser

260                 265                 270

Ala Leu Gln
        275

<210>  47
<211>  4
<212>  PRT
<213>  artificial sequence

<220>
<223>  placeholder

<400>  47

Gln Gln Gln Gln
1

<210>  48
<211>  275
<212>  PRT
<213>  artificial sequence

<220>
<223>  BPN' mutant

<400>  48

Ala Gln Ser Val Pro Tyr Gly Val Ser Gln Ile Lys Ala Pro Ala Leu
1               5                   10                  15

His Ser Gln Gly Tyr Thr Gly Ser Asn Val Lys Val Ala Val Ile Asp
            20                  25                  30

Ser Gly Ile Asp Ser Ser His Pro Asp Leu Lys Val Ala Gly Gly Ala
        35                  40                  45

Ser Met Val Pro Ser Glu Thr Asn Pro Phe Gln Asp Asn Asn Ser His
    50                  55                  60

Gly Thr His Val Ala Gly Thr Val Ala Ala Leu Asn Asn Ser Ile Gly
65                  70                  75                  80

Val Leu Gly Val Ala Pro Ser Ala Ser Leu Tyr Ala Val Lys Val Leu
                85                  90                  95

Gly Ala Asp Gly Ser Gly Gln Tyr Ser Trp Ile Ile Asn Gly Ile Glu
            100                 105                 110

Trp Ala Ile Ala Asn Asn Met Asp Val Ile Asn Met Ser Leu Gly Gly
        115                 120                 125

Pro Ser Gly Ser Ala Ala Leu Lys Ala Ala Val Asp Lys Ala Val Ala
    130                 135                 140

Ser Gly Val Val Val Val Ala Ala Ala Gly Asn Glu Gly Thr Ser Gly
145                 150                 155                 160

Ser Ser Ser Thr Val Gly Tyr Pro Gly Lys Tyr Pro Ser Val Ile Ala
                165                 170                 175

Val Gly Ala Val Asp Ser Ser Asn Gln Arg Ala Ser Phe Ser Ser Val
            180                 185                 190

Gly Pro Glu Leu Asp Val Met Ala Pro Gly Val Ser Ile Gln Ser Thr
        195                 200                 205

Leu Pro Gly Asn Lys Tyr Gly Ala Leu Asn Gly Thr Ser Met Ala Ser
    210                 215                 220

Pro His Val Ala Gly Ala Ala Ala Leu Ile Leu Ser Lys His Pro Asn
225                 230                 235                 240

Trp Thr Asn Thr Gln Val Arg Ser Ser Leu Glu Asn Thr Thr Thr Lys
                245                 250                 255

Leu Gly Asp Ser Phe Tyr Tyr Gly Lys Gly Leu Ile Asn Val Gln Ala
            260                 265                 270

Ala Ala Gln
        275

<210>  49
<211>  275
<212>  PRT
<213>  artificial sequence

<220>
<223>  LG12-clade protease variant

<400>  49

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His

50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  50
<211>  275
<212>  PRT
<213>  artificial sequence

<220>
<223>  LG12-clade protease variant

<400>  50

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Arg Ile Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser

225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  51
<211>  275
<212>  PRT
<213>  artificial sequence

<220>
<223>  LG12-clade protease variant

<400>  51

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  52
<211>  275
<212>  PRT
<213>  artificial sequence

<220>
<223>  LG12-clade protease variant

<400>  52

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Met
        115                 120                 125

Val Phe Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met His Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Phe Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  53
<211>  275
<212>  PRT
<213>  artificial sequence

<220>
<223>  LG12-clade protease variant

<400>  53

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Arg Ile Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Arg
                245                 250                 255

Leu Gly Asp Pro Phe Phe Tyr Gly Lys Gly Val Ile Asn Val Glu Ser
            260                 265                 270

Ala Leu Gln
        275

<210>  54
<211>  275
<212>  PRT
<213>  artificial sequence

<220>
<223>  LG12-clade protease variant

<400>  54

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Lys
            260                 265                 270

Ala Leu Gln
        275

<210>  55
<211>  275
<212>  PRT
<213>  artificial sequence

<220>
<223>  LG12-clade protease variant

<400>  55

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Phe Tyr Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu

            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Arg
                245                 250                 255

Leu Gly Asp Pro Phe Phe Tyr Gly Lys Gly Val Ile Asn Val Glu Lys
            260                 265                 270

Ala Leu Gln
        275

<210> 56
<211> 275
<212> PRT
<213> artificial sequence

<220>
<223> LG12-clade protease variant

<400> 56

Gly Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Asp Asp Leu Asn Val Lys Gly Gly Gln
        35                  40                  45

Ser Phe Val Thr Glu Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu His Ala Val Lys Val Leu
                85                  90                  95

Ser Ala Ser Gly Ser Gly Thr Leu Ser Gly Ile Ala Gln Gly Ile Glu
            100                 105                 110

Trp Thr Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ala Val Val Ile Ala Ala Ala Gly Asn Asn Gly Ser Asn Gly
145                 150                 155                 160

Asn Arg Asn Thr Met His Tyr Pro Ala Arg Tyr Ser Ser Val Ile Ala
                165                 170                 175

Val Gly Ala Val Ser Glu Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Phe Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln

        275

<210>  57
<211>  275
<212>  PRT
<213>  Bacillus sp.

<400>  57

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Val Ala Asn Gly Met Asn Val Ile Asn Met Ser Leu Gly Gly
        115                 120                 125

Ser Ser Gly Ser Ser Ala Leu Gln Gln Ala Cys Asp Asn Ala Tyr Ala
    130                 135                 140

Arg Gly Ile Val Val Val Ala Ala Ala Gly Asn Ser Gly Ser Arg Gly
145                 150                 155                 160

Lys Gln Asn Thr Ile Gly Tyr Pro Ala Arg Tyr Ser Ser Val Ile Ala
                165                 170                 175

Val Gly Ala Val Asp Ser Ser Asn Asn Arg Ala Ser Phe Ser Ser Val
            180                 185                 190

Gly Asn Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ser

210                 215                 220

Pro His Val Ala Gly Ala Ala Ala Leu Ile Lys Ala Lys Tyr Pro Ser
225                 230                 235                 240

Met Ser Asn Val Gln Ile Arg Asp Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  58
<211>  275
<212>  PRT
<213>  Bacillus sp.

<400>  58

Ala Gln Thr Val Pro Trp Gly Ile Pro His Ile Lys Ala Asp Lys Ala
1               5                   10                  15

His Gly Thr Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ala Asn Asp Met Asp Val Ile Asn Met Ser Leu Gly Gly
        115                 120                 125

Ser Ser Gly Ser Thr Thr Leu Gln Gln Ala Cys Asp Asn Ala Tyr Ala
    130                 135                 140

Ser Gly Ile Val Val Ile Ala Ala Ala Gly Asn Ser Gly Ser Lys Gly

145                 150                 155                 160

Lys Arg Asn Thr Ile Gly Tyr Pro Ala Lys Tyr Asn Ser Val Ile Ala
                165                 170                 175

Val Gly Ala Val Asp Ser Ser Asn Asn Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Lys Tyr Ser Ser Phe Asn Gly Thr Ser Met Ala Ser
    210                 215                 220

Pro His Val Ala Gly Ala Ala Ala Leu Ile Ile Ala Lys Tyr Pro Asn
225                 230                 235                 240

Met Thr Asn Val Gln Ile Arg Glu Arg Leu Lys Asn Thr Ala Thr Asn
                245                 250                 255

Leu Gly Asp Pro Phe Phe Phe Gly Lys Gly Val Ile Asn Val Glu Ser
            260                 265                 270

Ala Leu Gln
        275

<210>  59
<211>  275
<212>  PRT
<213>  artificial sequence

<220>
<223>  LG12-clade protease variant

<400>  59

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ala Gly Glu Pro Asn Ala Leu Gln Asp Gly Asn Gly His
    50                  55                  60

Gly Thr His Val Ala Gly Thr Val Ala Ala Leu Asp Asn Ser Ile Gly
65                  70                  75                  80

Val Leu Gly Val Ala Pro Asn Val Ser Leu Tyr Ala Val Lys Val Leu
                85                  90                  95

Gly Ala Ser Gly Ser Gly Ser Val Ser Ser Ile Ala Gln Gly Leu Glu
            100                 105                 110

Trp Ala Gly Asn Asn Gly Met His Val Ala Asn Leu Ser Leu Gly Ser
        115                 120                 125

Pro Ser Pro Ser Ala Thr Leu Glu Gln Ala Val Asn Ser Ala Thr Ser
    130                 135                 140

Arg Gly Val Leu Val Val Ala Ala Ala Gly Asn Ser Gly Thr Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Leu Asn Gly Thr Ser Met Ala Thr
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  60
<211>  275
<212>  PRT
<213>  artificial sequence

<220>
<223>  LG12-clade protease variant

<400>  60

Thr Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Ser Ile Gly
65                  70                  75                  80

Val Leu Gly Val Ala Pro Asn Ala Glu Leu Tyr Ala Val Lys Val Leu
                85                  90                  95

Gly Ala Ser Gly Ser Gly Ser Val Ser Ser Ile Ala Gln Gly Leu Glu
            100                 105                 110

Trp Ala Gly Asn Asn Gly Met His Val Ala Asn Leu Ser Leu Gly Ser
        115                 120                 125

Pro Ser Pro Ser Ala Thr Leu Glu Gln Ala Val Asn Ser Ala Thr Ser
    130                 135                 140

Arg Gly Val Leu Val Val Ala Ala Ala Gly Asn Ser Gly Thr Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Tyr Pro Gly Ser Thr Tyr Ala Thr Leu Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  61
<211>  275
<212>  PRT
<213>  artificial sequence

<220>
<223>  LG12-clade protease variant

<400>  61

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ala Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Pro Ser Val Ser Leu Tyr Ala Val Lys Val Leu
                85                  90                  95

Gly Ala Ser Gly Ser Gly Ser Val Ser Ser Ile Ala Gln Gly Leu Glu
            100                 105                 110

Trp Ala Gly Asn Asn Gly Met His Val Ala Asn Leu Ser Leu Gly Ser
        115                 120                 125

Pro Ser Pro Ser Ala Thr Leu Glu Gln Ala Val Asn Ser Ala Thr Ser
    130                 135                 140

Arg Gly Val Leu Val Val Ala Ala Ala Gly Asn Ser Gly Thr Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Tyr Pro Gly Ser Thr Tyr Ala Thr Leu Asn Gly Thr Ser Met Ala Thr
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210> 62
<211> 275
<212> PRT
<213> artificial sequence

<220>
<223> LG12-clade protease variant

<400> 62

Thr Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Pro Ser Val Ser Leu Tyr Ala Val Lys Val Leu
                85                  90                  95

Gly Ala Ser Gly Ser Gly Ser Val Ser Ser Ile Ala Gln Gly Leu Glu
            100                 105                 110

Trp Ala Gly Asn Asn Gly Met His Val Ala Asn Leu Ser Leu Gly Ser
        115                 120                 125

Pro Ser Pro Ser Ala Thr Leu Glu Gln Ala Val Asn Ser Ala Thr Ser
    130                 135                 140

Arg Gly Val Leu Val Val Ala Ala Ser Gly Asn Ser Gly Thr Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Tyr Pro Gly Ser Thr Tyr Ala Thr Leu Asn Gly Thr Ser Met Ala Thr
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  63
<211>  275
<212>  PRT
<213>  artificial sequence

<220>
<223>  LG12-clade protease variant

<400>  63

Thr Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp

20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Pro Ser Val Ser Leu Tyr Ala Val Lys Val Leu
                85                  90                  95

Gly Ala Ser Gly Ser Gly Ser Val Ser Ser Ile Ala Gln Gly Leu Glu
            100                 105                 110

Trp Ala Gly Asn Asn Gly Met His Val Ala Asn Leu Ser Leu Gly Ser
        115                 120                 125

Pro Ser Pro Ser Ala Thr Leu Glu Gln Ala Val Asn Ser Ala Thr Ser
    130                 135                 140

Arg Gly Val Leu Val Val Ala Ala Ala Gly Asn Ser Gly Thr Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Tyr Pro Gly Ser Thr Tyr Ala Thr Leu Asn Gly Thr Ser Met Ala Thr
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu His
        275

<210>  64
<211>  275
<212>  PRT
<213>  artificial sequence

<220>
<223>  LG12-clade protease variant

<400>  64

Thr Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ala Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Pro Ser Val Ser Leu Tyr Ala Val Lys Val Leu
                85                  90                  95

Gly Ala Ser Gly Ser Gly Ser Val Ser Ser Ile Ala Gln Gly Leu Glu
            100                 105                 110

Trp Ala Gly Asn Asn Gly Met His Val Ala Asn Leu Ser Leu Gly Ser
        115                 120                 125

Pro Ser Pro Ser Ala Thr Leu Glu Gln Ala Val Asn Ser Ala Thr Ser
    130                 135                 140

Arg Gly Val Leu Val Val Ala Ala Ser Gly Asn Ser Gly Thr Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr

195                 200                 205

Tyr Pro Gly Ser Thr Tyr Ala Thr Leu Asn Gly Thr Ser Met Ala Thr
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  65
<211>  275
<212>  PRT
<213>  artificial sequence

<220>
<223>  LG12-clade protease variant

<400>  65

Thr Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Ser Ile Gly
65                  70                  75                  80

Val Leu Gly Val Ala Pro Ser Val Ser Leu Tyr Ala Val Lys Val Leu
                85                  90                  95

Gly Ala Ser Gly Ser Gly Ser Val Ser Ser Ile Ala Gln Gly Leu Glu
            100                 105                 110

Trp Ala Gly Asn Asn Gly Met His Val Ala Asn Leu Ser Leu Gly Ser
        115                 120                 125

Pro Ser Pro Ser Ala Thr Leu Glu Gln Ala Val Asn Ser Ala Thr Ser
    130                 135                 140

Arg Gly Val Leu Val Val Ala Ala Ala Gly Asn Ser Gly Thr Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Tyr Pro Gly Ser Thr Tyr Ala Thr Leu Asn Gly Thr Ser Met Ala Thr
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  66
<211>  275
<212>  PRT
<213>  Bacillus sp.

<400>  66

Ala Gln Thr Val Pro Trp Gly Ile Pro His Ile Lys Ala Asp Lys Ala
1               5                   10                  15

His Ala Ser Gly Val Thr Gly Ser Gly Val Lys Val Ala Val Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ala Asn Asp Met Asp Val Ile Asn Met Ser Leu Gly Gly
        115                 120                 125

Ser Thr Gly Ser Thr Ala Leu Gln Gln Ala Cys Asp Asn Ala Tyr Ala
    130                 135                 140

Ser Gly Ile Val Val Val Ala Ala Ala Gly Asn Ser Gly Ser Lys Gly
145                 150                 155                 160

Lys Arg Asn Thr Met Gly Tyr Pro Ala Arg Tyr Ser Ser Val Ile Ala
                165                 170                 175

Val Gly Ala Val Asp Ser Ser Asn Asn Arg Ala Ser Phe Ser Ser Val
            180                 185                 190

Gly Ser Glu Leu Glu Val Met Ala Pro Gly Val Ser Ile Leu Ser Thr
        195                 200                 205

Thr Pro Gly Asn Asn Tyr Ser Ser Phe Asn Gly Thr Ser Met Ala Ser
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Lys Leu Lys Asn Thr Ala Thr Asn
                245                 250                 255

Leu Gly Asp Ala Phe Tyr Tyr Gly His Gly Val Ile Asn Val Glu Ser
            260                 265                 270

Ala Leu Gln
        275

<210>  67
<211>  275
<212>  PRT
<213>  artificial sequence

<220>
<223>  LG12-clade protease variant

<400>  67

Gly Gln Thr Val Pro Trp Gly Ile Pro His Ile Lys Ala Asp Lys Ala
1               5                   10                  15

His Ala Ala Gly Val Thr Gly Arg Gly Val Lys Val Ala Val Leu Asp
            20                  25                  30

Thr Gly Ile Asp Ala Asn His Ala Asp Leu Asn Val Lys Gly Gly Gln
        35                  40                  45

Ser Phe Val Gln Asn Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Thr Ile Ser Asn Gly Met Asn Val Ile Asn Met Ser Leu Gly Asn
        115                 120                 125

Ser Ser Gly Ser Thr Ala Leu Gln Gln Ala Cys Asn Asn Ala Tyr Asn
    130                 135                 140

Arg Gly Ile Val Asn Ile Ala Ala Ala Gly Asn Ser Gly Ser Ser Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ser Gly Ala Ala Ala Leu Ile Lys Asn Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  68
<211>  275
<212>  PRT
<213>  artificial sequence

<220>
<223>  LG12-clade protease variant

<400>  68

Gly Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Asn Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Pro Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Ser Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Gly Ile Ala Ala Ala Gly Asn Ser Gly Ser Glu Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Phe Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Asn
            260                 265                 270

Ala Leu Gln
        275

<210>  69
<211>  275
<212>  PRT
<213>  artificial sequence

<220>
<223>  LG12-clade protease variant

<400>  69

Ala Gln Val Val Pro Trp Gly Ile Val His Ile Lys Ala Asp Lys Ala
1               5                   10                  15

His Ala Arg Gly Val Thr Gly Thr Gly Val Lys Val Ala Val Leu Asp
            20                  25                  30

Thr Gly Ile Asp Ala Asn His Leu Asp Leu Asn Val Lys Gly Gly Ala
        35                  40                  45

Ser Phe Val Ser Gly Glu Pro Asn Pro Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Thr Gly Thr Leu Ser Gly Val Ala Gln Gly Ile Glu
            100                 105                 110

Trp Ser Ile Glu Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ala Val Val Ile Ala Ala Ala Gly Asn Ala Gly Ser Ser Gly
145                 150                 155                 160

Asn Arg Asn Thr Met Tyr Tyr Pro Ala Arg Tyr Ser Ser Val Ile Ala
                165                 170                 175

Val Gly Ala Val Ser Ser Asn Asn Gln Arg Ala Ser Phe Ser Ser Val
            180                 185                 190

Gly Pro Glu Leu Glu Val Met Ala Pro Gly Val Asn Ile Leu Ser Thr
        195                 200                 205

Thr Pro Gly Asn Asn Tyr Glu Ser Phe Asn Gly Thr Ser Met Ala Ala
    210                 215                 220

Pro His Val Ala Gly Ala Ala Ala Leu Ile Lys Ala Lys Tyr Pro Phe
225                 230                 235                 240

Met Thr Asn Val Gln Ile Arg Glu Arg Leu Lys Asn Thr Ala Thr Lys
                245                 250                 255

Leu Gly Asp Pro Phe Phe Tyr Gly Lys Gly Val Ile Asn Val Glu Lys
            260                 265                 270

Ala Leu Gln
        275

<210>  70
<211>  275
<212>  PRT
<213>  artificial sequence

<220>
<223>  LG12-clade protease variant

<400>  70

Gly Gln Thr Val Pro Trp Gly Ile Pro His Ile Lys Ala Asp Lys Ala

1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Gln
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asn Asn Gly Thr Gly
65                  70                  75                  80

Val Leu Gly Val Ala Tyr Asn Ala Asp Leu His Ala Val Lys Val Leu
                85                  90                  95

Ser Ala Ser Gly Ser Gly Thr Leu Ser Gly Ile Ala Gln Gly Ile Glu
            100                 105                 110

Trp Thr Ile Ser Ser Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ala Val Val Ile Ala Ala Ala Gly Asn Asn Gly Ser Asn Gly
145                 150                 155                 160

Asn Arg Asn Thr Met His Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Leu Ala Ala
    210                 215                 220

Pro His Val Ala Gly Ala Ala Ala Leu Ile Lys Asn Lys Phe Pro Ser
225                 230                 235                 240

Met Thr Asn Val Asn Ile Arg Glu Arg Leu Lys Asn Thr Ala Thr Asn
                245                 250                 255

Leu Gly Asp Pro Phe Phe Tyr Gly Lys Gly Asn Ile Asn Val Glu Ser
            260                 265                 270

Ala Leu Gln
        275

<210>  71
<211>  275
<212>  PRT
<213>  artificial sequence

<220>
<223>  LG12-clade protease variant

<400>  71

Gly Gln Thr Val Pro Trp Gly Ile Pro His Ile Lys Ala Asp Lys Ala
1               5                   10                  15

His Ala Ala Gly Val Thr Gly Ser Gly Val Thr Val Ala Ile Leu Asp
            20                  25                  30

Thr Gly Ile Asp Ala Gln His Leu Asp Leu Ser Val Lys Gly Gly Ala
        35                  40                  45

Ser Phe Val Gln Asp Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Lys Ser Gly Thr Leu Ser Gly Ile Ala Gln Gly Ile Glu
            100                 105                 110

Trp Ser Ile Ser Asn Gly Met Asn Val Ile Asn Met Ser Leu Gly Asn
        115                 120                 125

Ser Ser Gly Ser Thr Ala Leu Gln Gln Ala Cys Asn Asn Ala Tyr Asn
    130                 135                 140

Asn Gly Ile Val Asn Ile Ala Ala Ala Gly Asn Ser Gly Ser Asp Gly
145                 150                 155                 160

Asn Arg Asn Thr Met Gly Tyr Pro Ala Arg Tyr Ser Ser Val Ile Ala
                165                 170                 175

Val Gly Ala Val Ala Ser Asn Asn Thr Arg Ala Ser Phe Ser Ser Val

            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Arg Asn Thr Ala Thr Arg
                245                 250                 255

Leu Gly Asp Pro Phe Phe Tyr Gly Lys Gly Val Ile Asn Val Trp Gln
            260                 265                 270

Ala Leu Gln
        275

<210> 72
<211> 275
<212> PRT
<213> artificial sequence

<220>
<223> LG12-clade protease variant

<400> 72

Gly Gln Thr Val Pro Trp Gly Ile Thr His Ile Lys Ala Asp Lys Ala
1               5                   10                  15

His Ala Arg Gly Val Thr Gly Ser Gly Val Lys Val Ala Ile Leu Asp
            20                  25                  30

Thr Gly Ile Asp Ala Ala His Ala Asp Leu Asn Val Lys Gly Gly Ala
        35                  40                  45

Ser Phe Val Ser Gly Glu Pro Asn Pro Leu Gln Asp Gly Asn Gly His
    50                  55                  60

Gly Thr His Val Ala Gly Thr Ala Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Leu Gly Ser Gly Glu Leu Ser Gly Ile Ala Gln Gly Ile Glu
            100                 105                 110

Trp Thr Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Trp Gly Ile Val Ser Ile Ala Ala Ala Gly Asn Asn Gly Ser Ser Gly
145                 150                 155                 160

Asn Arg Asn Thr Met His Tyr Pro Ala Arg Tyr Ser Ser Val Ile Ala
                165                 170                 175

Val Gly Ala Val Ser Ser Asn Asn Gln Arg Ala Ser Phe Ser Ser Val
            180                 185                 190

Gly Pro Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Phe Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Ser Gly Val Ile Asn Val Glu Lys
            260                 265                 270

Ala Leu Gln
        275

<210>  73
<211>  275
<212>  PRT
<213>  artificial sequence

<220>
<223>  LG12-clade protease variant

<400>  73

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asp Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Asp Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Phe Pro Gly Asn Asn Tyr Ala Ser Phe Asn Gly Thr Ser Leu Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Asn Ile Asn Val Glu Ser
            260                 265                 270

Ala Leu Gln
        275

<210>  74
<211>  275
<212>  PRT
<213>  artificial sequence

<220>
<223>  LG12-clade protease variant

<400>  74

Gly Gln Thr Val Pro Trp Gly Ile Ile His Ile Lys Ala Asp Lys Ala
1               5                   10                  15

His Ala Arg Gly Val Thr Gly Thr Gly Val Thr Val Ala Val Leu Asp
            20                  25                  30

Thr Gly Ile Asp Ala Gln His Ala Asp Leu His Val Lys Gly Gly Gln
        35                  40                  45

Ser Phe Val Ser Asp Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Thr Gly Thr Leu Ser Gly Thr Ala Gln Gly Ile Glu
            100                 105                 110

Trp Ser Ile Ser Asn Gly Met Asn Val Ile Asn Met Ser Leu Gly Asn
        115                 120                 125

Ser Ser Gly Ser Asp Ala Leu Gln Gln Ala Cys Asn Asn Ala Tyr Asn
    130                 135                 140

Arg Gly Ile Val Ser Ile Ala Ala Ala Gly Asn Ser Gly Ser Ser Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Gln Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ser Gly Ala Ala Ala Leu Ile Lys Ala Lys Tyr Pro Ser
225                 230                 235                 240

Met Ser Asn Val Gln Ile Arg Glu Arg Leu Arg Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  75
<211>  275
<212>  PRT
<213>  artificial sequence

<220>
<223>  LG12-clade protease variant

<400>  75

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Gln Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asp Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Ser Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Phe Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Pro
                245                 250                 255

Leu Gly Asp Pro Phe Phe Tyr Gly Lys Gly Val Ile Asn Val Glu Ser
            260                 265                 270

Ala Leu Gln
        275

<210> 76
<211> 275
<212> PRT
<213> artificial sequence

<220>
<223> LG12-clade protease variant

<400> 76

Gly Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Gln
        35                  40                  45

Ser Phe Val Thr Gly Glu Pro Asn Ala Leu Gln Asp Gly Asn Gly His

50                  55                  60

Gly Thr His Val Ala Gly Thr Val Ala Ala Leu Asn Asn Gly Thr Gly
65                  70                  75                  80

Val Leu Gly Val Ala Tyr Asn Ala Asp Leu His Ala Val Lys Val Leu
                85                  90                  95

Ser Ala Ser Gly Ser Gly Thr Leu Ser Gly Ile Ala Gln Gly Ile Glu
            100                 105                 110

Trp Thr Ile Ser Ser Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ala Val Val Ile Ala Ala Ala Gly Asn Asn Gly Ser Asn Gly
145                 150                 155                 160

Asn Arg Asn Thr Met His Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Leu Ala Ala
    210                 215                 220

Pro His Val Ala Gly Ala Ala Ala Leu Ile Lys Asn Lys Phe Pro Ser
225                 230                 235                 240

Met Thr Asn Val Asn Ile Arg Glu Arg Leu Lys Asn Thr Ala Thr Asn
                245                 250                 255

Leu Gly Asp Pro Phe Phe Tyr Gly Lys Gly Asn Ile Asn Val Glu Ser
            260                 265                 270

Ala Leu Gln
        275

<210>  77
<211>  275
<212>  PRT
<213>  artificial sequence

<220>
<223>  LG12-clade protease variant

<400>  77

Gly Gln Thr Val Pro Trp Gly Ile Pro His Ile Lys Ala Asp Lys Ala
1               5                   10                  15

His Ala Ala Gly Val Thr Gly Ser Gly Val Lys Val Ala Val Leu Asp
            20                  25                  30

Thr Gly Ile Asp Ala Asn His Ala Asp Leu Asn Val Lys Gly Gly Ala
        35                  40                  45

Ser Phe Val Gln Asn Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Thr Ile Ser Asn Gly Met Asn Val Ile Asn Met Ser Leu Gly Asn
        115                 120                 125

Ser Ser Gly Ser Thr Ala Leu Gln Gln Ala Cys Asn Asn Ala Tyr Asn
    130                 135                 140

Arg Gly Ile Val Asn Ile Ala Ala Ala Gly Asn Ser Gly Ser Asn Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ser Gly Ala Ala Ala Leu Ile Lys Asn Lys Tyr Pro Ser

225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  78
<211>  275
<212>  PRT
<213>  artificial sequence

<220>
<223>  LG12-clade protease variant

<400>  78

Gly Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Gln
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asn Asn Gly Thr Gly
65                  70                  75                  80

Val Leu Gly Val Ala Tyr Asn Ala Asp Leu His Ala Val Lys Val Leu
                85                  90                  95

Ser Ala Ser Gly Ser Gly Thr Leu Ser Gly Ile Ala Gln Gly Ile Glu
            100                 105                 110

Trp Thr Ile Ser Ser Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ala Val Val Ile Ala Ala Ala Gly Asn Asn Gly Ser Asn Gly
145                 150                 155                 160

Asn Arg Asn Thr Met His Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Leu Ala Ala
    210                 215                 220

Pro His Val Ala Gly Ala Ala Ala Leu Ile Lys Asn Lys Phe Pro Ser
225                 230                 235                 240

Met Thr Asn Val Asn Ile Arg Glu Arg Leu Lys Asn Thr Ala Thr Glu
                245                 250                 255

Leu Gly Asp Pro Phe Phe Tyr Gly Lys Gly Asn Ile Asn Val Glu Ser
            260                 265                 270

Ala Leu Gln
        275

<210>  79
<211>  275
<212>  PRT
<213>  artificial sequence

<220>
<223>  LG12-clade protease variant

<400>  79

Gly Gln Thr Val Pro Trp Gly Ile Pro His Ile Lys Ala Asp Lys Ala
1               5                   10                  15

His Ala Ala Gly Val Thr Gly Ser Gly Val Lys Val Ala Val Leu Asp
            20                  25                  30

Thr Gly Ile Asp Ala Asn His Glu Asp Leu Asp Val Lys Gly Gly Gln
        35                  40                  45

Ser Phe Val Gln Asn Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Thr Ile Ser Asn Gly Met Asn Val Ile Asn Met Ser Leu Gly Asn
        115                 120                 125

Ser Ser Gly Ser Thr Ala Leu Gln Gln Ala Cys Asn Asn Ala Tyr Asn
    130                 135                 140

Arg Gly Ile Val Asn Val Ala Ala Ala Gly Asn Asn Gly Ser Asp Gly
145                 150                 155                 160

Asn Arg Asn Thr Met Asn Tyr Pro Ala Arg Tyr Ser Ser Val Ile Ala
                165                 170                 175

Val Gly Ala Val Ala Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Thr Phe Asn Gly Thr Ser Leu Ala Ala
    210                 215                 220

Pro His Val Ser Gly Ala Ala Ala Leu Ile Lys Asn Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275


<210>  80
<211>  275
<212>  PRT
<213>  artificial sequence

<220>
<223>  LG12-clade protease variant

<400>  80

Gly Gln Glu Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Asp Asp Leu Asn Val Lys Gly Gly Gln
        35                  40                  45

Ser Phe Val Thr Glu Glu Pro Asn Ala Leu Gln Asp Gly Asn Gly His
    50                  55                  60

Gly Thr His Val Ala Gly Thr Val Ala Ala Leu Asn Asn Gly Thr Gly
65                  70                  75                  80

Val Leu Gly Val Ala Tyr Asn Ala Asp Leu His Ala Val Lys Val Leu
                85                  90                  95

Ser Ala Ser Gly Ser Gly Thr Leu Ser Gly Ile Ala Gln Gly Ile Glu
            100                 105                 110

Trp Thr Ile Ser Ser Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ala Val Val Ile Ala Ala Ala Gly Asn Asn Gly Ser Asp Gly
145                 150                 155                 160

Asn Arg Asn Thr Met His Tyr Pro Ala Arg Tyr Ser Ser Val Ile Ala
                165                 170                 175

Val Gly Ala Val Ser Glu Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Asp Ser Arg Asn Gly Thr Ser Leu Ala Ala
    210                 215                 220

Pro His Val Ala Gly Ala Ala Ala Leu Ile Lys Asn Lys Phe Pro Ser
225                 230                 235                 240

Met Thr Asn Val Asn Ile Arg Glu Arg Leu Lys Asn Thr Ala Thr Glu
                245                 250                 255

Leu Gly Asp Pro Phe Phe Tyr Gly Lys Gly Asn Ile Asn Val Glu Ser
            260                 265                 270

Ala Leu Gln
        275

<210>  81
<211>  275
<212>  PRT
<213>  artificial sequence

<220>
<223>  LG12-clade protease variant

<400>  81

Gly Gln Thr Val Pro Trp Gly Ile Pro His Ile Lys Ala Asp Lys Ala
1               5                   10                  15

His Ala Ala Gly Val Thr Gly Arg Gly Val Lys Val Ala Val Leu Asp
            20                  25                  30

Thr Gly Ile Asp Ala Asn His Ala Asp Leu Asn Val Lys Gly Gly Gln
        35                  40                  45

Ser Phe Val Gln Asn Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Thr Ile Ser Asn Gly Met Asn Val Ile Asn Met Ser Leu Gly Asn
        115                 120                 125

Ser Ser Gly Ser Thr Ala Leu Gln Gln Ala Cys Asn Asn Ala Tyr Asn
    130                 135                 140

Arg Gly Ile Val Asn Ile Ala Ala Ala Gly Asn Ser Gly Ser Asp Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ser Gly Ala Ala Ala Leu Ile Lys Asn Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  82
<211>  275
<212>  PRT
<213>  artificial sequence

<220>
<223>  LG12-clade protease variant

<400>  82

Gly Gln Thr Val Pro Trp Gly Ile Pro His Ile Lys Ala Asp Lys Ala
1               5                   10                  15

His Ala Ala Gly Val Thr Gly Ser Gly Val Ser Val Ala Ile Leu Asp
            20                  25                  30

Thr Gly Ile Asp Ala Thr His Ala Asp Leu Asn Val Lys Gly Gly Ala
        35                  40                  45

Ser Phe Val Ser Glu Glu Pro Asn Ala Leu Gln Asp Gly Asn Gly His
    50                  55                  60

Gly Thr His Val Ala Gly Thr Val Ala Ala Leu Asn Asn Asp Thr Gly
65                  70                  75                  80

Val Leu Gly Val Ala Tyr Asn Ala Asp Pro Tyr Ala Thr Lys Val Leu
                85                  90                  95

Ser Ala Ser Gly Ser Gly Glu Leu Ser Gly Ile Ala Gln Gly Ile Glu

            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Asp Gly
145                 150                 155                 160

Asn Arg Asn Thr Met Ser Tyr Pro Ala Arg Tyr Ser Ser Val Ile Ala
                165                 170                 175

Val Gly Ala Val Ser Ser Asn Asn Gln Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Asn Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210> 83
<211> 275
<212> PRT
<213> artificial sequence

<220>
<223> LG12-clade protease variant

<400> 83

Gly Gln Thr Val Pro Trp Gly Ile Pro His Ile Lys Ala Asp Lys Ala
1               5                   10                  15

His Ala Ala Gly Val Thr Gly Ser Gly Val Thr Val Ala Ile Leu Asp
            20                  25                  30

Thr Gly Ile Asp Ala Asn His Leu Asp Leu Ser Val Lys Gly Gly Ala
        35                  40                  45

Ser Phe Val Gln Asp Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Asn Ile Ala Ala Ala Gly Asn Ser Gly Ser Asp Gly
145                 150                 155                 160

Asn Arg Asn Thr Met Tyr Tyr Pro Ala Arg Tyr Ser Ser Val Ile Ala
                165                 170                 175

Val Gly Ala Val Ala Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Leu Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Arg
                245                 250                 255

Leu Gly Asp Pro Phe Phe Tyr Gly Lys Gly Val Ile Asn Val Trp Gln
            260                 265                 270

Ala Leu Gln

        275

<210>  84
<211>  275
<212>  PRT
<213>  artificial sequence

<220>
<223>  LG12-clade protease variant

<400>  84

Ala Gln Val Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Leu Val Lys Gly Gly Ala
        35                  40                  45

Ser Phe Val Ser Glu Glu Pro Asn Pro Leu Gln Asp Gly Asn Gly His
    50                  55                  60

Gly Thr His Val Ala Gly Thr Ala Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Thr Gly Thr Leu Ser Gly Thr Ala Gln Gly Ile Glu
            100                 105                 110

Trp Ser Ile Glu Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ala Val Val Ile Ala Ala Ala Gly Asn Ser Gly Ser Gln Gly
145                 150                 155                 160

Asn Arg Asn Thr Met Ala Tyr Pro Ala Arg Tyr Ser Ser Val Ile Ala
                165                 170                 175

Val Gly Ala Val Ser Ser Asn Asn Thr Arg Ala Tyr Phe Ser Ser Val
            180                 185                 190

Gly Pro Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Gln Lys Tyr Pro Phe
225                 230                 235                 240

Met Thr Asn Val Gln Val Arg Glu Arg Leu Lys Asn Thr Ala Thr Asn
                245                 250                 255

Leu Gly Asp Pro Phe Phe Tyr Gly Lys Gly Val Ile Asn Val Glu Gln
            260                 265                 270

Ala Leu Gln
        275

<210>  85
<211>  275
<212>  PRT
<213>  artificial sequence

<220>
<223>  LG12-clade protease variant

<400>  85

Gly Gln Glu Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Asp Asp Leu Asn Val Lys Gly Gly Glu
        35                  40                  45

Ser Phe Val Thr Glu Glu Pro Asn Ala Leu Gln Asp Gly Asn Gly His
    50                  55                  60

Gly Thr His Val Ala Gly Thr Val Ala Ala Leu Asn Asn Gly Thr Gly
65                  70                  75                  80

Val Leu Gly Val Ala Tyr Asn Ala Asp Leu His Ala Val Lys Val Leu
                85                  90                  95

Ser Ala Ser Gly Ser Gly Thr Leu Ser Gly Ile Ala Gln Gly Ile Glu
            100                 105                 110

Trp Thr Ile Ser Ser Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ala Val Val Ile Ala Ala Ala Gly Asn Asn Gly Ser Asp Gly
145                 150                 155                 160

Asn Arg Asn Thr Met His Tyr Pro Ala Arg Tyr Ser Ser Val Ile Ala
                165                 170                 175

Val Gly Ala Val Ser Glu Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Asp Ser Phe Asn Gly Thr Ser Leu Ala Ala
    210                 215                 220

Pro His Val Ala Gly Ala Ala Ala Leu Ile Lys Asn Lys Phe Pro Ser
225                 230                 235                 240

Met Thr Asn Val Asn Ile Arg Glu Arg Leu Lys Asn Thr Ala Thr Glu
                245                 250                 255

Leu Gly Asp Pro Phe Phe Tyr Gly Lys Gly Asn Ile Asn Val Glu Ser
            260                 265                 270

Ala Leu Gln
        275

<210>  86
<211>  275
<212>  PRT
<213>  artificial sequence

<220>
<223>  LG12-clade protease variant

<400>  86

Gly Gln Thr Val Pro Trp Gly Ile Pro His Ile Lys Ala Asp Lys Ala
1               5                   10                  15

His Ala Ala Gly Val Thr Gly Ser Gly Val Thr Val Ala Ile Leu Asp
            20                  25                  30

Thr Gly Ile Asp Ala Gln His Leu Asp Leu Ser Val Lys Gly Gly Ala
        35                  40                  45

Ser Phe Val Gln Asp Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Glu Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Asn Gly Ile Val Asn Ile Ala Ala Ala Gly Asn Ser Gly Ser Asp Gly
145                 150                 155                 160

Asn Arg Asn Thr Met Tyr Tyr Pro Ala Arg Tyr Ser Ser Val Ile Ala
                165                 170                 175

Val Gly Ala Val Ala Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Leu Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Arg Asn Thr Ala Thr Arg
                245                 250                 255

Leu Gly Asp Pro Phe Phe Tyr Gly Lys Gly Val Ile Asn Val Trp Gln
            260                 265                 270

Ala Leu Gln
        275

<210>  87
<211>  275
<212>  PRT

<213>  artificial sequence

<220>
<223>  LG12-clade protease variant

<400>  87

Gly Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Asp Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asn Asn Gly Thr Gly
65                  70                  75                  80

Val Leu Gly Val Ala Tyr Asn Ala Asp Leu His Ala Val Lys Val Leu
                85                  90                  95

Ser Ala Ser Gly Ser Gly Thr Leu Ser Gly Ile Ala Gln Gly Ile Glu
            100                 105                 110

Trp Thr Ile Ser Ser Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ala Val Val Ile Ala Ala Ala Gly Asn Asn Gly Ser Asn Gly
145                 150                 155                 160

Asn Arg Asn Thr Met His Tyr Pro Ala Arg Tyr Ser Ser Val Ile Ala
                165                 170                 175

Val Gly Ala Val Ser Glu Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Leu Ala Ala
    210                 215                 220

Pro His Val Ala Gly Ala Ala Ala Leu Ile Lys Asn Lys Phe Pro Ser
225                 230                 235                 240

Met Thr Asn Val Asn Ile Arg Glu Arg Leu Lys Asn Thr Ala Thr Asn
                245                 250                 255

Leu Gly Asp Pro Phe Phe Tyr Gly Lys Gly Asn Ile Asn Val Glu Ser
            260                 265                 270

Ala Leu Gln
        275

<210>  88
<211>  275
<212>  PRT
<213>  artificial sequence

<220>
<223>  LG12-clade protease variant

<400>  88

Gly Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Gln
        35                  40                  45

Ser Phe Val Gln Asn Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Thr Ile Ser Asn Gly Met Asn Val Ile Asn Met Ser Leu Gly Asn
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Asn Gly

145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Thr Phe Asn Gly Thr Ser Met Ala Ala
    210                 215                 220

Pro His Val Ser Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  89
<211>  275
<212>  PRT
<213>  artificial sequence

<220>
<223>  LG12-clade protease variant

<400>  89

Gly Gln Thr Val Pro Trp Gly Ile Thr His Ile Lys Ala Asp Lys Ala
1               5                   10                  15

His Ala Ala Gly Val Thr Gly Thr Gly Val Ser Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Pro Leu Gln Asp Gly Asn Gly His
    50                  55                  60

Gly Thr His Val Ala Gly Thr Ala Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Thr Gly Thr Leu Ser Gly Ile Ala Gln Gly Ile Glu
            100                 105                 110

Trp Ser Ile Glu Asn Gly Met Asn Val Ile Asn Met Ser Leu Gly Gly
        115                 120                 125

Ser Ser Gly Ser Thr Ala Leu Gln Gln Ala Cys Tyr Asn Ala Tyr Asn
    130                 135                 140

Arg Gly Ile Val Asn Ile Ala Ala Ala Gly Asn Ser Gly Ser Asn Gly
145                 150                 155                 160

Asn Arg Asn Thr Met Asn Tyr Pro Ala Arg Tyr Ser Ser Val Ile Ala
                165                 170                 175

Val Gly Ala Val Ser Ser Asn Asn Gln Arg Ala Tyr Phe Ser Ser Val
            180                 185                 190

Gly Pro Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Val Lys Ala Lys Tyr Pro Phe
225                 230                 235                 240

Met Thr Asn Val Gln Ile Arg Glu Arg Leu Arg Asn Thr Ala Thr Asn
                245                 250                 255

Leu Gly Asp Pro Phe Phe Tyr Gly Lys Gly Val Ile Asn Val Phe Ser
            260                 265                 270

Ala Leu Gln
        275

<210>  90
<211>  275
<212>  PRT
<213>  artificial sequence

<220>
<223>  LG12-clade protease variant

<400>  90

Gly Gln Thr Val Pro Trp Gly Ile Pro His Ile Lys Ala Asp Lys Ala
1               5                   10                  15

His Ala Ala Gly Val Thr Gly Ser Gly Val Lys Val Ala Val Leu Asp
            20                  25                  30

Thr Gly Ile Asp Ala Asn His Ala Asp Leu Asp Val Lys Gly Gly Gln
        35                  40                  45

Ser Phe Val Gln Asn Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Thr Ile Ser Asn Gly Met Asn Val Ile Asn Met Ser Leu Gly Asn
        115                 120                 125

Ser Ser Gly Ser Thr Ala Leu Gln Gln Ala Cys Asn Asn Ala Tyr Asn
    130                 135                 140

Arg Gly Ile Val Asn Ile Ala Ala Ala Gly Asn Ser Gly Ser Asn Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Thr Phe Asn Gly Thr Ser Met Ala Ala
    210                 215                 220

Pro His Val Ser Gly Ala Ala Ala Leu Ile Lys Asn Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  91
<211>  275
<212>  PRT
<213>  artificial sequence

<220>
<223>  LG12-clade protease variant

<400>  91

Gly Gln Thr Val Pro Trp Gly Ile Pro His Ile Lys Ala Asp Lys Ala
1               5                   10                  15

His Ala Ala Gly Val Thr Gly Ser Gly Val Lys Val Ala Val Leu Asp
            20                  25                  30

Thr Gly Ile Asp Ala Asn His Val Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Asp Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Glu Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Ser Tyr Pro Ala Arg Tyr Ser Ser Val Ile Ala
                165                 170                 175

Val Gly Ala Val Ser Ser Asn Asn Gln Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210> 92
<211> 275
<212> PRT
<213> artificial sequence

<220>
<223> LG12-clade protease variant

<400> 92

Gly Gln Thr Val Pro Trp Gly Ile Pro His Ile Lys Ala Asp Lys Ala
1               5                   10                  15

His Ala Ala Gly Val Thr Gly Ser Gly Val Ser Val Ala Ile Leu Asp
            20                  25                  30

Thr Gly Ile Asp Ala Asn His Val Asp Leu Asn Val Lys Gly Gly Ala
        35                  40                  45

Ser Phe Val Ser Asn Glu Pro Asn Ala Leu Gln Asp Gly Asn Gly His
    50                  55                  60

Gly Thr His Val Ala Gly Thr Val Ala Ala Leu Asn Asn Asp Thr Gly
65                  70                  75                  80

Val Leu Gly Val Ala Tyr Asn Ala Asp Pro Tyr Ala Val Lys Val Leu
                85                  90                  95

Ser Ala Ser Gly Ser Gly Glu Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Asp Gly
145                 150                 155                 160

Asn Arg Asn Thr Met Ser Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210> 93
<211> 275
<212> PRT
<213> artificial sequence

<220>
<223> LG12-clade protease variant

<400> 93

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp

20                  25                  30

Thr Gly Ile Asp Ala Gln His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Ala Ser Gly Thr Leu Ser Gly Ile Ala Gln Gly Ile Glu
            100                 105                 110

Trp Ser Ile Ser Ser Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ala Val Val Ile Ala Ala Ala Gly Asn Ser Gly Thr Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Pro
                245                 250                 255

Leu Gly Asp Pro Phe Phe Tyr Gly Lys Gly Val Ile Asn Val Glu Lys
            260                 265                 270

Ala Leu Gln
        275

<210>  94
<211>  275
<212>  PRT
<213>  artificial sequence

<220>
<223>  LG12-clade protease variant

<400>  94

Gly Gln Thr Val Pro Trp Gly Ile Pro His Ile Lys Ala Asp Lys Ala
1               5                   10                  15

His Ala Ala Gly Val Thr Gly Ser Gly Val Lys Val Ala Val Leu Asp
            20                  25                  30

Thr Gly Ile Asp Ala Asn His Ala Asp Leu Asn Val Lys Gly Gly Gln
        35                  40                  45

Ser Phe Val Gln Asn Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Thr Ile Ser Asn Gly Met Asn Val Ile Asn Met Ser Leu Gly Asn
        115                 120                 125

Ser Ser Gly Ser Thr Ala Leu Gln Gln Ala Cys Asn Asn Ala Tyr Asn
    130                 135                 140

Arg Gly Ile Val Asn Val Ala Ala Ala Gly Asn Asn Gly Ser Asn Gly
145                 150                 155                 160

Asn Arg Asn Thr Met Gly Tyr Pro Ala Arg Tyr Ser Ser Val Ile Ala
                165                 170                 175

Val Gly Ala Val Ala Ser Asn Asn Thr Arg Ala Ser Phe Ser Ser Val
            180                 185                 190

Gly Ser Glu Leu Glu Val Met Ala Pro Gly Val Asn Ile Leu Ser Thr

195                 200                 205

Thr Pro Gly Asn Asn Tyr Ala Thr Phe Asn Gly Thr Ser Leu Ala Ala
    210                 215                 220

Pro His Val Ser Gly Ala Ala Ala Leu Ile Lys Asn Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  95
<211>  275
<212>  PRT
<213>  artificial sequence

<220>
<223>  LG12-clade protease variant

<400>  95

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Gln His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Ala Ser Gly Thr Leu Ser Gly Ile Ala Gln Gly Ile Glu
            100                 105                 110

Trp Ser Ile Ser Ser Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ala Val Val Ile Ala Ala Ala Gly Asn Ser Gly Thr Ser Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Pro
                245                 250                 255

Leu Gly Asp Pro Phe Phe Tyr Gly Lys Gly Val Ile Asn Val Glu Lys
            260                 265                 270

Ala Leu Gln
        275

<210>  96
<211>  275
<212>  PRT
<213>  artificial sequence

<220>
<223>  LG12-clade protease variant

<400>  96

Gly Gln Thr Val Pro Trp Gly Ile Pro His Ile Lys Ala Asp Lys Ala
1               5                   10                  15

His Ala Ala Gly Val Thr Gly Ser Gly Val Lys Val Ala Val Leu Asp
            20                  25                  30

Thr Gly Ile Asp Ala Asn His Ala Asp Leu Asp Val Lys Gly Gly Glu
        35                  40                  45

Ser Phe Val Gln Asn Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Thr Ile Ser Asn Gly Met Asn Val Ile Asn Met Ser Leu Gly Asn
        115                 120                 125

Ser Ser Gly Ser Thr Ala Leu Gln Gln Ala Cys Asn Asn Ala Tyr Asn
    130                 135                 140

Arg Gly Ile Val Asn Ile Ala Ala Ala Gly Asn Ser Gly Ser Asp Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Thr Phe Asn Gly Thr Ser Met Ala Ala
    210                 215                 220

Pro His Val Ser Gly Ala Ala Ala Leu Ile Lys Asn Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  97
<211>  275

<212>  PRT
<213>  artificial sequence

<220>
<223>  LG12-clade protease variant

<400>  97
   Gly Gln Thr Val Pro Trp Gly Ile Pro His Ile Lys Ala Asp Lys Ala
   1               5                   10                  15
   His Ala Ala Gly Val Thr Gly Ser Gly Val Thr Val Ala Ile Leu Asp
               20                  25                  30
   Thr Gly Ile Asp Ala Gln His Leu Asp Leu Ser Val Lys Gly Gly Ala
           35                  40                  45
   Ser Phe Val Gln Asp Glu Pro Asn Ala Leu Gln Asp Gly Asn Gly His
       50                  55                  60
   Gly Thr His Val Ala Gly Thr Val Ala Ala Leu Asn Asn Thr Thr Gly
   65                  70                  75                  80
   Val Leu Gly Val Ala Tyr Asn Ala Asp Leu Tyr Ala Val Lys Val Leu
                   85                  90                  95
   Ser Ala Ser Gly Ser Gly Thr Leu Ser Gly Ile Ala Gln Gly Ile Glu
               100                 105                 110
   Trp Ser Ile Ser Glu Gly Met Asn Val Ile Asn Met Ser Leu Gly Gly
           115                 120                 125
   Ser Ser Gly Ser Thr Ala Leu Gln Gln Ala Cys Asn Asn Ala Tyr Asn
       130                 135                 140
   Asn Gly Ile Val Asn Ile Ala Ala Ala Gly Asn Ser Gly Ser Asp Gly
   145                 150                 155                 160
   Asn Arg Asn Thr Met Tyr Tyr Pro Ala Arg Tyr Ser Ser Val Ile Ala
                   165                 170                 175
   Val Gly Ala Val Ala Ser Asn Asn Thr Arg Ala Ser Phe Ser Ser Val
               180                 185                 190
   Gly Ser Glu Leu Glu Val Met Ala Pro Gly Val Asn Ile Leu Ser Thr
           195                 200                 205
   Thr Pro Gly Asn Asn Tyr Ala Ser Arg Asn Gly Thr Ser Leu Ala Ala
       210                 215                 220
   Pro His Val Ala Gly Ala Ala Ala Leu Ile Lys Ala Lys Tyr Pro Ser
   225                 230                 235                 240
   Met Thr Asn Val Gln Ile Arg Glu Arg Leu Arg Asn Thr Ala Thr Arg
                   245                 250                 255
   Leu Gly Asp Pro Phe Phe Tyr Gly Lys Gly Val Ile Asn Val Trp Ser
               260                 265                 270
   Ala Leu Gln
           275

<210>  98
<211>  275
<212>  PRT
<213>  artificial sequence

<220>
<223>  LG12-clade protease variant

<400>  98

Ala Gln Thr Val Pro Trp Gly Ile Pro His Ile Lys Ala Asp Lys Ala
1               5                   10                  15

His Ala Ala Gly Val Thr Gly Arg Gly Val Lys Val Ala Val Leu Asp
            20                  25                  30

Thr Gly Ile Asp Ala Asn His Glu Asp Leu Asp Val Lys Gly Gly Glu
        35                  40                  45

Ser Phe Val Gln Asn Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Thr Ile Ser Asn Gly Met Asn Val Ile Asn Met Ser Leu Gly Asn
        115                 120                 125

Ser Ser Gly Ser Thr Ala Leu Gln Gln Ala Cys Asn Asn Ala Tyr Asn
    130                 135                 140

Arg Gly Ile Val Asn Val Ala Ala Ala Gly Asn Asn Gly Ser Asp Gly
145                 150                 155                 160

Asn Arg Asn Thr Met Asp Tyr Pro Ala Arg Tyr Ser Ser Val Ile Ala
                165                 170                 175

Val Gly Ala Val Ala Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Thr Arg Asn Gly Thr Ser Met Ala Ala
    210                 215                 220

Pro His Val Ser Gly Ala Ala Ala Leu Ile Lys Asn Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Trp Lys
            260                 265                 270

Ala Leu Gln
        275

<210>  99
<211>  275
<212>  PRT
<213>  artificial sequence

<220>
<223>  LG12-clade protease variant

<400>  99

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly

65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Ala
        115                 120                 125

Met Thr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  100
<211>  275
<212>  PRT
<213>  artificial sequence

<220>
<223>  LG12-clade protease variant

<400>  100

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Glu Leu Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn

245                 250                 255

Leu Gly Asp Pro Phe Phe Tyr Gly Lys Gly Val Ile Asn Val Glu Ser
            260                 265                 270

Ala Leu Gln
        275

<210>  101
<211>  275
<212>  PRT
<213>  artificial sequence

<220>
<223>  LG12-clade protease variant

<400>  101

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Lys His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  102
<211>  275
<212>  PRT
<213>  artificial sequence

<220>
<223>  LG12-clade protease variant

<400>  102

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  103
<211>  275
<212>  PRT
<213>  artificial sequence

<220>
<223>  LG12-clade protease variant

<400>  103

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Asn Met Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  104
<211>  275
<212>  PRT
<213>  artificial sequence

<220>
<223>  LG12-clade protease variant

<400>  104

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Asn Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  105
<211>  275
<212>  PRT
<213>  artificial sequence

<220>
<223>  LG12-clade protease variant

<400>  105

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp

115                 120                 125

Pro Phe Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  106
<211>  275
<212>  PRT
<213>  artificial sequence

<220>
<223>  LG12-clade protease variant

<400>  106

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Pro Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  107

<211>  275
<212>  PRT
<213>  artificial sequence

<220>
<223>  LG12-clade protease variant

<400>  107

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Arg Thr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  108
<211>  275
<212>  PRT
<213>  artificial sequence

<220>
<223>  LG12-clade protease variant

<400>  108

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Ser Asn Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Asp
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  109
<211>  275
<212>  PRT
<213>  artificial sequence

<220>
<223>  LG12-clade protease variant

<400>  109

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Thr Phe Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  110
<211>  275
<212>  PRT
<213>  artificial sequence

<220>
<223>  LG12-clade protease variant

<400>  110

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Tyr Arg Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  111
<211>  275
<212>  PRT
<213>  artificial sequence

<220>
<223>  LG12-clade protease variant

<400>  111

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Glu
        115                 120                 125

Gly Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala

165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  112
<211>  275
<212>  PRT
<213>  artificial sequence

<220>
<223>  LG12-clade protease variant

<400>  112

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Glu
        115                 120                 125

His Thr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210> 113
<211> 275
<212> PRT
<213> artificial sequence

<220>
<223> LG12-clade protease variant

<400> 113

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Glu
        115                 120                 125

Ser Arg Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  114
<211>  275
<212>  PRT
<213>  artificial sequence

<220>
<223>  LG12-clade protease variant

<400>  114

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Glu
        115                 120                 125

Val Met Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210> 115
<211> 275
<212> PRT
<213> artificial sequence

<220>
<223> LG12-clade protease variant

<400> 115

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Gly
        115                 120                 125

Asp Ala Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210> 116
<211> 275
<212> PRT
<213> artificial sequence

<220>
<223> LG12-clade protease variant

<400> 116

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala

35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Gly
        115                 120                 125

Ile Ala Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  117
<211>  275
<212>  PRT
<213>  artificial sequence

<220>
<223>  LG12-clade protease variant

<400>  117

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Gly
        115                 120                 125

Val Gln Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala

210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Arg
        275

<210>  118
<211>  275
<212>  PRT
<213>  artificial sequence

<220>
<223>  LG12-clade protease variant

<400>  118

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly His
        115                 120                 125

Lys Trp Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  119
<211>  275
<212>  PRT
<213>  artificial sequence

<220>
<223>  LG12-clade protease variant

<400>  119

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly His
        115                 120                 125

Leu Leu Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  120
<211>  275
<212>  PRT
<213>  artificial sequence

<220>

<223>  LG12-clade protease variant

<400>  120

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly His
        115                 120                 125

Met Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  121
<211>  275
<212>  PRT
<213>  artificial sequence

<220>
<223>  LG12-clade protease variant

<400>  121

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly His
        115                 120                 125

Asn Val Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  122
<211>  275
<212>  PRT
<213>  artificial sequence

<220>
<223>  LG12-clade protease variant

<400>  122

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu

85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly His
        115                 120                 125

Pro Leu Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  123
<211>  275
<212>  PRT
<213>  artificial sequence

<220>
<223>  LG12-clade protease variant

<400>  123

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly His
        115                 120                 125

Pro Asn Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser

260                 265                 270

Ala Leu Gln
        275

<210>  124
<211>  275
<212>  PRT
<213>  artificial sequence

<220>
<223>  LG12-clade protease variant

<400>  124

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly His
        115                 120                 125

Pro Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210> 125
<211> 275
<212> PRT
<213> artificial sequence

<220>
<223> LG12-clade protease variant

<400> 125

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly His
        115                 120                 125

Gln Ile Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  126
<211>  275
<212>  PRT
<213>  artificial sequence

<220>
<223>  LG12-clade protease variant

<400>  126

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly His
        115                 120                 125

Gln Trp Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  127
<211>  275
<212>  PRT
<213>  artificial sequence

<220>
<223>  LG12-clade protease variant

<400>  127

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly His
        115                 120                 125

Arg His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  128
<211>  275
<212>  PRT
<213>  artificial sequence

<220>
<223>  LG12-clade protease variant

<400>  128

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly His
        115                 120                 125

Ser Ile Gly Ser Thr Ala Leu Gln Gln Ala Cys Asn Asn Ala Tyr Asn

130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  129
<211>  275
<212>  PRT
<213>  artificial sequence

<220>
<223>  LG12-clade protease variant

<400>  129

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly His
        115                 120                 125

Thr His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  130
<211>  275
<212>  PRT
<213>  artificial sequence

<220>
<223>  LG12-clade protease variant

<400>  130

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly His
        115                 120                 125

Thr Thr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  131
<211>  275
<212>  PRT
<213>  artificial sequence

<220>
<223>  LG12-clade protease variant

<400>  131

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly His
        115                 120                 125

Val His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  132
<211>  275
<212>  PRT
<213>  artificial sequence

<220>
<223>  LG12-clade protease variant

<400>  132

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Lys
        115                 120                 125

Ile Asn Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  133
<211>  275
<212>  PRT
<213>  artificial sequence

<220>
<223>  LG12-clade protease variant

<400>  133

Ala Gln Thr Val Pro Trp Gly Ile Pro His Ile Lys Ala Asp Lys Ala

1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asn
        115                 120                 125

Gly Asn Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  134
<211>  275
<212>  PRT
<213>  artificial sequence

<220>
<223>  LG12-clade protease variant

<400>  134

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asn
        115                 120                 125

Met Gly Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val

180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  135
<211>  275
<212>  PRT
<213>  artificial sequence

<220>
<223>  LG12-clade protease variant

<400>  135

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asn
        115                 120                 125

Asn His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  136
<211>  275
<212>  PRT
<213>  artificial sequence

<220>
<223>  LG12-clade protease variant

<400>  136

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asn
        115                 120                 125

Thr Gln Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210> 137
<211> 275
<212> PRT
<213> artificial sequence

<220>
<223> LG12-clade protease variant

<400> 137

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asn
        115                 120                 125

Val Val Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  138
<211>  275
<212>  PRT
<213>  artificial sequence

<220>
<223>  LG12-clade protease variant

<400>  138

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asn
        115                 120                 125

Tyr Met Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  139
<211>  275
<212>  PRT
<213>  artificial sequence

<220>
<223>  LG12-clade protease variant

<400>  139

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His

50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Gln
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  140
<211>  275
<212>  PRT
<213>  artificial sequence

<220>
<223>  LG12-clade protease variant

<400>  140

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Gln
        115                 120                 125

Arg Asn Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser

225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  141
<211>  275
<212>  PRT
<213>  artificial sequence

<220>
<223>  LG12-clade protease variant

<400>  141

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Arg
        115                 120                 125

Val Glu Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  142
<211>  275
<212>  PRT
<213>  artificial sequence

<220>
<223>  LG12-clade protease variant

<400>  142

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Ser
        115                 120                 125

Glu Trp Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  143
<211>  275
<212>  PRT
<213>  artificial sequence

<220>
<223>  LG12-clade protease variant

<400>  143

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Ser
        115                 120                 125

His Gln Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  144
<211>  275
<212>  PRT
<213>  artificial sequence

<220>
<223>  LG12-clade protease variant

<400>  144

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Ser
        115                 120                 125

Ile Arg Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  145
<211>  275
<212>  PRT
<213>  artificial sequence

<220>
<223>  LG12-clade protease variant

<400>  145

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu

100                 105                 110

Trp Ser Ile Ser Asn Arg Met Asn Val Ile Asn Met Ser Leu Gly Ser
        115                 120                 125

Leu Glu Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  146
<211>  275
<212>  PRT
<213>  artificial sequence

<220>
<223>  LG12-clade protease variant

<400>  146

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Ser
        115                 120                 125

Leu Met Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln

        275

<210>  147
<211>  275
<212>  PRT
<213>  artificial sequence

<220>
<223>  LG12-clade protease variant

<400>  147

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Ser
        115                 120                 125

Asn Val Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  148
<211>  275
<212>  PRT
<213>  artificial sequence

<220>
<223>  LG12-clade protease variant

<400>  148

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Ser
        115                 120                 125

Pro Ala Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  149
<211>  275
<212>  PRT
<213>  artificial sequence

<220>
<223>  LG12-clade protease variant

<400>  149

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Ser
        115                 120                 125

Gln Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  150
<211>  275
<212>  PRT

<213>  artificial sequence

<220>
<223>  LG12-clade protease variant

<400>  150

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Ser
        115                 120                 125

Val Leu Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  151
<211>  275
<212>  PRT
<213>  artificial sequence

<220>
<223>  LG12-clade protease variant

<400>  151

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Ser
        115                 120                 125

Val Gln Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly

145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  152
<211>  275
<212>  PRT
<213>  artificial sequence

<220>
<223>  LG12-clade protease variant

<400>  152

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Thr
        115                 120                 125

Lys Leu Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  153
<211>  275
<212>  PRT
<213>  artificial sequence

<220>
<223>  LG12-clade protease variant

<400>  153

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Val
        115                 120                 125

His Glu Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  154
<211>  275
<212>  PRT
<213>  artificial sequence

<220>
<223>  LG12-clade protease variant

<400>  154

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Trp
        115                 120                 125

His Asp Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  155
<211>  275
<212>  PRT
<213>  artificial sequence

<220>
<223>  LG12-clade protease variant

<400>  155

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Trp
        115                 120                 125

Leu Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  156
<211>  275
<212>  PRT
<213>  artificial sequence

<220>
<223>  LG12-clade protease variant

<400>  156

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp

20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Trp
        115                 120                 125

Gln Phe Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  157
<211>  275
<212>  PRT
<213>  artificial sequence

<220>
<223>  LG12-clade protease variant

<400>  157

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Trp
        115                 120                 125

Tyr Asp Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr

195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  158
<211>  275
<212>  PRT
<213>  artificial sequence

<220>
<223>  LG12-clade protease variant

<400>  158

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Tyr
        115                 120                 125

Gly Thr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  159
<211>  275
<212>  PRT
<213>  artificial sequence

<220>
<223>  LG12-clade protease variant

<400>  159

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Tyr
        115                 120                 125

Pro Asp Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  160
<211>  275

<212>  PRT
<213>  artificial sequence

<220>
<223>  LG12-clade protease variant

<400>  160

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Tyr
        115                 120                 125

Val Met Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  161
<211>  275
<212>  PRT
<213>  artificial sequence

<220>
<223>  LG12-clade protease variant

<400>  161

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Thr
        115                 120                 125

His Asp Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  162
<211>  275
<212>  PRT
<213>  artificial sequence

<220>
<223>  LG12-clade protease variant

<400>  162

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly

65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Leu
        115                 120                 125

Met Asn Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  163
<211>  275
<212>  PRT
<213>  artificial sequence

<220>
<223>  LG12-clade protease variant

<400> 163

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Met
        115                 120                 125

Leu Gln Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn

245                 250                 255

Leu Gly Asp Pro Phe Phe Tyr Gly Lys Gly Val Ile Asn Val Glu Ser
            260                 265                 270

Ala Leu Gln
        275

<210>  164
<211>  275
<212>  PRT
<213>  artificial sequence

<220>
<223>  LG12-clade protease variant

<400>  164

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Asp Gly
145                 150                 155                 160

Asn Arg Asn Thr Met His Tyr Pro Ala Arg Tyr Ser Ser Val Ile Ala
                165                 170                 175

Val Gly Ala Val Ser Ser Asn Asn Thr Arg Ala Ser Phe Ser Ser Val
                180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
                260                 265                 270

Ala Leu Gln
        275

<210>  165
<211>  275
<212>  PRT
<213>  artificial sequence

<220>
<223>  LG12-clade protease variant

<400>  165

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
                20                  25                  30

Thr Gly Ile Asp Ala Thr His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asp Asn Ala Tyr Asn
    130                 135                 140

Arg Gly Ile Val Val Ile Ala Ala Ala Gly Asn Ser Gly Ser Asp Gly
145                 150                 155                 160

Asn Arg Asn Thr Met Tyr Tyr Pro Ala Arg Tyr Ser Ser Val Ile Ala
                165                 170                 175

Val Gly Ala Val Ser Ser Asn Asn Gln Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210> 166
<211> 275
<212> PRT
<213> artificial sequence

<220>
<223> LG12-clade protease variant

<400> 166

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
                20                  25                  30

Thr Gly Ile Asp Ala Thr His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn His Gly Ser Asp Gly
145                 150                 155                 160

Asn Arg Asn Thr Met Tyr Tyr Pro Ala Arg Tyr Ser Ser Val Ile Ala
                165                 170                 175

Val Gly Ala Val Ser Ser Asn Asn Gln Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Gln
            260                 265                 270

Ala Leu Gln
        275

<210>  167
<211>  275
<212>  PRT
<213>  artificial sequence

<220>
<223>  LG12-clade protease variant

<400>  167

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Thr His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn His Gly Ser Ser Gly
145                 150                 155                 160

Asn Arg Asn Thr Met Tyr Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Leu Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  168
<211>  275
<212>  PRT
<213>  artificial sequence

<220>
<223>  LG12-clade protease variant

<400>  168

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Thr His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly

115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn His Gly Ser Asp Gly
145                 150                 155                 160

Asn Arg Asn Thr Met Tyr Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Gln
            260                 265                 270

Ala Leu Gln
        275

<210>  169
<211>  275
<212>  PRT
<213>  artificial sequence

<220>
<223>  LG12-clade protease variant

<400>  169

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asp Asn Ala Tyr Asn
    130                 135                 140

Arg Gly Ile Val Leu Ile Ala Ala Ala Gly Asn His Gly Ser Ser Gly
145                 150                 155                 160

Asn Arg Asn Thr Met Asn Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Gln
            260                 265                 270

Ala Leu Gln
        275

<210>  170

<211>  275
<212>  PRT
<213>  artificial sequence

<220>
<223>  LG12-clade protease variant

<400>  170
   Ala Gln Thr Val Pro Trp Gly Ile Pro His Ile Lys Ala Asp Lys Ala
   1               5                   10                  15
   His Ala Ala Gly Val Thr Gly Ser Gly Val Lys Val Ala Ile Leu Asp
               20                  25                  30
   Thr Gly Ile Asp Ala Asn His Ala Asp Leu Asn Val Lys Gly Gly Ala
           35                  40                  45
   Ser Phe Val Ser Gly Glu Pro Asn Ala Leu Gln Asp Gly Asn Gly His
       50                  55                  60
   Gly Thr His Val Ala Gly Thr Val Ala Ala Leu Asn Asn Thr Thr Gly
   65                  70                  75                  80
   Val Leu Gly Val Ala Tyr Asn Ala Asp Leu Tyr Ala Val Lys Val Leu
                   85                  90                  95
   Ser Ala Ser Gly Ser Gly Thr Leu Ser Gly Ile Ala Gln Gly Ile Glu
               100                 105                 110
   Trp Ser Ile Ser Asn Gly Met Asn Val Ile Asn Met Ser Leu Gly Gly
           115                 120                 125
   Ser Ser Gly Ser Thr Ala Leu Gln Gln Ala Cys Asp Asn Ala Tyr Asn
       130                 135                 140
   Arg Gly Ile Val Leu Ile Ala Ala Ala Gly Asn Ser Gly Ser Asp Gly
   145                 150                 155                 160
   Asn Arg Asn Thr Met Tyr Tyr Pro Ala Arg Tyr Ser Ser Val Ile Ala
                   165                 170                 175
   Val Gly Ala Val Ser Ser Asn Asn Gln Arg Ala Ser Phe Ser Ser Val
               180                 185                 190
   Gly Ser Glu Leu Glu Val Met Ala Pro Gly Val Asn Ile Leu Ser Thr
           195                 200                 205
   Thr Pro Gly Asn Asn Tyr Ala Ser Phe Asn Gly Thr Ser Met Ala Ala
       210                 215                 220
   Pro His Val Ala Gly Ala Ala Ala Leu Ile Lys Ala Lys Tyr Pro Ser
   225                 230                 235                 240
   Met Thr Asn Val Gln Ile Arg Glu Arg Leu Lys Asn Thr Ala Thr Asn
                   245                 250                 255
   Leu Gly Asp Pro Phe Phe Tyr Gly Lys Gly Val Ile Asn Val Glu Ser
               260                 265                 270
   Ala Leu Gln
           275

<210>  171
<211>  275
<212>  PRT
<213>  artificial sequence

<220>
<223>  LG12-clade protease variant

<400>  171

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Thr His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asp Asn Ala Tyr Asn
    130                 135                 140

Arg Gly Ile Val Leu Ile Ala Ala Ala Gly Asn His Gly Ser Asp Gly
145                 150                 155                 160

Asn Arg Asn Thr Met Tyr Tyr Pro Ala Arg Tyr Ser Ser Val Ile Ala
                165                 170                 175

Val Gly Ala Val Ser Ser Asn Asn Gln Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Gln
            260                 265                 270

Ala Leu Gln
        275

<210>  172
<211>  275
<212>  PRT
<213>  artificial sequence

<220>
<223>  LG12-clade protease variant

<400>  172

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asp Asn Ala Tyr Asn
    130                 135                 140

Arg Gly Ile Val Val Ile Ala Ala Ala Gly Asn Ser Gly Ser Asp Gly
145                 150                 155                 160

Asn Arg Asn Thr Met Tyr Tyr Pro Ala Arg Tyr Ser Ser Val Ile Ala
                165                 170                 175

Val Gly Ala Val Ser Ser Asn Asn Gln Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  173
<211>  275
<212>  PRT
<213>  artificial sequence

<220>
<223>  LG12-clade protease variant

<400>  173

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Asp Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  174
<211>  275
<212>  PRT
<213>  artificial sequence

<220>
<223>  LG12-clade protease variant

<400>  174

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Asp Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala

                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Arg
                245                 250                 255

Leu Gly Asp Pro Phe Phe Tyr Gly Lys Gly Val Ile Asn Val Glu Lys
            260                 265                 270

Ala Leu Gln
        275

<210> 175
<211> 275
<212> PRT
<213> artificial sequence

<220>
<223> LG12-clade protease variant

<400> 175

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Asp Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
                100                 105                 110

Trp Ser Ile Ser Asn Arg Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
                180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Arg
                245                 250                 255

Leu Gly Asp Pro Phe Phe Tyr Gly Lys Gly Val Ile Asn Val Glu Lys
                260                 265                 270

Ala Leu Gln
        275

<210>  176
<211>  275
<212>  PRT
<213>  artificial sequence

<220>
<223>  LG12-clade protease variant

<400>  176

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Asp Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  177
<211>  275
<212>  PRT
<213>  artificial sequence

<220>
<223>  LG12-clade protease variant

<400>  177

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Asp Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  178
<211>  275
<212>  PRT
<213>  artificial sequence

<220>
<223>  LG12-clade protease variant

<400>  178

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Asp Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  179
<211>  275
<212>  PRT
<213>  artificial sequence

<220>
<223>  LG12-clade protease variant

<400>  179

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala

35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  180
<211>  275
<212>  PRT
<213>  artificial sequence

<220>
<223>  LG12-clade protease variant

<400>  180

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala

210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Arg
                245                 250                 255

Leu Gly Asp Pro Phe Phe Tyr Gly Lys Gly Val Ile Asn Val Glu Ser
            260                 265                 270

Ala Leu Gln
        275

<210>  181
<211>  275
<212>  PRT
<213>  artificial sequence

<220>
<223>  LG12-clade protease variant

<400>  181

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Lys Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  182
<211>  275
<212>  PRT
<213>  artificial sequence

<220>
<223>  LG12-clade protease variant

<400>  182

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  183
<211>  275
<212>  PRT
<213>  artificial sequence

<220>

<223>  LG12-clade protease variant

<400>  183

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Arg
                245                 250                 255

Leu Gly Asp Pro Phe Phe Tyr Gly Lys Gly Val Ile Asn Val Glu Ser
            260                 265                 270

Ala Leu Gln
        275

<210>  184
<211>  275
<212>  PRT
<213>  artificial sequence

<220>
<223>  LG12-clade protease variant

<400>  184

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  185
<211>  275
<212>  PRT
<213>  artificial sequence

<220>
<223>  LG12-clade protease variant

<400>  185

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu

85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Arg
                245                 250                 255

Leu Gly Asp Pro Phe Phe Tyr Gly Lys Gly Val Ile Asn Val Glu Lys
            260                 265                 270

Ala Leu Gln
        275

<210>  186
<211>  275
<212>  PRT
<213>  artificial sequence

<220>
<223>  LG12-clade protease variant

<400>  186

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Asp Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Lys

260                 265                 270

Ala Leu Gln
        275

<210>  187
<211>  275
<212>  PRT
<213>  artificial sequence

<220>
<223>  LG12-clade protease variant

<400>  187

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Asp Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Arg
                245                 250                 255

Leu Gly Asp Pro Phe Phe Tyr Gly Lys Gly Val Ile Asn Val Glu Lys
            260                 265                 270

Ala Leu Gln
        275

<210>  188
<211>  275
<212>  PRT
<213>  artificial sequence

<220>
<223>  LG12-clade protease variant

<400>  188

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Asp Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Lys Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  189
<211>  275
<212>  PRT
<213>  artificial sequence

<220>
<223>  LG12-clade protease variant

<400>  189

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  190
<211>  275
<212>  PRT
<213>  artificial sequence

<220>
<223>  LG12-clade protease variant

<400>  190

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Arg
                245                 250                 255

Leu Gly Asp Pro Phe Phe Tyr Gly Lys Gly Val Ile Asn Val Glu Lys
            260                 265                 270

Ala Leu Gln
        275

<210>  191
<211>  275
<212>  PRT
<213>  artificial sequence

<220>
<223>  LG12-clade protease variant

<400>  191

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Glu
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn

130                 135                 140

Arg Gly Ile Val Gly Ile Ala Ala Ala Gly Asn Asn Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  192
<211>  275
<212>  PRT
<213>  artificial sequence

<220>
<223>  LG12-clade protease variant

<400>  192

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Gly Ile Ala Ala Ala Gly Asn Asn Gly Ser Asp Gly
145                 150                 155                 160

Asn Arg Asn Thr Met His Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  193
<211>  275
<212>  PRT
<213>  artificial sequence

<220>
<223>  LG12-clade protease variant

<400>  193

Ala Gln Thr Val Pro Trp Gly Ile Pro His Ile Lys Ala Asp Lys Ala
1               5                   10                  15

His Ala Ala Gly Val Thr Gly Ser Gly Val Ser Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Glu Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Asp Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Pro
                245                 250                 255

Leu Gly Asp Pro Phe Phe Tyr Gly Lys Gly Val Ile Asn Val Glu Ser
            260                 265                 270

Ala Leu Gln
        275

<210>  194
<211>  275
<212>  PRT
<213>  artificial sequence

<220>
<223>  LG12-clade protease variant

<400>  194

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Gln Gly Gly Ala
        35                  40                  45

Ser Phe Val Ser Glu Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Asp Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Pro
                245                 250                 255

Leu Gly Asp Pro Phe Phe Tyr Gly Lys Gly Val Ile Asn Val Glu Ser
            260                 265                 270

Ala Leu Gln
        275

<210>  195
<211>  272
<212>  PRT
<213>  artificial sequence

<220>
<223>  LG12-clade protease variant

<400>  195

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Gln Asp Gly Asn Gly His Gly Thr His
    50                  55                  60

Val Ala Gly Thr Val Ala Ala Leu Asn Asn Thr Thr Gly Val Leu Gly
65                  70                  75                  80

Val Ala Tyr Asn Ala Asp Leu Tyr Ala Val Lys Val Leu Ser Ala Ser
                85                  90                  95

Gly Ser Gly Thr Leu Ser Gly Ile Ala Gln Gly Ile Glu Trp Ser Ile
            100                 105                 110

Ser Asn Gly Met Asn Val Ile Asn Met Ser Leu Gly Gly Ser Ser Gly
        115                 120                 125

Ser Thr Ala Leu Gln Gln Ala Cys Asn Asn Ala Tyr Asn Arg Gly Ile
    130                 135                 140

Val Val Ile Ala Ala Ala Gly Asn Ser Gly Ser Ser Gly Asn Arg Asn
145                 150                 155                 160

Thr Met Gly Tyr Pro Ala Arg Tyr Ser Ser Val Ile Ala Val Gly Ala
                165                 170                 175

Val Ser Ser Asn Asn Thr Arg Ala Ser Phe Ser Ser Val Gly Ser Glu
            180                 185                 190

Leu Glu Val Met Ala Pro Gly Val Asn Ile Leu Ser Thr Thr Pro Gly
        195                 200                 205

Asn Asn Tyr Ala Ser Phe Asn Gly Thr Ser Met Ala Ala Pro His Val
    210                 215                 220

Ala Gly Ala Ala Ala Leu Ile Lys Ala Lys Tyr Pro Ser Met Thr Asn
225                 230                 235                 240

Val Gln Ile Arg Glu Arg Leu Lys Asn Thr Ala Thr Asn Leu Gly Asp
                245                 250                 255

Pro Phe Phe Tyr Gly Lys Gly Val Ile Asn Val Glu Ser Ala Leu Gln
            260                 265                 270

<210> 196
<211> 275
<212> PRT
<213> artificial sequence

<220>
<223> LG12-clade protease variant

<400> 196

Ala Gln Thr Val Pro Trp Gly Ile Thr His Ile Lys Ala Asp Lys Ala
1               5                   10                  15

His Ala Ala Gly Val Thr Gly Ser Gly Val Lys Val Ala Ile Leu Asp

20                  25                  30

Thr Gly Ile Asp Ala Asn His Ala Asp Leu Asn Val Arg Gly Gly Glu
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Ala Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Asn
        115                 120                 125

Ser Ser Gly Ser Thr Ala Leu Gln Gln Ala Cys Asp Asn Ala Tyr Asn
    130                 135                 140

Arg Gly Ile Val Val Ile Ala Ala Ala Gly Asn Ser Gly Val Ser Gly
145                 150                 155                 160

Asn Arg Asn Thr Met Gly Tyr Pro Ala Arg Tyr Ser Ser Val Ile Ala
                165                 170                 175

Val Gly Ala Val Asn Ser Asn Asn Thr Arg Ala Ser Phe Ser Ser Val
            180                 185                 190

Gly Pro Glu Leu Glu Val Met Ala Pro Gly Val Asn Ile Leu Ser Thr
        195                 200                 205

Val Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Ala Ile Arg Glu Arg Leu Lys Asn Thr Ala Thr Pro
                245                 250                 255

Leu Gly Asp Pro Phe Phe Tyr Gly Lys Gly Val Ile Asn Val Glu Gln
            260                 265                 270

Ala Leu Gln
        275

<210>  197
<211>  274
<212>  PRT
<213>  artificial sequence

<220>
<223>  LG12-clade protease variant

<400>  197

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Glu Pro Asn Ala Leu Gln Asp Gly Asn Gly His Gly
    50                  55                  60

Thr His Val Ala Gly Thr Val Ala Ala Leu Asn Asn Thr Thr Gly Val
65                  70                  75                  80

Leu Gly Val Ala Tyr Asn Ala Asp Leu Tyr Ala Val Lys Val Leu Ser
                85                  90                  95

Ala Ser Gly Ser Gly Thr Leu Ser Gly Ile Ala Gln Gly Ile Glu Trp
            100                 105                 110

Ser Ile Ser Asn Gly Met Asn Val Ile Asn Met Ser Leu Gly Gly Ser
        115                 120                 125

Ser Gly Ser Thr Ala Leu Gln Gln Ala Cys Asn Asn Ala Tyr Asn Arg
    130                 135                 140

Gly Ile Val Val Ile Ala Ala Ala Gly Asn Ser Gly Ser Ser Gly Asn
145                 150                 155                 160

Arg Asn Thr Met Gly Tyr Pro Ala Arg Tyr Ser Ser Val Ile Ala Val
                165                 170                 175

Gly Ala Val Ser Ser Asn Asn Thr Arg Ala Ser Phe Ser Ser Val Gly
            180                 185                 190

Ser Glu Leu Glu Val Met Ala Pro Gly Val Asn Ile Leu Ser Thr Thr

195                 200                 205

Pro Gly Asn Asn Tyr Ala Ser Phe Asn Gly Thr Ser Met Ala Ala Pro
    210                 215                 220

His Val Ala Gly Ala Ala Ala Leu Ile Lys Ala Lys Tyr Pro Ser Met
225                 230                 235                 240

Thr Asn Val Gln Ile Arg Glu Arg Leu Lys Asn Thr Ala Thr Asn Leu
                245                 250                 255

Gly Asp Pro Phe Phe Tyr Gly Lys Gly Val Ile Asn Val Glu Ser Ala
            260                 265                 270

Leu Gln

<210>  198
<211>  274
<212>  PRT
<213>  artificial sequence

<220>
<223>  LG12-clade protease variant

<400>  198

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Asn Ala Leu Gln Asp Gly Asn Gly His Gly
    50                  55                  60

Thr His Val Ala Gly Thr Val Ala Ala Leu Asn Asn Thr Thr Gly Val
65                  70                  75                  80

Leu Gly Val Ala Tyr Asn Ala Asp Leu Tyr Ala Val Lys Val Leu Ser
                85                  90                  95

Ala Ser Gly Ser Gly Thr Leu Ser Gly Ile Ala Gln Gly Ile Glu Trp
            100                 105                 110

Ser Ile Ser Asn Gly Met Asn Val Ile Asn Met Ser Leu Gly Gly Ser
        115                 120                 125

Ser Gly Ser Thr Ala Leu Gln Gln Ala Cys Asn Asn Ala Tyr Asn Arg
    130                 135                 140

Gly Ile Val Val Ile Ala Ala Ala Gly Asn Ser Gly Ser Ser Gly Asn
145                 150                 155                 160

Arg Asn Thr Met Gly Tyr Pro Ala Arg Tyr Ser Ser Val Ile Ala Val
                165                 170                 175

Gly Ala Val Ser Ser Asn Asn Thr Arg Ala Ser Phe Ser Ser Val Gly
            180                 185                 190

Ser Glu Leu Glu Val Met Ala Pro Gly Val Asn Ile Leu Ser Thr Thr
        195                 200                 205

Pro Gly Asn Asn Tyr Ala Ser Phe Asn Gly Thr Ser Met Ala Ala Pro
    210                 215                 220

His Val Ala Gly Ala Ala Ala Leu Ile Lys Ala Lys Tyr Pro Ser Met
225                 230                 235                 240

Thr Asn Val Gln Ile Arg Glu Arg Leu Lys Asn Thr Ala Thr Asn Leu
                245                 250                 255

Gly Asp Pro Phe Phe Tyr Gly Lys Gly Val Ile Asn Val Glu Ser Ala
            260                 265                 270

Leu Gln

<210>  199
<211>  274
<212>  PRT
<213>  artificial sequence

<220>
<223>  LG12-clade protease variant

<400>  199

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Leu Gln Asp Gly Asn Gly His Gly
    50                  55                  60

Thr His Val Ala Gly Thr Val Ala Ala Leu Asn Asn Thr Thr Gly Val
65                  70                  75                  80

Leu Gly Val Ala Tyr Asn Ala Asp Leu Tyr Ala Val Lys Val Leu Ser
                85                  90                  95

Ala Ser Gly Ser Gly Thr Leu Ser Gly Ile Ala Gln Gly Ile Glu Trp
            100                 105                 110

Ser Ile Ser Asn Gly Met Asn Val Ile Asn Met Ser Leu Gly Gly Ser
        115                 120                 125

Ser Gly Ser Thr Ala Leu Gln Gln Ala Cys Asn Asn Ala Tyr Asn Arg
    130                 135                 140

Gly Ile Val Val Ile Ala Ala Ala Gly Asn Ser Gly Ser Ser Gly Asn
145                 150                 155                 160

Arg Asn Thr Met Gly Tyr Pro Ala Arg Tyr Ser Ser Val Ile Ala Val
                165                 170                 175

Gly Ala Val Ser Ser Asn Asn Thr Arg Ala Ser Phe Ser Ser Val Gly
            180                 185                 190

Ser Glu Leu Glu Val Met Ala Pro Gly Val Asn Ile Leu Ser Thr Thr
        195                 200                 205

Pro Gly Asn Asn Tyr Ala Ser Phe Asn Gly Thr Ser Met Ala Ala Pro
    210                 215                 220

His Val Ala Gly Ala Ala Ala Leu Ile Lys Ala Lys Tyr Pro Ser Met
225                 230                 235                 240

Thr Asn Val Gln Ile Arg Glu Arg Leu Lys Asn Thr Ala Thr Asn Leu
                245                 250                 255

Gly Asp Pro Phe Phe Tyr Gly Lys Gly Val Ile Asn Val Glu Ser Ala
            260                 265                 270

Leu Gln

<210>  200
<211>  275

<212>  PRT
<213>  artificial sequence

<220>
<223>  LG12-clade protease variant

<400>  200

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Asp Gly
145                 150                 155                 160

Asn Arg Asn Thr Met Asp Tyr Pro Ala Arg Tyr Ser Ser Val Ile Ala
                165                 170                 175

Val Gly Ala Val Ser Glu Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  201
<211>  274
<212>  PRT
<213>  artificial sequence

<220>
<223>  LG12-clade protease variant

<400>  201

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Gln Asp Gly Asn Gly His Gly
    50                  55                  60

Thr His Val Ala Gly Thr Val Ala Ala Leu Asn Asn Thr Thr Gly Val
65                  70                  75                  80

Leu Gly Val Ala Tyr Asn Ala Asp Leu Tyr Ala Val Lys Val Leu Ser
                85                  90                  95

Ala Ser Gly Ser Gly Thr Leu Ser Gly Ile Ala Gln Gly Ile Glu Trp
            100                 105                 110

Ser Ile Ser Asn Gly Met Asn Val Ile Asn Met Ser Leu Gly Gly Ser
        115                 120                 125

Ser Gly Ser Thr Ala Leu Gln Gln Ala Cys Asn Asn Ala Tyr Asn Arg
    130                 135                 140

Gly Ile Val Val Ile Ala Ala Ala Gly Asn Ser Gly Ser Ser Gly Asn
145                 150                 155                 160

Arg Asn Thr Met Gly Tyr Pro Ala Arg Tyr Ser Ser Val Ile Ala Val
                165                 170                 175

Gly Ala Val Ser Ser Asn Asn Thr Arg Ala Ser Phe Ser Ser Val Gly
            180                 185                 190

Ser Glu Leu Glu Val Met Ala Pro Gly Val Asn Ile Leu Ser Thr Thr
        195                 200                 205

Pro Gly Asn Asn Tyr Ala Ser Phe Asn Gly Thr Ser Met Ala Ala Pro
    210                 215                 220

His Val Ala Gly Ala Ala Ala Leu Ile Lys Ala Lys Tyr Pro Ser Met
225                 230                 235                 240

Thr Asn Val Gln Ile Arg Glu Arg Leu Lys Asn Thr Ala Thr Asn Leu
                245                 250                 255

Gly Asp Pro Phe Phe Tyr Gly Lys Gly Val Ile Asn Val Glu Ser Ala
            260                 265                 270

Leu Gln

<210>  202
<211>  275
<212>  PRT
<213>  artificial sequence

<220>
<223>  LG12-clade protease variant

<400>  202

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly

65                  70                  75                  80

Val Leu Gly Val Ala Tyr Asn Ala Asp Leu Tyr Ala Val Lys Val Leu
                85                  90                  95

Ser Ala Ser Ala Ser Gly Thr Leu Ser Gly Ile Ala Gln Gly Ile Glu
            100                 105                 110

Trp Ser Ile Ser Asn Gly Met Asn Val Ile Asn Met Ser Leu Gly His
        115                 120                 125

Phe Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  203
<211>  275
<212>  PRT
<213>  artificial sequence

<220>
<223>  LG12-clade protease variant

<400> 203

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Gln His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Ala Ser Gly Thr Leu Ser Gly Ile Ala Gln Gly Ile Glu
            100                 105                 110

Trp Ser Ile Ser Ser Arg Met Asn Val Ile Asn Met Ser Leu Gly His
        115                 120                 125

Ser Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn

245                 250                 255

Leu Gly Asp Pro Phe Phe Tyr Gly Lys Gly Val Ile Asn Val Glu Ser
            260                 265                 270

Ala Leu Gln
        275

<210>  204
<211>  275
<212>  PRT
<213>  artificial sequence

<220>
<223>  LG12-clade protease variant

<400>  204

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Asp Asp Leu Asn Val Lys Gly Gly Ala
        35                  40                  45

Ser Phe Val Gln Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Met
        115                 120                 125

Ser Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met His Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Phe Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Lys Cys Gly Lys
            260                 265                 270

Val Leu Tyr
        275

<210> 205
<211> 275
<212> PRT
<213> artificial sequence

<220>
<223> LG12-clade protease variant

<400> 205

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Asp Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Met
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met His Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210> 206
<211> 275
<212> PRT
<213> artificial sequence

<220>
<223> LG12-clade protease variant

<400> 206

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Ser
        115                 120                 125

Phe Phe Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  207
<211>  275
<212>  PRT
<213>  artificial sequence

<220>
<223>  LG12-clade protease variant

<400>  207

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Gly
        115                 120                 125

Phe Ser Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  208
<211>  275
<212>  PRT
<213>  artificial sequence

<220>
<223>  LG12-clade protease variant

<400>  208

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Met

115                 120                 125

Phe Phe Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Phe Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  209
<211>  275
<212>  PRT
<213>  artificial sequence

<220>
<223>  LG12-clade protease variant

<400>  209

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Asp Asp Leu Asn Val Lys Gly Gly Ala
        35                  40                  45

Ser Phe Val Gln Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Ser
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Phe Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  210

<211>  275
<212>  PRT
<213>  artificial sequence

<220>
<223>  LG12-clade protease variant

<400>  210

Ala Gln Thr Val Pro Trp Gly Ile Pro His Ile Lys Ala Asp Lys Ala
1               5                   10                  15

His Ala Ala Gly Val Thr Gly Ser Gly Val Met Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Tyr Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Asp
        115                 120                 125

Arg Ile Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Lys Arg Leu Lys Asn Thr Ala Thr Glu
                245                 250                 255

Leu Gly Asp Pro Phe Phe Tyr Gly Lys Gly Val Ile Asn Val Glu Lys
            260                 265                 270

Ala Leu Gln
        275

<210>  211
<211>  275
<212>  PRT
<213>  artificial sequence

<220>
<223>  LG12-clade protease variant

<400>  211

Ala Gln Thr Val Pro Trp Cys Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Asp
        115                 120                 125

Arg Ile Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  212
<211>  275
<212>  PRT
<213>  artificial sequence

<220>
<223>  LG12-clade protease variant

<400>  212

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Asp
        115                 120                 125

Arg Ile Gly Ser Thr Ala Leu Gln Gln Ala Cys Asn Asn Ala Tyr Asn
    130                 135                 140

Arg Gly Ile Val Val Ile Ala Ala Ala Gly Asn Thr Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  213
<211>  275
<212>  PRT
<213>  artificial sequence

<220>
<223>  LG12-clade protease variant

<400>  213

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Asp
        115                 120                 125

Arg Ile Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met His Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  214
<211>  275
<212>  PRT
<213>  artificial sequence

<220>
<223>  LG12-clade protease variant

<400>  214

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Asp Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Arg Ile Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Ser Gly
145                 150                 155                 160

Asn Arg Asn Thr Met Asp Tyr Pro Ala Arg Tyr Ser Ser Val Ile Ala

165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Lys Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  215
<211>  275
<212>  PRT
<213>  artificial sequence

<220>
<223>  LG12-clade protease variant

<400>  215

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Tyr Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Glu
                245                 250                 255

Leu Gly Asp Pro Phe Phe Tyr Gly Lys Gly Val Ile Asn Val Glu Lys
            260                 265                 270

Ala Leu Gln
        275

<210>  216
<211>  275
<212>  PRT
<213>  artificial sequence

<220>
<223>  LG12-clade protease variant

<400>  216

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Asp Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Ser Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Glu
                245                 250                 255

Leu Gly Asp Pro Phe Phe Tyr Gly Lys Gly Val Ile Asn Val Glu Lys
            260                 265                 270

Ala Leu Gln
        275

<210>  217
<211>  275
<212>  PRT
<213>  artificial sequence

<220>
<223>  LG12-clade protease variant

<400>  217

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Phe Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  218
<211>  275
<212>  PRT
<213>  artificial sequence

<220>
<223>  LG12-clade protease variant

<400>  218

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Phe Tyr Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Lys
            260                 265                 270

Ala Leu Gln
        275

<210>  219
<211>  275
<212>  PRT
<213>  artificial sequence

<220>
<223>  LG12-clade protease variant

<400>  219

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala

35                  40                  45

Ser Phe Val Phe Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  220
<211>  275
<212>  PRT
<213>  artificial sequence

<220>
<223>  LG12-clade protease variant

<400>  220

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala

210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Lys
            260                 265                 270

Ala Leu Gln
        275

<210>  221
<211>  275
<212>  PRT
<213>  artificial sequence

<220>
<223>  LG12-clade protease variant

<400>  221

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Ser
            260                 265                 270

Ala Leu Gln
        275

<210>  222
<211>  275
<212>  PRT
<213>  artificial sequence

<220>
<223>  LG12-clade protease variant

<400>  222

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  223
<211>  275
<212>  PRT
<213>  artificial sequence

<220>

<223>  LG12-clade protease variant

<400>  223

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  224
<211>  275
<212>  PRT
<213>  artificial sequence

<220>
<223>  LG12-clade protease variant

<400>  224

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  225
<211>  275
<212>  PRT
<213>  artificial sequence

<220>
<223>  LG12-clade protease variant

<400>  225

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Tyr Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu

85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  226
<211>  275
<212>  PRT
<213>  artificial sequence

<220>
<223>  LG12-clade protease variant

<400>  226

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Phe Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Lys

260                 265                 270

Ala Leu Gln
        275

<210>  227
<211>  275
<212>  PRT
<213>  artificial sequence

<220>
<223>  LG12-clade protease variant

<400>  227

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Phe Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Ser
            260                 265                 270

Ala Leu Gln
        275

<210> 228
<211> 275
<212> PRT
<213> artificial sequence

<220>
<223> LG12-clade protease variant

<400> 228

Ala Gln Thr Val Pro Trp Cys Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Asp Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Ser
            260                 265                 270

Ala Leu Gln
        275

<210>  229
<211>  275
<212>  PRT
<213>  artificial sequence

<220>
<223>  LG12-clade protease variant

<400>  229

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Phe Tyr Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Lys
            260                 265                 270

Ala Leu Gln
        275

<210>  230
<211>  275
<212>  PRT
<213>  artificial sequence

<220>
<223>  LG12-clade protease variant

<400>  230

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  231
<211>  275
<212>  PRT
<213>  artificial sequence

<220>
<223>  LG12-clade protease variant

<400>  231

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Phe Tyr Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn

130                 135                 140

Arg Gly Ile Val Val Ile Ala Ala Ala Gly Asn Asn Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Lys
            260                 265                 270

Ala Leu Gln
        275

<210>  232
<211>  275
<212>  PRT
<213>  artificial sequence

<220>
<223>  LG12-clade protease variant

<400>  232

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Asp Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  233
<211>  275
<212>  PRT
<213>  artificial sequence

<220>
<223>  LG12-clade protease variant

<400>  233

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Phe Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Arg
        275

<210>  234
<211>  275
<212>  PRT
<213>  artificial sequence

<220>
<223>  LG12-clade protease variant

<400>  234

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Phe Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Arg
                245                 250                 255

Leu Gly Asp Pro Phe Phe Tyr Gly Lys Gly Val Ile Asn Val Glu Ser
            260                 265                 270

Ala Leu Gln
        275

<210>  235
<211>  275
<212>  PRT
<213>  artificial sequence

<220>
<223>  LG12-clade protease variant

<400>  235

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Phe Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Arg
                245                 250                 255

Leu Gly Asp Pro Phe Phe Tyr Gly Lys Gly Val Ile Asn Val Glu Lys
            260                 265                 270

Ala Leu Gln
        275

<210>  236
<211>  275
<212>  PRT
<213>  artificial sequence

<220>
<223>  LG12-clade protease variant

<400>  236

Ala Gln Thr Val Pro Trp Gly Ile Pro His Ile Lys Ala Asp Lys Ala

1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Phe Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  237
<211>  275
<212>  PRT
<213>  artificial sequence

<220>
<223>  LG12-clade protease variant

<400>  237

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Phe Tyr Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val

180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  238
<211>  275
<212>  PRT
<213>  artificial sequence

<220>
<223>  LG12-clade protease variant

<400>  238

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Phe Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met His Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Lys
            260                 265                 270

Ala Leu Gln
        275

<210>  239
<211>  275
<212>  PRT
<213>  artificial sequence

<220>
<223>  LG12-clade protease variant

<400>  239

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Tyr Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  240
<211>  275
<212>  PRT
<213>  artificial sequence

<220>
<223>  LG12-clade protease variant

<400>  240

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Lys
            260                 265                 270

Ala Leu Gln
        275

<210>  241
<211>  275
<212>  PRT
<213>  artificial sequence

<220>
<223>  LG12-clade protease variant

<400>  241

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Arg
                245                 250                 255

Leu Gly Asp Pro Phe Phe Tyr Gly Lys Gly Val Ile Asn Val Glu Ser
            260                 265                 270

Ala Leu Gln
        275

<210>  242
<211>  275
<212>  PRT
<213>  artificial sequence

<220>
<223>  LG12-clade protease variant

<400>  242

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His

50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Ser Gly
145                 150                 155                 160

Asn Arg Asn Thr Met His Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Arg
                245                 250                 255

Leu Gly Asp Pro Phe Phe Tyr Gly Lys Gly Val Ile Asn Val Glu Ser
            260                 265                 270

Ala Leu Gln
        275

<210>  243
<211>  275
<212>  PRT
<213>  artificial sequence

<220>
<223>  LG12-clade protease variant

<400>  243

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Phe Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser

225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  244
<211>  275
<212>  PRT
<213>  artificial sequence

<220>
<223>  LG12-clade protease variant

<400>  244

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Val Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  245
<211>  275
<212>  PRT
<213>  artificial sequence

<220>
<223>  LG12-clade protease variant

<400>  245

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Arg
                245                 250                 255

Leu Gly Asp Pro Phe Phe Tyr Gly Lys Gly Val Ile Asn Val Glu Ser
            260                 265                 270

Ala Leu Gln
        275


<210>  246
<211>  275
<212>  PRT
<213>  artificial sequence

<220>
<223>  LG12-clade protease variant

<400>  246

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Phe Tyr Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Ser
            260                 265                 270

Ala Leu Gln
        275

<210>  247
<211>  275
<212>  PRT
<213>  artificial sequence

<220>
<223>  LG12-clade protease variant

<400>  247

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Phe Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Asp
        115                 120                 125

Phe Tyr Gly Ser Thr Ala Leu Gln Gln Ala Cys Asn Asn Ala Tyr Asn
    130                 135                 140

Arg Gly Ile Val Val Ile Ala Ala Ala Gly Asn Asn Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  248
<211>  275
<212>  PRT
<213>  artificial sequence

<220>
<223>  LG12-clade protease variant

<400>  248

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu

100                 105                 110

Trp Ser Ile Ser Asn Gly Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Asp Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  249
<211>  275
<212>  PRT
<213>  artificial sequence

<220>
<223>  LG12-clade protease variant

<400>  249

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln

        275

<210>  250
<211>  275
<212>  PRT
<213>  artificial sequence

<220>
<223>  LG12-clade protease variant

<400>  250

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Tyr Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Asp Gly Ser Ser Gly
145                 150                 155                 160

Asn Arg Asn Thr Met His Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Ser
            260                 265                 270

Ala Leu Gln
        275

<210>  251
<211>  275
<212>  PRT
<213>  artificial sequence

<220>
<223>  LG12-clade protease variant

<400>  251

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  252
<211>  275
<212>  PRT
<213>  artificial sequence

<220>
<223>  LG12-clade protease variant

<400>  252

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Lys
            260                 265                 270

Ala Leu Gln
        275

<210>  253
<211>  275
<212>  PRT

<213>  artificial sequence

<220>
<223>  LG12-clade protease variant

<400>  253

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Glu
                245                 250                 255

Leu Gly Asp Pro Phe Phe Tyr Gly Lys Gly Val Ile Asn Val Glu Lys
            260                 265                 270

Ala Leu Gln
        275

<210>  254
<211>  275
<212>  PRT
<213>  artificial sequence

<220>
<223>  LG12-clade protease variant

<400>  254

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Asp Gly Ser Ser Gly

145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  255
<211>  275
<212>  PRT
<213>  artificial sequence

<220>
<223>  LG12-clade protease variant

<400>  255

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Lys
            260                 265                 270

Ala Leu Gln
        275

<210>  256
<211>  275
<212>  PRT
<213>  artificial sequence

<220>
<223>  LG12-clade protease variant

<400>  256

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  257
<211>  275
<212>  PRT
<213>  artificial sequence

<220>
<223>  LG12-clade protease variant

<400>  257

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210> 258
<211> 275
<212> PRT
<213> artificial sequence

<220>
<223> LG12-clade protease variant

<400> 258

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Asp Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210> 259
<211> 275
<212> PRT
<213> artificial sequence

<220>
<223> LG12-clade protease variant

<400> 259

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp

20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Lys
            260                 265                 270

Ala Leu Gln
        275

<210>  260
<211>  275
<212>  PRT
<213>  artificial sequence

<220>
<223>  LG12-clade protease variant

<400>  260

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Asp Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr

195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  261
<211>  275
<212>  PRT
<213>  artificial sequence

<220>
<223>  LG12-clade protease variant

<400>  261

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Tyr Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Asp Gly Ser Ser Gly
145                 150                 155                 160

Asn Arg Asn Thr Met His Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Glu
                245                 250                 255

Leu Gly Asp Pro Phe Phe Tyr Gly Lys Gly Val Ile Asn Val Glu Lys
            260                 265                 270

Ala Leu Gln
        275

<210>  262
<211>  275
<212>  PRT
<213>  artificial sequence

<220>
<223>  LG12-clade protease variant

<400>  262

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  263
<211>  275

<212>  PRT
<213>  artificial sequence

<220>
<223>  LG12-clade protease variant

<400>  263

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Tyr Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  264
<211>  275
<212>  PRT
<213>  artificial sequence

<220>
<223>  LG12-clade protease variant

<400>  264

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Tyr Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  265
<211>  275
<212>  PRT
<213>  artificial sequence

<220>
<223>  LG12-clade protease variant

<400>  265

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Tyr Glu Pro Asn Ala Leu Gln Asp Gly Asn Gly His
    50                  55                  60

Gly Thr His Val Ala Gly Thr Val Ala Ala Leu Asn Asn Thr Thr Gly

65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Lys
            260                 265                 270

Ala Leu Gln
        275

<210>  266
<211>  275
<212>  PRT
<213>  artificial sequence

<220>
<223>  LG12-clade protease variant

<400> 266

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asp Arg Leu Lys Asn Thr Ala Thr Asn

245                 250                 255

Leu Gly Asp Pro Phe Phe Tyr Gly Lys Gly Val Ile Asn Val Glu Lys
            260                 265                 270

Ala Leu Gln
        275

<210>  267
<211>  275
<212>  PRT
<213>  artificial sequence

<220>
<223>  LG12-clade protease variant

<400>  267

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210> 268
<211> 275
<212> PRT
<213> artificial sequence

<220>
<223> LG12-clade protease variant

<400> 268

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Phe Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Lys
            260                 265                 270

Ala Leu Gln
        275

<210>  269
<211>  275
<212>  PRT
<213>  artificial sequence

<220>
<223>  LG12-clade protease variant

<400>  269

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Tyr Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Asp Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Lys
            260                 265                 270

Ala Leu Gln
        275

<210>  270
<211>  275
<212>  PRT
<213>  artificial sequence

<220>
<223>  LG12-clade protease variant

<400>  270

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Glu Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

<210>  271
<211>  275
<212>  PRT
<213>  artificial sequence

<220>
<223>  LG12-clade protease variant

<400>  271

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Thr Gly Glu Pro Asn Ala Leu Gln Asp Gly Asn Gly His
    50                  55                  60

Gly Thr His Val Ala Gly Thr Val Ala Ala Leu Asp Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Arg Met Asn Val Ile Asn Met Ser Leu Gly Val

115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Asp Gly Ser Ser Gly
145                 150                 155                 160

Asn Arg Asn Thr Met Asp Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Glu
                245                 250                 255

Leu Gly Asp Pro Phe Phe Tyr Gly Lys Gly Val Ile Asn Val Glu Lys
            260                 265                 270

Ala Leu Gln
        275

<210>  272
<211>  275
<212>  PRT
<213>  artificial sequence

<220>
<223>  LG12-clade protease variant

<400>  272

Ala Gln Thr Val Pro Trp Gly Ile Pro His Ile Lys Ala Asp Lys Ala
1               5                   10                  15

His Ala Ala Gly Val Thr Gly Ser Gly Val Lys Val Ala Ile Leu Asp
            20                  25                  30

Thr Gly Ile Asp Ala Asn His Ala Asp Leu Asn Val Lys Gly Gly Ala
        35                  40                  45

Ser Phe Val Ser Gly Glu Pro Asn Ala Leu Gln Asp Gly Asn Gly His
    50                  55                  60

Gly Thr His Val Ala Gly Thr Val Ala Ala Leu Asn Asn Thr Thr Gly
65                  70                  75                  80

Val Leu Gly Val Ala Tyr Asn Ala Asp Leu Tyr Ala Val Lys Val Leu
                85                  90                  95

Ser Ala Ser Gly Ser Gly Thr Leu Ser Gly Ile Ala Gln Gly Ile Glu
            100                 105                 110

Trp Ser Ile Ser Asn Gly Met Asn Val Ile Asn Met Ser Leu Gly Val
        115                 120                 125

Met His Gly Ser Thr Ala Leu Gln Gln Ala Cys Asn Asn Ala Tyr Asn
    130                 135                 140

Arg Gly Ile Val Val Ile Ala Ala Ala Gly Asn Ser Gly Ser Ser Gly
145                 150                 155                 160

Asn Arg Asn Thr Met Gly Tyr Pro Ala Arg Tyr Ser Ser Val Ile Ala
                165                 170                 175

Val Gly Ala Val Ser Ser Asn Asn Thr Arg Ala Ser Phe Ser Ser Val
            180                 185                 190

Gly Ser Glu Leu Glu Val Met Ala Pro Gly Val Asn Ile Leu Ser Thr
        195                 200                 205

Thr Pro Gly Asn Asn Tyr Ala Ser Arg Asn Gly Thr Ser Met Ala Ala
    210                 215                 220

Pro His Val Ala Gly Ala Ala Ala Leu Ile Lys Ala Lys Tyr Pro Ser
225                 230                 235                 240

Met Thr Asn Val Gln Ile Arg Asn Arg Leu Lys Asn Thr Ala Thr Asn
                245                 250                 255

Leu Gly Asp Pro Phe Phe Tyr Gly Lys Gly Val Ile Asn Val Glu Ser
            260                 265                 270

Ala Leu Gln
        275

## Description

Serine proteases are a class of enzymes that cleave peptide bonds in proteins. The serine proteases contain a nucleophilic serine residue at the active site. Subtilisins are a large family of serine proteases, and although they have long been known in the art of industrial enzymes, there remains a need for engineered proteases that are suitable for particular conditions and uses.

The present disclosure provides, *inter alia,* LG12-clade protease enzymes, including variant LG12-clade protease enzymes, nucleic acids encoding the same, and compositions and methods related to the production and use thereof.

In some embodiments, the invention is a composition comprising at least one variant disclosed herein. In some embodiments, the invention is a method of cleaning using a cleaning composition comprising at least one variant disclosed herein.

Variants, compositions and methods disclosed herein relate to a protease variant, or a recombinant polypeptide or an active fragment thereof generated through conventional molecular biology techniques (see, e.g., Sambrook et al, Molecular Cloning: Cold Spring Harbor Laboratory Press). Some embodiments are directed to a protease variant, or a recombinant polypeptide or an active fragment thereof comprising an amino acid sequence comprising two or more substitutions at one or more positions selected from: (i) 1, 3, 7, 9, 19, 24, 27, 30, 38, 40, 43, 45, 48, 52, 53, 57, 72, 76, 78, 79, 86, 87, 88, 89, 90, 91, 93, 97, 99, 100, 101, 103, 104, 106, 107, 111, 114, 115, 116, 117, 118, 120, 122, 123, 124, 128, 129, 130, 131, 133, 134, 136, 139, 140, 141, 143, 144, 145, 147, 148, 149, 150, 153, 156, 158, 159, 160, 166, 181, 182, 185, 188, 194, 209, 212, 213, 215, 216, 217, 222, 224, 228, 234, 236, 238, 240, 242, 245, 246, 248, 251, 252, 256, 265, 267, 269, 270, 271, 272, 273, and 275; (ii) 1, 3, 9, 19, 24, 27, 30, 38, 40, 43, 45, 48, 52, 53, 57, 72, 76, 78, 79, 86, 87, 88, 89, 90, 91, 93, 97, 99, 100, 101, 103, 104, 106, 107, 111, 114, 115, 116, 117, 118, 120, 122, 123, 124, 128, 129, 130, 131, 133, 134, 136, 139, 140, 141, 143, 144, 145, 147, 148, 149, 150, 153, 156, 158, 159, 160, 166, 181, 182, 185, 188, 194, 209, 212, 213, 215, 216, 217, 222, 224, 228, 234, 236, 238, 240, 242, 245, 246, 248, 251, 252, 256, 265, 267, 271, 272, and 275; (iii) 1, 7, 9, 27, 38, 40, 45, 48, 52, 53, 76, 78, 91, 100, 114, 117, 118, 122, 128, 129, 130, 140, 147, 149, 156, 158, 159, 160, 166, 181, 182, 194, 209, 217, 222, 228, 234, 236, 238, 245, 248, 256, 269, 270, 271, 272, 273, and 275; (iv) 1, 24, 30, 38, 40, 48, 52, 53, 76, 78, 79, 86, 87, 88, 89, 91, 93, 97, 100, 103, 104, 106, 111, 114, 115, 116, 117, 118, 120, 122, 124, 128, 129, 130, 131, 133, 134, 136, 139, 141, 143, 144, 147, 148, 149, 150, 153, 156, 158, 159, 166, 182, 185, 194, 209, 212, 213, 216, 217, 222, 224, 236, 238, 248, 251, 252, 256, 272, and 275; (v) 1, 24, 30, 38, 40, 52, 53, 76, 78, 79, 86, 87, 88, 89, 93, 97, 100, 103, 104, 106, 111, 114, 115, 116, 117, 118, 120, 122, 124, 128, 129, 131, 133, 134, 136, 139, 141, 143, 144, 147, 148, 149, 150, 153, 156, 158, 159, 166, 182, 185, 194, 209, 212, 213, 216, 217, 222, 224, 236, 238, 248, 251, 252, 256, 272, and 275; or (vi) 1, 38, 40, 48, 52, 53, 76, 91,114, 117, 118, 128,129, 130, 147, 149, 156, 158, 159, 166, 182, 217, 238, 248, 256, and 272; wherein the amino acid positions of the variant or recombinant polypeptide or active fragment thereof are numbered by correspondence with the amino acid sequence of SEQ ID NO:10. Another embodiment is directed to a protease variant, or a recombinant polypeptide or an active fragment thereof comprising an amino acid sequence comprising two or more substitutions at one or more position selected from: (i) 52, 53, 76, 86, 97, 103, 104, 106, 111, 114, 115, 116, 118, 120, 122, 124, 128, 129, 130, 131, 134, 136, 139, 141, 143, 144, 147, 148, 150, 156, 158, 159, 166, 217, 248, and 272; (ii) 53, 76, 106, 128, 131, 134, 139, 158, 159, 166, 217, 248, and 272; (iii) 52, 53, 76, 106, 118, 128, 129, 130, 131, 134, 139, 156, 158, 166, 217, 248, and 272; (iv) 52, 53, 76, 128, 118, 156, 158, 166, 217, and 248; (v) 53, 76, 128, 158, 166, and 248; (vi) 52, 53, 76, 118, 128, 156, 158, 166, 217, and 248; or (vii) 52-129, 53-166, 76-166, 166-159, 166-272, 53-76-166, 53-118-166, 53-166-272, 76-118-166, 76-166-248, 76-166-256, and 76-166-272; wherein the amino acid positions of the variant or recombinant polypeptide or active fragment thereof are numbered by correspondence with the amino acid sequence of SEQ ID NO:10.

A still further embodiment is directed to a protease variant, or a recombinant polypeptide or an active fragment thereof comprising an amino acid sequence comprising two or more substitutions selected from: (i) A1X, T3X, G7X, P9X, A19X, S24X, K27X, I30X, N38X, A40X, N43X, K45X, A48X, S52X, G53X, A57X, V72X, N76X, T78X, T79X, Y86X, N87X, A88X, D89X, L90X, Y91X, V93X, S97X, S99X, G100X, S101X, T103X, L104X, G106X, I107X, I111X, S114X, I115X, S116X, N117X, G118X, N120X, I122X, N123X, M124X, G128X, S129X, S130X, G131X, T133X, A134X, Q136X, C139X, N140X, N141X, Y143X, N144X, R145X, I147X, V148X, V149X, I150X, A153X, S156X, S158X, S159X, G160X, G166X, S181X, S182X, T185X, S188X, S194X, T209X, N212X, N213X, A215X, S216X, F217X, M222X, A224X, A228X, I234X, A236X, Y238X, S240X, T242X, Q245X, I246X, E248X, K251X, N252X, N256X, K265X, V267X, N269X, V270X, E271X, S272X, A273X, and Q275X; (ii) A1X, T3X, P9X, A19X, S24X, K27X, I30X, N38X, A40X, N43X, K45X, A48X, S52X, G53X, A57X, V72X, N76X, T78X, T79X, Y86X, N87X, A88X, D89X, L90X, Y91X, V93X, S97X, S99X, G100X, S101X, T103X, L104X, G106X, I107X, I111X, S114X, I115X, S116X, N117X, G118X, N120X, I122X, N123X, M124X, G128X, S129X, S130X, G131X, T133X, A134X, Q136X, C139X, N140X, N141X, Y143X, N144X, R145X, I147X, V148X, V149X, I150X, A153X, S156X, S158X, S159X, G160X, G166X, S181X, S182X, T185X, S188X, S194X, T209X, N212X, N213X, A215X, S216X, F217X, M222X, A224X, A228X, I234X, A236X, Y238X, S240X, T242X, Q245X, I246X, E248X, K251X, N252X, N256X, K265X, V267X, E271X, S272X, and Q275X; (iii) A1X, G7X, P9X, K27X, N38X, A40X, K45X, A48X, S52X, G53X, N76X, T78X, Y91X, G100X, S114X, N117X, G118X, I122X, G128X, S129X, S130X, N140X, I147X, V149X, S156X, S158X, S159X, G160X, G166X, S181X, S182X, S194X, T209X, F217X, M222X, A228X, I234X, A236X, Y238X, Q245X, E248X, N256X, N269X, V270X, E271X, S272X, A273X, and Q275X; (iv) A1X, S24X, I30X, N38X, A40X, A48X, S52X, G53X, N76X, T78X, T79X, Y86X, N87X, A88X, D89X, Y91X, V93X, S97X, G100X, T103X, L104X, G106X, I111X, S114X, I115X, S116X, N117X, G118X, N120X, I122X, M124X, G128X, S129X, S130X, G131X, T133X, A134X, Q136X, C139X, N141X, Y143X, N144X, I147X, V148X, V149X, I150X, A153X, S156X, S158X, S159X, G166X, S182X, T185X, S194X, T209X, N212X, N213X, S216X, F217X, M222X, A224X, A236X, Y238X, E248X, K251X, N252X, N256X, S272X, and Q275X; (v) A1X, S24X, I30X, N38X, A40X, S52X, G53X, N76X, T78X, T79X, Y86X, N87X, A88X, D89X, V93X, S97X, G100X, T103X, L104X, G106X, I111X, S114X, I115X, S116X, N117X, G118X, N120X, I122X, M124X, G128X, S129X, G131X, T133X, A134X, Q136X, C139X, N141X, Y143X, N144X, I147X, V148X, V149X, I150X, A153X, S156X, S158X, S159X, G166X, S182X, T185X, S194X, T209X, N212X, N213X, S216X, F217X, M222X, A224X, A236X, Y238X, E248X, K251X, N252X, N256X, S272X, and Q275X; or (vi) A1X, N38X, A40X, A48X, S52X, G53X, N76X, Y91X, S114X, N117X, G118X, G128X, S129X, S130X, I147X, V149X, S156X, S158X, S159X, G166X, S182X, F217X, Y238X, E248X, N256X, and S272X; wherein X is any amino acid and the amino acid positions of the variant or recombinant polypeptide or active fragment thereof are numbered by correspondence with the amino acid sequence of SEQ ID NO:10. Yet still another embodiment is directed to a protease variant, or a recombinant polypeptide or an active fragment thereof comprising an amino acid sequence comprising two or more substitutions selected from: (i) S52X, G53X, N76X, Y86X, S97X, T103X, L104X, G106X, I111X, S114X, I115X, S116X, G118X, N120X, I122X, M124X, G128X, S129X, S130X, G131X, A134X, Q136X, C139X, N141X, Y143X, N144X, I147X, V148X, I150X, S156X, S158X, S159X, G166X, F217X, E248X, and S272X; (ii) G53X, N76X, G106X, G128X, G131X, A134X, C139X, S158X, S159X, G166X, F217X, E248X, and S272X; (iii) S52X, G53X, N76X, G106X, G118X, G128X, S129X, S130X, G131X, A134X, C139X, S156X, G166X, F217X, E248X, and S272X; (iv) S52X, G53X, N76X, G118X, G128X, S156X, S158X, G166X, F217X, and E248X; (v) G53X, N76X, G128X, S158X, G166X, and E248X; (vi) S52X, G53X, N76X, G118X, G128X, S156X, S158X, G166X, F217X, and E248X; or (vii) S52X-S129X, G53X-G166X, N76X-G166X, G166X-S159X, G166X-S272X, G53X-N76X-G166X, G53X-G118X-G166X, G53X-G166X-S272X, N76X-G118X-G166X, N76X-G166X-E248X, N76X-G166X-N256X, and N76X-G166X-S272X; wherein X is any amino acid and the amino acid positions of the variant or recombinant polypeptide or active fragment thereof are numbered by correspondence with the amino acid sequence of SEQ ID NO:10.

An even still further embodiment is directed to a protease variant, or a recombinant polypeptide or an active fragment thereof comprising an amino acid sequence comprising two or more substitutions selected from: (i) X1G, X1T, X3E, X3V, X7C, X9I, X9T, X9V, X19R, X24R, X24T, X27S, X27T, X30V, X38A, X38Q, X38T, X40D, X40E, X40L, X40V, X43D, X43H, X43L, X43S, X45Q, X45R, X48E, X48Q, X52A, X52N, X52Q, X52T, X53D, X53E, X53N, X57P, X72A, X76D, X78A, X78D, X78G, X78S, X79I, X86P, X87S, X88V, X89E, X89S, X90P, X91H, X93T, X97G, X99L, X100A, X100K, X101T, X103E, X103S, X104V, X106S, X107T, X107V, X111L, X114A, X114T, X115G, X116E, X116N, X117E, X117S, X118R, X120H, X122A, X123D, X124L, X128A, X128D, X128E, X128H, X128K, X128M, X128N, X128Q, X128S, X128Y, X129D, X129E, X129G, X129H, X129I, X129K, X129L, X129M, X129N, X129P, X129Q, X129R, X129T, X129V, X129Y, X130A, X130E, X130F, X130G, X130H, X130I, X130L, X130M, X130N, X130Q, X130R, X130T, X130V, X130W, X130Y, X131P, X133A, X133D, X133V, X134T, X136E, X139V, X140D, X140Y, X141S, X143T, X144S, X145N, X145W, X147A, X147V, X148L, X149G, X149L, X149N, X149S, X150V, X153S, X156A, X156H, X156N, X158T, X158V, X159D, X159E, X159N, X159Q, X160D, X166A, X166D, X166H, X166N, X166S, X166Y, X181A, X181N, X182E, X185Q, X188Y, X194P, X209F, X209V, X209Y, X212S, X213T, X215D, X215E, X216T, X217L, X217R, X222L, X224T, X228S, X234V, X236N, X236Q, X238F, X240F, X242S, X245A, X245N, X246V, X248K, X248N, X251R, X251S, X252S, X256E, X256K, X256P, X256R, X256Y, X265S, X267N, X269K, X270C, X271F, X271W, X272K, X272N, X272Q, X273V, X275H, and X275R; (ii) X1G, X1T, X3E, X3V, X9I, X9T, X9V, X19R, X24R, X24T, X27S, X27T, X30V, X38A, X38Q, X38T, X40D, X40E, X40L, X40V, X43D, X43H, X43L, X43S, X45Q, X45R, X48E, X48Q, X52A, X52N, X52Q, X52T, X53D, X53E, X53N, X57P, X72A, X76D, X78A, X78D, X78G, X78S, X79I, X86P, X87S, X88V, X89E, X89S, X90P, X91H, X93T, X97G, X99L, X100A, X100K, X101T, X103E, X103S, X104V, X106S, X107T, X107V, X111L, X114A, X114T, X115G, X116E, X116N, X117E, X117S, X118R, X120H, X122A, X123D, X124L, X128A, X128D, X128E, X128H, X128K, X128M, X128N, X128Q, X128S, X128Y, X129D, X129E, X129G, X129H, X129I, X129K, X129L, X129M, X129N, X129P, X129Q, X129R, X129T, X129V, X129Y, X130A, X130E, X130F, X130G, X130H, X130I, X130L, X130M, X130N, X130Q, X130R, X130T, X130V, X130W, X130Y, X131P, X133A, X133D, X133V, X134T, X136E, X139V, X140D, X140Y, X141S, X143T, X144S, X145N, X145W, X147A, X147V, X148L, X149G, X149L, X149N, X149S, X150V, X153S, X156A, X156H, X156N, X158T, X158V, X159D, X159E, X159N, X159Q, X160D, X166A, X166D, X166H, X166N, X166S, X166Y, X181A, X181N, X182E, X185Q, X188Y, X194P, X209F, X209V, X209Y, X212S, X213T, X215D, X215E, X216T, X217L, X217R, X222L, X224T, X228S, X234V, X236N, X236Q, X238F, X240F, X242S, X245A, X245N, X246V, X248K, X248N, X251R, X251S, X252S, X256E, X256K, X256P, X256R, X256Y, X265S, X267N, X271F, X271W, X272K, X272N, X272Q, X275H, and X275R; (iii) X1G, X7C, X9T, X27M, X38Q, X38T, X40D, X45Q, X45R, X48E, X48Q, X52F, X52Q, X52T, X53D, X53E, X53Y, X76D, X78A, X91H, X100A, X114T, X117S, X118R, X122V, X128A, X128D, X128E, X128H, X128K, X128L, X128M, X128N, X128Q, X128R, X128S, X128T, X128V, X128W, X128Y, X129D, X129E, X129F, X129G, X129H, X129I, X129K, X129L, X129M, X129N, X129P, X129Q, X129R, X129T, X129V, X129Y, X130A, X130D, X130E, X130F, X130G, X130H, X130I, X130L, X130M, X130N, X130Q, X130R, X130T, X130V, X130W, X130Y, X140D, X147A, X149G, X156D, X156H, X156N, X156T, X158T, X158V, X159D, X159N, X160D, X166D, X166H, X166Y, X181N, X182E, X194P, X209V, X217R, X222L, X228S, X234V, X236N, X236Q, X238F, X245A, X248D, X248K, X248N, X256E, X256P, X256R, X269K, X270C, X271G, X272K, X272Q, X273V, X275R, and X275Y; (iv) X1G, X1T, X24R, X24T, X30V, X38A, X38Q, X38T, X40D, X40E, X40L, X40V, X48Q, X52A, X52N, X52Q, X52T, X53D, X53E, X53N, X76D, X78A, X78D, X78G, X78S, X79I, X86P, X87S, X88V, X89E, X91H, X93T, X97G, X100A, X100K, X103E, X103S, X104V, X106S, X111L, X114A, X114T, X115G, X116E, X116N, X117E, X117S, X118R, X120H, X122A, X124L, X128A, X128D, X128E, X128H, X128K, X128M, X128N, X128Q, X128S, X128Y, X129D, X129E, X129G, X129H, X129I, X129K, X129L, X129M, X129N, X129P, X129Q, X129R, X129T, X129V, X129Y, X130F, X130H, X130I, X130Y, X131P, X133A, X133D, X133V, X134T, X136E, X139V, X141S, X143T, X144S, X147A, X147V, X148L, X149G, X149L, X149N, X149S, X150V, X153S, X156A, X156H, X156N, X158T, X158V, X159D, X159E, X159N, X159Q, X166A, X166D, X166H, X166N, X166S, X166Y, X182E, X185Q, X194P, X209F, X209V, X209Y, X212S, X213T, X216T, X217L, X217R, X222L, X224T, X236N, X236Q, X238F, X248K, X248N, X251R, X251S, X252S, X256E, X256K, X256P, X256R, X256Y, X272K, X272N, X272Q, X275H, and X275R; (v) X1G, X1T, X24R, X24T, X30V, X38A, X38Q, X38T, X40D, X40E, X40L, X40V, X52A, X52N, X52Q, X52T, X53D, X53E, X53N, X76D, X78A, X78D, X78G, X78S, X79I, X86P, X87S, X88V, X89E, X93T, X97G, X100A, X100K, X103E, X103S, X104V, X106S, X111L, X114A, X114T, X115G, X116E, X116N, X117E, X117S, X118R, X120H, X122A, X124L, X128A, X128D, X128E, X128H, X128K, X128M, X128N, X128Q, X128S, X128Y, X129D, X129E, X129G, X129H, X129I, X129K, X129L, X129M, X129N, X129P, X129Q, X129R, X129T, X129V, X129Y, X131P, X133A, X133D, X133V, X134T, X136E, X139V, X141S, X143T, X144S, X147A, X147V, X148L, X149G, X149L, X149N, X149S, X150V, X153S, X156A, X156H, X156N, X158T, X158V, X159D, X159E, X159N, X159Q, X166A, X166D, X166H, X166N, X166S, X166Y, X182E, X185Q, X194P, X209F, X209V, X209Y, X212S, X213T, X216T, X217L, X217R, X222L, X224T, X236N, X236Q, X238F, X248K, X248N, X251R, X251S, X252S, X256E, X256K, X256P, X256R, X256Y, X272K, X272N, X272Q, X275H, and X275R; or (vi) X1G, X38Q, X40D, X48Q, X52F, X52T, X53E, X53Y, X76D, X91H, X114T, X117S, X118R, X128D, X128M, X128V, X129F, X129M, X129R, X129V, X130F, X130H, X130I, X130Y, X147A, X149G, X156N, X158T, X159N, X166D, X166H, X182E, X217R, X238F, X248N, X256E, X256P, X256R, and X272K; wherein X is any amino acid and the amino acid positions of the variant or recombinant polypeptide or active fragment thereof are numbered by correspondence with the amino acid sequence of SEQ ID NO:10. Another embodiment is directed to a protease variant, or a recombinant polypeptide or an active fragment thereof comprising an amino acid sequence comprising two or more substitutions selected from: (i) X52T, X53D, X76D, X86P, X97G, X103S, X104V, X106S, X111L, X114A, X115G, X116N, X118R, X120H, X122A, X124L, X128D, X128S, X129F, X130Y, X131P, X134T, X136E, X139V, X141S, X143T, X144S, X147V, X148L, X150V, X156N, X158T, X159D, X166D, X217R, X248N, and X272K; (ii) X53D, X76D, X106S, X128S, X131P, X134T, X139V, X158T, X159D, X166D, X217R, X248N, and X272K; (iii) X52T, X53D, X76D, X106S, X118R, X128D, X129F, X130Y, X131P, X134T, X139V, X156N, X166D, X217R, X248N, and X272K; (iv) X52T, X53D, X76D, X118R, X128S, X156N, X158T, X166D, X217R, and X248N; (v) X53D, X76D, X128S, X158T, X166D, and X248N; (vi) X52T, X53D, X76D, X118R, X128S, X156N, X158T, X166D, X217R, and X248N; or (vii) X52T-X129F, X53D-X166D, X76D-X166D, X166H-X159D, X166D-X272K, X53D-X76D-X166D, X53D-X118R-X166D, X53D-X166D-X272K, X76D-X118R-X166D, X76D-X166D-X248K, X76D-X166D-X256R, and X76D-X166D-X272K; wherein X is any amino acid and the amino acid positions of the variant or recombinant polypeptide or active fragment thereof are numbered by correspondence with the amino acid sequence of SEQ ID NO:10.

An even further embodiment is directed to a protease variant, or a recombinant polypeptide or an active fragment thereof comprising an amino acid sequence comprising two or more substitutions selected from: (i) A001G, A001T, T003E, T003V, G007C, P009I, P009T, P009V, A019R, S024R, S024T, K027S, K027T, 1030V, N038A, N038Q, N038T, A040D, A040E, A040L, A040V, N043D, N043H, N043L, N043S, K045Q, K045R, A048E, A048Q, S052A, S052N, S052Q, S052T, G053D, G053E, G053N, A057P, V072A, N076D, T078A, T078D, T078G, T078S, T079I, Y086P, N087S, A088V, D089E, D089S, L090P, Y091H, V093T, S097G, S099L, G100A, G100K, S101T, T103E, T103S, L104V, G106S, I107T, I107V, I111L, S114A, S114T, I115G, S116E, S116N, N117E, N117S, G118R, N120H, I122A, N123D, M124L, G128A, G128D, G128E, G128H, G128K, G128M, G128N, G128Q, G128S, G128Y, S129D, S129E, S129G, S129H, S129I, S129K, S129L, S129M, S129N, S129P, S129Q, S129R, S129T, S129V, S129Y, S130A, S130E, S130F, S130G, S130H, S130I, S130L, S130M, S130N, S130Q, S130R, S130T, S130V, S130W, S130Y, G131P, T133A, T133D, T133V, A134T, Q136E, C139V, N140D, N140Y, N141S, Y143T, N144S, R145N, R145W, I147A, I147V, V148L, V149G, V149L, V149N, V149S, I150V, A153S, S156A, S156H, S156N, S158T, S158V, S159D, S159E, S159N, S159Q, G160D, G166A, G166D, G166H, G166N, G166S, G166Y, S181A, S181N, S182E, T185Q, S188Y, S194P, T209F, T209V, T209Y, N212S, N213T, A215D, A215E, S216T, F217L, F217R, M222L, A224T, A228S, I234V, A236N, A236Q, Y238F, S240F, T242S, Q245A, Q245N, I246V, E248K, E248N, K251R, K251S, N252S, N256E, N256K, N256P, N256R, N256Y, K265S, V267N, N269K, V270C, E271F, E271W, S272K, S272N, S272Q, A273V, Q275H, and Q275R; (ii) A001G, A001T, T003E, T003V, P009I, P009T, P009V, A019R, S024R, S024T, K027S, K027T, I030V, N038A, N038Q, N038T, A040D, A040E, A040L, A040V, N043D, N043H, N043L, N043S, K045Q, K045R, A048E, A048Q, S052A, S052N, S052Q, S052T, G053D, G053E, G053N, A057P, V072A, N076D, T078A, T078D, T078G, T078S, T079I, Y086P, N087S, A088V, D089E, D089S, L090P, Y091H, V093T, S097G, S099L, G100A, G100K, S101T, T103E, T103S, L104V, G106S, I107T, I107V, I111L, S114A, S114T, I115G, S116E, S116N, N117E, N117S, G118R, N120H, I122A, N123D, M124L, G128A, G128D, G128E, G128H, G128K, G128M, G128N, G128Q, G128S, G128Y, S129D, S129E, S129G, S129H, S129I, S129K, S129L, S129M, S129N, S129P, S129Q, S129R, S129T, S129V, S129Y, S130A, S130E, S130F, S130G, S130H, S130I, S130L, S130M, S130N, S130Q, S130R, S130T, S130V, S130W, S130Y, G131P, T133A, T133D, T133V, A134T, Q136E, C139V, N140D, N140Y, N141S, Y143T, N144S, R145N, R145W, I147A, I147V, V148L, V149G, V149L, V149N, V149S, I150V, A153S, S156A, S156H, S156N, S158T, S158V, S159D, S159E, S159N, S159Q, G160D, G166A, G166D, G166H, G166N, G166S, G166Y, S181A, S181N, S182E, T185Q, S188Y, S194P, T209F, T209V, T209Y, N212S, N213T, A215D, A215E, S216T, F217L, F217R, M222L, A224T, A228S, I234V, A236N, A236Q, Y238F, S240F, T242S, Q245A, Q245N, I246V, E248K, E248N, K251R, K251S, N252S, N256E, N256K, N256P, N256R, N256Y, K265S, V267N, E271F, E271W, S272K, S272N, S272Q, Q275H, and Q275R; (iii) A001G, G007C, P009T, K027M, N038Q, N038T, A040D, K045Q, K045R, A048E, A048Q, S052F, S052Q, S052T, G053D, G053E, G053Y, N076D, T078A, Y091H, G100A, S114T, N117S, G118R, I122V, G128A, G128D, G128E, G128H, G128K, G128L, G128M, G128N, G128Q, G128R, G128S, G128T, G128V, G128W, G128Y, S129D, S129E, S129F, S129G, S129H, S129I, S129K, S129L, S129M, S129N, S129P, S129Q, S129R, S129T, S129V, S129Y, S130A, S130D, S130E, S130F, S130G, S130H, S130I, S130L, S130M, S130N, S130Q, S130R, S130T, S130V, S130W, S130Y, N140D, I147A, V149G, S156D, S156H, S156N, S156T, S158T, S158V, S159D, S159N, G160D, G166D, G166H, G166Y, S181N, S182E, S194P, T209V, F217R, M222L, Y238F, Q245A, E248D, E248K, E248N, N256E, N256P, N256R, N269K, V270C, E271G, S272K, S272Q, A273V, Q275R, and Q275Y; (iv) A001G, A001T, S024T, I030V, N038Q, A040D, A048Q, S052A, S052Q, S052T, G053D, G053E, N076D, T078D, T078S, T079I, Y086P, N087S, A088V, D089E, D089S, Y091H,V093T, S097G, G100A, T103E, T103S, L104V, G106S, I111L, S114A, S114T, I115G, S116N, N117S, G118R, N120H, I122A, M124L, G128S, S129P, S130F, S130H, S130I, S130Y, G131P, T133A, T133V, A134T, Q136E, C139V, N141S, Y143T, N144S, I147A, I147V, V148L, V149G, V149L, I150V, A153S, S156H, S156N, S158T, S159D, S159N, G166D, G166H, G166S, S182E, T185Q, S194P, T209Y, N212S, N213T, S216T, F217L, F217R, M222L, A224T, A236N, Y238F, E248N, K251S, N252S, N256P, N256R, N256Y, S272K, S272Q, and Q275H; (v) A001G, A001T, S024T, I030V, N038Q, A040D, S052A, S052Q, S052T, G053D, G053E, N076D, T078D, T078S, T079I, Y086P, N087S, A088V, D089E, D089S, V093T, S097G, G100A, T103E, T103S, L104V, G106S, I111L, S114A, S114T, I115G, S116N, N117S, G118R, N120H, I122A, M124L, G128S, S129P, G131P, T133A, T133V, A134T, Q136E, C139V, N141S, Y143T, N144S, I147A, I147V, V148L, V149G, V149L, I150V, A153S, S156H, S156N, S158T, S159D, S159N, G166D, G166H, G166S, S182E, T185Q, S194P, T209Y, N212S, N213T, S216T, F217L, F217R, M222L, A224T, A236N, Y238F, E248N, K251S, N252S, N256P, N256R, N256Y, S272K, S272Q, and Q275H; or (vi) A001G, N038Q, A040D, A048Q, S052F, S052T, G053E, G053Y, N076D, Y091H, S114T, N117S, G118R, G128D, G128M, G128V, S129F, S129M, S129R, S129V, S130F, S130H, S130I, S130Y, I147A, V149G, S156N, S158T, S159N, G166D, G166H, S182E, F217R, Y238F, E248N, N256E, N256P, N256R, and S272K; wherein the amino acid positions of the variant or recombinant polypeptide or active fragment thereof are numbered by correspondence with the amino acid sequence of SEQ ID NO:10. A still further embodiment is directed to a protease variant, or a recombinant polypeptide or an active fragment thereof comprising an amino acid sequence comprising two or more substitutions selected from: (i) S52T, G53D, N76D, Y086P, S097G, T103S, L104V, G106S, I111L, S114A, I115G, S116N, G118R, N120H, I122A, M124L, G128D, G128S, S129F, S129P, S130Y, G131P, A134T, Q136E, C139V, N141S, Y143T, N144S, I147V, V148L, I150V, S156N, S158T, S159D, G166D, F217R, E248N, and S272K; (ii) G53D, N76D, G106S, G128S, G131P, A134T, C139V, S158T, S159D, G166D, F217R, E248N, and S272K; (iii) S52T, G53D, N76D, G106S, G118R, G128D, S129F, S130Y, G131P, A134T, C139V, S156N, G166D, F217R, E248N, and S272K; (iv) S52T, G53D, N76D, G118R, G128S, S156N, S158T, G166D, F217R, and E248N; (v) G53D, N76D, G128S, S158T, G166D, and E248N; (vi) S52T, G53D, N76D, G118R, S156N, S158T, G166D, F217R, and E248N; or (vii) S52T-S129F, G53D-G166D, N76D-G166D, G166H-S159D, G166D-S272K, G53D-N76D-G166D, G53D-G118R-G166D, G53D-G166D-S272K, N76D-G118R-G166D, N76D-G166D-E248K, N76D-G166D-N256R, and N76D-G166D-S272K; wherein the amino acid positions of the variant or recombinant polypeptide or active fragment thereof are numbered by correspondence with the amino acid sequence of SEQ ID NO:10.

An even still further embodiment is directed to a protease variant, or a recombinant polypeptide or an active fragment thereof comprising an amino acid sequence comprising one or more deletions at one or more positions selected from 53, 55, 56, 57, and 58 or G053, P055, N056, A057 and L058; wherein the amino acid positions of the variant, or recombinant polypeptide or active fragment thereof are numbered by correspondence with the amino acid sequence of SEQ ID NO:10.

Another embodiment is directed to an LG12-Clade of subtilisins comprising one or more protease variants described herein, or recombinant polypeptide or an active fragment thereof, wherein said variant, or recombinant polypeptide or active fragment thereof comprises an EPNALQDGNGHGTH (SEQ ID NO:44) motif. In a still further embodiment, one or more protease variant described herein, or recombinant polypeptide or an active fragment thereof comprises an amino acid sequence having at least 75% amino acid sequence identity to SEQ ID NO:10. In yet still other embodiments, one or more protease variant described herein, or recombinant polypeptide or an active fragment thereof has protease activity in the presence of a surfactant, cleaning activity in a detergent composition, or a combination thereof. In an even further embodiment, one or more protease variant described herein, or recombinant polypeptide or an active fragment thereof has one or more improved property selected from improved cleaning performance, improved stability, and improved residual protease activity when compared to SEQ ID NO:10 and/or 48. A still further embodiment is directed to a polynucleotide comprising a nucleic acid sequence that encodes a protease variant, or recombinant polypeptide or active fragment thereof described herein.

An even further embodiment is directed to a composition containing one or more protease variants described herein, or recombinant polypeptide or an active fragment thereof. In yet still an even further embodiment, the composition described herein contains phosphate, is phosphate-free, contains borate, is boron-free, or combinations thereof. In other embodiments, the composition is a boron-free composition. In some embodiments, a boron-free composition is a composition to which a borate stabilizer has not been added. In another embodiment, a boron-free composition is a composition that contains less than 5.5% boron. In a still further embodiment, a boron-free composition is a composition that contains less than 4.5% boron. In yet still another embodiment, a boron-free composition is a composition that contains less than 3.5% boron. In yet still further embodiments, a boron-free composition is a composition that contains less than 2.5% boron. In even further embodiments, a boron-free composition is a composition that contains less than 1.5% boron. In another embodiment, a boron-free composition is a composition that contains less than 1.0% boron. In still further embodiments, a boron-free composition is a composition that contains less than 0.5% boron. In other embodiments, the composition is a cleaning composition. In yet further embodiments, the cleaning composition is a detergent composition. In still further embodiments, a boron-free composition is a composition substantially-free of boron.

In some embodiments, the serine protease enzymes disclosed herein find use in soil removal, grain ethanol production, animal feed, food processing and digestive aids, leather processing, and/or personal care. In still further embodiments, the serine protease enzyme variants disclosed herein can be combined with other enzymes useful in detergent compositions. Still further embodiments provide methods for using the serine protease enzyme variants disclosed herein.

Serine protease enzymes can be improved by engineering the enzyme to have better cleaning performance, stability in the presence of chemical agents, and/or thermostability over currently available serine protease enzymes. In some embodiments, the serine protease enzyme variants disclosed herein have a minimum performing index (PI) for wash performance, stability of the enzyme in detergent compositions and thermostability of the enzyme, while having at least one of these characteristics improved from a parent serine protease enzyme. In further embodiments, the serine protease enzymes disclosed herein are particularly well suited to and useful in a variety of cleaning applications. In some embodiments, the serine protease enzymes are LG12-clade serine protease variants. In other embodiments, the serine protease enzyme variants disclosed herein have one or more modifications, such as a substitution, insertion or deletion, as compared to a parent serine protease enzyme. In some embodiments, the protease variant, or recombinant polypeptide or active fragment thereof is derived from a reference polypeptide selected from SEQ ID NOs:10, 18, 19, 45, 46, 57, 58, and 66. In other embodiments the reference polypeptide is LG12 wild-type protease (SEQ ID NO:10).
Figure 1A-1B provides a Clustal W protein sequence alignment of LG12-Clade proteases (LG12-CP022, LG12-CP180, LG12-CP446, LG12-CP455, LG12-CP460, LG12-CP462, and LG12-CP465) and parent LG12 wild-type protease.
Figure 2 provides a structure of the LG12 wild-type protease depicting the key loops comprising residues 97-104, 126-130, 150-170 and 207-220 that form the substrate binding region in which the overall folding is represented as a schematic in light gray; the catalytic triad formed by Aspartic acid 32, Histidine 64 and Serine 221 is shown as black stick figures; the two calcium sites common to all commercial bacterial subtilisins are represented as dark gray spheres; and the key loops forming the substrate binding region are set forth as black lines.
Figure 3 provides a 3D structure of the LG12 wild-type protease depicting (i) side chains of substitutions common to the best LG12-Clade protease variants, which substitutions are primarily localized in two segments that form the walls of the substrate binding site and the following helical segments that serve to anchor the residue 97-148 segment, (ii) substitutions 158, 166 and 217, which line the substrate binding cavity, and (iii) substitutions at positions 76, 78, 79 and 86, which are near one of the critical calcium binding sites.
Figure 4A-4F provides the Clustal W alignment of various *Bacillus* subtilisin mature protease sequences including members of the LG12-clade.
Figure 5 provides the phylogenetic tree of LG12-clade proteases and various other *Bacillus* subtilisins.
Figure 6 provides a structure of LG12 wild-type protease depicting the catalytic triad formed by Aspartic acid 32, Histidine 64 and Serine 221 and the region including residues 54-59 leading into His 64 of the triad in which the overall fold of the main chain is shown in light gray; the side chains of the catalytic triad formed by Aspartic acid 32, Histidine 64 and Serine 221 are shown as black sticks; Histidine 64 is indicated by the arrow; the region including residues 54-59 leading into Histidine 64 of the triad are also shown as black sticks.
Figure 7 provides a crystallographic structure of LG12 wild-type protease in which the region 54-59 in the sequence EPNALQ of LG12, shown as black sticks, is compared with the equivalent loop segment in subtilisin BPN' (pdb entry 2ST1), shown as gray lines.
Figure 8 provides a crystallographic structure of LG12 wild-type protease in which the region 54-59 in the sequence EPNALQ of LG12, shown as black sticks, is compared with the equivalent loop segment in subtilisin Carlsberg (pdb entry 3UNX), shown as gray lines.
Figure 9 provides a crystallographic structure of LG12 wild-type protease in which the region 54-59 in the sequence EPNALQ of LG12, shown as black sticks, is compared with the equivalent loop segment in subtilisin from *B. Lentus*, shown as gray lines.
Figure 10 A-10F provides the Clustal W alignment of various LG12-clade mature protease sequences.
Figure 11 provides a phylogenetic tree of LG12-clade proteases.

It is to be appreciated those certain feature of the invention, which are, for clarity, described above and below in the context of separate embodiments, may also be provided in combination in a single embodiment. Conversely, various features of the invention that are, for brevity, described in the context of a single embodiment, may also be provided separately or in any sub-combination.

Unless otherwise indicated, the practice of the present invention involves conventional techniques commonly used in molecular biology, protein engineering, microbiology, and recombinant DNA, which are within the skill of the art. Such techniques are known to those of skill in the art and are described in numerous texts and reference works well known to those of skill in the art. All patents, patent applications, articles and publications mentioned herein, both *supra* and *infra*, are hereby expressly incorporated herein by reference.

Unless defined otherwise herein, all technical and scientific terms used herein have the same meaning as commonly understood by one of ordinary skill in the art to which this invention pertains. Many technical dictionaries are known to those of skill in the art. Although any methods and materials similar or equivalent to those described herein find use in the practice of the present invention, some suitable methods and materials are described herein. Accordingly, the terms defined immediately below are more fully described by reference to the Specification as a whole. Also, as used herein, the singular "a", "an" and "the" includes the plural reference unless the context clearly indicates otherwise. Numeric ranges are inclusive of the numbers defining the range. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context they are used by those of skill in the art.

The practice of the present invention employs, unless otherwise indicated, conventional techniques of protein purification, molecular biology, microbiology, recombinant DNA techniques and protein sequencing, all of which are within the skill of those in the art.

Furthermore, the headings provided herein are not limitations of the various aspects or embodiments of the invention which can be had by reference to the specification as a whole. Accordingly, the terms defined immediately below are more fully defined by reference to the specification as a whole. Nonetheless, in order to facilitate understanding of the invention, a number of terms are defined below.

It is intended that every maximum numerical limitation given throughout this specification include every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The terms "protease" and "proteinase" refer to an enzyme protein that has the ability to break down other proteins. A protease has the ability to conduct "proteolysis," which begins protein catabolism by hydrolysis of peptide bonds that link amino acids together in a peptide or polypeptide chain forming the protein. This activity of a protease as a protein-digesting enzyme is referred to as "proteolytic activity." Many well known procedures exist for measuring proteolytic activity (*See e.g.*, Kalisz, "Microbial Proteinases," In: Fiechter (ed.), Advances in Biochemical Engineering/Biotechnology, (1988)). For example, proteolytic activity may be ascertained by comparative assays which analyze the respective protease's ability to hydrolyze a commercial substrate. Exemplary substrates useful in the analysis of protease or proteolytic activity, include, but are not limited to, di-methyl casein (Sigma C-9801), bovine collagen (Sigma C-9879), bovine elastin (Sigma E-1625), and bovine keratin (ICN Biomedical 902111). Colorimetric assays utilizing these substrates are well known in the art (*See e.g.*, WO 99/34011 and U.S. Pat. No. 6,376,450, both of which are incorporated herein by reference). The pNA assay (*See e.g.*, Del Mar et al., Anal. Biochem. 99:316-320 [1979]) also finds use in determining the active enzyme concentration for fractions collected during gradient elution. This assay measures the rate at which p-nitroaniline is released as the enzyme hydrolyzes a soluble synthetic peptide substrate, such as succinyl-alanine-alanine-proline-phenylalanine-p-nitroanilide (suc-AAPF-pNA), and cleavage occurs between the C-terminal amino acid (phenylalanine) and the p-NA, causing the production of yellow color from the hydrolysis reaction, which is measured at 410 nm on a spectrophotometer and is proportional to the active enzyme concentration. Measurement of the color change allows calculation of the rate of the reaction. In addition, absorbance measurements at 280 nanometers (nm) can be used to determine the total protein concentration. The active enzyme/total protein ratio gives the enzyme purity when a reference standard is used.

The phrase "composition(s) substantially-free of boron" or "detergent(s) substantially-free of boron" refers to composition(s) or detergents), respectively, that contain trace amounts of boron, for example, about 1000 ppm (1mg/kg or litre equals 1 ppm), about 100 ppm, about 50 ppm, about 10 ppm, or about 5 ppm, or about 1 ppm, perhaps from other composition or detergent constituents.

The phrase "variant polypeptide" refers to a polypeptide comprising an amino acid sequence that differs in at least one amino acid residue from the amino acid sequence of a parent or reference polypeptide (including but not limited to wild-type polypeptides).

The phrase "protease variant" refers to a polypeptide that is derived from a reference polypeptide by the substitution, addition, or deletion, of one or more amino acids, typically by recombinant DNA techniques.

The phrase "the genus *Bacillus*" includes all species within the genus "*Bacillus*," as known to those of skill in the art, including but not limited to *B. subtilis, B. licheniformis, B. lentils*, *B. brevis*, *B. stearothermophilus*, *B. alkalophilus*, *B. amyloliquefaciens, B. clausii, B. halodurans, B. pumilus, B. gibsonii, B. pseudofirmus, B. lehensis, B. megaterium, B. coagulans, B. circulans, B. lautus, and B. thuringiensis.* It is recognized that the genus *Bacillus* continues to undergo taxonomical reorganization. Thus, it is intended that the genus include species that have been reclassified, including but not limited to such organisms as *B. stearothermophilus,* which is now named "*Geobacillus stearothermophilus.*" The production of resistant endospores in the presence of oxygen is considered the defining feature of the genus *Bacillus*, although this characteristic also applies to the recently named *Alicyclobacillus*, *Amphibacillus*, *Aneurinibacillus*, *Anoxybacillus*, *Brevibacillus*, *Filobacillus*, *Gracilibacillus*, *Halobacillus*, *Paenibacillus*, *Salibacillus*, *Thermobacillus*, *Ureibacillus*, and *Virgibacillus.*

The terms "polynucleotide" and "nucleic acid," which are used interchangeably herein, refer to a polymer of any length of nucleotide monomers covalently bonded in a chain. DNA (deoxyribonucleic acid), a polynucleotide comprising deoxyribonucleotides, and RNA (ribonucleic acid), a polymer of ribonucleotides, are examples of polynucleotides or nucleic acids having distinct biological function. Polynucleotides or nucleic acids include, but are not limited to, a single-, double- or triple-stranded DNA, genomic DNA, cDNA, RNA, DNA-RNA hybrid, or a polymer comprising purine and pyrimidine bases, or other natural, chemically, biochemically modified, non-natural or derivatized nucleotide bases. The following are non-limiting examples of polynucleotides: genes, gene fragments, chromosomal fragments, expressed sequence tag(s) (EST(s)), exons, introns, messenger RNA (mRNA), transfer RNA (tRNA), ribosomal RNA (rRNA), ribozymes, complementary DNA (cDNA), recombinant polynucleotides, branched polynucleotides, plasmids, vectors, isolated DNA of any sequence, isolated RNA of any sequence, nucleic acid probes, and primers. In some embodiments, polynucleotides comprise modified nucleotides, such as methylated nucleotides and nucleotide analogs, uracyl, other sugars and linking groups such as fluororibose and thioate, and nucleotide branches. In a particular embodiment, a sequence of nucleotides is interrupted by non-nucleotide components.

The term "mutation" refers to changes made in a starting amino acid or nucleic acid sequence. It is intended that the term encompass substitutions, insertions and deletions.

The term "vector" refers to a nucleic acid construct or polynucleotide construct used to introduce or transfer nucleic acid(s) or polynucleotide(s) into a target cell or tissue. A vector is typically used to introduce foreign DNA into another cell or tissue. A vector generally comprises a DNA sequence that is a transgene and a larger polynucleotide sequence that serves as the "backbone" of the vector. The vector typically serves to transfers genetic information, such as the inserted transgene, to a target cell or tissue so as to isolate, multiply, or express the insert in the target cell or tissue. Vectors include plasmids, cloning vectors, bacteriophages, viruses (*e.g*., viral vector), cosmids, expression vectors, shuttle vectors, cassettes, and the like. A vector typically includes an origin of replication, a multicloning site, and a selectable marker. The process of inserting a vector into a target cell is typically referred to as transfection. The transfection of a cell with a viral vector is typically referred to as transduction. The present invention includes, in some embodiments, a vector that comprises a DNA sequence encoding a variant protease (*e.g*., precursor or mature variant protease) that is operably linked to a suitable pro sequence (*e.g*., secretory, signal peptide sequence, etc.) capable of effecting the expression of the DNA sequence in a suitable host.

The phrase "expression cassette," "expression plasmid" or "expression vector" refers to a nucleic acid construct or vector generated recombinantly or synthetically for the expression of a nucleic acid of interest (*e.g*., a foreign nucleic acid or transgene) in a target cell. The nucleic acid of interest typically expresses a protein of interest. An expression vector or expression cassette typically comprises a promoter nucleotide sequence that drives or promotes expression of the foreign nucleic acid. The expression vector or cassette also typically includes any other specified nucleic acid elements that permit transcription of a particular nucleic acid in a target cell. A recombinant expression cassette can be incorporated into a plasmid, chromosome, mitochondrial DNA, plastid DNA, virus, or nucleic acid fragment. Some expression vectors have the ability to incorporate and express heterologous DNA fragments in a host cell. Many prokaryotic and eukaryotic expression vectors are commercially available. Selection of appropriate expression vectors is within the knowledge of those of skill in the art. Selection of appropriate expression vectors for expression of a protein from a nucleic acid sequence incorporated into the expression vector is within the knowledge of those of skill in the art.

A DNA construct is an artificially constructed segment of nucleic acid that may be introduced into a target cell or tissue. A DNA construct typically comprises a DNA insert comprising a nucleotide sequence encoding a protein of interest that has been subcloned into a vector. The vector may contain bacterial resistance genes for growth in bacteria and a promoter for expression of the protein of interest in an organism. The DNA may be generated *in vitro* by PCR or any other suitable technique(s) known to those in the art. In some embodiments, the DNA construct comprises a nucleic acid sequence of interest. In some embodiments, the sequence is operably linked to additional elements such as control elements (*e.g*., promoters, etc.). The DNA construct may further comprise a selectable marker and may further comprise an incoming sequence flanked by homology boxes. The construct may comprise other non-homologous sequences, added to the ends (*e.g*., stuffer sequences or flanks). In some embodiments, the ends of the sequence are closed such that the DNA construct forms a closed circle. The nucleic acid sequence of interest, which is incorporated into the DNA construct, using techniques well known in the art, may be a wild-type, mutant, or modified nucleic acid. In some embodiments, the DNA construct comprises one or more nucleic acid sequences homologous to the host cell chromosome. In other embodiments, the DNA construct comprises one or more non-homologous nucleotide sequences. Once the DNA construct is assembled *in vitro*, it may be used, for example, to: 1) insert heterologous sequences into a desired target sequence of a host cell; and/or 2) mutagenize a region of the host cell chromosome (*i.e*., replace an endogenous sequence with a heterologous sequence); 3) delete target genes; and/or 4) introduce a replicating plasmid into the host. "DNA construct" is used interchangeably herein with "expression cassette."

The term "plasmid" refers to an extrachromosomal DNA molecule which is capable of replicating independently from the chromosomal DNA. A plasmid is double stranded (ds) and may be circular and is typically used as a cloning vector.

As used herein in the context of introducing a nucleic acid sequence into a cell, the term "introduced" refers to any method suitable for transferring the nucleic acid sequence into the cell. Such methods for introduction include but are not limited to protoplast fusion, transfection, transformation, electroporation, conjugation, and transduction (*See e.g.*, Ferrari et al., "Genetics," in Hardwood et al. (eds.), Bacillus, Plenum Publishing Corp., pp. 57-72 [1989]).

The term "trεmsformation T refers to the genetic alteration of a cell which results from the uptake, genomic incorporation, and expression of genetic material (*e.g*., DNA).

As used herein, a nucleic acid is "operably linked" with another nucleic acid sequence when it is placed into a functional relationship with another nucleic acid sequence. For example, a promoter or enhancer is operably linked to a nucleotide coding sequence if the promoter affects the transcription of the coding sequence. A ribosome binding site may be operably linked to a coding sequence if it is positioned so as to facilitate translation of the coding sequence. Typically, "operably linked" DNA sequences are contiguous. However, enhancers do not have to be contiguous. Linking is accomplished by ligation at convenient restriction sites. If such sites do not exist, synthetic oligonucleotide adaptors or linkers may be used in accordance with conventional practice.

The term "gene" refers to a polynucleotide (*e.g*., a DNA segment), that encodes a polypeptide and includes regions preceding and following the coding regions as well as intervening sequences (introns) between individual coding segments (exons).

The term "recombinant" when used with reference to a cell typically indicates that the cell has been modified by the introduction of a heterologous nucleic acid sequence or that the cell is derived from a cell so modified. For example, a recombinant cell may comprise a gene not found in identical form within the native (non-recombinant) form of the cell, or a recombinant cell may comprise a native gene (found in the native form of the cell) but which has been modified and re-introduced into the cell. A recombinant cell may comprise a nucleic acid endogenous to the cell that has been modified without removing the nucleic acid from the cell; such modifications include those obtained by gene replacement, site-specific mutation, and related techniques known to those of ordinary skill in the art. Recombinant DNA technology includes techniques for the production of recombinant DNA *in vitro* and transfer of the recombinant DNA into cells where it may be expressed or propagated, thereby producing a recombinant polypeptide. "Recombination," "recombining," and "recombined" of polynucleotides or nucleic acids refer generally to the assembly or combining of two or more nucleic acid or polynucleotide strands or fragments to generate a new polynucleotide or nucleic acid. The recombinant polynucleotide or nucleic acid is sometimes referred to as a chimera. A nucleic acid or polypeptide is "recombinant" when it is artificial or engineered, or derived from an artificial or engineered protein or nucleic acid.

A nucleic acid or polynucleotide is said to "encode" a polypeptide if, in its native state or when manipulated by methods known to those of skill in the art, it can be transcribed and/or translated to produce a protease variant described herein, or a recombinant polypeptide or a fragment thereof. The anti-sense strand of such a nucleic acid is also said to encode the sequence.

The phrase "host strain" or "host cell" refers to a suitable host for an expression vector comprising a polynucleotide sequence that expresses a protease variant described herein, or recombinant polypeptide or active fragment thereof in the host strain or host cell.

A "protein" or "polypeptide" comprises a polymeric sequence of amino acid residues. The terms "protein" and "polypeptide" are used interchangeably herein. The single and 3-letter code for amino acids as defined in conformity with the IUPAC-IUB Joint Commission on Biochemical Nomenclature (JCBN) is used throughout this disclosure. The single letter X refers to any of the twenty amino acids. It is also understood that a polypeptide may be coded for by more than one nucleotide sequence due to the degeneracy of the genetic code. Mutations are named by the one letter code for the parent amino acid, followed by a three or two digit position number and then the one letter code for the variant amino acid. For example, mutating glycine (G) at position 87 to serine (S) is represented as "G087S" or "G87S". Multiple mutations are indicated by inserting a "-" between the mutations. Mutations at positions 87 and 90 are represented as either "G087S-A090Y" or "G87S-A90Y" or "G87S + A90Y" or "G087S + A090Y".

A "prosequence" or "propeptide sequence" refers to an amino acid sequence between the signal peptide sequence and mature protease sequence that is necessary for proper folding and the secretion of the protease. Cleavage of the prosequence or propeptide sequence results in a mature active protease. The prosequence may be endogenous or the sequence may occur is a highly related protein such as another subtilisin. The prosequence may be a naturally occurring sequence or a variant sequence derived by recombinant technology, such as, for example, is described in U.S. provisional patent application No. 62/181,192, filed June 17, 2015

The phrase "signal sequence" or "signal peptide" refers to a sequence of amino acid residues that may participate in the secretion or direct transport of the mature or precursor form of a protein. The signal sequence is typically located N-terminal to the precursor or mature protein sequence. The signal sequence may be endogenous or exogenous. A signal sequence is normally absent from the mature protein. A signal sequence is typically cleaved from the protein by a signal peptidase after the protein is transported.

The phrase "hybrid signal sequence" refers to signal sequences in which part of sequence is obtained from the expression host fused to the signal sequence of the gene to be expressed. In some embodiments, synthetic sequences are utilized.

The term "mature" form of a protein, polypeptide, or peptide refers to the functional form of the protein, polypeptide, or peptide without the signal peptide sequence and propeptide sequence.

The term "precursor" form of a protein or peptide refers to a mature form of the protein having a prosequence operably linked to the amino or carbonyl terminus of the protein. The precursor may also have a "signal" sequence operably linked to the amino terminus of the prosequence. The precursor may also have additional polynucleotides that are involved in post-translational activity (e.g., polynucleotides cleaved therefrom to leave the mature form of a protein or peptide).

The term "wild-type" in reference to an amino acid sequence or nucleic acid sequence indicates that the amino acid sequence or nucleic acid sequence is native or naturally occurring sequence. As used herein, the term "naturally-occurring" refers to anything (*e.g*., proteins, amino acids, or nucleic acid sequences) that are found in nature (*i.e*., have not been manipulated by means of recombinant methods).

The phrase "non-naturally occurring" refers to anything that is not found in nature (*e.g*., recombinant polynucleotides or polypeptides produced in the laboratory).

As used herein with regard to amino acid residue positions, "corresponding to" or "corresponds to" or "corresponds" refers to an amino acid residue at the enumerated position in a protein or peptide, or an amino acid residue that is analogous, homologous, or equivalent to an enumerated residue in a protein or peptide. As used herein, "corresponding region" generally refers to an analogous position in a related proteins or a reference protein.

The phrases "derived from" and "obtained from" refer to not only a protein produced or producible by a strain of the organism in question, but also a protease encoded by a DNA sequence isolated from such strain and produced in a host organism containing such DNA sequence. Additionally, the term refers to a protein which is encoded by a DNA sequence of synthetic and/or cDNA origin and which has the identifying characteristics of the protease in question. To exemplify, "proteases derived from *Bacillus*" refers to those enzymes having proteolytic activity which are naturally produced by *Bacillus*, as well as to serine proteases like those produced by *Bacillus* sources but which through the use of genetic engineering techniques are produced by a *Bacillus* strain or *non-Bacillus* organisms transformed with a nucleic acid encoding the serine proteases.

The phrase "homologous genes" refers to a pair of genes from different, but usually related species, which correspond to each other and which are identical or very similar to each other. The term encompasses genes that are separated by speciation (i.e., the development of new species) (e.g., orthologous genes), as well as genes that have been separated by genetic duplication (*e.g*., paralogous genes).

The term "homology" refers to sequence similarity or identity, with identity being preferred. Homology may be determined using standard techniques known in the art (*See e.g.*, Smith and Waterman, Adv. Appl. Math. 2:482 [1981]; Needleman and Wunsch, J. Mol. Biol. 48:443 [1970]; Pearson and Lipman, Proc. Natl. Acad. Sci. USA 85:2444 [1988]; software programs such as GAP, BESTFIT, FASTA, and TFASTA in the Wisconsin Genetics Software Package (Genetics Computer Group, Madison, WI); and Devereux et al., Nucl. Acid Res. 12:387-395 [1984]). One example of a useful algorithm is PILEUP. PILEUP creates a multiple sequence alignment from a group of related sequences using progressive, pair-wise alignments. It can also plot a tree showing the clustering relationships used to create the alignment. PILEUP uses a simplification of the progressive alignment method of Feng and Doolittle (*See,* Feng and Doolittle, J. Mol. Evol. 35:351-360 [1987]). The method is similar to that described by Higgins and Sharp (*See,* Higgins and Sharp, CABIOS 5:151-153 [1989]). Useful PILEUP parameters including a default gap weight of 3.00, a default gap length weight of 0.10, and weighted end gaps.

Percent "identical" or "identity" in the context of two or more nucleic acid or polypeptide sequences refers to two or more sequences that are the same or have a specified percentage of nucleic acid residues or amino acid residues, respectively, that are the same, when compared and aligned for maximum similarity, as determined using a sequence comparison algorithm or by visual inspection. "Percent sequence identity" or "% identity" or "% sequence identity or "% amino acid sequence identity" of a subject amino acid sequence to a reference (*i*.*e*., query) amino acid sequence means that the subject amino acid sequence is identical (*i.e.,* on an amino acid-by-amino acid basis) by a specified percentage to the query amino acid sequence over a comparison length when the sequences are optimally aligned. Thus, 80% amino acid sequence identity or 80% identity with respect to two amino acid sequences means that 80% of the amino acid residues in two optimally aligned amino acid sequences are identical.

In some embodiments, the "percent sequence identity" or "% sequence identity" or "% identity" of a subject sequence to a query sequence can be calculated by optimally aligning the two sequences and comparing the two optimally aligned sequences over the comparison length. The number of positions in the optimal alignment at which identical residues occur in both sequences is determined, thereby providing the number of matched positions, and the number of matched positions is then divided by the total number of positions of the comparison length (which, unless otherwise specified, is the length of the query sequence). The resulting number is multiplied by 100 to yield the percent sequence identity of the subject sequence to the query sequence. The percent sequence identity between a reference sequence and a protease variant described herein may be readily determined by one skilled in the art. The percent identity shared by polynucleotide or polypeptide sequences is determined by direct comparison of the sequence information between the molecules by aligning the sequences and determining the identity by methods known in the art.

Sequence identity may be determined using known programs such as BLAST, ALIGN, and CLUSTAL using standard parameters. (See, e.g., Altschul et al. [1990] J. Mol. Biol. 215:403-410; Henikoff et al. [1989] Proc. Natl. Acad. Sci. USA 89:10915; Karin et al. [1993] Proc. Natl. Acad. Sci USA 90:5873; and Higgins et al. [1988] Gene 73:237-244). Software for performing BLAST analyses is publicly available through the National Center for Biotechnology Information. Databases may also be searched using FASTA (Pearson et al. [1988] Proc. Natl. Acad. Sci. USA 85:2444-2448). One indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative substitution. Another useful algorithm for comparison of multiple protein sequences is the MUSCLE program from Geneious software (Biomatters Ltd.) (Robert C. Edgar. MUSCLE: multiple sequence alignment with high accuracy and high throughput Nucl. Acids Res. (2004) 32 (5): 1792-1797).

The phrase "optimal alignment" or "optimally aligned" refers to the alignment of two (or more) sequences giving the highest percent identity score. For example, optimal alignment of two protein sequences can be achieved by manually aligning the sequences such that the maximum number of identical amino acid residues in each sequence are aligned together or by using software programs or procedures described herein or known in the art. Optimal alignment of two nucleic acid sequences can be achieved by manually aligning the sequences so that the maximum number of identical nucleotides in each sequence are aligned together or by using software programs or procedures described herein or known in the art. In some embodiments, two polypeptide sequences are deemed "optimally aligned" when they are aligned using defined parameters, such as a defined amino acid substitution matrix, gap existence penalty (also termed gap open penalty), and gap extension penalty, so as to achieve the highest similarity score possible for that pair of sequences. The BLOSUM62 scoring matrix (*See*, Henikoff and Henikoff, *supra*) is often used as a default scoring substitution matrix in polypeptide sequence alignment algorithms (*e.g*., BLASTP). The gap existence penalty is imposed for the introduction of a single amino acid gap in one of the aligned sequences, and the gap extension penalty is imposed for each residue position in the gap. Exemplary alignment parameters employed are: BLOSUM62 scoring matrix, gap existence penalty=11, and gap extension penalty=1. The alignment score is defined by the amino acid positions of each sequence at which the alignment begins and ends (e.g., the alignment window), and optionally by the insertion of a gap or multiple gaps into one or both sequences, so as to achieve the highest possible similarity score.

A nucleic acid or polynucleotide is "isolated" when it is partially or completely separated from other components, including but not limited to for example, other proteins, nucleic acids, cells, etc. Similarly, a polypeptide, protein or peptide is "isolated" when it is partially or completely separated from other components, including but not limited to for example, other proteins, nucleic acids, cells, etc. On a molar basis, an isolated species is more abundant than are other species in a composition. For example, an isolated species may comprise at least about 50%, about 70%, about 80%, about 85%, about 90%, about 91%, about 92%, about 93%, about 94%, about 95%, about 96%, about 97%, about 98%, about 99%, or about 100% (on a molar basis) of all macromolecular species present. Preferably, the species of interest is purified to essential homogeneity (*i*.*e*., contaminant species cannot be detected in the composition by conventional detection methods). Purity and homogeneity can be determined using a number of techniques well known in the art, such as agarose or polyacrylamide gel electrophoresis of a protein or nucleic acid sample, followed by visualization upon staining. If desired, a high-resolution technique, such as high performance liquid chromatography (HPLC) or a similar means can be utilized for purification of the material.

The term "purified" as applied to nucleic acids or polypeptides generally denotes a nucleic acid or polypeptide that is essentially free from other components as determined by analytical techniques well known in the art (*e.g.,* a purified polypeptide or polynucleotide forms a discrete band in an electrophoretic gel, chromatographic eluate, and/or a media subjected to density gradient centrifugation). For example, a nucleic acid or polypeptide that gives rise to essentially one band in an electrophoretic gel is "purified." A purified nucleic acid or polypeptide is at least about 50% pure, usually at least about 75%, about 80%, about 85%, about 90%, about 91%, about 92%, about 93%, about 94%, about 95%, about 96%, about 97%, about 98%, about 99%, about 99.5%, about 99.6%, about 99.7%, about 99.8% or more pure (*e.g*., percent by weight on a molar basis). In a related sense, the invention provides methods of enriching compositions for one or more molecules of the invention, such as one or more polypeptides or polynucleotides of the invention. A composition is enriched for a molecule when there is a substantial increase in the concentration of the molecule after application of a purification or enrichment technique. A substantially pure polypeptide or polynucleotide of the invention (*e.g*., substantially pure variant protease or polynucleotide encoding a variant protease of the invention, respectively) will typically comprise at least about 55%, about 60%, about 70%, about 80%, about 85%, about 90%, about 91%, about 92%, about 93%, about 94%, about 95%, about 96%, about 97%, about 98, about 99%, about 99.5% or more by weight (on a molar basis) of all macromolecular species in a particular composition.

In a related sense, the invention provides methods of enriching compositions for one or more molecules of the invention, such as one or more polypeptides of the invention (*e.g*., one or more variant proteases of the invention) or one or more nucleic acids of the invention (*e.g*., one or more nucleic acids encoding one or more variant proteases of the invention). A composition is enriched for a molecule when there is a substantial increase in the concentration of the molecule after application of a purification or enrichment technique.

The phrase "improved property" when used in connection with a protease variant described herein refers to a protease variant with improved residual protease activity, improved cleaning performance and/or improved stability optionally with retained cleaning performance relative to the corresponding reference polypeptide.

The phrase "functional assay" refers to an assay that provides an indication of a protein's activity. In some embodiments, the term refers to assay systems in which a protein is analyzed for its ability to function in its usual capacity. For example, in the case of enzymes, a functional assay involves determining the effectiveness of the enzyme in catalyzing a reaction.

The phrase "thermally stable" and "thermostable" and "thermostability" refer to proteases that retain a specified amount of enzymatic activity after exposure to identified temperatures over a given period of time under conditions prevailing during the proteolytic, hydrolyzing, cleaning or other process of the invention, while being exposed to altered temperatures. "Altered temperatures" encompass increased or decreased temperatures. In some embodiments, the proteases retain at least about 50%, about 60%, about 70%, about 75%, about 80%, about 85%, about 90%, about 92%, about 95%, about 96%, about 97%, about 98%, or about 99% proteolytic activity after exposure to altered temperatures over a given time period, for example, at least about 60 minutes, about 120 minutes, about 180 minutes, about 240 minutes, about 300 minutes, etc.

The phrase "improved stability" in the context of an oxidation, chelator, thermal and/or pH stable protease refers to a higher retained proteolytic activity over time as compared to other proteases (*e.g*., LG12 and BPN'-Y217L proteases) and/or wild-type enzymes.

The phrase "diminished stability" in the context of an oxidation, chelator, thermal and/or pH stable protease refers to a lower retained proteolytic activity over time as compared to other proteases (*e.g.*, LG12 proteases) and/or wild-type enzymes.

The phrase "cleaning activity" refers to a cleaning performance achieved by a variant protease or reference protease under conditions prevailing during the proteolytic, hydrolyzing, cleaning, soil/stain removal, or other process of the invention. In some embodiments, cleaning performance of a protease variant described herein or reference protease/polypeptide may be determined by using various assays for cleaning one or more various enzyme sensitive stains on an item or surface (*e.g*., a stain resulting from food, grass, blood, ink, milk, oil, and/or egg protein). Cleaning performance of a protease variant or reference protease/polypeptide can be determined by subjecting a protein stain on the item or surface to standard wash condition(s) and assessing the degree to which the stain is removed by using various chromatographic, spectrophotometric, or other quantitative methodologies. A protein stain includes, for example, egg yolk, blood, milk, ink, grass, pigment, oil, crème brulee, pasta, cheese, and combinations thereof. Exemplary cleaning assays and methods are known in the art and include, but are not limited to those described in WO 99/34011 and U.S. Pat. 6,605,458, both of which are herein incorporated by reference, as well as those cleaning assays and methods included in the Examples provided below.

The phrase "cleaning effective amount" of a variant protease or reference protease refers to the amount of protease that achieves a desired level of enzymatic activity in a specific cleaning composition. Such effective amounts are readily ascertained by one of ordinary skill in the art and are based on many factors, such as the particular protease used, the cleaning application, the specific composition of the cleaning composition, and whether a liquid or dry (*e.g*., granular, tablet, bar) composition is required, etc. The term "soil removal index" (SRI) of a variant protease or reference protease refers to the degree of soil removal achieved by a certain quantity of enzyme or enzymatic activity.

The phrase "cleaning adjunct material" refers to any liquid, solid, or gaseous material included in cleaning composition other than a variant protease of the invention. In some embodiments, the cleaning compositions of the present invention include one of more cleaning adjunct materials. Each cleaning adjunct material is typically selected depending on the particular type and form of cleaning composition (*e.g*., liquid, granule, powder, bar, paste, spray, tablet, gel, foam, or other composition). Preferably, each cleaning adjunct material is compatible with the protease enzyme used in the composition.

The phrase "improved cleaning performance" in the context of cleaning activity refers to an increased or greater cleaning activity by an enzyme on certain enzyme sensitive stains such as, for example, egg, milk, grass, ink, oil, pigment, and/or blood, as determined by usual evaluation after a standard wash cycle and/or multiple wash cycles. The phrase "diminished cleaning performance" in the context of cleaning activity refers to a decreased or lesser cleaning activity by an enzyme on certain enzyme sensitive stains such as, for example, egg, milk, grass, ink, oil pigment and/or blood, as determined by usual evaluation after a standard wash cycle. Cleaning performance can be determined by comparing the protease variants of the present invention with reference proteases in various cleaning assays concerning enzyme sensitive stains such as, for example, egg, grass, blood, ink, oil, pigment and/or milk as determined by usual spectrophotometric or analytical methodologies after standard wash cycle conditions.

The phrase "consumer product" means fabric and home care product.

The phrase "fabric and home care product" or "fabric and household care product" includes products generally intended to be used or consumed in the form in which they are sold and that are for treating fabrics, hard surfaces and any other surfaces, and cleaning systems all for the care and cleaning of inanimate surfaces, as well as fabric conditioner products and other products designed specifically for the care and maintenance of fabrics, and air care products, including: air care including air fresheners and scent delivery systems, car care, pet care, livestock care, personal care, jewelry care, dishwashing, fabric conditioning (including softening and/or freshening), laundry detergency, laundry and rinse additive and/or care, pre-treatment cleaning compositions, hard surface cleaning and/or treatment including floor and toilet bowl cleaners, glass cleaners and/or treatments, tile cleaners and /or treatments, ceramic cleaners and/or treatments, and other cleaning for consumer or institutional use. In some embodiments, the fabric and home care products are suitable for use on wounds and/or skin. "Fabric and home care product" includes consumer and institutional products.

The phrase "institutional cleaning composition" refers to products suitable for use in institutions including but not limited to schools, hospitals, factories, stores, corporations, buildings, restaurants, office complexes and buildings, processing and/or manufacturing plants, veterinary hospitals, factory farms, factory ranches, etc.

The phrase "cleaning and/or treatment composition" is a subset of fabric and home care products that includes, unless otherwise indicated, compositions suitable for cleaning and/or treating items. Such products include, but are not limited to, products for treating fabrics, hard surfaces and any other surfaces in the area of fabric and home care, including: air care including air fresheners and scent delivery systems, car care, dishwashing, fabric conditioning (including softening and/or freshening), laundry detergency, laundry and rinse additive and/or care, hard surface cleaning and/or treatment including floor and toilet bowl cleaners, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use: car or carpet shampoos, bathroom cleaners including toilet bowl cleaners; as well as cleaning auxiliaries such as bleach additives and "stain-stick" or pre-treat types, substrate-laden products such as dryer added sheets.

The phrase "cleaning composition" or "cleaning formulation" of the invention refers to any composition of the invention useful for removing or eliminating a compound (*e.g*., undesired compound) from an object, item or surface to be cleaned, including, but not limited to for example, a fabric, fabric item, dishware item, tableware item, glassware item, contact lens, other solid substrate, hair (shampoo) (including human or animal hair), skin (soap or and cream), teeth (mouthwashes, toothpastes), surface of an item or object (*e.g*., hard surfaces, such as the hard surface of a table, table top, wall, furniture item, floor, ceiling, non-dishware item, non-tableware item, etc.), filters, membranes (*e.g*., filtration membranes, including but not limited to ultrafiltration membranes), etc. The term encompasses any material and/or added compound selected for the particular type of cleaning composition desired and the form of the product (*e.g*., liquid, gel, granule, spray, or other composition), as long as the composition is compatible with the protease and other enzyme(s) used in the composition. The specific selection of cleaning composition materials are readily made by considering the surface, object, item, or fabric to be cleaned, and the desired form of the composition for the cleaning conditions during use.

Cleaning compositions and cleaning formulations include any composition that is suited for cleaning, bleaching, disinfecting, and/or sterilizing any object, item, and/or surface. Such compositions and formulations include, but are not limited to for example, liquid and/or solid compositions, including cleaning or detergent compositions, such as, for example, liquid, tablet, gel, bar, granule, and/or solid laundry cleaning or detergent compositions and fine fabric detergent compositions; hard surface cleaning compositions and formulations, such as, for example, glass, wood, ceramic and metal counter tops and windows; carpet cleaners; oven cleaners; fabric fresheners; fabric softeners; and textile, laundry booster cleaning or detergent compositions, laundry additive cleaning compositions, and laundry pre-spotter cleaning compositions; dishwashing compositions, including hand or manual dishwash compositions (*e.g.*, "hand" or "manual" dishwashing detergents) and automatic dishwashing (ADW) compositions (*e.g*., "automatic dishwashing detergents").

Cleaning composition or cleaning formulations, as used herein, include, unless otherwise indicated, granular or powder-form all-purpose or heavy-duty washing agents, especially cleaning detergents; liquid, granular, gel, solid, tablet, or paste-form all-purpose washing agents, especially the so-called heavy-duty liquid (HDL) detergent or heavy-duty powder detergent (HDD) types; liquid fine-fabric detergents; hand or manual dishwashing agents, including those of the high-foaming type; hand or manual dishwashing, automatic dishwashing, or dishware or tableware washing agents, including the various tablet, powder, solid, granular, liquid, gel, and rinse-aid types for household and institutional use; liquid cleaning and disinfecting agents, including antibacterial hand-wash types, cleaning bars, mouthwashes, denture cleaners, car shampoos, carpet shampoos, bathroom cleaners; hair shampoos and/or hair-rinses for humans and other animals; shower gels and foam baths and metal cleaners; as well as cleaning auxiliaries, such as bleach additives and "stain-stick" or pre-treat types. In some embodiments, granular compositions are in "compact" form; in some embodiments, liquid compositions are in a "concentrated" form.

As used herein, "fabric cleaning compositions" include hand and machine laundry detergent compositions including laundry additive compositions and compositions suitable for use in the soaking and/or pretreatment of stained fabrics (*e.g*., clothes, linens, and other textile materials).

The phrase "non-fabric cleaning compositions" include non-textile (*i.e*., non-fabric) surface cleaning compositions, including, but not limited to for example, hand or manual or automatic dishwashing detergent compositions, oral cleaning compositions, denture cleaning compositions, and personal cleansing compositions.

The phrase "fabric and/or hard surface cleaning and/or treatment composition" is a subset of cleaning and treatment compositions that includes, unless otherwise indicated, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, car or carpet shampoos, bathroom cleaners including toilet bowl cleaners; fabric conditioning products including softening and/or freshening that may be in liquid, solid and/or dryer sheet form; as well as cleaning auxiliaries such as bleach additives and "stain-stick" or pre-treat types, substrate-laden products such as dryer added sheets. All of such products which are applicable may be in standard, concentrated or even highly concentrated form even to the extent that such products may in certain aspect be non-aqueous.

The phrase "detergent composition" or "detergent formulation" is used in reference to a composition intended for use in a wash medium for the cleaning of soiled or dirty objects, including particular fabric and/or non-fabric objects or items. Such compositions of the present invention are not limited to any particular detergent composition or formulation. Indeed, in some embodiments, the detergents of the invention comprise at least one variant protease of the invention and, in addition, one or more surfactants, transferase(s), hydrolytic enzymes, oxido reductases, builders (*e.g*., a builder salt), bleaching agents, bleach activators, bluing agents, fluorescent dyes, caking inhibitors, masking agents, enzyme activators, antioxidants, and/or solubilizers. In some instances, a builder salt is a mixture of a silicate salt and a phosphate salt, preferably with more silicate (*e.g*., sodium metasilicate) than phosphate (*e.g*., sodium tripolyphosphate). Some compositions of the invention, such as, but not limited to, cleaning compositions or detergent compositions, do not contain any phosphate (*e.g*., phosphate salt or phosphate builder).

The term "bleaching" refers to the treatment of a material (*e.g*., fabric, laundry, pulp, etc.) or surface for a sufficient length of time and/or under appropriate pH and/or temperature conditions to effect a brightening (*i*.*e*., whitening) and/or cleaning of the material. Examples of chemicals suitable for bleaching include, but are not limited to, for example, ClO₂, H₂O₂, peracids, NO₂, etc.

The term "disinfecting" refers to the removal of contaminants from the surfaces, as well as the inhibition or killing of microbes on the surfaces of items. It is not intended that the present invention be limited to any particular surface, item, or contaminants) or microbes to be removed.

The term "compact" form of the cleaning compositions herein is best reflected by density and, in terms of composition, by the amount of inorganic filler salt. Inorganic filler salts are conventional ingredients of detergent compositions in powder form. In conventional detergent compositions, the filler salts are present in substantial amounts, typically about 17 to about 35% by weight of the total composition. In contrast, in compact compositions, the filler salt is present in amounts not exceeding about 15% of the total composition. In some embodiments, the filler salt is present in amounts that do not exceed about 10%, or more preferably, about 5%, by weight of the composition. In some embodiments, the inorganic filler salts are selected from the alkali and alkaline-earth-metal salts of sulfates and chlorides. In some embodiments, the filler salt is sodium sulfate.

The position of an amino acid residue in a given amino acid sequence is typically numbered herein using the numbering of the position of the corresponding amino acid residue of the LG12 SprC (described as LG-12 SprC in Example 2, page 86, line 16 to page 87, line 14 of PCT Published Application WO2015/038792, filed September 11, 2014, which is hereby incorporated herein by reference) amino acid sequence shown in SEQ ID NO:10, which is also referred to interchangeably throughout as LG12 wild-type. A given amino acid sequence, such as a variant protease amino acid sequence described herein, can be aligned with the LG12 SprC sequence using an alignment algorithm as described herein, and an amino acid residue in the given amino acid sequence that aligns (preferably optimally aligns) with an amino acid residue in the LG12 SprC sequence can be conveniently numbered by reference to the corresponding amino acid residue in the LG12 SprC sequence.

Variants, compositions and methods disclosed herein relate to a protease variant, or a recombinant polypeptide or an active fragment thereof generated through conventional molecular biology techniques (see, e.g., Sambrook et al, Molecular Cloning: Cold Spring Harbor Laboratory Press). Some embodiments are directed to a protease variant, or a recombinant polypeptide or an active fragment thereof comprising an amino acid sequence comprising two or more substitutions at one or more positions selected from: (i) 1, 3, 7, 9, 19, 24, 27, 30, 38, 40, 43, 45, 48, 52, 53, 57, 72, 76, 78, 79, 86, 87, 88, 89, 90, 91, 93, 97, 99, 100, 101, 103, 104, 106, 107, 111, 114, 115, 116, 117, 118, 120, 122, 123, 124, 128, 129, 130, 131, 133, 134, 136, 139, 140, 141, 143, 144, 145, 147, 148, 149, 150, 153, 156, 158, 159, 160, 166, 181, 182, 185, 188, 194, 209, 212, 213, 215, 216, 217, 222, 224, 228, 234, 236, 238, 240, 242, 245, 246, 248, 251, 252, 256, 265, 267, 269, 270, 271, 272, 273, and 275; (ii) 1, 3, 9, 19, 24, 27, 30, 38, 40, 43, 45, 48, 52, 53, 57, 72, 76, 78, 79, 86, 87, 88, 89, 90, 91, 93, 97, 99, 100, 101, 103, 104, 106, 107, 111, 114, 115, 116, 117, 118, 120, 122, 123, 124, 128, 129, 130, 131, 133, 134, 136, 139, 140, 141, 143, 144, 145, 147, 148, 149, 150, 153, 156, 158, 159, 160, 166, 181, 182, 185, 188, 194, 209, 212, 213, 215, 216, 217, 222, 224, 228, 234, 236, 238, 240, 242, 245, 246, 248, 251, 252, 256, 265, 267, 271, 272, and 275; (iii) 1, 7, 9, 27, 38, 40, 45, 48, 52, 53, 76, 78, 91, 100, 114, 117, 118, 122, 128, 129, 130, 140, 147, 149, 156, 158, 159, 160, 166, 181, 182, 194, 209, 217, 222, 228, 234, 236, 238, 245, 248, 256, 269, 270, 271, 272, 273, and 275; (iv) 1, 24, 30, 38, 40, 48, 52, 53, 76, 78, 79, 86, 87, 88, 89, 91, 93, 97, 100, 103, 104, 106, 111, 114, 115, 116, 117, 118, 120, 122, 124, 128, 129, 130,131,133, 134, 136, 139, 141, 143, 144, 147, 148, 149, 150, 153, 156, 158, 159, 166, 182, 185, 194, 209, 212, 213, 216, 217, 222, 224, 236, 238, 248, 251, 252, 256, 272, and 275; (v) 1, 24, 30, 38, 40, 52, 53, 76, 78, 79, 86, 87, 88, 89, 93, 97, 100, 103, 104, 106, 111, 114, 115, 116, 117, 118, 120, 122, 124, 128, 129, 131, 133, 134, 136, 139, 141, 143, 144, 147, 148, 149, 150, 153, 156, 158, 159, 166, 182, 185, 194, 209, 212, 213, 216, 217, 222, 224, 236, 238, 248, 251, 252, 256, 272, and 275; or (vi) 1, 38, 40, 48, 52, 53, 76, 91, 114, 117, 118, 128, 129, 130, 147, 149, 156, 158, 159, 166, 182, 217, 238, 248, 256, and 272; wherein the amino acid positions of the variant or recombinant polypeptide or active fragment thereof are numbered by correspondence with the amino acid sequence of SEQ ID NO:10. A further embodiment is directed to a protease variant, or a recombinant polypeptide or an active fragment thereof comprising an amino acid sequence comprising two or more substitutions at one or more positions selected from: (i) 1, 3, 9, 19, 24, 27, 30, 38, 40, 43, 45, 48, 52, 53, 57, 72, 76, 78, 79, 86, 87, 88, 89, 90, 91, 93, 97, 99, 100, 101, 103, 104, 106, 107, 111, 114, 115, 116, 117, 118, 120, 122, 123, 124, 128, 129, 130, 131, 133, 134, 136, 139, 140, 141, 143, 144, 145, 147, 148, 149, 150, 153, 156, 158, 159, 160, 166, 181, 182, 185, 188, 194, 209, 212, 213, 215, 216, 217, 222, 224, 228, 234, 236, 238, 240, 242, 245, 246, 248, 251, 252, 256, 265, 267, 271, 272, and 275; (ii) 1, 3, 9, 19, 24,27, 30, 38, 40, 43, 45, 48, 52, 53, 57, 72, 76, 78, 79, 86, 87, 88, 89, 90, 91, 93, 97, 99, 100, 101, 103, 104, 106, 107, 111, 114, 115, 116, 117, 118, 120, 122, 123, 124, 128, 129, 130, 131, 133, 134, 136, 139, 140, 141, 143, 144, 145, 147, 148, 149, 150, 156, 158, 159, 160, 166, 181, 182, 185, 188, 194, 209, 212, 213, 215, 216, 217, 222, 224, 228, 234, 236, 238, 240, 242, 245, 246, 248, 251, 252, 256, 265, 267, 271, 272, and 275; (iii) 9, 38, 40, 45, 48, 52, 53, 76, 78, 100, 114, 117, 118, 128, 129, 130, 140, 147, 149, 156, 158, 159, 160, 166, 181, 182, 194, 209, 217, 234, 236, 238, 245, 248, 256, 272, and 275; (iv) 1, 24, 30, 38, 40, 48, 52, 53, 76, 78, 79, 86, 87, 88, 89, 91, 93, 97, 100, 103, 104, 106, 111, 114, 115, 116, 117, 118, 120, 122, 124, 128, 129, 130, 131, 133, 134, 136, 139, 141,143,144, 147, 148, 149, 150, 156, 158, 159, 166, 182, 185, 194, 209, 212, 213, 216, 217, 222, 224, 236, 238, 248, 251, 252, 256, 272, and 275; (v) 1, 24, 30, 38, 40, 52, 53, 76, 78, 79, 86, 87, 88, 89, 93, 97, 100, 103, 104, 106, 111, 114, 115, 116, 117, 118, 120, 122, 124, 128, 129, 131, 133, 134, 136, 139, 141, 143, 144, 147, 148, 149, 150, 156, 158, 159, 166, 182, 185, 194, 209, 212, 213, 216, 217, 222, 224, 236, 238, 248, 251,252, 256, 272, and 275; or (vi) 38, 40, 48, 52, 53, 76, 114, 117, 118, 128, 129, 130, 147, 149, 156, 158, 159, 166, 182, 217, 238, 248, 256, and 272; wherein the amino acid positions of the variant or recombinant polypeptide or active fragment thereof are numbered by correspondence with the amino acid sequence of SEQ ID NO:10. Another embodiment is directed to a protease variant, or a recombinant polypeptide or an active fragment thereof comprising an amino acid sequence comprising two or more substitutions at one or more position selected from: (i) 52, 53, 76, 86, 97, 103, 104, 106, 111, 114, 115, 116, 118, 120, 122, 124, 128, 129, 130, 131, 134, 136, 139, 141, 143, 144, 147, 148, 150, 156, 158, 159, 166, 217, 248, and 272; (ii) 53, 76, 106, 128, 131, 134, 139, 158, 159, 166, 217, 248, and 272; (iii) 52, 53, 76, 106, 118, 128, 129, 130, 131, 134, 139, 156, 158, 166, 217, 248, and 272; (iv) 52, 53, 76, 128, 118, 156, 158, 166, 217, and 248; (v) 53, 76, 128, 158, 166, and 248; (vi) 52, 53, 76, 118, 128, 156, 158, 166, 217, and 248; or (vii) 52-129, 53-166, 76-166, 166-159, 166-272, 53-76-166, 53-118-166, 53-166-272, 76-118-166, 76-166-248, 76-166-256, and 76-166-272; wherein the amino acid positions of the variant or recombinant polypeptide or active fragment thereof are numbered by correspondence with the amino acid sequence of SEQ ID NO:10.

A still further embodiment is directed to a protease variant, or a recombinant polypeptide or an active fragment thereof comprising an amino acid sequence comprising two or more substitutions selected from: (i) A1X, T3X, G7X, P9X, A19X, S24X, K27X, I30X, N38X, A40X, N43X, K45X, A48X, S52X, G53X, A57X, V72X, N76X, T78X, T79X, Y86X, N87X, A88X, D89X, L90X, Y91X, V93X, S97X, S99X, G100X, S101X, T103X, L104X, G106X, I107X, I111X, S114X, I115X, S116X, N117X, G118X, N120X, I122X, N123X, M124X, G128X, S129X, S130X, G131X, T133X, A134X, Q136X, C139X, N140X, N141X, Y143X, N144X, R145X, I147X, V148X, V149X, I150X, A153X, S156X, S158X, S159X, G160X, G166X, S181X, S182X, T185X, S188X, S194X, T209X, N212X, N213X, A215X, S216X, F217X, M222X, A224X, A228X, I234X, A236X, Y238X, S240X, T242X, Q245X, I246X, E248X, K251X, N252X, N256X, K265X, V267X, N269X, V270X, E271X, S272X, A273X, and Q275X; (ii) A1X, T3X, P9X, A19X, S24X, K27X, I30X, N38X, A40X, N43X, K45X, A48X, S52X, G53X, A57X, V72X, N76X, T78X, T79X, Y86X, N87X, A88X, D89X, L90X, Y91X, V93X, S97X, S99X, G100X, S101X, T103X, L104X, G106X, I107X, I111X, S114X, I115X, S116X, N117X, G118X, N120X, I122X, N123X, M124X, G128X, S129X, S130X, G131X, T133X, A134X, Q136X, C139X, N140X, N141X, Y143X, N144X, R145X, I147X, V148X, V149X, I150X, A153X, S156X, S158X, S159X, G160X, G166X, S181X, S182X, T185X, S188X, S194X, T209X, N212X, N213X, A215X, S216X, F217X, M222X, A224X, A228X, I234X, A236X, Y238X, S240X, T242X, Q245X, I246X, E248X, K251X, N252X, N256X, K265X, V267X, E271X, S272X, and Q275X; (iii) A1X, G7X, P9X, K27X, N38X, A40X, K45X, A48X, S52X, G53X, N76X, T78X, Y91X, G100X, S114X, N117X, G118X, I122X, G128X, S129X, S130X, N140X, I147X, V149X, S156X, S158X, S159X, G160X, G166X, S181X, S182X, S194X, T209X, F217X, M222X, A228X, I234X, A236X, Y238X, Q245X, E248X, N256X, N269X, V270X, E271X, S272X, A273X, and Q275X; (iv) A1X, S24X, I30X, N38X, A40X, A48X, S52X, G53X, N76X, T78X, T79X, Y86X, N87X, A88X, D89X, Y91X, V93X, S97X, G100X, T103X, L104X, G106X, I111X, S114X, I115X, S116X, N117X, G118X, N120X, I122X, M124X, G128X, S129X, S130X, G131X, T133X, A134X, Q136X, C139X, N141X, Y143X, N144X, I147X, V148X, V149X, I150X, A153X, S156X, S158X, S159X, G166X, S182X, T185X, S194X, T209X, N212X, N213X, S216X, F217X, M222X, A224X, A236X, Y238X, E248X, K251X, N252X, N256X, S272X, and Q275X; (v) A1X, S24X, I30X, N38X, A40X, S52X, G53X, N76X, T78X, T79X, Y86X, N87X, A88X, D89X, V93X, S97X, G100X, T103X, L104X, G106X, I111X, S114X, I115X, S116X, N117X, G118X, N120X, I122X, M124X, G128X, S129X, G131X, T133X, A134X, Q136X, C139X, N141X, Y143X, N144X, I147X, V148X, V149X, I150X, A153X, S156X, S158X, S159X, G166X, S182X, T185X, S194X, T209X, N212X, N213X, S216X, F217X, M222X, A224X, A236X, Y238X, E248X, K251X, N252X, N256X, S272X, and Q275X; or (vi) A1X, N38X, A40X, A48X, S52X, G53X, N76X, Y91X, S114X, N117X, G118X, G128X, S129X, S130X, I147X, V149X, S156X, S158X, S159X, G166X, S182X, F217X, Y238X, E248X, N256X, and S272X; wherein X is any amino acid and the amino acid positions of the variant or recombinant polypeptide or active fragment thereof are numbered by correspondence with the amino acid sequence of SEQ ID NO:10. An an even still further embodiment is directed to a protease variant, or a recombinant polypeptide or an active fragment thereof comprising an amino acid sequence comprising two or more substitutions selected from: (i) A1X, T3X, P9X, A19X, S24X, K27X, I30X, N38X, A40X, N43X, K45X, A48X, S52X, G53X, A57X, V72X, N76X, T78X, T79X, Y86X, N87X, A88X, D89X, L90X, Y91X, V93X, S97X, S99X, G100X, S101X, T103X, L104X, G106X, I107X, I111X, S114X, I115X, S116X, N117X, G118X, N120X, I122X, N123X, M124X, G128X, S129X, S130X, G131X, T133X, A134X, Q136X, C139X, N140X, N141X, Y143X, N144X, R145X, I147X, V148X, V149X, I150X, A153X, S156X, S158X, S159X, G160X, G166X, S181X, S182X, T185X, S188X, S194X, T209X, N212X, N213X, A215X, S216X, F217X, M222X, A224X, A228X, I234X, A236X, Y238X, S240X, T242X, Q245X, I246X, E248X, K251X, N252X, N256X, K265X, V267X, E271X, S272X, and Q275X; (ii) A1X, T3X, P9X, A19X, S24X, K27X, I30X, N38X, A40X, N43X, K45X, A48X, S52X, G53X, A57X, V72X, N76X, T78X, T79X, Y86X, N87X, A88X, D89X, L90X, Y91X, V93X, S97X, S99X, G100X, S101X, T103X, L104X, G106X, I107X, I111X, S114X, I115X, S116X, N117X, G118X, N120X, I122X, N123X, M124X, G128X, S129X, S130X, G131X, T133X, A134X, Q136X, C139X, N140X, N141X, Y143X, N144X, R145X, I147X, V148X, V149X, I150X, S156X, S158X, S159X, G160X, G166X, S181X, S182X, T185X, S188X, S194X, T209X, N212X, N213X, A215X, S216X, F217X, M222X, A224X, A228X, I234X, A236X, Y238X, S240X, T242X, Q245X, I246X, E248X, K251X, N252X, N256X, K265X, V267X, E271X, S272X, and Q275X; (iii) P9X, N38X, A40X, K45X, A48X, S52X, G53X, N76X, T78X, G100X, S114X, N117X, G118X, G128X, S129X, S130X, N140X, I147X, V149X, S156X, S158X, S159X, G160X, G166X, S181X, S182X, S194X, T209X, F217X, I234X, A236X, Y238X, Q245X, E248X, N256X, S272X, and Q275X; (iv) A1X, S24X, I30X, N38X, A40X, A48X, S52X, G53X, N76X, T78X, T79X, Y86X, N87X, A88X, D89X, Y91X, V93X, S97X, G100X, T103X, L104X, G106X, I111X, S114X, I115X, S116X, N117X, G118X, N120X, I122X, M124X, G128X, S129X, S130X, G131X, T133X, A134X, Q136X, C139X, N141X, Y143X, N144X, I147X, V148X, V149X, I150X, S156X, S158X, S159X, G166X, S182X, T185X, S194X, T209X, N212X, N213X, S216X, F217X, M222X, A224X, A236X, Y238X, E248X, K251X, N252X, N256X, S272X, and Q275X; (v) A1X, S24X, I30X, N38X, A40X, S52X, G53X, N76X, T78X, T79X, Y86X, N87X, A88X, D89X, V93X, S97X, G100X, T103X, L104X, G106X, I111X, S114X, 1115X, S116X, N117X, G118X, N120X, I122X, M124X, G128X, S129X, G131X, T133X, A134X, Q136X, C139X, N141X, Y143X, N144X, I147X, V148X, V149X, I150X, S156X, S158X, S159X, G166X, S182X, T185X, S194X, T209X, N212X, N213X, S216X, F217X, M222X, A224X, A236X, Y238X, E248X, K251X, N252X, N256X, S272X, and Q275X; or (vi) N38X, A40X, A48X, S52X, G53X, N76X, S114X, N117X, G118X, G128X, S129X, S130X, I147X, V149X, S156X, S158X, S159X, G166X, S182X, F217X, Y238X, E248X, N256X, and S272X; wherein X is any amino acid and the amino acid positions of the variant or recombinant polypeptide or active fragment thereof are numbered by correspondence with the amino acid sequence of SEQ ID NO:10. Yet still another embodiment is directed to a protease variant, or a recombinant polypeptide or an active fragment thereof comprising an amino acid sequence comprising two or more substitutions selected from: (i) S52X, G53X, N76X, Y86X, S97X, T103X, L104X, G106X, I111X, S114X, I115X, S116X, G118X, N120X, I122X, M124X, G128X, S129X, S130X, G131X, A134X, Q136X, C139X, N141X, Y143X, N144X, I147X, V148X, I150X, S156X, S158X, S159X, G166X, F217X, E248X, and S272X; (ii) G53X, N76X, G106X, G128X, G131X, A134X, C139X, S158X, S159X, G166X, F217X, E248X, and S272X; (iii) S52X, G53X, N76X, G106X, G118X, G128X, S129X, S130X, G131X, A134X, C139X, S156X, G166X, F217X, E248X, and S272X; (iv) S52X, G53X, N76X, G118X, G128X, S156X, S158X, G166X, F217X, and E248X; (v) G53X, N76X, G128X, S158X, G166X, and E248X; (vi) S52X, G53X, N76X, G118X, G128X, S156X, S158X, G166X, F217X, and E248X; or (vii) S52X-S129X, G53X-G166X, N76X-G166X, G166X-S159X, G166X-S272X, G53X-N76X-G166X, G53X-G118X-G166X, G53X-G166X-S272X, N76X-G118X-G166X, N76X-G166X-E248X, N76X-G166X-N256X, and N76X-G166X-S272X; wherein X is any amino acid and the amino acid positions of the variant or recombinant polypeptide or active fragment thereof are numbered by correspondence with the amino acid sequence of SEQ ID NO:10.

An even still further embodiment is directed to a protease variant, or a recombinant polypeptide or an active fragment thereof comprising an amino acid sequence comprising two or more substitutions selected from: (i) X1G, X1T, X3E, X3V, X7C, X9I, X9T, X9V, X19R, X24R, X24T, X27S, X27T, X30V, X38A, X38Q, X38T, X40D, X40E, X40L, X40V, X43D, X43H, X43L, X43S, X45Q, X45R, X48E, X48Q, X52A, X52N, X52Q, X52T, X53D, X53E, X53N, X57P, X72A, X76D, X78A, X78D, X78G, X78S, X79I, X86P, X87S, X88V, X89E, X89S, X90P, X91H, X93T, X97G, X99L, X100A, X100K, X101T, X103E, X103S, X104V, X106S, X107T, X107V, X111L, X114A, X114T, X115G, X116E, X116N, X117E, X117S, X118R, X120H, X122A, X123D, X124L, X128A, X128D, X128E, X128H, X128K, X128M, X128N, X128Q, X128S, X128Y, X129D, X129E, X129G, X129H, X129I, X129K, X129L, X129M, X129N, X129P, X129Q, X129R, X129T, X129V, X129Y, X130A, X130E, X130F, X130G, X130H, X130I, X130L, X130M, X130N, X130Q, X130R, X130T, X130V, X130W, X130Y, X131P, X133A, X133D, X133V, X134T, X136E, X139V, X140D, X140Y, X141S, X143T, X144S, X145N, X145W, X147A, X147V, X148L, X149G, X149L, X149N, X149S, X150V, X153S, X156A, X156H, X156N, X158T, X158V, X159D, X159E, X159N, X159Q, X160D, X166A, X166D, X166H, X166N, X166S, X166Y, X181A, X181N, X182E, X185Q, X188Y, X194P, X209F, X209V, X209Y, X212S, X213T, X215D, X215E, X216T, X217L, X217R, X222L, X224T, X228S, X234V, X236N, X236Q, X238F, X240F, X242S, X245A, X245N, X246V, X248K, X248N, X251R, X251S, X252S, X256E, X256K, X256P, X256R, X256Y, X265S, X267N, X269K, X270C, X271F, X271W, X272K, X272N, X272Q, X273V, X275H, and X275R; (ii) X1G, X1T, X3E, X3V, X9I, X9T, X9V, X19R, X24R, X24T, X27S, X27T, X30V, X38A, X38Q, X38T, X40D, X40E, X40L, X40V, X43D, X43H, X43L, X43S, X45Q, X45R, X48E, X48Q, X52A, X52N, X52Q, X52T, X53D, X53E, X53N, X57P, X72A, X76D, X78A, X78D, X78G, X78S, X79I, X86P, X87S, X88V, X89E, X89S, X90P, X91H, X93T, X97G, X99L, X100A, X100K, X101T, X103E, X103S, X104V, X106S, X107T, X107V, X111L, X114A, X114T, X115G, X116E, X116N, X117E, X117S, X118R, X120H, X122A, X123D, X124L, X128A, X128D, X128E, X128H, X128K, X128M, X128N, X128Q, X128S, X128Y, X129D, X129E, X129G, X129H, X129I, X129K, X129L, X129M, X129N, X129P, X129Q, X129R, X129T, X129V, X129Y, X130A, X130E, X130F, X130G, X130H, X130I, X130L, X130M, X130N, X130Q, X130R, X130T, X130V, X130W, X130Y, X131P, X133A, X133D, X133V, X134T, X136E, X139V, X140D, X140Y, X141S, X143T, X144S, X145N, X145W, X147A, X147V, X148L, X149G, X149L, X149N, X149S, X150V, X153S, X156A, X156H, X156N, X158T, X158V, X159D, X159E, X159N, X159Q, X160D, X166A, X166D, X166H, X166N, X166S, X166Y, X181A, X181N, X182E, X185Q, X188Y, X194P, X209F, X209V, X209Y, X212S, X213T, X215D, X215E, X216T, X217L, X217R, X222L, X224T, X228S, X234V, X236N, X236Q, X238F, X240F, X242S, X245A, X245N, X246V, X248K, X248N, X251R, X251S, X252S, X256E, X256K, X256P, X256R, X256Y, X265S, X267N, X271F, X271W, X272K, X272N, X272Q, X275H, and X275R; (iii) X1G, X7C, X9T, X27M, X38Q, X38T, X40D, X45Q, X45R, X48E, X48Q, X52F, X52Q, X52T, X53D, X53E, X53Y, X76D, X78A, X91H, X100A, X114T, X117S, X118R, X122V, X128A, X128D, X128E, X128H, X128K, X128L, X128M, X128N, X128Q, X128R, X128S, X128T, X128V, X128W, X128Y, X129D, X129E, X129F, X129G, X129H, X129I, X129K, X129L, X129M, X129N, X129P, X129Q, X129R, X129T, X129V, X129Y, X130A, X130D, X130E, X130F, X130G, X130H, X130I, X130L, X130M, X130N, X130Q, X130R, X130T, X130V, X130W, X130Y, X140D, X147A, X149G, X156D, X156H, X156N, X156T, X158T, X158V, X159D, X159N, X160D, X166D, X166H, X166Y, X181N, X182E, X194P, X209V, X217R, X222L, X228S, X234V, X236N, X236Q, X238F, X245A, X248D, X248K, X248N, X256E, X256P, X256R, X269K, X270C, X271G, X272K, X272Q, X273V, X275R, and X275Y; (iv) X1G, X1T, X24R, X24T, X30V, X38A, X38Q, X38T, X40D, X40E, X40L, X40V, X48Q, X52A, X52N, X52Q, X52T, X53D, X53E, X53N, X76D, X78A, X78D, X78G, X78S, X79I, X86P, X87S, X88V, X89E, X91H, X93T, X97G, X100A, X100K, X103E, X103S, X104V, X106S, X111L, X114A, X114T, X115G, X116E, X116N, X117E, X117S, X118R, X120H, X122A, X124L, X128A, X128D, X128E, X128H, X128K, X128M, X128N, X128Q, X128S, X128Y, X129D, X129E, X129G, X129H, X129I, X129K, X129L, X129M, X129N, X129P, X129Q, X129R, X129T, X129V, X129Y, X130F, X130H, X130I, X130Y, X131P, X133A, X133D, X133V, X134T, X136E, X139V, X141S, X143T, X144S, X147A, X147V, X148L, X149G, X149L, X149N, X149S, X150V, X153S, X156A, X156H, X156N, X158T, X158V, X159D, X159E, X159N, X159Q, X166A, X166D, X166H, X166N, X166S, X166Y, X182E, X185Q, X194P, X209F, X209V, X209Y, X212S, X213T, X216T, X217L, X217R, X222L, X224T, X236N, X236Q, X238F, X248K, X248N, X251R, X251S, X252S, X256E, X256K, X256P, X256R, X256Y, X272K, X272N, X272Q, X275H, and X275R; (v) X1G, X1T, X24R, X24T, X30V, X38A, X38Q, X38T, X40D, X40E, X40L, X40V, X52A, X52N, X52Q, X52T, X53D, X53E, X53N, X76D, X78A, X78D, X78G, X78S, X79I, X86P, X87S, X88V, X89E, X93T, X97G, X100A, X100K, X103E, X103S, X104V, X106S, X111L, X114A, X114T, X115G, X116E, X116N, X117E, X117S, X118R, X120H, X122A, X124L, X128A, X128D, X128E, X128H, X128K, X128M, X128N, X128Q, X128S, X128Y, X129D, X129E, X129G, X129H, X129I, X129K, X129L, X129M, X129N, X129P, X129Q, X129R, X129T, X129V, X129Y, X131P, X133A, X133D, X133V, X134T, X136E, X139V, X141S, X143T, X144S, X147A, X147V, X148L, X149G, X149L, X149N, X149S, X150V, X153S, X156A, X156H, X156N, X158T, X158V, X159D, X159E, X159N, X159Q, X166A, X166D, X166H, X166N, X166S, X166Y, X182E, X185Q, X194P, X209F, X209V, X209Y, X212S, X213T, X216T, X217L, X217R, X222L, X224T, X236N, X236Q, X238F, X248K, X248N, X251R, X251S, X252S, X256E, X256K, X256P, X256R, X256Y, X272K, X272N, X272Q, X275H, and X275R; or (vi) X1G, X38Q, X40D, X48Q, X52F, X52T, X53E, X53Y, X76D, X91H, X114T, X117S, X118R, X128D, X128M, X128V, X129F, X129M, X129R, X129V, X130F, X130H, X130I, X130Y, X147A, X149G, X156N, X158T, X159N, X166D, X166H, X182E, X217R, X238F, X248N, X256E, X256P, X256R, and X272K; wherein X is any amino acid and the amino acid positions of the variant or recombinant polypeptide or active fragment thereof are numbered by correspondence with the amino acid sequence of SEQ ID NO:10. An even yet still further embodiment is directed to a protease variant, or a recombinant polypeptide or an active fragment thereof comprising an amino acid sequence comprising two or more substitutions selected from: (i) X1G, X1T, X3E, X3V, X9I, X9T, X9V, X19R, X24R, X24T, X27S, X27T, X30V, X38A, X38Q, X38T, X40D, X40E, X40L, X40V, X43D, X43H, X43L, X43S, X45Q, X45R, X48E, X48Q, X52A, X52N, X52Q, X52T, X53D, X53E, X53N, X57P, X72A, X76D, X78A, X78D, X78G, X78S, X79I, X86P, X87S, X88V, X89E, X89S, X90P, X91H, X93T, X97G, X99L, X100A, X100K, X101T, X103E, X103S, X104V, X106S, X107T, X107V, X111L, X114A, X114T, X115G, X116E, X116N, X117E, X117S, X118R, X120H, X122A, X123D, X124L, X128A, X128D, X128E, X128H, X128K, X128M, X128N, X128Q, X128S, X128Y, X129D, X129E, X129G, X129H, X129I, X129K, X129L, X129M, X129N, X129P, X129Q, X129R, X129T, X129V, X129Y, X130A, X130E, X130F, X130G, X130H, X130I, X130L, X130M, X130N, X130Q, X130R, X130T, X130V, X130W, X130Y, X131P, X133A, X133D, X133V, X134T, X136E, X139V, X140D, X140Y, X141S, X143T, X144S, X145N, X145W, X147A, X147V, X148L, X149G, X149L, X149N, X149S, X150V, X153S, X156A, X156H, X156N, X158T, X158V, X159D, X159E, X159N, X159Q, X160D, X166A, X166D, X166H, X166N, X166S, X166Y, X181A, X181N, X182E, X185Q, X188Y, X194P, X209F, X209V, X209Y, X212S, X213T, X215D, X215E, X216T, X217L, X217R, X222L, X224T, X228S, X234V, X236N, X236Q, X238F, X240F, X242S, X245A, X245N, X246V, X248K, X248N, X251R, X251S, X252S, X256E, X256K, X256P, X256R, X256Y, X265S, X267N, X271F, X271W, X272K, X272N, X272Q, and X275R; (ii) X1G, X1T, X3E, X3V, X9I, X9T, X9V, X19R, X24R, X24T, X27S, X27T, X30V, X38A, X38Q, X38T, X40D, X40E, X40L, X40V, X43D, X43H, X43L, X43S, X45Q, X45R, X48E, X48Q, X52A, X52N, X52Q, X52T, X53D, X53E, X53N, X57P, X72A, X76D, X78A, X78D, X78G, X78S, X79I, X86P, X87S, X88V, X89E, X89S, X90P, X91H, X93T, X97G, X99L, X100A, X100K, X101T, X103E, X103S, X104V, X106S, X107T, X107V, X111L, X114A, X114T, X115G, X116E, X116N, X117E, X117S, X118R, X120H, X122A, X123D, X124L, X128A, X128D, X128E, X128H, X128K, X128M, X128N, X128Q, X128S, X128Y, X129D, X129E, X129G, X129H, X129I, X129K, X129L, X129M, X129N, X129P, X129Q, X129R, X129T, X129V, X129Y, X130A, X130E, X130F, X130G, X130H, X130I, X130L, X130M, X130N, X130Q, X130R, X130T, X130V, X130W, X130Y, X131P, X133A, X133D, X133V, X134T, X136E, X139V, X140D, X140Y, X141S, X143T, X144S, X145N, X145W, X147A, X147V, X148L, X149G, X149L, X149N, X149S, X150V, X156A, X156H, X156N, X158T, X158V, X159D, X159E, X159N, X159Q, X160D, X166A, X166D, X166H, X166N, X166S, X166Y, X181A, X181N, X182E, X185Q, X188Y, X194P, X209F, X209V, X209Y, X212S, X213T, X215D, X215E, X216T, X217L, X217R, X222L, X224T, X228S, X234V, X236N, X236Q, X238F, X240F, X242S, X245A, X245N, X246V, X248K, X248N, X251R, X251S, X252S, X256E, X256K, X256P, X256R, X256Y, X265S, X267N, X271F, X271W, X272K, X272N, X272Q, and X275R; (iii) X9T, X38Q, X38T, X40D, X45Q, X45R, X48E, X52T, X53D, X53E, X76D, X78A, X100A, X114T, X117S, X118R, X128A, X128D, X128E, X128H, X128K, X128M, X128N, X128Q, X128S, X128Y, X129D, X129E, X129G, X129H, X129I, X129K, X129L, X129M, X129N, X129Q, X129R, X129T, X129V, X129Y, X130A, X130E, X130F, X130G, X130H, X130I, X130L, X130M, X130N, X130Q, X130R, X130T, X130V, X130W, X130Y, X140D, X147A, X149G, X156N, X158T, X158V, X159D, X159N, X160D, X166D, X166H, X181N, X182E, X194P, X209V, X217R, X234V X236N, X236Q, X245A, X248K, X248N, X256P, X256R, X272K, X272Q, and X275R; (iv) X1G, X1T, X24R, X24T, X30V, X38A, X38Q, X38T, X40D, X40E, X40L, X40V, X48Q, X52A, X52N, X52Q, X52T, X53D, X53E, X53N, X76D, X78A, X78D, X78G, X78S, X79I, X86P, X87S, X88V, X89E, X91H, X93T, X97G, X100A, X100K, X103E, X103S, X104V, X106S, X111L, X114A, X114T, X115G, X116E, X116N, X117E, X117S, X118R, X120H, X122A, X124L, X128A, X128D, X128E, X128H, X128K, X128M, X128N, X128Q, X128S, X128Y, X129D, X129E, X129G, X129H, X1291, X129K, X129L, X129M, X129N, X129P, X129Q, X129R, X129T, X129V, X129Y, X130F, X130H, X130I, X130Y, X131P, X133A, X133D, X133V, X134T, X136E, X139V, X141S, X143T, X144S, X147A, X147V, X148L, X149G, X149L, X149N, X149S, X150V, X156A, X156H, X156N, X158T, X158V, X159D, X159E, X159N, X159Q, X166A, X166D, X166H, X166N, X166S, X166Y, X182E, X185Q, X194P, X209F, X209V, X209Y, X212S, X213T, X216T, X217L, X217R, X222L, X224T, X236N, X236Q, X238F, X248K, X248N, X251R, X251S, X252S, X256E, X256K, X256P, X256R, X256Y, X272K, X272N, X272Q, and X275R; (v) X1G, X1T, X24R, X24T, X30V, X38A, X38Q, X38T, X40D, X40E, X40L, X40V, X52A, X52N, X52Q, X52T, X53D, X53E, X53N, X76D, X78A, X78D, X78G, X78S, X79I, X86P, X87S, X88V, X89E, X93T, X97G, X100A, X100K, X103E, X103S, X104V, X106S, X111L, X114A, X114T, X115G, X116E, X116N, X117E, X117S, X118R, X120H, X122A, X124L, X128A, X128D, X128E, X128H, X128K, X128M, X128N, X128Q, X128S, X128Y, X129D, X129E, X129G, X129H, X129I, X129K, X129L, X129M, X129N, X129P, X129Q, X129R, X129T, X129V, X129Y, X131P, X133A, X133D, X133V, X134T, X136E, X139V, X141S, X143T, X144S, X147A, X147V, X148L, X149G, X149L, X149N, X149S, X150V, X156A, X156H, X156N, X158T, X158V, X159D, X159E, X159N, X159Q, X166A, X166D, X166H, X166N, X166S, X166Y, X182E, X185Q, X194P, X209F, X209V, X209Y, X212S, X213T, X216T, X217L, X217R, X222L, X224T, X236N, X236Q, X238F, X248K, X248N, X251R, X251S, X252S, X256E, X256K, X256P, X256R, X256Y, X272K, X272N, X272Q, and X275R; or (vi) X38Q, X40D, X52T, X53E, X76D, X114T, X117S, X118R, X128D, X128M, X129M, X129R, X129V, X130F, X130H, X130I, X130Y, X147A, X149G, X156N, X158T, X159N, X166D, X166H, X182E, X217R, X248N, X256P, X256R, and X272K; wherein X is any amino acid and the amino acid positions of the variant or recombinant polypeptide or active fragment thereof are numbered by correspondence with the amino acid sequence of SEQ ID NO:10. Another embodiment is directed to a protease variant, or a recombinant polypeptide or an active fragment thereof comprising an amino acid sequence comprising two or more substitutions selected from: (i) X52T, X53D, X76D, X86P, X97G, X103S, X104V, X106S, X111L, X114A, X115G, X116N, X118R, X120H, X122A, X124L, X128D, X128S, X129F, X130Y, X131P, X134T, X136E, X139V, X141S, X143T, X144S, X147V, X148L, X150V, X156N, X158T, X159D, X166D, X217R, X248N, and X272K; (ii) X53D, X76D, X106S, X128S, X131P, X134T, X139V, X158T, X159D, X166D, X217R, X248N, and X272K; (iii) X52T, X53D, X76D, X106S, X118R, X128D, X129F, X130Y, X131P, X134T, X139V, X156N, X166D, X217R, X248N, and X272K; (iv) X52T, X53D, X76D, X118R, X128S, X156N, X158T, X166D, X217R, and X248N; (v) X53D, X76D, X128S, X158T, X166D, and X248N; (vi) X52T, X53D, X76D, X118R, X128S, X156N, X158T, X166D, X217R, and X248N; or (vii) X52T-X129F, X53D-X166D, X76D-X166D, X166H-X159D, X166D-X272K, X53D-X76D-X166D, X53D-X118R-X166D, X53D-X166D-X272K, X76D-X118R-X166D, X76D-X166D-X248K, X76D-X166D-X256R, and X76D-X166D-X272K; wherein X is any amino acid and the amino acid positions of the variant or recombinant polypeptide or active fragment thereof are numbered by correspondence with the amino acid sequence of SEQ ID NO:10.

An even further embodiment is directed to a protease variant, or a recombinant polypeptide or an active fragment thereof comprising an amino acid sequence comprising two or more substitutions selected from: (i) A001G, A001T, T003E, T003V, G007C, P009I, P009T, P009V, A019R, S024R, S024T, K027S, K027T, I030V, N038A, N038Q, N038T, A040D, A040E, A040L, A040V, N043D, N043H, N043L, N043S, K045Q, K045R, A048E, A048Q, S052A, S052N, S052Q, S052T, G053D, G053E, G053N, A057P, V072A, N076D, T078A, T078D, T078G, T078S, T079I, Y086P, N087S, A088V, D089E, D089S, L090P, Y091H, V093T, S097G, S099L, G100A, G100K, S101T, T103E, T103S, L104V, G106S, I107T, I107V, I111L, S114A, S114T, I115G, S116E, S116N, N117E, N117S, G118R, N120H, I122A, N123D, M124L, G128A, G128D, G128E, G128H, G128K, G128M, G128N, G128Q, G128S, G128Y, S129D, S129E, S129G, S129H, S129I, S129K, S129L, S129M, S129N, S129P, S129Q, S129R, S129T, S129V, S129Y, S130A, S130E, S130F, S130G, S130H, S130I, S130L, S130M, S130N, S130Q, S130R, S130T, S130V, S130W, S130Y, G131P, T133A, T133D, T133V, A134T, Q136E, C139V, N140D, N140Y, N141S, Y143T, N144S, R145N, R145W, I147A, I147V, V148L, V149G, V149L, V149N, V149S, I150V, A153S, S156A, S156H, S156N, S158T, S158V, S159D, S159E, S159N, S159Q, G160D, G166A, G166D, G166H, G166N, G166S, G166Y, S181A, S181N, S182E, T185Q, S188Y, S194P, T209F, T209V, T209Y, N212S, N213T, A215D, A215E, S216T, F217L, F217R, M222L, A224T, A228S, I234V, A236N, A236Q, Y238F, S240F, T242S, Q245A, Q245N, I246V, E248K, E248N, K251R, K251S, N252S, N256E, N256K, N256P, N256R, N256Y, K265S, V267N, N269K, V270C, E271F, E271W, S272K, S272N, S272Q, A273V, Q275H, and Q275R; (ii) A001G, A001T, T003E, T003V, P009I, P009T, P009V, A019R, S024R, S024T, K027S, K027T, I030V, N038A, N038Q, N038T, A040D, A040E, A040L, A040V, N043D, N043H, N043L, N043S, K045Q, K045R, A048E, A048Q, S052A, S052N, S052Q, S052T, G053D, G053E, G053N, A057P, V072A, N076D, T078A, T078D, T078G, T078S, T079I, Y086P, N087S, A088V, D089E, D089S, L090P, Y091H, V093T, S097G, S099L, G100A, G100K, S101T, T103E, T103S, L104V, G106S, I107T, I107V, I111L, S114A, S114T, I115G, S116E, S116N, N117E, N117S, G118R, N120H, I122A, N123D, M]24L, G128A, G128D, G128E, G128H, G128K, G128M, G128N, G128Q, G128S, G128Y, S129D, S129E, S129G, S129H, S129I, S129K, S129L, S129M, S129N, S129P, S129Q, S129R, S129T, S129V, S129Y, S130A, S130E, S130F, S130G, S130H, S130I, S130L, S130M, S130N, S130Q, S130R, S130T, S130V, S130W, S130Y, G131P, T133A, T133D, T133V, A134T, Q136E, C139V, N140D, N140Y, N141S, Y143T, N144S, R145N, R145W, I147A, I147V, V148L, V149G, V149L, V149N, V149S, I150V, A153S, S156A, S156H, S156N, S158T, S158V, S159D, S159E, S159N, S159Q, G160D, G166A, G166D, G166H, G166N, G166S, G166Y, S181A, S181N, S182E, T185Q, S188Y, S194P, T209F, T209V, T209Y, N212S, N213T, A215D, A215E, S216T, F217L, F217R, M222L, A224T, A228S, I234V, A236N, A236Q, Y238F, S240F, T242S, Q245A, Q245N, I246V, E248K, E248N, K251R, K251S, N252S, N256E, N256K, N256P, N256R, N256Y, K265S, V267N, E271F, E271W, S272K, S272N, S272Q, Q275H, and Q275R; (iii) A001G, G007C, P009T, K027M, N038Q, N038T, A040D, K045Q, K045R, A048E, A048Q, S052F, S052Q, S052T, G053D, G053E, G053Y, N076D, T078A, Y091H, G100A, S114T, N117S, G118R, I122V, G128A, G128D, G128E, G128H, G128K, G128L, G128M, G128N, G128Q, G128R, G128S, G128T, G128V, G128W, G128Y, S129D, S129E, S129F, S129G, S129H, S129I, S129K, S129L, S129M, S129N, S129P, S129Q, S129R, S129T, S129V, S129Y, S130A, S130D, S130E, S130F, S130G, S130H, S130I, S130L, S130M, S130N, S130Q, S130R, S130T, S130V, S130W, S130Y, N140D, I147A, V149G, S156D, S156H, S156N, S156T, S158T, S158V, S159D, S159N, G160D, G166D, G166H, G166Y, S181N, S182E, S194P, T209V, F217R, M222L, Y238F, Q245A, E248D, E248K, E248N, N256E, N256P, N256R, N269K, V270C, E271G, S272K, S272Q, A273V, Q275R, and Q275Y; (iv) A001G, A001T, S024T, I030V, N038Q, A040D, A048Q, S052A, S052Q, S052T, G053D, G053E, N076D, T078D, T078S, T079I, Y086P, N087S, A088V, D089E, D089S, Y091H, V093T, S097G, G100A, T103E, T103S, L104V, G106S, I111L, S114A, S114T, I115G, S116N, N117S, G118R, N120H, I122A, M124L, G128S, S129P, S130F, S130H, S130I, S130Y, G131P, T133A, T133V, A134T, Q136E, C139V, N141S, Y143T, N144S, I147A, I147V, V148L, V149G, V149L, I150V, A153S, S156H, S156N, S158T, S159D, S159N, G166D, G166H, G166S, S182E, T185Q, S194P, T209Y, N212S, N213T, S216T, F217L, F217R, M222L, A224T, A236N, Y238F, E248N, K251S, N252S, N256P, N256R, N256Y, S272K, S272Q, and Q275H; (v) A001G, A001T, S024T, I030V, N038Q, A040D, S052A, S052Q, S052T, G053D, G053E, N076D, T078D, T078S, T079I, Y086P, N087S, A088V, D089E, D089S, V093T, S097G, G100A, T103E, T103S, L104V, G106S, I111L, S114A, S114T, I115G, S116N, N117S, G118R, N120H, I122A, M124L, G128S, S129P, G131P, T133A, T133V, A134T, Q136E, C139V, N141S, Y143T, N144S, I147A, I147V, V148L, V149G, V149L, I150V, A153S, S156H, S156N, S158T, S159D, S159N, G166D, G166H, G166S, S182E, T185Q, S194P, T209Y, N212S, N213T, S216T, F217L, F217R, M222L, A224T, A236N, Y238F, E248N, K251S, N252S, N256P, N256R, N256Y, S272K, S272Q, and Q275H; or (vi) A001G, N038Q, A040D, A048Q, S052F, S052T, G053E, G053Y, N076D, Y091H, S114T, N117S, G118R, G128D, G128M, G128V, S129F, S129M, S129R, S129V, S130F, S130H, S130I, S130Y, I147A, V149G, S156N, S158T, S159N, G166D, G166H, S182E, F217R, Y238F, E248N, N256E, N256P, N256R, and S272K; wherein the amino acid positions of the variant or recombinant polypeptide or active fragment thereof are numbered by correspondence with the amino acid sequence of SEQ ID NO:10. A still even further embodiment is directed to a protease variant, or a recombinant polypeptide or an active fragment thereof comprising an amino acid sequence comprising two or more substitutions selected from: (i) A001G, A001T, T003E, T003V, P009I, P009T, P009V, A019R, S024R, S024T, K027S, K027T, I030V, N038A, N038Q, N038T, A040D, A040E, A040L, A040V, N043D, N043H, N043L, N043S, K045Q, K045R, A048E, A048Q, S052A, S052N, S052Q, S052T, G053D, G053E, G053N, A057P, V072A, N076D, T078A, T078D, T078G, T078S, T0791, Y086P, N087S, A088V, D089E, D089S, L090P, Y091H, V093T, S097G, S099L, G100A, G100K, S101T, T103E, T103S, L104V, G106S, I107T, 1107V, I111L, S114A, S114T, I115G, S116E, S116N, N117E, N117S, G118R, N120H, I122A, N123D, M124L, G128A, G128D, G128E, G128H, G128K, G128M, G128N, G128Q, G128S, G128Y, S129D, S129E, S129G, S129H, S129I, S129K, S129L, S129M, S129N, S129P, S129Q, S129R, S129T, S129V, S129Y, S130A, S130E, S130F, S130G, S130H, S130I, S130L, S130M, S130N, S130Q, S130R, S130T, S130V, S130W, S130Y, G131P, T133A, T133D, T133V, A134T, Q136E, C139V, N140D, N140Y, N141S, Y143T, N144S, R145N, R145W, I147A, I147V, V148L, V149G, V149L, V149N, V149S, I150V, S156A, S156H, S156N, S158T, S158V, S159D, S159E, S159N, S159Q, G160D, G166A, G166D, G166H, G166N, G166S, G166Y, S181A, S181N, S182E, T185Q, S188Y, S194P, T209F, T209V, T209Y, N212S, N213T, A215D, A215E, S216T, F217L, F217R, M222L, A224T, A228S, I234V, A236N, A236Q, Y238F, S240F, T242S, Q245A, Q245N, I246V, E248K, E248N, K251R, K251S, N252S, N256E, N256K, N256P, N256R, N256Y, K265S, V267N, E271F, E271W, S272K, S272N, S272Q, and Q275R; (ii) A001G, A001T, T003E, T003V, P009I, P009T, P009V, A019R, S024R, S024T, K027S, K027T, I030V, N038A, N038Q, N038T, A040D, A040E, A040L, A040V, N043D, N043H, N043L, N043S, K045Q, K045R, A048E, A048Q, S052A, S052N, S052Q, S052T, G053D, G053E, G053N, A057P, V072A, N076D, T078A, T078D, T078G, T078S, T079I, Y086P, N087S, A088V, D089E, D089S, L090P, Y091H, V093T, S097G, S099L, G100A, G100K, S101T, T103E, T103S, L104V, G106S, I107T, I107V, I111L, S114A, S114T, I115G, S116E, S116N, N117E, N117S, G118R, N120H, I122A, N123D, M124L, G128A, G128D, G128E, G128H, G128K, G128M, G128N, G128Q, G128S, G128Y, S129D, S129E, S129G, S129H, S129I, S129K, S129L, S129M, S129N, S129P, S129Q, S129R, S129T, S129V, S129Y, S130A, S130E, S130F, S130G, S130H, S130I, S130L, S130M, S130N, S130Q, S130R, S130T, S130V, S130W, S130Y, G131P, T133A, T133D, T133V, A134T, Q136E, C139V, N140D, N140Y, N141S, Y143T, N144S, R145N, R145W, I147A, I147V, V148L, V149G, V149L, V149N, V149S, I150V, S156A, S156H, S156N, S158T, S158V, S159D, S159E, S159N, S159Q, G160D, G166A, G166D, G166H, G166N, G166S, G166Y, S181A, S181N, S182E, T185Q, S188Y, S194P, T209F, T209V, T209Y, N212S, N213T, A215D, A215E, S216T, F217L, F217R, M222L, A224T, A228S, I234V, A236N, A236Q, Y238F, S240F, T242S, Q245A, Q245N, I246V, E248K, E248N, K251R, K251S, N252S, N256E, N256K, N256P, N256R, N256Y, K265S, V267N, E271F, E271W, S272K, S272N, S272Q, and Q275R; (iii) P009T, N038Q, A040D, K045Q, K045R, A048E S052T, G053D, G053E, N076D, T078A, G100A, S114T, N117S, G118R, G128A, G128D, G128E, G128H, G128K, G128M, G128N, G128Q, G128S, G128Y, S129D, S129E, S129G, S129H, S129I, S129K, S129L, S129M, S129N, S129Q, S129R, S129T, S129V, S129Y, S130A, S130E, S130F, S130G, S130H, S130I, S130L, S130M, S130N, S130Q, S130R, S130T, S130V, S130W, S130Y, N140D, I147A, V149G, S156N, S158T, S158V, S159D, S159N, G160D, G166D, G166H, S181N, S182E, S194P, T209V, F217R, Q245A, E248K, E248N, N256P, N256R, S272K, S272Q, and Q275R; (iv) A001G, A001T, S024T, I030V, N038Q, A040D, A048Q, S052A, S052Q, S052T, G053D, G053E, N076D, T078D, T078S, T079I, Y086P, N087S, A088V, D089E, D089S, Y091H,V093T, S097G, G100A, T103E, T103S, L104V, G106S, I111L, S114A, S114T, I115G, S116N, N117S, G118R, N120H, I122A, M124L, G128S, S129P, S130F, S130H, S130I, S130Y, G131P, T133A, T133V, A134T, Q136E, C139V, N141S, Y143T, N144S, I147A, I147V, V148L, V149G, V149L, I150V, S156H, S156N, S158T, S159D, S159N, G166D, G166H, G166S, S182E, T185Q, S194P, T209Y, N212S, N213T, S216T, F217L, F217R, M222L, A224T, A236N, Y238F, E248N, K251S, N252S, N256P, N256R, N256Y, S272K, S272Q, and Q275H; (v) A001G, A001T, S024T, I030V, N038Q, A040D, S052A, S052Q, S052T, G053D, G053E, N076D, T078D, T078S, T079I, Y086P, N087S, A088V, D089E, D089S, V093T, S097G, G100A, T103E, T103S, L104V, G106S, I111L, S114A, S114T, I115G, S116N, N117S, G118R, N120H, I122A, M124L, G128S, S129P, G131P, T133A, T133V, A134T, Q136E, C139V, N141S, Y143T, N144S, I147A, I147V, V148L, V149G, V149L, I150V, S156H, S156N, S158T, S159D, S159N, G166D, G166H, G166S, S182E, T185Q, S194P, T209Y, N212S, N213T, S216T, F217L, F217R, M222L, A224T, A236N, Y238F, E248N, K251S, N252S, N256P, N256R, N256Y, S272K, S272Q, and Q275H; or (vi) N038Q, A040D, S052T, G053E, N076D, S114T, N117S, G118R, G128D, G128M, S129M, S129R, S129V, S130F, S130H, S130I, S130Y, I147A, V149G, S156N, S158T, S159N, G166D, G166H, S182E, F217R, E248N, N256P, N256R, and S272K; wherein the amino acid positions of the variant or recombinant polypeptide or active fragment thereof are numbered by correspondence with the amino acid sequence of SEQ ID NO:10. A still further embodiment is directed to a protease variant, or a recombinant polypeptide or an active fragment thereof comprising an amino acid sequence comprising two or more substitutions selected from: (i) S52T, G53D, N76D, Y086P, S097G, T103S, L104V, G106S, I111L, S114A, I115G, S116N, G118R, N120H, I122A, M124L, G128D, G128S, S129F, S129P, S130Y, G131P, A134T, Q136E, C139V, N141S, Y143T, N144S, I147V, V148L, I150V, S156N, S158T, S159D, G166D, F217R, E248N, and S272K; (ii) G53D, N76D, G106S, G128S, G131P, A134T, C139V,S158T, S159D, G166D, F217R, E248N, and S272K; (iii) S52T, G53D, N76D, G106S, G118R, G128D, S129F, S130Y, G131P, A134T, C139V, S156N, G166D, F217R, E248N, and S272K; (iv) S52T, G53D, N76D, G118R, G128S, S156N, S158T, G166D, F217R, and E248N; (v) G53D, N76D, G128S, S158T, G166D, and E248N; (vi) S52T, G53D, N76D, G118R, S156N, S158T, G166D, F217R, and E248N; or (vii) S52T-S129F, G53D-G166D, N76D-G166D, G166H-S159D, G166D-S272K, G53D-N76D-G166D, G53D-G118R-G166D, G53D-G166D-S272K, N76D-G118R-G166D, N76D-G166D-E248K, N76D-G166D-N256R, and N76D-G166D-S272K; wherein the amino acid positions of the variant or recombinant polypeptide or active fragment thereof are numbered by correspondence with the amino acid sequence of SEQ ID NO:10.

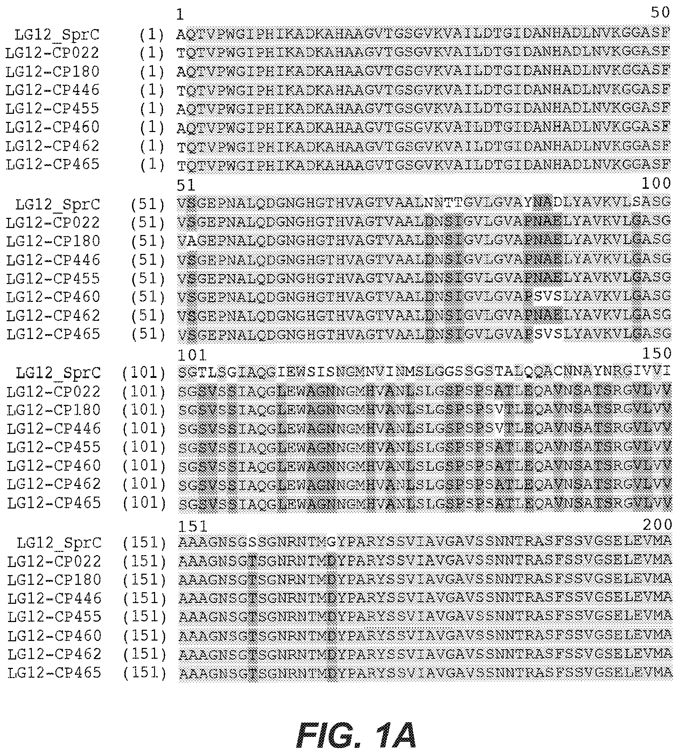

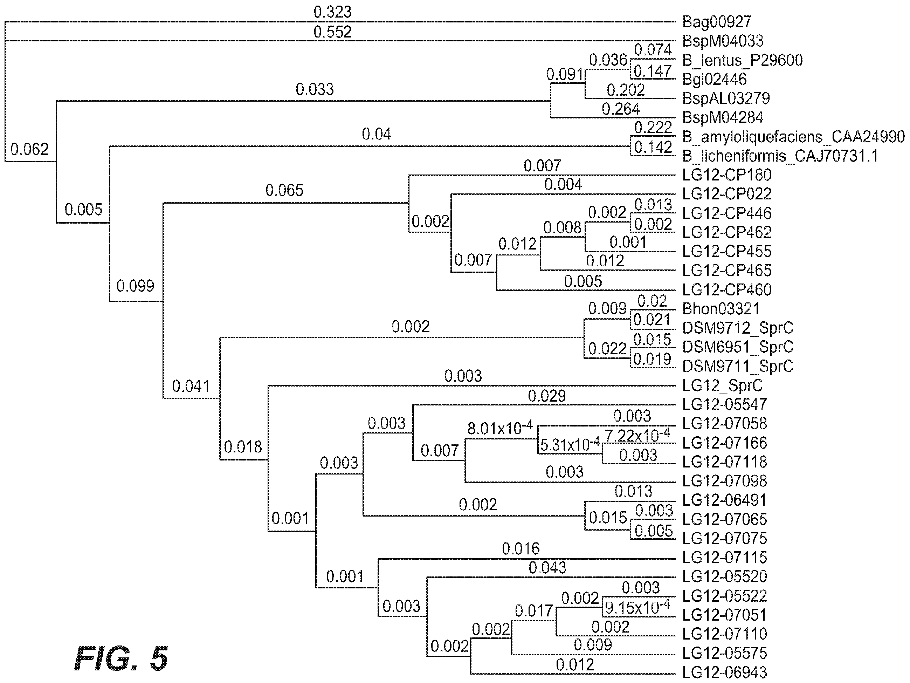

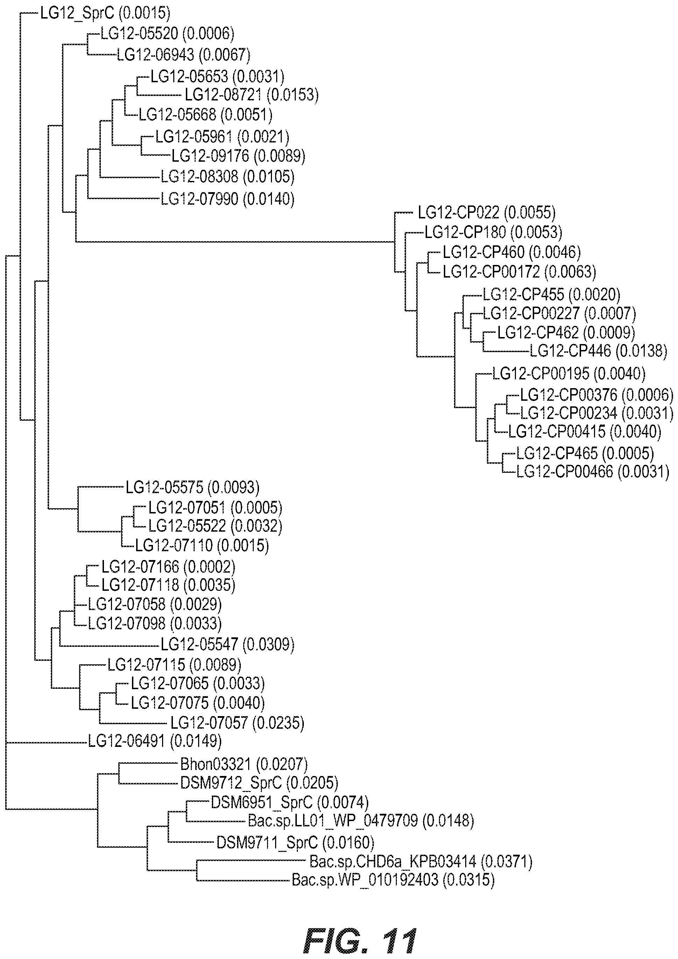

As used herein, a LG12-clade protease enzyme includes an enzyme, polypeptide, or protein, or an active fragment thereof, exhibiting a proteolytic activity. This includes members of the LG12-clade, as described in the Examples, alignments shown in Figures 1A-1B, 4A-4F, and Figure 10A-10F and/or the phylogenetic tree of Figures 5 and/or 11. Members of the LG12-clade were identified by selecting subtilisin molecules with close homology to LG12 SprC, generating a structure-based alignment of these sequences to other subtilisins, and identifying regions of unique sequences conserved for all the LG12-clade enzymes. One embodiment is directed to an LG12-Clade of subtilisins comprising one or more protease variants described herein, or recombinant polypeptide or an active fragment thereof. In some embodiments, the LG12 enzyme clade comprises subtilisin proteases where the parent, wild-type and/or protease variant sequence contains one or more of the following amino acid motifs: a catalytic triad comprising a Ser221, His64, and Asp32 motif and an EPNALQDGNGHGTH (SEQ ID NO:44) motif. In a further embodiment, the protease variant, or recombinant polypeptide or active fragment thereof comprises an EPNALQDGNGHGTH (SEQ ID NO:44) motif. In a still further embodiment, the protease variant, or recombinant polypeptide or active fragment thereof comprises an EPNALQDGNGHGTH (SEQ ID NO:44) motif, with the proviso that the variant or recombinant polypeptide or active fragment thereof does not comprise WP_047970940, WP_010192403, KPB_03414, BAD11988, BAD21128, Bhon03321, DSM9712_SprC, DSM6951_SprC, or DSM9711_SprC. In another embodiment, the motif extends from residue E54 to H67, wherein the amino acid positions of the motif are numbered by correspondence with the amino acid sequence of LG12 wild-type set forth in SEQ ID NO:10. Any LG12-clade protease enzyme sequence can be used as a parent sequence for generation of variant subtilisin enzymes of the present invention. In one embodiment, the protease variant, or recombinant polypeptide or active fragment thereof is derived from a reference polypeptide selected from SEQ ID NOs:10, 18, 19, 45, 46, 57, 58, and 66. In another embodiment, the protease variant, or recombinant polypeptide or active fragment thereof is derived from a reference polypeptide selected from SEQ ID NO:10. In another embodiment, the protease variant, or recombinant polypeptide or active fragment thereof is derived from a reference polypeptide selected from SEQ ID NOs:10 and said protease variant, or recombinant polypeptide or active fragment thereof has one or more substitutions in one or more molecular segments selected from Segment 1 comprising positions 76-79 and 86, Segment 2 comprising positions 97-148, Segment 3 comprising positions 150-170, and Segment 4 comprising positions 207-220. Members of the LG12-Clade were confirmed to cluster to the same branching points in a phylogentic tree created using the Neighbor Joining method (Saitou, N. and Nei, M. (1987) Mol. Biol. Evol. 4:406-425).

In some embodiments, the sequence identity for the LG12-clade includes enzymes that are at least 60%, 65%, 70%, 75%, 80%, 85%, 90%, 91%, 92%, 93%, 94%, 95%, 96%, 97%, 98%, 99% or 100% identical to the LG12 SprC sequence (SEQ ID NO:10). In other embodiments, the protease variant, or recombinant polypeptide or active fragment thereof comprises an amino acid sequence having at least 75, 80, 85, 90, 95, 96, 97, 98, or 99% amino acid sequence identity to SEQ ID NO:10. In yet other embodiments, the protease variant, or recombinant polypeptide or active fragment thereof comprises an amino acid sequence having at least 75% amino acid sequence identity to SEQ ID NO:10, with the proviso that the variant or recombinant polypeptide or active fragment thereof does not comprise WP_047970940, WP_010192403, KPB_03414, BAD11988, BAD21128, Bhon03321, DSM9712_SprC, DSM6951_SprC, or DSM9711_SprC. In a still further embodiment, the protease variant, or recombinant polypeptide or active fragment thereof comprises an amino acid sequence having at least 80% amino acid sequence identity to SEQ ID NO:10, with the proviso that the variant or recombinant polypeptide or active fragment thereof does not comprise WP_047970940, WP_010192403, KPB_03414, Bhon03321, DSM9712_SprC, DSM6951_SprC, or DSM9711_SprC. In an even still further embodiment, the protease variant, or recombinant polypeptide or active fragment thereof comprises an amino acid sequence having at least 95% amino acid sequence identity to SEQ ID NO:10, with the proviso that the variant or recombinant polypeptide or active fragment thereof does not comprise Bhon03321, DSM9712_SprC, or DSM6951_SprC.

The serine protease polypeptides of the present disclosure include isolated, recombinant, substantially pure, or non-naturally occurring polypeptides. In some embodiments, the polypeptides are useful in cleaning applications and can be incorporated into cleaning compositions that are useful in methods of cleaning an item or a surface in need thereof.

In some embodiments, the polypeptide has a specified degree of amino acid sequence homology to an LG12-clade protease variant disclosed herein. In some embodiments, an LG12-clade protease variant has 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity to an amino acid sequence selected from SEQ ID NO:1, 3, 4, 5, 6, 7, 8, 29, 30, 31, 32, 33, 34, 35, 36, 37, 38, 39, 40, 41, 42, and 43. In further embodiments, an LG12-clade protease variant has 70%, 75%, 80%, 85%, 90%, 95%, 96%, 97%, 98%, 99% or 100% sequence identity to an amino acid sequence selected from SEQ ID NO:1, 3, 4, 5, 6, 7, 8, 29, 30, 31, 32, 33, 34, 35, 36, 37, 38, 39, 40, 41, 42, and 43. In another embodiment, an isolated, recombinant, substantially pure, or non-naturally occurring LG12-clade protease variant has protease activity and comprises a polypeptide sequence having at least about 60%, at least about 65%, at least about 70%, at least about 75%, at least about 76%, at least about 77%, at least about 78%, at least about 79%, at least about 80%, at least about 81%, at least about 82%, at least about 83%, at least about 84%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, at least about 99.5%, or 100% sequence identity to an amino acid sequence selected from SEQ ID NO:1, 3, 4, 5, 6, 7, 8, 29, 30, 31, 32, 33, 34, 35, 36, 37, 38, 39, 40, 41, 42, and 43. In a still further embodiment, an LG12-clade protease variant is selected from SEQ ID NO:1, 3, 4, 5, 6, 7, 8, 29, 30, 31, 32, 33, 34, 35, 36, 37, 38, 39, 40, 41, 42, and 43.

Homology can be determined by amino acid sequence alignment, e.g., using a program such as BLAST, ALIGN, or CLUSTAL, as described herein. In some embodiments, the polypeptide is an isolated, recombinant, substantially pure, or non-naturally occurring enzyme having protease activity, such as subtilisin activity, or casein hydrolysis activity (for example, dimethylcasein hydrolysis activity).

Also provided is an LG12-clade protease variant having protease activity, such as alkaline protease activity, said variant comprising an amino acid sequence which differs from the amino acid sequence of SEQ ID NOs: 1, 3, 4, 5, 6, 7, 8, 29, 30, 31, 32, 33, 34, 35, 36, 37, 38, 39, 40, 41, 42, or 43 by no more than 50, no more than 45, no more than 40, no more than 35, no more than 30, no more than 25, no more than 20, no more than 19, no more than 18, no more than 17, no more than 16, no more than 15, no more than 14, no more than 13, no more than 12, no more than 11, no more than 10, no more than 9, no more than 6, no more than 5, no more than 4, no more than 3, no more than 2 amino acid residues, or no more than 1 amino acid residue(s), as determined by alignment of the variant protease amino acid sequence with the LG12-clade wild-type amino acid sequence.

In another embodiment, a protease variant described herein, or recombinant polypeptide or active fragment thereof has one or more improved property selected from improved cleaning performance, improved stability, and improved residual protease activity when compared to SEQ ID NO:10 and/or 48. In another embodiment, the improved property is (i) improved residual protease activity and said protease variant, or recombinant polypeptide or active fragment thereof has 15-100%, 20-100%, 25-100%, 30-100%, 35-100%, or 40-100% residual protease activity when measured in accordance with the Stability Assay of Example 1; (ii) improved cleaning performance and said protease variant, or recombinant polypeptide or active fragment thereof has a BMI cleaning PI of ≥0.7 in comparison to SEQ ID NO:48 when measured in accordance with the Cleaning Performance in Detergent assay of Example 1; (iii) improved stability and said protease variant, or recombinant polypeptide or active fragment thereof has a stability PI ≥1.01 or 1.1 or 1.2 in comparison to SEQ ID NO:10 when measured in accordance with the Stability Assay of Example 1; and/or (iv) improved cleaning performance and said protease variant, or recombinant polypeptide or active fragment thereof has a cleaning PI ≥1.01 or 1.1 or 1.2 in comparison to SEQ ID NO:10 when measured in accordance with the Cleaning Performance in Detergent Assay of Example 1.

In some embodiments, one or more LG12-clade protease variants has at least one beneficial mutation compared to the parent and/or BPN'-Y217L, wherein the benefit can be any one or more of the following: % residual protease activity in 10% HDL non-boron detergent (e.g., Persil non-bio HDL and Test HDL) that is greater than BPN'-Y217L; % residual protease activity in 10% HDL non-boron detergent (e.g., Persil non-bio HDL and Test HDL) that is 5-10 fold greater than BPN'-Y217L; laundry stability PI>1.01 over LG12 wild-type protease in 10% OMO HDL pH 8.2 at 51°C and/or LAS/EDTA at 53.5°C; laundry stability PI>0.75 over LG12 wild-type in 10% Kirkland HDL pH 8.2 at 56°C; HDD cleaning PI>1.01 over LG12 wild-type protease in Kirkland Ultra HDD pH 10.6, OMO Color HDD pH 10.6, and/or Surf Excel HDD pH 10.6; or HDL cleaning PI>1.01 over LG12 wild-type in OMO HDL pH 8.2 and/or Kirkland ultraclean pH 8.2. In still other embodiments, one or more LG12-clade protease variants has at least one beneficial mutation compared to the parent and/or BPN'-Y217L, wherein the benefit can be any one or more of the following: laundry stability PI and laundry cleaning PI >1.01 over LG12 wild-type protease in all Example 1 stability and cleaning performance assays; ADW cleaning PI>1.01 over LG12 wild-type protease in GSM-B detergent pH10.5; ADW stability PI >1.01 in buffer and ADW cleaning PI>1.01 over LG12 wild-type protease in GSM-B detergent pH 10.5

In yet a further embodiment, the protease variant described herein, or recombinant polypeptide or active fragment thereof has protease activity in the presence of a surfactant, cleaning activity in a detergent composition, or a combination thereof. In still yet a further embodiment, the protease variant described herein, or recombinant polypeptide or active fragment thereof has protease activity in the presence of a surfactant, cleaning activity in a detergent composition, or a combination thereof, wherein the detergent composition is an automatic dish washing detergent or a laundry detergent. In an even still yet a further embodiment, the protease variant described herein, or recombinant polypeptide or active fragment thereof has protease activity in the presence of a surfactant, cleaning activity in a detergent composition, or a combination thereof, wherein the cleaning activity comprises hydrolysis of a protein stain. In another embodiment the protein stains is selected from egg yolk, blood, milk, ink, oil, and pigment.

In yet still an even further embodiment, the composition described herein contains phosphate, is phosphate-free, contains borate, is boron-free, or combinations thereof. In other embodiments, the composition is a boron-free composition. In some embodiments, a boron-free composition is a composition to which a borate stabilizer has not been added. In another embodiment, a boron-free composition is a composition that contains less than 5.5% boron. In a still further embodiment, a boron-free composition is a composition that contains less than 4.5% boron. In yet still another embodiment, a boron-free composition is a composition that contains less than 3.5% boron. In yet still further embodiments, a boron-free composition is a composition that contains less than 2.5% boron. In even further embodiments, a boron-free composition is a composition that contains less than 1.5% boron. In another embodiment, a boron-free composition is a composition that contains less than 1.0% boron. In still further embodiments, a boron-free composition is a composition that contains less than 0.5% boron. In other embodiments, the composition is a cleaning composition. In yet further embodiments, the cleaning composition is a detergent composition. In still further embodiments, a boron-free composition is a composition substantially-free of boron.

In another embodiment, an LG12-clade enzyme variant or an active fragment thereof contains at least two, three, four, five, six, seven, eight, nine or ten or more amino acid modifications to a parent LG12-clade enzyme, wherein a first amino acid modification is at a position of the enzyme variant selected from 52, 53, 76, 78, 79, 86, 97, 103, 104, 106, 111, 114, 115, 116, 118, 120, 122, 124, 128, 129, 130, 131, 134, 136, 139, 141, 143, 144, 147, 148, 149, 150, 156, 158, 159, 166, 217, 248, 256, and 272, wherein the amino acid positions of the variant are numbered by correspondence with the amino acid sequence of LG12 wild-type set forth in SEQ ID NO:10.

In another embodiment, a LG12-clade enzyme variant or an active fragment thereof contains at least two, three, four, five, six, seven, eight, nine or ten or more amino acid modifications to a parent LG12-clade enzyme, wherein a first amino acid modification is at a position of the enzyme variant selected from 76, 78, 79, 86, 97, 103, 104, 106, 111, 114, 115, 116, 120, 122, 124, 128, 129, 131, 134, 136, 139, 141, 143, 144, 147, 148, 150, 158, 166, 217 and 248, wherein the amino acid positions of the variant are numbered by correspondence with the amino acid sequence of LG12 wild-type set forth in SEQ ID NO:10.

In another embodiment, a LG12-clade enzyme variant or an active fragment thereof contains at least two, three, four, five, six, seven, eight, nine or ten or more amino acid modifications to a parent LG12-clade enzyme, wherein a first amino acid modification is at a position of the enzyme variant selected from 52, 53, 118, 129, 130, 149, 156, 159, 166, 248, 256, and 272, wherein the amino acid positions of the variant are numbered by correspondence with the amino acid sequence of LG12 wild-type set forth in SEQ ID NO:10.

In another embodiment, a LG12-clade enzyme variant or an active fragment thereof contains amino acid modifications to a parent LG12-clade enzyme, wherein said enzyme variant comprises amino acid modifications at positions 76 and 217 in combination with at least one amino acid modification selected from 52, 53, 118, 129, 130, 149, 156, 159, 166, 248, 256, and 272, wherein the amino acid positions of the variant are numbered by correspondence with the amino acid sequence of LG12 wild-type set forth in SEQ ID NO:10.

In another embodiment, a LG12-clade enzyme variant or an active fragment thereof contains amino acid modifications to a parent LG12-clade enzyme, wherein said enzyme variant comprises amino acid modifications at positions 76 and 166 in combination with at least one amino acid modification selected from 52, 53, 118, 129, 130, 149, 156, 159, 217, 248, 256, and 272, wherein the amino acid positions of the variant are numbered by correspondence with the amino acid sequence of LG12 wild-type set forth in SEQ ID NO:10.

In another embodiment, a LG12-clade enzyme variant or an active fragment thereof contains at least two, three, four, five, six, seven, eight, nine or ten or more amino acid modifications to a parent LG12-clade enzyme, wherein a first amino acid modification is at a position of the enzyme variant selected from N76D, T78S, T79I, Y86P, S97G, T103S, L104V, G106S, I111L, S114A, I115G, S116N, N120H, I122A, M124L, G128S, G129P, G131P, A134T, Q136E, C139V, N141S, Y143T, N144S, I147V, V148L, I150V, S158T, G166D, F217L and E248N, wherein the amino acid positions of the variant are numbered by correspondence with the amino acid sequence of LG12 wild-type set forth in SEQ ID NO:10.

An even still further embodiment is directed to a protease variant, or a recombinant polypeptide or an active fragment thereof comprising an amino acid sequence comprising one or more deletions at one or more positions selected from 53, 55, 56, 57, and 58 or G053, P055, N056, A057 and L058; wherein the amino acid positions of the variant, or recombinant polypeptide or active fragment thereof are numbered by correspondence with the amino acid sequence of SEQ ID NO:10.

In another embodiment, a LG12-clade enzyme variant or an active fragment thereof contains at least one, two, or three or more amino acid modifications to a parent LG12-clade enzyme, wherein a first amino acid modification is a deletion at a position of the enzyme variant selected from 53, 54, 55, 56, 57, and 58, wherein the amino acid positions of the variant are numbered by correspondence with the amino acid sequence of LG12 wild-type set forth in SEQ ID NO:10.

In another embodiment, a LG12-clade enzyme variant or an active fragment thereof contains at least two, three or more amino acid modifications to a parent LG12-clade enzyme, wherein an amino acid modification is a deletion of two or more positions of the enzyme variant selected from 53, 54, 55, 56, 57, and 58, wherein the amino acid positions of the variant are numbered by correspondence with the amino acid sequence of LG12 wild-type set forth in SEQ ID NO:10.

In another embodiment, a LG12-clade enzyme variant or an active fragment thereof contains at least three or more amino acid modifications to a parent LG12-clade enzyme, wherein an amino acid modification is a deletion of three or more positions of the enzyme variant selected from 53, 54, 55, 56, 57, and 58, wherein the amino acid positions of the variant are numbered by correspondence with the amino acid sequence of LG12 wild-type set forth in SEQ ID NO:10.

In a still further embodiment, the protease variant, or recombinant polypeptide or active fragment thereof comprises an amino acid sequence comprising two or more substitutions selected from S052T-S129F; G053D-G166D; N076D-G166D; G166H-S159D; G166D-S272K; G053D-N076D-G166D; G053D-G118R-G166D; G053D-G166D-S272K; N076D-G118R-G166D; N076D-G166D-E248K; N076D-G166D-N256R; N076D-G166D-S272K;[ S024T-G053E-G166D-M222L-N256P; V149L-S156H-T185Q-S272Q-S159D; G053D-N076D-G118R-G166D-N256R; S052T-V149G-S156N-G166H-F217R; S052T-V149G-S156N-G166D-F217R; S052T-V149G-S156N-S159D-G166D-F217R; S052T-V149G-S156N-G166D-S182E-F217R; A040D-S114T-1147A-S159N-G166D-S182E; N038Q-G053E-G100A-N117S-I147A-S158T-G166D-S272K; N038Q-G053E-N117S-1147A-S158T-G166D-N256P-S272K; A001G-S052Q-V149G-S156N-S159N-S182E-Y238F-S272Q; A001G-S052Q-S114T-S156N-S159N-S182E-Y238F-S272Q; N038Q-G053E-G100A-N117S-I147A-S158T-G166D-N256P-S272K; A001G-I030V-T078D-V093T-T103E-S156N-S159D-G166S-S194P-F217R-A236N; A001T-N076D-T078S-T079I-Y086P-D089E-S097G-T103S-L104V-G106S-I111L-S114A-I115G-S116N-N120H-1122A-M124L-G128S-S129P-G131P-T133A-A134T-Q136E-C139V-N141S-Y143T-N144S-I147V-V148L-I150V-S158T-G166D-E248N; S052A-N076D-T078S-T079I-Y086P-D089E-S097G-T103S-L104V-G106S-I111L-S114A-I115G-S116N-N120H-I122A-M124L-G128S-S129P-G131P-T133V-A134T-Q136E-C139V-N141S-Y143T-N144S-I147V-V148L-I150V-S158T-G166D-F217L-E248N; A001T-N076D-T078S-T079I-Y086P-D089E-S097G-T103S-L104V-G106S-I111L-S114A-I115G-S116N-N120H-I122A-M124L-G128S-S129P-G131P-T133V-A134T-Q136E-C139V-N141S-Y143T-N144S-I147V-V148L-1150V-S158T-G166D-T209Y-N212S-N213T-S216T-F217L-A224T-E248N-K251S-N252S-N256Y; N076D-T078S-T079I-Y086P-D089E-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N; N076D-T078S-T079I-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-F217L-E248N; A001T-N076D-T078S-T079I-Y086P-D089E-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N; A001T-N076D-T078S-T079I-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-E248N;S052A-N076D-T078S-T079I-Y086P-A088V-D089S-S097G-T103S-L104V-G106S-I111L-S114A-T115G-S116N-N120H-T122A-M124L-G128S-S129P-G131P-T133A-A134T-Q136E-C139V-N141S-Y143T-N144S-I147V-V148L-I150V-S158T-G166D-F217L-A224T-E248N; S052A-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N; A001T-N076D-T078S-T079I-Y086P-D089E-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-E248N; A001T-S052A-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-A153S-S158T-G166D-T209Y-N212S-N213T-S216T-F217L-A224T-E248N; A001T-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-A153S-S158T-G166D-T209Y-N212S-N213T-S216T-F217L-A224T-E248N; A001T-N076D-Y086P-N087S-A088V-D089S-S097G-T103S-L104V-G106S-I111L-S114A-I115G-S116N-N120H-1122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N-Q275H; A001T-N076D-T078S-T079I-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N; N076D-G118R-G128D-S129R-S130I-G166D-E248H; N076D-G118R-G128D-S129F-S130Y-G166D-E248N; N076D-G118R-G128V-S129M-S130H-G166D-E248N; S052T-N076D-G118R-G128M-S129V-S130F-G166H-Y238F; S052T-N076D-G118R-G128D-S129R-S130I-G166D-F217R-N256R; S052T-N076D-G118R-G128V-S129M-S130H-G166D-E248N-N256E-S272K; S052F-G053Y-N076D-G118R-G128D-S129F-S130Y-G166D-F217R-E248N-N256R-S272K; and A001G-A040D-A048Q-S052T-G053E-Y091H-S114T-I147A-S156N-S159N-G166H-S182E-Y238F; wherein the amino acid positions of the variant or recombinant polypeptide or active fragment thereof are numbered by correspondence with the amino acid sequence of SEQ ID NO:10.

In an even still further embodiment, the protease variant, or recombinant polypeptide or active fragment thereof comprises an amino acid sequence comprising two or more substitutions selected from S052T-S129F; G053D-G166D; N076D-G166D; G166H-S159D; G166D-S272K; G053D-N076D-G166D; G053D-G118R-G166D; G053D-G166D-S272K; N076D-G118R-G166D; N076D-G166D-E248K; N076D-G166D-N256R; N076D-G166D-S272K; S024T-G053E-G166D-M222L-N256P; V149L-S156H-T185Q-S272Q-S159D; G053D-N076D-G118R-G166D-N256R; S052T-V149G-S156N-G166H-F217R; S052T-V149G-S156N-G166D-F217R; S052T-V149G-S156N-S159D-G166D-F217R; S052T-V149G-S156N-G166D-S182E-F217R; A040D-S114T-I147A-S159N-G166D-S182E; N038Q-G053E-G100A-N117S-I147A-S158T-G166D-S272K; N038Q-G053E-N117S-I147A-S158T-G166D-N256P-S272K; A001G-S052Q-V149G-S156N-S159N-S182E-Y238F-S272Q; A001G-S052Q-S114T-S156N-S159N-S182E-Y238F-S272Q; N038Q-G053E-G100A-N117S-I147A-S158T-G166D-N256P-S272K; A001G-I030V-T078D-V093T-T103E-S156N-S159D-G166S-S194P-F21 7R-A236N; A001T-N076D-T078S-T079I-Y086P-D089E-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-E248N; S052A-N076D-T078S-T0791-Y086P-D089E-S097G-T103S-L104V-G106S-I111L-S114A-I115G-S116N-N120H-I122A-M124L-G128S-S129P-G131P-T133V-A134T-Q136E-C139V-N141S-Y143T-N144S-I147V-V148L-I150V-S158T-G166D-F217L-E248N; A001T-N076D-T078S-T079I-Y086P-D089E-S097G-T103S-L104V-G106S-I111L-S114A-I115G-S116N-N120H-I122A-M124L-G128S-S129P-G131P-T133V-A134T-Q136E-C139V-N141S-Y143T-N144S-I147V-V148L-I150V-S158T-G166D-T209Y-N212S-N213T-S216T-F217L-A224T-E248N-K251S-N252S-N256Y;N076D-T078S-T079I-Y086P-D089E-S097G-T103S-L104V-G106S-I111L-S114A-I115G-S116N-N120H-1122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N; N076D-T078S-T079I-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-F217L-E248N; A001T-N076D-T078S-T079I-Y086P-D089E-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N; A001T-N076D-T078S-T079I-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-E248N; and N076D-G118R-G128D-S129R-S130I-G166D-E248N; wherein the amino acid positions of the variant or recombinant polypeptide or active fragment thereof are numbered by correspondence with the amino acid sequence of SEQ ID NO:10.

In yet another embodiment, the protease variant, or recombinant polypeptide or active fragment thereof comprises an amino acid sequence comprising two or more substitutions selected from S052A-N076D-T078S-T079I-Y086P-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-F217L-A224T-E248N; S052A-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N; A001T-N076D-T078S-T0791-Y086P-D089E-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-E248N; A001T-S052A-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-A153S-S158T-G166D-T209Y-N212S-N213T-S216T-F217L-A224T-E248N; A001T-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-A153S-S158T-G166D-T209Y-N212S-N213T-S216T-F217L-A224T-E248N; A001T-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-1150V-S158T-G166D-T209Y-N212S-N213T-S216T-F217L-A224T-E248N-Q275H; A001T-N076D-T078S-T079I-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-M141S-Y143T-N144S-I147V-V148L-I150V-S158T-G166D-T209Y-N212S-N213T-S216T-F217L-A224T-E248N; N076D-G118R-G128D-S129F-S130Y-G166D-E248N; N076D-G118R-G128V-S129M-S130H-G166D-E248N; S052T-N076D-G118R-G128M-S129V-S130F-G166H-Y238F; S052T-N076D-G118R-G128D-S129R-S130I-G166D-F217R-N256R; S052T-N076D-G118R-G128V-S129M-S130H-G166D-E248N-N256E-S272K; S052F-G053Y-N076D-G118R-G128D-S129F-S130Y-G166D-F217R-E248N-N256R-S272K; and A001G-A040D-A048Q-S052T-G053E-Y091H-S114T-I147A-S156N-S159N-G166H-S182E-Y238F; wherein the amino acid positions of the variant or recombinant polypeptide or active fragment thereof are numbered by correspondence with the amino acid sequence of SEQ ID NO:10.

In some embodiments of the invention, LG12-clade enzymes of the present invention comprise substitutions selected from: G053D-N076D-G166D; N038Q-G053E-G100A-N117S-I147A-S158T-G166D-N256P-S272K; A001G-1030V-T078D-V093T-T103E-S156N-S159D-G166S-S194P-F217R-A236N; S024T-G053E-G166D-M222L-N256P; V149L-S156H-T185Q-S272Q-S159D; G053D-N076D-G118R-G166D-N256R; N038Q-G053E-G100A-N117S-I147A-S158T-G166D-S272K; S052T-V149G-S156N-G166H-F217R; A001G-S052Q-V149G-S156N-S159N-S182E-Y238F-S272Q; A001G-S052Q-S114T-S156N-S159N-S182E-Y238F-S272Q; S052T-V149G-S156N-S159D-G166D-F217R; N038Q-G053E-N117S-1147A-S158T-G166D-N256P-S272K; A040D-S114T-1147A-S159N-G166D-S182E; S052T-V149G-S156N-G166D-S182E-F217R; and S052T-V149G-S156N-G166D-F217R, wherein the amino acid positions of the variant are numbered by correspondence with the amino acid sequence of LG12 wild-type set forth in SEQ ID NO:10.

In some embodiments of the invention, LG12-clade enzymes of the present invention having a beneficial mutation in long-term stability in at least one boron-free HDL comprise substitutions selected from: G053D-N076D-G166D; V149L-S156H-T185Q-S272Q-S159D; G053D-N076D-G118R-G166D-N256R; N038Q-G053E-G100A-N117S-I147A-S158T-G166D-S272K; S052T-V149G-S156N-G166H-F217R; and S052T-V149G-S156N-G166D-F217R, wherein the amino acid positions of the variant are numbered by correspondence with the amino acid sequence of LG12 wild-type set forth in SEQ ID NO:10.

Also provided are LG12-clade protease variants useful in a detergent composition where favorable modifications result in a minimum performing index for cleaning performance, stability of the enzyme in detergent compositions and/or thermostability of the enzyme, while having at least one of these characteristics improved from its parent LG12-clade enzyme. In one embodiment, an LG12-clade protease variant is useful in a detergent composition and has a minimum performing index for cleaning performance, stability of the enzyme in detergent compositions and/or thermostability of the enzyme, while having at least one of these characteristics improved over the LG12-clade wild-type parent protease (SEQ ID NO:10).

The terms "thermal stability" and "thermostability" refer to LG12-clade protease variants of the present invention that retain a specified amount of enzymatic activity after exposure to an identified temperature, often over a given period of time under conditions prevailing during the proteolytic, hydrolyzing, cleaning or other process disclosed herein, for example while exposed to altered temperatures. Altered temperatures include increased or decreased temperatures. In some embodiments, the LG12-clade protease retains at least about 50%, about 60%, about 70%, about 75%, about 80%, about 85%, about 90%, about 92%, about 95%, about 96%, about 97%, about 98%, or about 99% protease activity after exposure to altered temperatures over a given time period, for example, at least about 60 minutes, about 120 minutes, about 180 minutes, about 240 minutes, about 300 minutes, etc.

As used herein, improved properties of an LG12-clade protease variant has improved residual protease activity, improved cleaning performance, and/or improved stability optionally with retained wash or cleaning performance, relative to the corresponding parent LG12-clade enzyme. In some embodiments, an LG12-clade protease variant has improved stability optionally with retained cleaning performance relative to the corresponding LG12-clade wild-type parent protease (SEQ ID NO:10). In some further embodiments, an LG12-clade protease variant has improved stability with retained cleaning performance relative to the corresponding LG12-clade wild-type parent protease (SEQ ID NO:10). The improved properties of an LG12-clade protease variant may comprise improved cleaning performance and/or improved stability. In some embodiments, the invention provides an LG12-clade protease variant that exhibits one of more of the following properties: improved hand wash performance, improved hand or manual dishwashing performance, improved automatic dishwashing performance, improved laundry performance, and/or improved stability relative to a reference parent LG12-clade enzyme. In some embodiments, the invention provides an LG12-clade protease variant that exhibits one of more of the following properties: improved hand wash performance, improved hand or manual dishwashing performance, improved automatic dishwashing performance, improved laundry performance, and/or improved stability relative to the corresponding LG12-clade wild-type parent protease (SEQ ID NO:10).

As noted above, the variant enzyme polypeptides of the invention have enzymatic activities (e.g., protease activities) and thus are useful in cleaning applications, including but not limited to, methods for cleaning dishware items, tableware items, fabrics, and items having hard surfaces (e.g., the hard surface of a table, table top, wall, furniture item, floor, ceiling, etc.). Exemplary cleaning compositions comprising one or more variant serine protease enzyme polypeptides of the invention described infra. The enzymatic activity (e.g., protease enzyme activity) of an enzyme polypeptide of the invention can be determined readily using procedures well known to those of ordinary skill in the art. The Examples presented infra describe methods for evaluating the enzymatic activity and cleaning performance. The performance of polypeptide enzymes of the invention in removing stains (e.g., a protein stain such as blood/milk/ink or egg yolk), cleaning hard surfaces, or cleaning laundry, dishware or tableware item(s) can be readily determined using procedures well known in the art and/or by using procedures set forth in the Examples.

The serine protease polypeptides of the present invention can have protease activity over a broad range of pH conditions. In some embodiments, the serine protease polypeptides have protease activity on dimethylcasein as a substrate, as demonstrated in Examples below. In some embodiments, the serine protease polypeptides have protease activity at a pH of from about 4.0 to about 12.0. In some embodiments, the serine protease polypeptides have protease activity at a pH of from about 6.0 to about 12.0. In some embodiments, the serine protease polypeptides have at least 50%, 60%, 70%, 80% or 90% of maximal protease activity at a pH of from about 6.0 to about 12.0, or from about 7.0 to about 12.0. In some embodiments, the serine protease polypeptides have protease activity at a pH above 6.0, 6.5, 7.0, 7.5, 8.0, 8.5, 9.0, 9.5, 10.0, 10.5, 11.0 or 11.5. In some embodiments, the serine protease polypeptides have protease activity at a pH below 12.0, 11.5, 11.0, 10.5, 10.0, 9.5, 9.0, 8.5, 8.0, 7.5, 7.0, or 6.5.

In some embodiments, the serine protease polypeptides of the present invention have protease activity at a temperature range from about 10°C to about 90°C, or from about 30°C to about 80°C. In some embodiments, the serine protease polypeptides of the present invention have protease activity at a temperature range of from about 55°C to about 75°C. In some embodiments, the serine protease polypeptides have at least 50%, 60%, 70%, 80% or 90% of maximal protease activity at a temperature of from about 55°C to about 75°C. In some embodiments, the serine proteases have activity at a temperature above 50°C, 55°C, 60°C, 65°C, or 70°C. In some embodiments, the serine proteases have activity at a temperature below 80°C, 75°C, 70°C, 65°C, 60°C, or 55°C.

In some embodiments, the serine protease polypeptides of the present invention demonstrate cleaning performance in a cleaning composition. Cleaning compositions often include ingredients harmful to the stability and performance of enzymes, making cleaning compositions a harsh environment for enzymes, e.g. serine proteases, to retain function. Thus, it is not trivial for an enzyme to be put in a cleaning composition and expect enzymatic function (e.g. serine protease activity, such as demonstrated by cleaning performance). In some embodiments, the serine protease polypeptides of the present invention demonstrate cleaning performance in automatic dishwashing (ADW) detergent compositions. In some embodiments, the cleaning performance in automatic dishwashing (ADW) detergent compositions includes cleaning of egg yolk stains. In some embodiments, the serine protease polypeptides of the present invention demonstrate cleaning performance in laundry detergent compositions. In some embodiments, the cleaning performance in laundry detergent compositions includes cleaning of blood/milk/ink stains. In each of the cleaning compositions, the serine protease polypeptides of the present invention demonstrate cleaning performance with or without a bleach component.

In some embodiments, the serine protease polypeptides of the present invention have stability in detergent compositions. In some embodiments, the serine protease polypeptides of the present invention have a thermostability T_{50%} value of at least 60°C.

A polypeptide of the invention can be subject to various changes, such as one or more amino acid insertions, deletions, and/or substitutions, either conservative or non-conservative, including where such changes do not substantially alter the enzymatic activity of the polypeptide. Similarly, a nucleic acid of the invention can also be subject to various changes, such as one or more substitutions of one or more nucleotides in one or more codons such that a particular codon encodes the same or a different amino acid, resulting in either a silent variation (e.g., when the encoded amino acid is not altered by the nucleotide mutation) or non-silent variation, one or more deletions of one or more nucleic acids (or codons) in the sequence, one or more additions or insertions of one or more nucleic acids (or codons) in the sequence, and/or cleavage of or one or more truncations of one or more nucleic acids (or codons) in the sequence. Many such changes in the nucleic acid sequence may not substantially alter the enzymatic activity of the resulting encoded polypeptide enzyme compared to the polypeptide enzyme encoded by the original nucleic acid sequence. A nucleic acid sequence of the invention can also be modified to include one or more codons that provide for optimum expression in an expression system (e.g., bacterial expression system), while, if desired, said one or more codons still encode the same amino acid(s).

In some embodiments, the LG12-clade protease variant can be a variant of a parent LG12-clade enzyme from the Genus *Bacillus.* In other embodiments, the LG12-clade protease variant of the present invention is a variant of a parent LG12-clade enzyme from a genus selected from *Bacillus, Geobacillus, Alicyclobacillus, Lactobacillus, Exiguobacterium, Brevibacillus, Paenibacillus, Herpetosiphon, Oceanobacillus, Shewanella, Clostridium, Staphylococcus, Flavobacterium, Stigmatella, Myxococcus, Vibrio, Methanosarcina, Chryseobacterium, Streptomyces, Kribbella, Janibacter, Nocardioides, Xanthamonas, Micromonospora, Burkholderia, Dehalococcoides, Croceibacter, Kordia, Microscilla, Thermoactinomyces, Chloroflexus, Listeria, Plesiocystis, Haliscomenobacter, Cytophaga, Hahella, Arthrobacter, Brachybacterium, Clavibacter, Microbacterium, Intrasporangium, Frankia, Meiothermus, Pseudomonas, Ricinus, Catenulispora, Anabaena, Nostoc, Halomonas, Chromohalobacter, Bordetella, Variavorax, Dickeya, Pectobacterium, Citrobacter, Enterobacter, Salmonella, Erwinia, Pantoea, Rahnella, Serratia, Geodermatophilus, Gemmata, Xenorhabdus, Photorhabdus, Aspergillus, Neosartorya, Pyrenophora, Saccharopolyspora, Nectria, Gibberella, Metarhizium, Waddlia, Cyanothece, Cellulphaga, Providencia, Bradyrhizobium, Agrobacterium, Mucilaginibacter, Serratia, Sorangium, Streptosporangium, Renibacterium, Aeromonas, Reinekea, Chromobacterium, Moritella, Haliangium, Kangiella, Marinomonas, Vibrionales, Listonella, Salinivibrio, Photobacterium, Alteromonadales, Legionella, Teredinibacter, Reinekea, Hydrogenivirga,* and *Pseudoalteromonas.*

A polypeptide of the invention can be subject to various changes, such as one or more amino acid insertions, deletions, and/or substitutions, either conservative or non-conservative, including where such changes do not substantially alter the enzymatic activity of the polypeptide. In some embodiments, the present invention provides a genus of polypeptides comprising LG12-clade protease variants having the desired enzymatic activity (e.g., LG12-clade enzyme activity or cleaning performance activity) which comprise sequences having the amino acid substitutions described herein and also which comprise one or more additional amino acid substitutions, such as conservative and non-conservative substitutions, wherein the polypeptide exhibits, maintains, or approximately maintains the desired enzymatic activity (e.g., LG12-clade enzyme activity or proteolytic activity, as reflected in the cleaning activity or performance of the variant LG12-clade enzyme). Amino acid substitutions in accordance with the invention may include, but are not limited to, one or more non-conservative substitutions and/or one or more conservative amino acid substitutions. A conservative amino acid residue substitution typically involves exchanging a member within one functional class of amino acid residues for a residue that belongs to the same functional class (identical amino acid residues are considered functionally homologous or conserved in calculating percent functional homology). A conservative amino acid substitution typically involves the substitution of an amino acid in an amino acid sequence with a functionally similar amino acid. For example, alanine, glycine, serine, and threonine are functionally similar and thus may serve as conservative amino acid substitutions for one another. Aspartic acid and glutamic acid may serve as conservative substitutions for one another. Asparagine and glutamine may serve as conservative substitutions for one another. Arginine, lysine, and histidine may serve as conservative substitutions for one another. Isoleucine, leucine, methionine, and valine may serve as conservative substitutions for one another. Phenylalanine, tyrosine, and tryptophan may serve as conservative substitutions for one another.

Other conservative amino acid substitution groups can be envisioned. For example, amino acids can be grouped by similar function or chemical structure or composition (e.g., acidic, basic, aliphatic, aromatic, sulfur-containing). For instance, an aliphatic grouping may comprise: Glycine (G), Alanine (A), Valine (V), Leucine (L), Isoleucine (I). Other groups containing amino acids that are considered conservative substitutions for one another include: aromatic: Phenylalanine (F), Tyrosine (Y), Tryptophan (W); sulfur-containing: Methionine (M), Cysteine (C); Basic: Arginine (R), Lysine (K), Histidine (H); Acidic: Aspartic acid (D), Glutamic acid (E); non-polar uncharged residues, Cysteine (C), Methionine (M), and Proline (P); hydrophilic uncharged residues: Serine (S), Threonine (T), Asparagine (N), and Glutamine (Q). Additional groupings of amino acids are well-known to those of skill in the art and described in various standard textbooks. Listing of a polypeptide sequence herein, in conjunction with the above substitution groups, provides an express listing of all conservatively substituted polypeptide sequences.

More conservative substitutions exist within the amino acid residue classes described above, which also or alternatively can be suitable. Conservation groups for substitutions that are more conservative include: valine-leucine-isoleucine, phenylalanine-tyrosine, lysine-arginine, alanine-valine, and asparagine-glutamine.

Conservatively substituted variations of a polypeptide sequence of the invention (e.g., variant proteases of the invention) include substitutions of a small percentage, sometimes less than 25%, 20%, 15%, 14%, 13%, 12%, 11%, 10%, 9%, 8%, 7%, or 6% of the amino acids of the polypeptide sequence, or less than 5%, 4%, 3%, 2%, or 1%, or less than 10, 9, 8, 7, 6, 5, 4, 3, 2, or 1 amino acid substitution of the amino acids of the polypeptide sequence, with a conservatively selected amino acid of the same conservative substitution group.

In some embodiments, the protease variant comprises one or more mutations, and having a total net charge of -5, -4, -3, -2, -1, 0, 1, 2, 3, 4, or 5 relative to LG12-clade wild-type parent (SEQ ID NO:10).

### Nucleic Acids of the Invention

The invention provides isolated, non-naturally occurring, or recombinant nucleic acids (also referred to herein as "polynucleotides"), which may be collectively referred to as "nucleic acids of the invention" or "polynucleotides of the invention", which encode polypeptides of the invention. Nucleic acids of the invention, including all described below, are useful in recombinant production (*e*.*g*., expression) of polypeptides of the invention, typically through expression of a plasmid expression vector comprising a sequence encoding the polypeptide of interest or fragment thereof. As discussed above, polypeptides include variant protease polypeptides, including LG12-clade protease variant polypeptides having enzymatic activity (*e*.*g*., proteolytic activity) which are useful in cleaning applications and cleaning compositions for cleaning an item or a surface (*e*.*g*., surface of an item) in need of cleaning.

In some embodiments, the invention provides an isolated, recombinant, substantially pure, or non-naturally occurring nucleic acid comprising a nucleotide sequence encoding any polypeptide (including any fusion protein, etc.) of the invention described above in the section entitled "Polypeptides of the Invention" and elsewhere herein. The present invention provides nucleic acids encoding a serine protease polypeptide of the present invention, wherein the serine protease polypeptide is a mature form having proteolytic activity. In some embodiments, the serine protease is expressed recombinantly with a homologous pro-peptide sequence. In other embodiments, the serine protease is expressed recombinantly with a heterologous pro-peptide sequence (e.g., GG36 pro-peptide sequence).

Some embodiments are directed to a polynucleotide comprising a nucleic acid sequence that encodes a protease variant described herein, or recombinant polypeptide or active fragment thereof. In some embodiments, a polynucleotide encodes an isolated, recombinant, substantially pure, or non-naturally occurring LG12-clade protease variant having protease activity, which polypeptide has at least about 60%, at least about 65%, at least about 70%, at least about 75%, at least about 76%, at least about 77%, at least about 78%, at least about 79%, at least about 80%, at least about 81%, at least about 82%, at least about 83%, at least about 84%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, at least about 99.5%, or 100% sequence to an amino acid sequence selected from SEQ ID NO: 1,2,3,4, 5, 6, 7, 8, 29, 30, 31, 32, 33, 34, 35, 36, 37, 38, 39, 40, 41, 42, and 43. In some embodiments, a polynucleotide encodes an isolated, recombinant, substantially pure, or non-naturally occurring LG12-clade protease variant having protease activity, which polypeptide has 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity to an amino acid sequence selected from SEQ ID NO:1, 2, 3, 4, 5, 6, 7, 8, 29, 30, 31, 32, 33, 34, 35, 36, 37, 38, 39, 40, 41, 42, and 43. In a still further embodiment, a polynucleotide encodes an isolated, recombinant, substantially pure, or non-naturally occurring LG12-clade protease variant having protease activity, which polypeptide has 70%, 75%, 80%, 85%, 90%, 95%, 96%, 97%, 98%, 99% or 100% sequence identity to an amino acid sequence selected from SEQ ID NO:1, 2, 3, 4, 5, 6, 7, 8, 29, 30, 31, 32, 33, 34, 35, 36, 37, 38, 39, 40, 41, 42, and 43. In an even still further embodiment, a polynucleotide encodes an isolated, recombinant, substantially pure, or non-naturally occurring LG12-clade protease variant having protease activity, wherein said polypeptide is selected from SEQ ID NO:1, 2, 3, 4, 5, 6, 7, 8, 29, 30, 31, 32, 33, 34, 35, 36, 37, 38, 39, 40, 41, 42, and 43.

Also provided is an isolated, recombinant, substantially pure, or non-naturally occurring nucleic acid comprising a polynucleotide sequence which encodes a LG12-clade protease variant having proteolytic activity, said variant comprising an amino acid sequence which differs from the amino acid sequence of SEQ ID NO:1, 2, 3, 4, 5, 6, 7, 8, 29, 30, 31, 32, 33, 34, 35, 36, 37, 38, 39, 40, 41, 42, or 43 by no more than 50, no more than 45, no more than 40, no more than 35, no more than 30, no more than 25, no more than 20, no more than 19, no more than 18, no more than 17, no more than 16, no more than 15, no more than 14, no more than 13, no more than 12, no more than 11, no more than 10, no more than 9, no more than 6, no more than 5, no more than 4, no more than 3, no more than 2 amino acid residues, or no more than 1 amino acid residue(s), as determined by alignment of the variant protease amino acid sequence with the LG12-clade wild-type amino acid sequence.

Nucleic acids of the invention can be generated by using any suitable synthesis, manipulation, and/or isolation techniques, or combinations thereof. For example, a polynucleotide of the invention may be produced using standard nucleic acid synthesis techniques, such as solid-phase synthesis techniques that are well-known to those skilled in the art. In such techniques, fragments of up to 50 or more nucleotide bases are typically synthesized, then joined (*e*.*g*., by enzymatic or chemical ligation methods, or polymerase mediated recombination methods) to form essentially any desired continuous nucleic acid sequence. The synthesis of the nucleic acids of the invention can be also facilitated (or alternatively accomplished) by any suitable method known in the art, including but not limited to chemical synthesis using the classical phosphoramidite method (*See e*.*g*., Beaucage et al. Tetrahedron Letters 22:1859-69 [1981]); or the method described by Matthes *et al.* (*See*, Matthes et al., EMBO J. 3:801-805 [1984], as is typically practiced in automated synthetic methods. Nucleic acids of the invention also can be produced by using an automatic DNA synthesizer. Customized nucleic acids can be ordered from a variety of commercial sources (*e*.*g*., The Midland Certified Reagent Company, the Great American Gene Company, Operon Technologies Inc., and DNA2.0). Other techniques for synthesizing nucleic acids and related principles are known in the art (*See e*.*g*., Itakura et al., Ann. Rev. Biochem. 53:323 [1984]; and Itakura et al., Science 198:1056 [1984]).

As indicated above, recombinant DNA techniques useful in modification of nucleic acids are well known in the art. For example, techniques such as restriction endonuclease digestion, ligation, reverse transcription and cDNA production, and polymerase chain reaction (*e*.*g*., PCR) are known and readily employed by those of skill in the art. Nucleotides of the invention may also be obtained by screening cDNA libraries (*e*.*g*., cDNA libraries generated using mutagenesis techniques commonly used in the art, including those described herein) using one or more oligonucleotide probes that can hybridize to or PCR-amplify polynucleotides which encode a variant protease polypeptide(s) of the invention. Procedures for screening and isolating cDNA clones and PCR amplification procedures are well known to those of skill in the art and described in standard references known to those skilled in the art. Some nucleic acids of the invention can be obtained by altering a naturally occurring polynucleotide backbone (*e*.*g*., that encodes an enzyme or parent protease) by, for example, a known mutagenesis procedure (*e*.*g*., site-directed mutagenesis, site saturation mutagenesis, and *in vitro* recombination).

A variety of methods are known in the art that are suitable for generating modified polynucleotides of the invention that encode variant proteases of the invention, including, but not limited to, for example, site-saturation mutagenesis, scanning mutagenesis, insertional mutagenesis, deletion mutagenesis, random mutagenesis, site-directed mutagenesis, synthetic gene construction by oligonucleotide synthesis and ligation, and directed-evolution, as well as various other recombinatorial approaches. Methods for making modified polynucleotides and proteins (*e*.*g*., variant proteases) include DNA shuffling methodologies, methods based on non-homologous recombination of genes, such as ITCHY (*See*, Ostermeier *et al.*, 7:2139-44 [1999]), SCRACHY (*See*, Lutz *et al.* 98:11248-53 [2001]), SHIPREC (*See*, Sieber *et al.*, 19:456-60 [2001]), and NRR (*See*, Bittker *et al*., 20:1024-9 [2001]; Bittker *et al.*, 101:7011-6 [2004]), and methods that rely on the use of oligonucleotides to insert random and targeted mutations, deletions and/or insertions (*See*, Ness *et al*., 20:1251-5 [2002]; Coco *et al.*, 20:1246-50 [2002]; Zha *et al.*, 4:34-9 [2003]; Glaser *et al.*, 149:3903-13 [1992]).

The present invention provides isolated or recombinant vectors comprising at least one polynucleotide of the invention described herein (*e*.*g*., a polynucleotide encoding a variant protease of the invention described herein), isolated or recombinant expression vectors or expression cassettes comprising at least one nucleic acid or polynucleotide of the invention, isolated, substantially pure, or recombinant DNA constructs comprising at least one nucleic acid or polynucleotide of the invention, isolated or recombinant cells comprising at least one polynucleotide of the invention, cell cultures comprising cells comprising at least one polynucleotide of the invention, cell cultures comprising at least one nucleic acid or polynucleotide of the invention, and compositions comprising one or more such vectors, nucleic acids, expression vectors, expression cassettes, DNA constructs, cells, cell cultures, or any combination or mixtures thereof.

In some embodiments, the invention provides recombinant cells comprising at least one vector (e.g., expression vector or DNA construct) of the invention which comprises at least one nucleic acid or polynucleotide of the invention. Some such recombinant cells are transformed or transfected with such at least one vector. Such cells are typically referred to as host cells. Some such cells comprise bacterial cells, including, but are not limited to *Bacillus sp.* cells, such as *B. subtilis* cells. The invention also provides recombinant cells (*e*.*g*., recombinant host cells) comprising at least one variant protease of the invention.

In some embodiments, the invention provides a vector comprising a nucleic acid or polynucleotide of the invention. In some embodiments, the vector is an expression vector or expression cassette in which a polynucleotide sequence of the invention which encodes a variant protease of the invention is operably linked to one or additional nucleic acid segments required for efficient gene expression (*e*.*g*., a promoter operably linked to the polynucleotide of the invention which encodes a variant protease of the invention). A vector may include a transcription terminator and/or a selection gene, such as an antibiotic resistance gene that enables continuous cultural maintenance of plasmid-infected host cells by growth in antimicrobial-containing media.

An expression vector may be derived from plasmid or viral DNA, or in alternative embodiments, contains elements of both. Exemplary vectors include, but are not limited to pHYT, pC194, pJH101, pE194, pHP13 (*See*, Harwood and Cutting [eds.], Chapter 3, Molecular Biological Methods for Bacillus, John Wiley & Sons [1990]; suitable replicating plasmids for *B. subtilis* include those listed on p. 92; *See also*, Perego, Integrational Vectors for Genetic Manipulations in Bacillus subtilis, in Sonenshein et al., [eds.] Bacillus subtilis and Other Gram-Positive Bacteria: Biochemistry, Physiology and Molecular Genetics, American Society for Microbiology, Washington, D.C. [1993],pp. 615-624).

For expression and production of a protein of interest (*e*.*g*., variant protease) in a cell, at least one expression vector comprising at least one copy of a polynucleotide encoding the modified protease, and preferably comprising multiple copies, is transformed into the cell under conditions suitable for expression of the protease. In some embodiments of the present invention, a polynucleotide sequence encoding the variant protease (as well as other sequences included in the vector) is integrated into the genome of the host cell, while in other embodiments, a plasmid vector comprising a polynucleotide sequence encoding the variant protease remains as autonomous extra-chromosomal element within the cell. The invention provides both extrachromosomal nucleic acid elements as well as incoming nucleotide sequences that are integrated into the host cell genome. The vectors described herein are useful for production of the variant proteases of the invention. In some embodiments, a polynucleotide construct encoding the variant protease is present on an integrating vector that enables the integration and optionally the amplification of the polynucleotide encoding the variant protease into the bacterial chromosome. Examples of sites for integration are well known to those skilled in the art. In some embodiments, transcription of a polynucleotide encoding a variant protease of the invention is effectuated by a promoter that is the wild-type promoter for the selected precursor protease. In some other embodiments, the promoter is heterologous to the precursor protease, but is functional in the host cell. Specifically, examples of suitable promoters for use in bacterial host cells include, but are not limited to, for example, the AprE amyE, amyL, pstS, sacB, pSPAC, pVeg, pHpaII promoters, the promoter of the *B. stearothermophilus* maltogenic amylase gene, the *B. amyloliquefaciens* (BAN) amylase gene, the *B. subtilis* alkaline protease gene, the *B. clausii* alkaline protease gene the *B. pumilis* xylosidase gene, the *B. thuringiensis* cryIIIA, and the *B. licheniformis* alpha-amylase gene. Additional promoters include, but are not limited to the A4 promoter, as well as phage Lambda P_{R} or P_{L} promoters, and the *E. coli* lac, trp or tac promoters.

Variant proteases of the present invention can be produced in host cells of any suitable microorganism, including bacteria and fungi. For example, in some embodiments, the variant protease is produced in host cells of fungal and/or bacterial origin. In some embodiments, the host cells are *Bacillus sp., Streptomyces sp., Escherichia sp. or Aspergillus sp.* In some embodiments, the variant proteases are produced by *Bacillus sp.* host cells. Examples of *Bacillus* sp. host cells that find use in the production of the variant proteases of the invention include, but are not limited to *B. licheniformis, B. lentus, B. subtilis, B. amyloliquefaciens, B. lentus, B. brevis, B. stearothermophilus, B. alkalophilus, B. coagulans, B. circulans, B. pumilis, B. thuringiensis, B. clausii,* and *B. megaterium,* as well as other organisms within the genus *Bacillus.* In some embodiments, *B. subtilis* host cells are used for production of variant proteases. U.S. Patents 5,264,366 and 4,760,025 (RE 34,606) describe various *Bacillus* host strains that can be used for producing variant proteases of the invention, although other suitable strains can be used.

Several industrial bacterial strains that can be used to produce variant proteases of the invention include non-recombinant (*i*.*e*., wild-type) *Bacillus sp.* strains, as well as variants of naturally-occurring strains and/or recombinant strains. In some embodiments, the host strain is a recombinant strain, wherein a polynucleotide encoding a polypeptide of interest has been introduced into the host. In some embodiments, the host strain is a *B. subtilis* host strain and particularly a recombinant *Bacillus subtilis* host strain. Numerous *B. subtilis* strains are known, including, but not limited to for example, 1A6 (ATCC 39085), 168 (1A01), SB19, W23, Ts85, B637, PB1753 through PB1758, PB3360, JH642, 1A243 (ATCC 39,087), ATCC 21332, ATCC 6051, MI113, DE100 (ATCC 39,094), GX4931, PBT 110, and PEP 211strain (*See e*.*g*., Hoch et al., Genetics 73:215-228 [1973]; *See also*, U.S. Patent Nos. 4,450,235 and 4,302,544, and EP 0134048, each of which is incorporated by reference in its entirety). The use of *B. subtilis* as an expression host cells is well known in the art (*See e.g.*, Palva et al., Gene 19:81-87 [1982]; Fahnestock and Fischer, J. Bacteriol., 165:796-804 [1986]; and Wang et al., Gene 69:39-47 [1988]).

In some embodiments, the *Bacillus* host cell is a *Bacillus* sp. that includes a mutation or deletion in at least one of the following genes, *degU, degS, degR* and *degQ.* Preferably the mutation is in a *degU* gene, and more preferably the mutation is *degU(Hy)32* (*See e*.*g*., Msadek et al., J. Bacteriol. 172:824-834 [1990]; and Olmos et al., Mol. Gen. Genet. 253:562-567 [1997]). One suitable host strain is a *Bacillus subtilis* carrying a *degU32(Hy)* mutation. In some embodiments, the *Bacillus* host comprises a mutation or deletion in *scoC4* (*See e*.*g*., Caldwell et al., J. Bacteriol. 183:7329-7340 [2001]); *spoIIE* (*See e*.*g*., Arigoni et al., Mol. MicrobioL 31:1407-1415 [1999]); and/or *oppA* or other genes of the *opp* operon (*See e*.*g*., Perego et al., Mol. Microbiol. 5:173-185 [1991]). Indeed, it is contemplated that any mutation in the *opp* operon that causes the same phenotype as a mutation in the *oppA* gene will find use in some embodiments of the altered *Bacillus* strain of the invention. In some embodiments, these mutations occur alone, while in other embodiments, combinations of mutations are present. In some embodiments, an altered *Bacillus* host cell strain that can be used to produce a variant protease of the invention is a *Bacillus* host strain that already includes a mutation in one or more of the above-mentioned genes. In addition, *Bacillus* sp. host cells that comprise mutation(s) and/or deletions of endogenous protease genes find use. In some embodiments, the *Bacillus* host cell comprises a deletion of the *aprE* and the *nprE* genes. In other embodiments, the *Bacillus* sp. host cell comprises a deletion of 5 protease genes, while in other embodiments, the *Bacillus sp.* host cell comprises a deletion of 9 protease genes (*See e*.*g*., U.S. Pat. Appln. Pub. No. 2005/0202535, incorporated herein by reference).

Host cells are transformed with at least one nucleic acid encoding at least one variant protease of the invention using any suitable method known in the art. Whether the nucleic acid is incorporated into a vector or is used without the presence of plasmid DNA, it is typically introduced into a microorganism, in some embodiments, preferably an *E. coli* cell or a competent *Bacillus* cell. Methods for introducing a nucleic acid (*e*.*g*., DNA) into *Bacillus* cells or *E. coli* cells utilizing plasmid DNA constructs or vectors and transforming such plasmid DNA constructs or vectors into such cells are well known. In some embodiments, the plasmids are subsequently isolated from *E. coli* cells and transformed into *Bacillus* cells. However, it is not essential to use intervening microorganisms such as *E. coli,* and in some embodiments, a DNA construct or vector is directly introduced into a *Bacillus* host.

Those of skill in the art are well aware of suitable methods for introducing nucleic acid or polynucleotide sequences of the invention into *Bacillus* cells (*See e*.*g*., Ferrari et al., "Genetics," in Harwood et al. [eds.], Bacillus, Plenum Publishing Corp. [1989], pp. 57-72; Saunders et al., J. Bacteriol. 157:718-726 [1984]; Hoch et al., J. Bacteriol. 93:1925 -1937 [1967]; Mann et al., Current Microbiol. 13:131-135 [1986]; Holubova, Folia Microbiol. 30:97 [1985]; Chang et al., Mol. Gen. Genet. 168:11-115 [1979]; Vorobjeva et al., FEMS Microbiol. Lett. 7:261-263 [1980]; Smith et al., Appl. Env. Microbiol. 51:634 [1986]; Fisher et al., Arch. Microbiol. 139:213-217 [1981]; and McDonald, J. Gen. Microbiol. 130:203 [1984]). Indeed, such methods as transformation, including protoplast transformation and congression, transduction, and protoplast fusion are well known and suited for use in the present invention. Methods of transformation are used to introduce a DNA construct or vector comprising a nucleic acid encoding a variant protease of the present invention into a host cell. Methods known in the art to transform *Bacillus* cells include such methods as plasmid marker rescue transformation, which involves the uptake of a donor plasmid by competent cells carrying a partially homologous resident plasmid (*See*, Contente et al., Plasmid 2:555-571 [1979]; Haima et al., Mol. Gen. Genet. 223:185-191 [1990]; Weinrauch et al., J. Bacteriol. 154:1077-1087 [1983]; and Weinrauch et al., J. Bacteriol. 169:1205-1211 [1987]). In this method, the incoming donor plasmid recombines with the homologous region of the resident "helper" plasmid in a process that mimics chromosomal transformation.

In addition to commonly used methods, in some embodiments, host cells are directly transformed with a DNA construct or vector comprising a nucleic acid encoding a variant protease of the invention (*i.e*., an intermediate cell is not used to amplify, or otherwise process, the DNA construct or vector prior to introduction into the host cell). Introduction of the DNA construct or vector of the invention into the host cell includes those physical and chemical methods known in the art to introduce a nucleic acid sequence (*e*.*g*., DNA sequence) into a host cell without insertion into a plasmid or vector. Such methods include, but are not limited to calcium chloride precipitation, electroporation, naked DNA, liposomes and the like. In additional embodiments, DNA constructs or vector are co-transformed with a plasmid, without being inserted into the plasmid. In further embodiments, a selective marker is deleted from the altered *Bacillus* strain by methods known in the art (*See*, Stahl et al., J. Bacteriol. 158:411-418 [1984]; and Palmeros et al., Gene 247:255 -264 [2000]).

In some embodiments, the transformed cells of the present invention are cultured in conventional nutrient media. The suitable specific culture conditions, such as temperature, pH and the like are known to those skilled in the art and are well described in the scientific literature. In some embodiments, the invention provides a culture (*e*.*g*., cell culture) comprising at least one variant protease or at least one nucleic acid of the invention. Also provided are compositions comprising at least one nucleic acid, vector, or DNA construct of the invention.

In some embodiments, host cells transformed with at least one polynucleotide sequence encoding at least one variant protease of the invention are cultured in a suitable nutrient medium under conditions permitting the expression of the present protease, after which the resulting protease is recovered from the culture. The medium used to culture the cells comprises any conventional medium suitable for growing the host cells, such as minimal or complex media containing appropriate supplements. Suitable media are available from commercial suppliers or may be prepared according to published recipes (*See e*.*g*., the catalogues of the American Type Culture Collection). In some embodiments, the protease produced by the cells is recovered from the culture medium by conventional procedures, including, but not limited to for example, separating the host cells from the medium by centrifugation or filtration, precipitating the proteinaceous components of the supernatant or filtrate by means of a salt (*e*.*g*., ammonium sulfate), chromatographic purification (e.g., ion exchange, gel filtration, affinity, etc.). Any method suitable for recovering or purifying a variant protease finds use in the present invention.

In some embodiments, a variant protease produced by a recombinant host cell is secreted into the culture medium. A nucleic acid sequence that encodes a purification facilitating domain may be used to facilitate purification of soluble proteins. A vector or DNA construct comprising a polynucleotide sequence encoding a variant protease may further comprise a nucleic acid sequence encoding a purification facilitating domain to facilitate purification of the variant protease (*See e*.*g*., Kroll et al., DNA Cell Biol. 12:441-53 [1993]). Such purification facilitating domains include, but are not limited to, for example, metal chelating peptides such as histidine-tryptophan modules that allow purification on immobilized metals (*See*, Porath, Protein Expr. Purif. 3:263-281 [1992]), protein A domains that allow purification on immobilized immunoglobulin, and the domain utilized in the FLAGS extension/affinity purification system (*e*.*g*., protein A domains available from Immunex Corp., Seattle, WA). The inclusion of a cleavable linker sequence such as Factor XA or enterokinase (*e*.*g*., sequences available from Invitrogen, San Diego, CA) between the purification domain and the heterologous protein also find use to facilitate purification.

Assays for detecting and measuring the enzymatic activity of an enzyme, such as a variant protease of the invention, are well known. Various assays for detecting and measuring activity of proteases (*e*.*g*., variant proteases of the invention), are also known to those of ordinary skill in the art. In particular, assays are available for measuring protease activity that are based on the release of acid-soluble peptides from casein or hemoglobin, measured as absorbance at 280 nm or colorimetrically using the Folin method, well known to those skilled in the art. Other exemplary assays involve the solubilization of chromogenic substrates (*See e*.*g*., Ward, "Proteinases," in Fogarty (ed.)., Microbial Enzymes and Biotechnology, Applied Science, London, [1983], pp. 251-317). Other exemplary assays include, but are not limited to succinyl-Ala-Ala-Pro-Phe-para nitroanilide assay (suc-AAPF-pNA) and the 2,4,6-trinitrobenzene sulfonate sodium salt assay (TNBS assay). Numerous additional references known to those in the art provide suitable methods (*See e.g.*, Wells et al., Nucleic Acids Res. 11:7911 - 7925 [1983]; Christianson et al., Anal. Biochem. 223:119 -129 [1994]; and Hsia et al., Anal Biochem. 242:221-227 [1999]).

A variety of methods can be used to determine the level of production of a mature protease (*e*.*g*., mature variant proteases of the present invention) in a host cell. Such methods include, but are not limited to, for example, methods that utilize either polyclonal or monoclonal antibodies specific for the protease. Exemplary methods include, but are not limited to enzyme-linked immunosorbent assays (ELISA), radioimmunoassays (RIA), fluorescent immunoassays (FIA), and fluorescent activated cell sorting (FACS). These and other assays are well known in the art (*See e.g.*, Maddox et al., J. Exp. Med. 158:1211 [1983]).

In some other embodiments, the invention provides methods for making or producing a mature variant protease of the invention. A mature variant protease does not include a signal peptide or a propeptide sequence. Some methods comprise making or producing a variant protease of the invention in a recombinant bacterial host cell, such as for example, a *Bacillus sp.* cell (*e*.*g*., a *B. subtilis* cell). In some embodiments, the invention provides a method of producing a variant protease of the invention, the method comprising cultivating a recombinant host cell comprising a recombinant expression vector comprising a nucleic acid encoding a variant protease of the invention under conditions conducive to the production of the variant protease. Some such methods further comprise recovering the variant protease from the culture.

In some embodiments the invention provides methods of producing a variant protease of the invention, the methods comprising: (a) introducing a recombinant expression vector comprising a nucleic acid encoding a variant protease of the invention into a population of cells (*e*.*g*., bacterial cells, such as *B. subtilis* cells); and (b) culturing the cells in a culture medium under conditions conducive to produce the variant protease encoded by the expression vector. Some such methods further comprise: (c) isolating the variant protease from the cells or from the culture medium.

Unless otherwise noted, all component or composition levels provided herein are made in reference to the active level of that component or composition, and are exclusive of impurities, for example, residual solvents or by-products, which may be present in commercially available sources. Enzyme components weights are based on total active protein. All percentages and ratios are calculated by weight unless otherwise indicated. All percentages and ratios are calculated based on the total composition unless otherwise indicated. In the exemplified detergent compositions, the enzymes levels are expressed by pure enzyme by weight of the total composition and unless otherwise specified, the detergent ingredients are expressed by weight of the total compositions.

As indicated herein, in some embodiments, the cleaning compositions of the present invention further comprise adjunct materials including, but not limited to, surfactants, builders, bleaches, bleach activators, bleach catalysts, other enzymes, enzyme stabilizing systems, chelants, optical brighteners, soil release polymers, dye transfer agents, dispersants, suds suppressors, dyes, perfumes, colorants, filler salts, hydrotropes, photoactivators, fluorescers, fabric conditioners, hydrolyzable surfactants, preservatives, anti-oxidants, anti-shrinkage agents, anti-wrinkle agents, germicides, fungicides, color speckles, silvercare, anti-tarnish and/or anti-corrosion agents, alkalinity sources, solubilizing agents, carriers, processing aids, pigments, and pH control agents (*See e*.*g*., U.S. Pat. Nos. 6,610,642, 6,605,458, 5,705,464, 5,710,115, 5,698,504, 5,695,679, 5,686,014 and 5,646,101, all of which are incorporated herein by reference). Embodiments of specific cleaning composition materials are exemplified in detail below. In embodiments in which the cleaning adjunct materials are not compatible with the variant proteases of the present invention in the cleaning compositions, then suitable methods of keeping the cleaning adjunct materials and the protease(s) separated (*i.e.*, not in contact with each other) until combination of the two components is appropriate are used. Such separation methods include any suitable method known in the art (*e*.*g*., gelcaps, encapsulation, tablets, physical separation, etc.).

The cleaning compositions of the present invention are advantageously employed for example, in laundry applications, hard surface cleaning, dishwashing applications, as well as cosmetic applications such as dentures, teeth, hair and skin. In addition, due to the unique advantages of increased effectiveness in lower temperature solutions, the enzymes of the present invention are ideally suited for laundry applications. Furthermore, the enzymes of the present invention find use in granular and liquid compositions.

The variant proteases of the present invention also find use in cleaning additive products. In some embodiments, low temperature solution cleaning applications find use. In some embodiments, the present invention provides cleaning additive products including at least one enzyme of the present invention is ideally suited for inclusion in a wash process when additional bleaching effectiveness is desired. Such instances include, but are not limited to low temperature solution cleaning applications. In some embodiments, the additive product is in its simplest form, one or more proteases. In some embodiments, the additive is packaged in dosage form for addition to a cleaning process. In some embodiments, the additive is packaged in dosage form for addition to a cleaning process where a source of peroxygen is employed and increased bleaching effectiveness is desired. Any suitable single dosage unit form finds use with the present invention, including but not limited to pills, tablets, gelcaps, or other single dosage units such as pre-measured powders or liquids. In some embodiments, filler(s) or carrier material(s) are included to increase the volume of such compositions. Suitable filler or carrier materials include, but are not limited to, various salts of sulfate, carbonate and silicate as well as talc, clay and the like. Suitable filler or carrier materials for liquid compositions include, but are not limited to water or low molecular weight primary and secondary alcohols including polyols and diols. Examples of such alcohols include, but are not limited to, methanol, ethanol, propanol and isopropanol. In some embodiments, the compositions contain from about 5% to about 90% of such materials. Acidic fillers find use to reduce pH. Alternatively, in some embodiments, the cleaning additive includes adjunct ingredients, as more fully described below.

The present cleaning compositions and cleaning additives require an effective amount of at least one of the protease variants provided herein, alone or in combination with other proteases and/or additional enzymes. The required level of enzyme is achieved by the addition of one or more protease variants of the present invention. Typically the present cleaning compositions comprise at least about 0.0001 weight percent, from about 0.0001 to about 10, from about 0.001 to about 1, or even from about 0.01 to about 0.1 weight percent of at least one of the variant proteases of the present invention.

The cleaning compositions herein are typically formulated such that, during use in aqueous cleaning operations, the wash water will have a pH of from about 5.0 to about 11.5 or even from about 7.5 to about 10.5. Liquid product formulations are typically formulated to have a neat pH from about 3.0 to about 9.0 or even from about 3 to about 5. Granular laundry products are typically formulated to have a pH from about 9 to about 11. Techniques for controlling pH at recommended usage levels include the use of buffers, alkalis, acids, etc., and are well known to those skilled in the art.

Suitable "low pH cleaning compositions" typically have a neat pH of from about 3 to about 5, and are typically free of surfactants that hydrolyze in such a pH environment. Such surfactants include sodium alkyl sulfate surfactants that comprise at least one ethylene oxide moiety or even from about 1 to about 16 moles of ethylene oxide. Such cleaning compositions typically comprise a sufficient amount of a pH modifier, such as sodium hydroxide, monoethanolamine or hydrochloric acid, to provide such cleaning composition with a neat pH of from about 3 to about 5. Such compositions typically comprise at least one acid stable enzyme. In some embodiments, the compositions are liquids, while in other embodiments, they are solids. The pH of such liquid compositions is typically measured as a neat pH. The pH of such solid compositions is measured as a 10% solids solution of said composition wherein the solvent is distilled water. In these embodiments, all pH measurements are taken at 20°C, unless otherwise indicated.

In some embodiments, when the variant protease(s) is/are employed in a granular composition or liquid, it is desirable for the variant protease to be in the form of an encapsulated particle to protect the variant protease from other components of the granular composition during storage. In addition, encapsulation is also a means of controlling the availability of the variant protease during the cleaning process. In some embodiments, encapsulation enhances the performance of the variant protease(s) and/or additional enzymes. In this regard, the variant proteases of the present invention are encapsulated with any suitable encapsulating material known in the art. In some embodiments, the encapsulating material typically encapsulates at least part of the catalyst for the variant protease(s) of the present invention. Typically, the encapsulating material is water-soluble and/or water-dispersible. In some embodiments, the encapsulating material has a glass transition temperature (Tg) of 0°C or higher. Glass transition temperature is described in more detail in WO97/11151. The encapsulating material is typically selected from consisting of carbohydrates, natural or synthetic gums, chitin, chitosan, cellulose and cellulose derivatives, silicates, phosphates, borates, polyvinyl alcohol, polyethylene glycol, paraffin waxes, and combinations thereof. When the encapsulating material is a carbohydrate, it is typically selected from monosaccharides, oligosaccharides, polysaccharides, and combinations thereof. In some typical embodiments, the encapsulating material is a starch (*See e.g.*, EP 0922499 and USPNs 4,977,252; 5,354,559 and US 5,935,826). In some embodiments, the encapsulating material is a microsphere made from plastic such as thermoplastics, acrylonitrile, methacrylonitrile, polyacrylonitrile, polymethacrylonitrile and mixtures thereof; commercially available microspheres that find use include, but are not limited to those supplied by EXPANCEL® (Stockviksverken, Sweden), and PM 6545, PM 6550, PM 7220, PM 7228, EXTENDOSPHERES®, LUXSIL®, Q-CEL®, and SPHERICEL® (PQ Corp., Valley Forge, PA).

As described herein, the variant proteases of the present invention find particular use in the cleaning industry, including, but not limited to laundry and dish detergents. These applications place enzymes under various environmental stresses. The variant proteases of the present invention provide advantages over many currently used enzymes, due to their stability under various conditions.

Indeed, there are a variety of wash conditions including varying detergent formulations, wash water volumes, wash water temperatures, and lengths of wash time, to which proteases involved in washing are exposed. In addition, detergent formulations used in different geographical areas have different concentrations of their relevant components present in the wash water. For example, European detergents typically have about 4500-5000 ppm of detergent components in the wash water, while Japanese detergents typically have approximately 667 ppm of detergent components in the wash water. In North America, particularly the United States, detergents typically have about 975 ppm of detergent components present in the wash water.

A low detergent concentration system includes detergents where less than about 800 ppm of the detergent components are present in the wash water. Japanese detergents are typically considered low detergent concentration system as they have approximately 667 ppm of detergent components present in the wash water.

A medium detergent concentration includes detergents where between about 800 ppm and about 2000ppm of the detergent components are present in the wash water. North American detergents are generally considered to be medium detergent concentration systems as they have approximately 975 ppm of detergent components present in the wash water. Brazil typically has approximately 1500 ppm of detergent components present in the wash water.

A high detergent concentration system includes detergents where greater than about 2000 ppm of the detergent components are present in the wash water. European detergents are generally considered to be high detergent concentration systems as they have approximately 4500-5000 ppm of detergent components in the wash water.

Latin American detergents are generally high suds phosphate builder detergents and the range of detergents used in Latin America can fall in both the medium and high detergent concentrations as they range from 1500 ppm to 6000 ppm of detergent components in the wash water. As mentioned above, Brazil typically has approximately 1500 ppm of detergent components present in the wash water. However, other high suds phosphate builder detergent geographies, not limited to other Latin American countries, may have high detergent concentration systems up to about 6000 ppm of detergent components present in the wash water.

In light of the foregoing, it is evident that concentrations of detergent compositions in typical wash solutions throughout the world varies from less than about 800 ppm of detergent composition ("low detergent concentration geographies"), for example about 667 ppm in Japan, to between about 800 ppm to about 2000 ppm ("medium detergent concentration geographies"), for example about 975 ppm in U.S. and about 1500 ppm in Brazil, to greater than about 2000 ppm ("high detergent concentration geographies"), for example about 4500 ppm to about 5000 ppm in Europe and about 6000 ppm in high suds phosphate builder geographies.

The concentrations of the typical wash solutions are determined empirically. For example, in the U.S., a typical washing machine holds a volume of about 64.4 L of wash solution. Accordingly, in order to obtain a concentration of about 975 ppm of detergent within the wash solution about 62.79 g of detergent composition must be added to the 64.4 L of wash solution. This amount is the typical amount measured into the wash water by the consumer using the measuring cup provided with the detergent.

As a further example, different geographies use different wash temperatures. The temperature of the wash water in Japan is typically less than that used in Europe. For example, the temperature of the wash water in North America and Japan is typically between about 10 and about 30°C (*e*.*g*., about 20°C), whereas the temperature of wash water in Europe is typically between about 30 and about 60°C (*e*.*g*., about 40°C). However, in the interest of saving energy, many consumers are switching to using cold water washing. In addition, in some further regions, cold water is typically used for laundry, as well as dish washing applications. In some embodiments, the "cold water washing" of the present invention utilizes "cold water detergent" suitable for washing at temperatures from about 10°C to about 40°C, or from about 20°C to about 30°C, or from about 15°C to about 25°C, as well as all other combinations within the range of about 15°C to about 35°C, and all ranges within 10°C to 40°C.

As a further example, different geographies typically have different water hardness. Water hardness is usually described in terms of the grains per gallon mixed Ca²⁺/Mg²⁺. Hardness is a measure of the amount of calcium (Ca²⁺) and magnesium (Mg²⁺) in the water. Most water in the United States is hard, but the degree of hardness varies. Moderately hard (60-120 ppm) to hard (121-181 ppm) water has 60 to 181 parts per million (parts per million converted to grains per U.S. gallon is ppm # divided by 17.1 equals grains per gallon) of hardness minerals.

| **Water** | **Grains per gallon** | **Parts per million** |
|---|---|---|
| Soft | less than 1.0 | less than 17 |
| Slightly hard | 1.0 to 3.5 | 17 to 60 |
| Moderately hard | 3.5 to 7.0 | 60 to 120 |
| Hard | 7.0 to 10.5 | 120 to 180 |
| Very hard | greater than 10.5 | greater than 180 |

European water hardness is typically greater than about 10.5 (for example about 10.5 to about 20.0) grains per gallon mixed Ca²⁺/Mg²⁺ (*e*.*g*., about 15 grains per gallon mixed Ca²⁺/Mg²⁺). North American water hardness is typically greater than Japanese water hardness, but less than European water hardness. For example, North American water hardness can be between about 3 to about 10 grains, about 3 to about 8 grains or about 6 grains. Japanese water hardness is typically lower than North American water hardness, usually less than about 4, for example about 3 grains per gallon mixed Ca²⁺/Mg²⁺.

Accordingly, in some embodiments, the present invention provides variant proteases that show surprising wash performance in at least one set of wash conditions (e.g., water temperature, water hardness, and/or detergent concentration). In some embodiments, the variant proteases of the present invention are comparable in wash performance to other LG12 proteases. In some embodiments of the present invention, the variant proteases provided herein exhibit enhanced oxidative stability, enhanced thermal stability, enhanced cleaning capabilities under various conditions, and/or enhanced chelator stability. In addition, the variant proteases of the present invention find use in cleaning compositions that do not include detergents, again either alone or in combination with builders and stabilizers.

In some embodiments of the present invention, the cleaning compositions comprise at least one variant protease of the present invention at a level from about 0.00001 to about 10% by weight of the composition and the balance (e.g., about 99.999 to about 90.0%) comprising cleaning adjunct materials by weight of composition. In some other embodiments of the present invention, the cleaning compositions of the present invention comprises at least one variant protease at a level of about 0.0001 to about 10%, about 0.001 to about 5%, about 0.001 to about 2%, about 0.005 to about 0.5% by weight of the composition and the balance of the cleaning composition (e.g., about 99.9999 to about 90.0%, about 99.999 to about 98%, about 99.995 to about 99.5% by weight) comprising cleaning adjunct materials.

In some embodiments, the cleaning compositions of the present invention comprise one or more additional detergent enzymes, which provide cleaning performance and/or fabric care and/or dishwashing benefits. Examples of suitable enzymes include, but are not limited to, hemicellulases, cellulases, peroxidases, proteases, xylanases, lipases, phospholipases, esterases, cutinases, pectinases, pectate lyases, mannanases, keratinases, reductases, oxidases, phenoloxidases, lipoxygenases, ligninases, pullulanases, tannases, pentosanases, malanases, B-glucanases, arabinosidases, hyaluronidases, chondroitinases, laccases, and amylases, or any combinations or mixtures thereof. In some embodiments, a combination of enzymes is used (*i.e.*, a "cocktail") comprising conventional applicable enzymes like protease, lipase, cutinase and/or cellulase in conjunction with amylase is used.

In addition to the protease variants provided herein, any other suitable protease finds use in the compositions of the present invention. Suitable proteases include those of animal, vegetable or microbial origin. In some embodiments, microbial proteases are used. In some embodiments, chemically or genetically modified mutants are included. In some embodiments, the protease is a serine protease, preferably an alkaline microbial protease or a trypsin-like protease. Examples of alkaline proteases include subtilisins, especially those derived from *Bacillus* (*e.g.,* subtilisin, *lentus, amyloliquefaciens,* subtilisin Carlsberg, subtilisin 309, subtilisin 147 and subtilisin 168). Additional examples include those mutant proteases described in USPNs RE 34,606, 5,955,340, 5,700,676, 6,312,936, and 6,482,628, all of which are incorporated herein by reference. Additional protease examples include, but are not limited to trypsin (*e.g.,* of porcine or bovine origin), and the *Fusarium* protease described in WO 89/06270. In some embodiments, commercially available protease enzymes that find use in the present invention include, but are not limited to MAXATASE®, MAXACAL™, MAXAPEM™, OPTICLEAN®, OPTIMASE®, PROPERASE®, PURAFECT®, PURAFECT® OXP, PURAMAX™, EXCELLASE™, and PURAFAST™ (Genencor); ALCALASE®, SAVINASE®, PRIMASE®, DURAZYM™, POLARZYME®, OVOZYME®, KANNASE®, LIQUANASE®, NEUTRASE®, RELASE® and ESPERASE® (Novozymes); BLAP™ and BLAP™ variants (Henkel Kommanditgesellschaft auf Aktien, Duesseldorf, Germany), and KAP (B. alkalophilus subtilisin; Kao Corp., Tokyo, Japan). Various proteases are described in WO95/23221, WO92/21760, U.S. Pat. Publ. No. 2008/0090747, and USPNs 5,801,039, 5,340,735, 5,500,364, 5,855,625, US RE 34,606, 5,955,340, 5,700,676, 6,312,936, and 6,482,628, and various other patents. In some further embodiments, serine proteases find use in the present invention, including but not limited to the neutral serine protease described in WO07/044993.

In addition, any suitable lipase finds use in the present invention. Suitable lipases include, but are not limited to those of bacterial or fungal origin. Chemically or genetically modified mutants are encompassed by the present invention. Examples of useful lipases include *H. lanuginosa* lipase (*See e.g.,* EP258068 and EP305216), *R. miehei* lipase *(See e.g.,* EP238023), *Candida* lipase, such as *C*. *antarctica* lipase (*e.g.,* the *C*. *antarctica* lipase A or B; *See e.g.,* EP214761), *Pseudomonas* lipases such as *P. alcaligenes* lipase and *P. pseudoalcaligenes* lipase *(See e.g.,* EP218272), *P. cepacia* lipase (*See e.g.,* EP331376), *P. stutzeri* lipase (*See e.g.,* GB1372034), *P. fluorescens* lipase, *Bacillus* lipase (*e.g., B. subtilis* lipase [Dartois et al., Biochem. Biophys. Acta 1131:253-260 [1993]); *B. stearothermophilus* lipase [*See e.g.,* JP 64/744992]; and *B.pumilus* lipase [*See e.g.,* WO 91/16422]).

Furthermore, a number of cloned lipases find use in some embodiments of the present invention, including but not limited to *P. camembertii* lipase (*See,* Yamaguchi et al., Gene 103:61-67 [1991]), *G*. *candidum* lipase (*See,* Schimada et al., J. Biochem., 106:383-388 [1989]), and various *Rhizopus* lipases such as *R. delemar* lipase (*See,* Hass et al., Gene 109:117-113 [1991]), a *R*. *niveus* lipase (Kugimiya et al., Biosci. Biotech. Biochem. 56:716-719 [1992]) and *R*. *oryzae* lipase.

Other types of lipolytic enzymes such as cutinases also find use in some embodiments of the present invention, including but not limited to the cutinase derived from *Pseudomonas mendocina* (*See,* WO88/09367), and the cutinase derived from *Fusarium solani pisi* (*See*, WO90/09446).

Additional suitable lipases include commercially available lipases such as M1 LIPASE™, LUMA FAST™, and LIPOMAX™ (Genencor); LIPEX®, LIPOLASE® and LIPOLASE® ULTRA (Novozymes); and LIPASE P™ "Amano" (Amano Pharmaceutical Co. Ltd., Japan).

In some embodiments of the present invention, the cleaning compositions of the present invention further comprise lipases at a level from about 0.00001 to about 10% of additional lipase by weight of the composition and the balance of cleaning adjunct materials by weight of composition. In some other embodiments of the present invention, the cleaning compositions of the present invention also comprise lipases at a level of about 0.0001 to about 10%, about 0.001 to about 5%, about 0.001 to about 2%, about 0.005 to about 0.5% lipase by weight of the composition.

In some embodiments of the present invention, any suitable amylase finds use in the present invention. In some embodiments, any amylase (*e*.*g*., alpha and/or beta) suitable for use in alkaline solutions also find use. Suitable amylases include, but are not limited to those of bacterial or fungal origin. Chemically or genetically modified mutants are included in some embodiments. Amylases that find use in the present invention, include, but are not limited to α-amylases obtained from *B*. *licheniformis* (*See e.g.,* GB1296839). Commercially available amylases that find use in the present invention include, but are not limited to DURAMYL®, TERMAMYL®, FUNGAMYL®, STAINZYME®, STAINZYME PLUS®, STAINZYME ULTRA®, and BAN™ (Novozymes), as well as POWERASE™, RAPIDASE® and MAXAMYL® P (Genencor).

In some embodiments of the present invention, the cleaning compositions of the present invention further comprise amylases at a level from about 0.00001 to about 10% of additional amylase by weight of the composition and the balance of cleaning adjunct materials by weight of composition. In some other embodiments of the present invention, the cleaning compositions of the present invention also comprise amylases at a level of about 0.0001 to about 10%, about 0.001 to about 5%, about 0.001 to about 2%, about 0.005 to about 0.5% amylase by weight of the composition.

In some further embodiments, any suitable cellulase finds used in the cleaning compositions of the present invention. Suitable cellulases include, but are not limited to those of bacterial or fungal origin. Chemically or genetically modified mutants are included in some embodiments. Suitable cellulases include, but are not limited to *H. insolens* cellulases (*See e.g.,* USPN 4,435,307). Especially suitable cellulases are the cellulases having color care benefits (*See e.g.,* EP0495257). Commercially available cellulases that find use in the present include, but are not limited to CELLUZYME®, CAREZYME® (Novozymes), and KAC-500(B)™ (Kao Corporation). In some embodiments, cellulases are incorporated as portions or fragments of mature wild-type or variant cellulases, wherein a portion of the N-terminus is deleted (*See e.g.,* USPN 5,874,276). In some embodiments, the cleaning compositions of the present invention further comprise cellulases at a level from about 0.00001 to about 10% of additional cellulase by weight of the composition and the balance of cleaning adjunct materials by weight of composition. In some other embodiments of the present invention, the cleaning compositions of the present invention also comprise cellulases at a level of about 0.0001 to about 10%, about 0.001 to about 5%, about 0.001 to about 2%, about 0.005% to about 0.5% cellulase by weight of the composition.

Any mannanase suitable for use in detergent compositions also finds use in the present invention. Suitable mannanases include, for example, those of bacterial or fungal origin. Chemically or genetically modified mutants are included in some embodiments. Various mannanases are known which find use in the present invention (*See e.g.,* USPNs 6,566,114, 6,602,842, and 6,440,991. In some embodiments, the cleaning compositions of the present invention further comprise mannanases at a level from about 0.00001 to about 10% of additional mannanase by weight of the composition and the balance of cleaning adjunct materials by weight of composition. In some embodiments of the present invention, the cleaning compositions of the present invention also comprise mannanases at a level of about 0.0001 to about 10%, about 0.001 to about 5%, about 0.001 to about 2%, about 0.005 to about 0.5% mannanase by weight of the composition.

In some embodiments, peroxidases are used in combination with hydrogen peroxide or a source thereof (*e*.*g*., a percarbonate, perborate or persulfate) in the compositions of the present invention. In some alternative embodiments, oxidases are used in combination with oxygen. Both types of enzymes are used for "solution bleaching" (*i.e.*, to prevent transfer of a textile dye from a dyed fabric to another fabric when the fabrics are washed together in a wash liquor), preferably together with an enhancing agent (*See e.g.,* WO94/12621 and WO95/01426). Suitable peroxidases/oxidases include, but are not limited to those of plant, bacterial or fungal origin. Chemically or genetically modified mutants are included in some embodiments. In some embodiments, the cleaning compositions of the present invention further comprise peroxidase and/or oxidase enzymes at a level from about 0.00001 to about 10% of additional peroxidase and/or oxidase by weight of the composition and the balance of cleaning adjunct materials by weight of composition. In some other embodiments of the present invention, the cleaning compositions of the present invention also comprise peroxidase and/or oxidase enzymes at a level of about 0.0001 to about 10%, about 0.001 to about 5%, about 0.001 to about 2%, about 0.005 to about 0.5% peroxidase and/or oxidase enzymes by weight of the composition.

In some embodiments, additional enzymes find use, including but not limited to perhydrolases (*See e.g.,* WO05/056782). In addition, in some embodiments, mixtures of the above mentioned enzymes are encompassed herein, in particular one or more additional protease, amylase, lipase, mannanase, and/or at least one cellulase. Indeed, it is contemplated that various mixtures of these enzymes will find use in the present invention. It is also contemplated that the varying levels of the variant protease(s) and one or more additional enzymes may both independently range to about 10%, the balance of the cleaning composition being cleaning adjunct materials. The specific selection of cleaning adjunct materials are readily made by considering the surface, item, or fabric to be cleaned, and the desired form of the composition for the cleaning conditions during use (*e*.*g*., through the wash detergent use).

Examples of suitable cleaning adjunct materials include, but are not limited to, surfactants, builders, bleaches, bleach activators, bleach catalysts, other enzymes, enzyme stabilizing systems, chelants, optical brighteners, soil release polymers, dye transfer agents, dye transfer inhibiting agents, catalytic materials, hydrogen peroxide, sources of hydrogen peroxide, preformed peracids, polymeric dispersing agents, clay soil removal agents, structure elasticizing agents, dispersants, suds suppressors, dyes, perfumes, colorants, filler salts, hydrotropes, photoactivators, fluorescers, fabric conditioners, fabric softeners, carriers, hydrotropes, processing aids, solvents, pigments, hydrolyzable surfactants, preservatives, anti-oxidants, anti-shrinkage agents, anti-wrinkle agents, germicides, fungicides, color speckles, silvercare, anti-tarnish and/or anti-corrosion agents, alkalinity sources, solubilizing agents, carriers, processing aids, pigments, and pH control agents *(See e.g.,* USPNs 6,610,642, 6,605,458, 5,705,464, 5,710,115, 5,698,504, 5,695,679, 5,686,014 and 5,646,101, all of which are incorporated herein by reference). Embodiments of specific cleaning composition materials are exemplified in detail below. In embodiments in which the cleaning adjunct materials are not compatible with the variant proteases of the present invention in the cleaning compositions, then suitable methods of keeping the cleaning adjunct materials and the protease(s) separated (*i.e.*, not in contact with each other) until combination of the two components is appropriate are used. Such separation methods include any suitable method known in the art (*e*.*g*., gelcaps, encapsulation, tablets, physical separation, etc.).

In some embodiments, an effective amount of one or more variant protease(s) provided herein is included in compositions useful for cleaning a variety of surfaces in need of proteinaceous stain removal. Such cleaning compositions include cleaning compositions for such applications as cleaning hard surfaces, fabrics, and dishes. Indeed, in some embodiments, the present invention provides fabric cleaning compositions, while in other embodiments the present invention provides non-fabric cleaning compositions. Notably, the present invention also provides cleaning compositions suitable for personal care, including oral care (including dentrifices, toothpastes, mouthwashes, etc., as well as denture cleaning compositions), skin, and hair cleaning compositions. It is intended that the present invention encompass detergent compositions in any form (*i.e.*, liquid, granular, bar, semi-solid, gels, emulsions, tablets, capsules, etc.).

By way of example, several cleaning compositions wherein the variant proteases of the present invention find use are described in greater detail below. In some embodiments in which the cleaning compositions of the present invention are formulated as compositions suitable for use in laundry machine washing method(s), the compositions of the present invention preferably contain at least one surfactant and at least one builder compound, as well as one or more cleaning adjunct materials preferably selected from organic polymeric compounds, bleaching agents, additional enzymes, suds suppressors, dispersants, lime-soap dispersants, soil suspension and anti-redeposition agents and corrosion inhibitors. In some embodiments, laundry compositions also contain softening agents (*i.e.,* as additional cleaning adjunct materials). The compositions of the present invention also find use detergent additive products in solid or liquid form. Such additive products are intended to supplement and/or boost the performance of conventional detergent compositions and can be added at any stage of the cleaning process. In some embodiments, the density of the laundry detergent compositions herein ranges from about 400 to about 1200 g/liter, while in other embodiments, it ranges from about 500 to about 950 g/liter of composition measured at 20°C.

In embodiments formulated as compositions for use in manual dishwashing methods, the compositions of the invention preferably contain at least one surfactant and preferably at least one additional cleaning adjunct material selected from organic polymeric compounds, suds enhancing agents, group II metal ions, solvents, hydrotropes and additional enzymes.

In some embodiments, various cleaning compositions such as those provided in USPN 6,605,458, find use with the variant proteases of the present invention. Thus, in some embodiments, the compositions comprising at least one variant protease of the present invention is a compact granular fabric cleaning composition, while in other embodiments, the composition is a granular fabric cleaning composition useful in the laundering of colored fabrics, in further embodiments, the composition is a granular fabric cleaning composition which provides softening through the wash capacity, in additional embodiments, the composition is a heavy duty liquid fabric cleaning composition. In some embodiments, the compositions comprising at least one variant protease of the present invention are fabric cleaning compositions such as those described in USPNs 6,610,642 and 6,376,450. In addition, the variant proteases of the present invention find use in granular laundry detergent compositions of particular utility under European or Japanese washing conditions *(See e.g.,* USPN 6,610,642).

In some alternative embodiments, the present invention provides hard surface cleaning compositions comprising at least one variant protease provided herein. Thus, in some embodiments, the compositions comprising at least one variant protease of the present invention is a hard surface cleaning composition such as those described in USPNs 6,610,642, 6,376,450, and 6,376,450.

In yet further embodiments, the present invention provides dishwashing compositions comprising at least one variant protease provided herein. Thus, in some embodiments, the compositions comprising at least one variant protease of the present invention is a hard surface cleaning composition such as those in USPNs 6,610,642 and 6,376,450. In some still further embodiments, the present invention provides dishwashing compositions comprising at least one variant protease provided herein. In some further embodiments, the compositions comprising at least one variant protease of the present invention comprise oral care compositions such as those in USPNs 6,376,450 and 6,376,450. The formulations and descriptions of the compounds and cleaning adjunct materials contained in the aforementioned USPNs 6,376,450, 6,605,458, 6,605,458, and 6,610,642, find use with the variant proteases provided herein.

The cleaning compositions of the present invention are formulated into any suitable form and prepared by any process chosen by the formulator, non-limiting examples of which are described in USPNs 5,879,584, 5,691,297, 5,574,005, 5,569,645, 5,565,422, 5,516,448, 5,489,392, and 5,486,303. When a low pH cleaning composition is desired, the pH of such composition is adjusted via the addition of a material such as monoethanolamine or an acidic material such as HCl.

While not essential for the purposes of the present invention, the non-limiting list of adjuncts illustrated hereinafter are suitable for use in the instant cleaning compositions. In some embodiments, these adjuncts are incorporated for example, to assist or enhance cleaning performance, for treatment of the substrate to be cleaned, or to modify the aesthetics of the cleaning composition as is the case with perfumes, colorants, dyes or the like. It is understood that such adjuncts are in addition to the variant proteases of the present invention. The precise nature of these additional components, and levels of incorporation thereof, will depend on the physical form of the composition and the nature of the cleaning operation for which it is to be used. Suitable adjunct materials include, but are not limited to, surfactants, builders, chelating agents, dye transfer inhibiting agents, deposition aids, dispersants, additional enzymes, and enzyme stabilizers, catalytic materials, bleach activators, bleach boosters, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids and/or pigments. In addition to the disclosure below, suitable examples of such other adjuncts and levels of use are found in USPNs 5,576,282, 6,306,812, and 6,326,348. The aforementioned adjunct ingredients may constitute the balance of the cleaning compositions of the present invention.

In some embodiments, the cleaning compositions according to the present invention comprise at least one surfactant and/or a surfactant system wherein the surfactant is selected from nonionic surfactants, anionic surfactants, cationic surfactants, ampholytic surfactants, zwitterionic surfactants, semi-polar nonionic surfactants and mixtures thereof. In some low pH cleaning composition embodiments (e.g., compositions having a neat pH of from about 3 to about 5), the composition typically does not contain alkyl ethoxylated sulfate, as it is believed that such surfactant may be hydrolyzed by such compositions the acidic contents. In some embodiments, the surfactant is present at a level of from about 0.1 to about 60%, while in alternative embodiments the level is from about 1 to about 50%, while in still further embodiments the level is from about 5 to about 40%, by weight of the cleaning composition.

In some embodiments, the cleaning compositions of the present invention comprise one or more detergent builders or builder systems. In some embodiments incorporating at least one builder, the cleaning compositions comprise at least about 1%, from about 3 to about 60% or even from about 5 to about 40% builder by weight of the cleaning composition. Builders include, but are not limited to, the alkali metal, ammonium and alkanolammonium salts of polyphosphates, alkali metal silicates, alkaline earth and alkali metal carbonates, aluminosilicates, polycarboxylate compounds, ether hydroxypolycarboxylates, copolymers of maleic anhydride with ethylene or vinyl methyl ether, 1, 3, 5-trihydroxy benzene-2, 4, 6-trisulphonic acid, and carboxymethyloxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, citric acid, oxydisuccinic acid, polymaleic acid, benzene 1,3,5-tricarboxylic acid, carboxymethyloxysuccinic acid, and soluble salts thereof. Indeed, it is contemplated that any suitable builder will find use in various embodiments of the present invention.

In some embodiments, the builders form water-soluble hardness ion complexes (e.g., sequestering builders), such as citrates and polyphosphates (e.g., sodium tripolyphosphate and sodium tripolyphospate hexahydrate, potassium tripolyphosphate, and mixed sodium and potassium tripolyphosphate, etc.). It is contemplated that any suitable builder will find use in the present invention, including those known in the art *(See e.g.,* EP2100949).

In some embodiments, the cleaning compositions of the present invention contain at least one chelating agent. Suitable chelating agents include, but are not limited to copper, iron and/or manganese chelating agents and mixtures thereof. In embodiments in which at least one chelating agent is used, the cleaning compositions of the present invention comprise from about 0.1 to about 15% or even from about 3.0 to about 10% chelating agent by weight of the subject cleaning composition.

In some still further embodiments, the cleaning compositions provided herein contain at least one deposition aid. Suitable deposition aids include, but are not limited to, polyethylene glycol, polypropylene glycol, polycarboxylate, soil release polymers such as polytelephthalic acid, clays such as kaolinite, montmorillonite, atapulgite, illite, bentonite, halloysite, and mixtures thereof.

As indicated herein, in some embodiments, anti-redeposition agents find use in some embodiments of the present invention. In some embodiments, non-ionic surfactants find use. For example, in automatic dishwashing embodiments, non-ionic surfactants find use for surface modification purposes, in particular for sheeting, to avoid filming and spotting and to improve shine. These non-ionic surfactants also find use in preventing the re-deposition of soils. In some embodiments, the anti-redeposition agent is a non-ionic surfactant as known in the art *(See e.g.,* EP2100949).

In some embodiments, the cleaning compositions of the present invention include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. In embodiments in which at least one dye transfer inhibiting agent is used, the cleaning compositions of the present invention comprise from about 0.0001 to about 10%, from about 0.01 to about 5%, or even from about 0.1 to about 3% by weight of the cleaning composition.

In some embodiments, silicates are included within the compositions of the present invention. In some such embodiments, sodium silicates (*e*.*g*., sodium disilicate, sodium metasilicate, and crystalline phyllosilicates) find use. In some embodiments, silicates are present at a level of from about 1 to about 20%. In some embodiments, silicates are present at a level of from about 5 to about 15% by weight of the composition.

In some still additional embodiments, the cleaning compositions of the present invention also contain dispersants. Suitable water-soluble organic materials include, but are not limited to the homo- or co-polymeric acids or their salts, in which the polycarboxylic acid comprises at least two carboxyl radicals separated from each other by not more than two carbon atoms.

In some further embodiments, the enzymes used in the cleaning compositions are stabilized by any suitable technique. In some embodiments, the enzymes employed herein are stabilized by the presence of water-soluble sources of calcium and/or magnesium ions in the finished compositions that provide such ions to the enzymes. In some embodiments, the enzyme stabilizers include oligosaccharides, polysaccharides, and inorganic divalent metal salts, including alkaline earth metals, such as calcium salts. It is contemplated that various techniques for enzyme stabilization will find use in the present invention. For example, in some embodiments, the enzymes employed herein are stabilized by the presence of water-soluble sources of zinc (II), calcium (II) and/or magnesium (II) ions in the finished compositions that provide such ions to the enzymes, as well as other metal ions (*e*.*g*., barium (II), scandium (II), iron (II), manganese (II), aluminum (III), Tin (II), cobalt (II), copper (II), nickel (II), and oxovanadium (IV). Chlorides and sulfates also find use in some embodiments of the present invention. Examples of suitable oligosaccharides and polysaccharides (*e*.*g*., dextrins) are known in the art (*See e.g.,* WO07/145964). In some embodiments, reversible protease inhibitors also find use, such as boron-containing compounds (*e*.*g*., borate, 4-formyl phenyl boronic acid) and/or a tripeptide aldehyde find use to further improve stability, as desired.

In some embodiments, bleaches, bleach activators and/or bleach catalysts are present in the compositions of the present invention. In some embodiments, the cleaning compositions of the present invention comprise inorganic and/or organic bleaching compound(s). Inorganic bleaches include, but are not limited to perhydrate salts (*e*.*g*., perborate, percarbonate, perphosphate, persulfate, and persilicate salts). In some embodiments, inorganic perhydrate salts are alkali metal salts. In some embodiments, inorganic perhydrate salts are included as the crystalline solid, without additional protection, although in some other embodiments, the salt is coated. Any suitable salt known in the art finds use in the present invention *(See e.g.,* EP2100949).

In some embodiments, bleach activators are used in the compositions of the present invention. Bleach activators are typically organic peracid precursors that enhance the bleaching action in the course of cleaning at temperatures of 60°C and below. Bleach activators suitable for use herein include compounds which, under perhydrolysis conditions, give aliphatic peroxoycarboxylic acids having preferably from about 1 to about 10 carbon atoms, in particular from about 2 to about 4 carbon atoms, and/or optionally substituted perbenzoic acid. Additional bleach activators are known in the art and find use in the present invention (*See e.g.,* EP2100949).

In addition, in some embodiments and as further described herein, the cleaning compositions of the present invention further comprise at least one bleach catalyst. In some embodiments, the manganese triazacyclononane and related complexes find use, as well as cobalt, copper, manganese, and iron complexes. Additional bleach catalysts find use in the present invention *(See e.g.,* USPNs 4,246,612, 5,227,084, and 4,810410, and WO 99/06521 and EP2100949).

In some embodiments, the cleaning compositions of the present invention contain one or more catalytic metal complexes. In some embodiments, a metal-containing bleach catalyst finds use. In some embodiments, the metal bleach catalyst comprises a catalyst system comprising a transition metal cation of defined bleach catalytic activity, (*e*.*g*., copper, iron, titanium, ruthenium, tungsten, molybdenum, or manganese cations), an auxiliary metal cation having little or no bleach catalytic activity (*e*.*g*., zinc or aluminum cations), and a sequestrate having defined stability constants for the catalytic and auxiliary metal cations, particularly ethylenediaminetetraacetic acid, ethylenediaminetetra (methylenephosphonic acid) and water-soluble salts thereof are used (*See e.g.,* USPN 4,430,243). In some embodiments, the cleaning compositions of the present invention are catalyzed by means of a manganese compound. Such compounds and levels of use are well known in the art (*See e.g.,* USPN 5,576,282). In additional embodiments, cobalt bleach catalysts find use in the cleaning compositions of the present invention. Various cobalt bleach catalysts are known in the art (*See e.g.,* USPN 5,597,936 and 5,595,967) and are readily prepared by known procedures.

In some additional embodiments, the cleaning compositions of the present invention include a transition metal complex of a macropolycyclic rigid ligand (MRL). As a practical matter, and not by way of limitation, in some embodiments, the compositions and cleaning processes provided by the present invention are adjusted to provide on the order of at least one part per hundred million of the active MRL species in the aqueous washing medium, and in some embodiments, provide from about 0.005 ppm to about 25 ppm, more preferably from about 0.05 ppm to about 10 ppm, and most preferably from about 0.1 ppm to about 5 ppm, of the MRL in the wash liquor.

In some embodiments, transition-metals in the instant transition-metal bleach catalyst include, but are not limited to manganese, iron and chromium. MRLs also include, but are not limited to special ultra-rigid ligands that are cross-bridged (*e*.*g*., 5,12-diethyl-1,5,8,12-tetraazabicyclo[6.6.2]hexadecane). Suitable transition metal MRLs are readily prepared by known procedures (*See e.g.*, WO 2000/32601, and US Patent No. 6,225,464).

In some embodiments, the cleaning compositions of the present invention comprise metal care agents. Metal care agents find use in preventing and/or reducing the tarnishing, corrosion, and/or oxidation of metals, including aluminum, stainless steel, and non-ferrous metals (e.g., silver and copper). Suitable metal care agents include those described in EP2100949, WO9426860 and WO9426859). In some embodiments, the metal care agent is a zinc salt. In some further embodiments, the cleaning compositions of the present invention comprise from about 0.1% to about 5% by weight of one or more metal care agent.

In some embodiments, the cleaning composition is a high density liquid (HDL) composition having a variant LG12 protease. The HDL liquid laundry detergent can comprise a detersive surfactant (10-40%) comprising anionic detersive surfactant (selected from a group of linear or branched or random chain, substituted or unsubstituted alkyl sulphates, alkyl sulphonates, alkyl alkoxylated sulphate, alkyl phosphates, alkyl phosphonates, alkyl carboxylates, and/or mixtures thereof); and optionally non-ionic surfactant (selected from a group of linear or branched or random chain, substituted or unsubstituted alkyl alkoxylated alcohol, for example a C₈-C₁₈ alkyl ethoxylated alcohol and/or C₆-C₁₂ alkyl phenol alkoxylates), optionally wherein the weight ratio of anionic detersive surfactant (with a hydrophilic index (HIc) of from 6.0-9) to non-ionic detersive surfactant is greater than 1:1.

The composition can comprise optionally, a surfactancy boosting polymer consisting of amphiphilic alkoxylated grease cleaning polymers (selected from a group of alkoxylated polymers having branched hydrophilic and hydrophobic properties, such as alkoxylated polyalkylenimines in the range of 0.05-10wt%) and/or random graft polymers (typically comprising of hydrophilic backbone comprising monomers selected from the group consisting of: unsaturated C₁-C₆ carboxylic acids, ethers, alcohols, aldehydes, ketones, esters, sugar units, alkoxy units, maleic anhydride, saturated polyalcohols such as glycerol, and mixtures thereof; and hydrophobic side chain(s) selected from the group consisting of: C₄-C₂₅ alkyl group, polypropylene, polybutylene, vinyl ester of a saturated C₁-C₆ mono-carboxylic acid, C₁-C₆ alkyl ester of acrylic or methacrylic acid, and mixtures thereof.

The composition can comprise additional polymers such as soil release polymers (include anionically end-capped polyesters, for example SRP1, polymers comprising at least one monomer unit selected from saccharide, dicarboxylic acid, polyol and combinations thereof, in random or block configuration, ethylene terephthalate-based polymers and co-polymers thereof in random or block configuration, for example Repel-o-tex SF, SF-2 and SRP6, Texcare SRA100, SRA300, SRN100, SRN170, SRN240, SRN300 and SRN325, Marloquest SL), anti-redeposition polymers (0.1 wt% to 10wt%, include carboxylate polymers, such as polymers comprising at least one monomer selected from acrylic acid, maleic acid (or maleic anhydride), fumaric acid, itaconic acid, aconitic acid, mesaconic acid, citraconic acid, methylenemalonic acid, and any mixture thereof, vinylpyrrolidone homopolymer, and/or polyethylene glycol, molecular weight in the range of from 500 to 100,000 Da); cellulosic polymer (including those selected from alkyl cellulose, alkyl alkoxyalkyl cellulose, carboxyalkyl cellulose, alkyl carboxyalkyl cellulose examples of which include carboxymethyl cellulose, methyl cellulose, methyl hydroxyethyl cellulose, methyl carboxymethyl cellulose, and mixures thereof) and polymeric carboxylate (such as maleate/acrylate random copolymer or polyacrylate homopolymer).

The composition can further comprise saturated or unsaturated fatty acid, preferably saturated or unsaturated C₁₂-C₂₄ fatty acid (0-10 wt%); deposition aids (examples for which include polysaccharides, preferably cellulosic polymers, poly diallyl dimethyl ammonium halides (DADMAC), and co-polymers of DAD MAC with vinyl pyrrolidone, acrylamides, imidazoles, imidazolinium halides, and mixtures thereof, in random or block configuration, cationic guar gum, cationic cellulose such as cationic hydoxyethyl cellulose, cationic starch, cationic polyacylamides, and mixtures thereof.

The composition can further comprise dye transfer inhibiting agents examples of which include manganese phthalocyanine, peroxidases, polyvinylpyrrolidone polymers, polyamine N-oxide polymers, copolymers of N-vinylpyrrolidone and N-vinylimidazole, polyvinyloxazolidones and polyvinylimidazoles and/or mixtures thereof; chelating agents examples of which include ethylenediamine-tetraacetic acid (EDTA); diethylene triamine penta methylene phosphonic acid (DTPMP); hydroxy-ethane diphosphonic acid (HEDP); ethylenediamine N,N'-disuccinic acid (EDDS); methyl glycine diacetic acid (MGDA); diethylene triamine penta acetic acid (DTPA); propylene diamine tetracetic acid (PDT A); 2-hydroxypyridine-N-oxide (HPNO); or methyl glycine diacetic acid (MGDA); glutamic acid N,N-diacetic acid (N,N-dicarboxymethyl glutamic acid tetrasodium salt (GLDA); nitrilotriacetic acid (NTA); 4,5-dihydroxy-m-benzenedisulfonic acid; citric acid and any salts thereof; N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP) and derivatives thereof.

The composition can further comprise enzymes (0.01-0.03wt% active enzyme) selected from a group of proteases; amylases; lipases; cellulases; choline oxidases; peroxidases/oxidases; pectate lyases; mannanases; cutinases; laccases; phospholipases; lysophospholipases; acyltransferase; perhydrolase; arylesterase and any mixture thereof. The composition may comprise an enzyme stabilizer (examples of which include polyols such as propylene glycol or glycerol, sugar or sugar alcohol, lactic acid, reversible protease inhibitor, boric acid, or a boric acid derivative, e.g., an aromatic borate ester, or a phenyl boronic acid derivative such as 4-formylphenyl boronic acid).

The composition can further comprise silicone or fatty-acid based suds suppressors; heuing dyes, calcium and magnesium cations, visual signaling ingredients, anti-foam (0.001-4.0wt%), and/or structurant/thickener (0.01-5wt%, selected from the group consisting of diglycerides and triglycerides, ethylene glycol distearate, microcrystalline cellulose, cellulose based materials, microfiber cellulose, biopolymers, xanthan gum, gellan gum, and mixtures thereof).

Suitable detersive surfactants also include cationic detersive surfactants (selected from a group of alkyl pyridinium compounds, alkyl quarternary ammonium compounds, alkyl quarternary phosphonium compounds, alkyl ternary sulphonium compounds, and/or mixtures thereof); zwitterionic and/or amphoteric detersive surfactants (selected from a group of alkanolamine sulpho-betaines); ampholytic surfactants; semi-polar non-ionic surfactants and mixtures thereof.

The composition can be any liquid form, for example a liquid or gel form, or any combination thereof. The composition may be in any unit dose form, for example a pouch.

In some embodiments, the cleaning composition is a HDD composition having a variant LG12 protease. The HDD laundry detergent can comprise a detersive surfactant including anionic detersive surfactants (selected from a group of linear or branched or random chain, substituted or unsubstituted alkyl sulphates, alkyl sulphonates, alkyl alkoxylated sulphate, alkyl phosphates, alkyl phosphonates, alkyl carboxylates and/or mixtures thereof), non-ionic detersive surfactant (selected from a group of linear or branched or random chain, substituted or unsubstituted C₈-C₁₈ alkyl ethoxylates, and/or C₆-C₁₂ alkyl phenol alkoxylates), cationic detersive surfactants (selected from a group of alkyl pyridinium compounds, alkyl quaternary ammonium compounds, alkyl quaternary phosphonium compounds, alkyl ternary sulphonium compounds, and mixtures thereof), zwitterionic and/or amphoteric detersive surfactants (selected from a group of alkanolamine sulpho-betaines); ampholytic surfactants; semi-polar non-ionic surfactants and mixtures thereof; builders (phosphate free builders [for example zeolite builders examples of which include zeolite A, zeolite X, zeolite P and zeolite MAP in the range of 0 to less than 10 wt%]; phosphate builders [examples of which include sodium tri-polyphosphate in the range of 0 to less than 10wt%]; citric acid, citrate salts and nitrilotriacetic acid or salt thereof in the range of less than 15 wt%); silicate salt (sodium or potassium silicate or sodium meta-silicate in the range of 0 to less than 10wt%, or layered silicate (SKS-6)); carbonate salt (sodium carbonate and/or sodium bicarbonate in the range of 0 to less than 10 wt%); and bleaching agents (photobleaches, examples of which include sulfonated zinc phthalocyanines, sulfonated aluminum phthalocyanines, xanthenes dyes, and mixtures thereof; hydrophobic or hydrophilic bleach activators (examples of which include dodecanoyl oxybenzene sulfonate, decanoyl oxybenzene sulfonate, decanoyl oxybenzoic acid or salts thereof, 3,5,5-trimethy hexanoyl oxybenzene sulfonate, tetraacelyl ethylene diamine-TAED, and nonanoyloxybenzene sulfonate-NOBS, nitrile quats, and mixtures thereof; hydrogen peroxide; sources of hydrogen peroxide (inorganic perhydrate salts examples of which include mono or tetra hydrate sodium salt of perborate, percarbonate, persulfate, perphosphate, or persilicate); preformed hydrophilic and/or hydrophobic peracids (selected from a group consisting of percarboxylic acids and salts, percarbonic acids and salts, perimidic acids and salts, peroxymonosulfuric acids and salts) & mixtures thereof and/or bleach catalyst (such as imine bleach boosters examples of which include iminium cations and polyions; iminium zwitterions; modified amines; modified amine oxides; N-sulphonyl imines; N-phosphonyl imines; N-acyl imines; thiadiazole dioxides; perfluoroimines; cyclic sugar ketones and mixtures thereof; metal-containing bleach catalyst for example copper, iron, titanium, ruthenium, tungsten, molybdenum, or manganese cations along with an auxiliary metal cations such as zinc or aluminum and a sequestrate such as ethylenediaminetetraacetic acid, ethylenediaminetetra(methylenephosphonic acid) and water-soluble salts thereof).

The composition can further comprise enzymes selected from a group of proteases; amylases; lipases; cellulases; choline oxidases; peroxidases/oxidases; pectate lyases; mannanases; cutinases; laccases; phospholipases; lysophospholipases; acyltransferase; perhydrolase; arylesterase and any mixture thereof.

The composition can further comprise additional detergent ingredients including perfume microcapsules, starch encapsulated perfume accord, hueing agents, additional polymers including fabric integrity and cationic polymers, dye lock ingredients, fabric-softening agents, brighteners (for example C.I. Fluorescent brighteners), flocculating agents, chelating agents, alkoxylated polyamines, fabric deposition aids, and/or cyclodextrin.

In some embodiments, the cleaning composition is an automatic dishwashing (ADW) detergent composition having a variant LG12 protease. The ADW detergent can comprise two or more non-ionic surfactants selected from a group of ethoxylated non-ionic surfactants, alcohol alkoxylated surfactants, epoxy-capped poly(oxyalkylated) alcohols, or amine oxide surfactants present in amounts from 0-10% by weight; builders in the range of 5-60% comprising either phosphate (mono-phosphates, di-phosphates, tri-polyphosphates or oligomeric-poylphosphates, preferred sodium tripolyphosphate-STPP or phosphate-free builders [amino acid based compounds, examples of which include MGDA (methyl-glycine-diacetic acid), and salts and derivatives thereof, GLDA (glutamic-N,Ndiacetic acid) and salts and derivatives thereof, IDS (iminodisuccinic acid) and salts and derivatives thereof, carboxy methyl inulin and salts and derivatives thereof and mixtures thereof, nitrilotriacetic acid (NTA), diethylene triamine penta acetic acid (DTPA), B-alaninediacetic acid (B-ADA) and their salts], homopolymers and copolymers of polycarboxylic acids and their partially or completely neutralized salts, monomeric polycarboxylic acids and hydroxycarboxylic acids and their salts in the range of 0.5-50% by weight; sulfonated/carboxylated polymers (provide dimensional stability to the product) in the range of about 0.1 to about 50% by weight; drying aids in the range of about 0.1 to about 10% by weight (selected from polyesters, especially anionic polyesters optionally together with further monomers with 3 to 6 functionalities which are conducive to polycondensation, specifically acid, alcohol or ester functionalities, polycarbonate-, polyurethane- and/or polyurea-polyorganosiloxane compounds or precursor compounds thereof of the reactive cyclic carbonate and urea type); silicates in the range from about 1 to about 20% by weight (sodium or potassium silicates for example sodium disilicate, sodium meta-silicate and crystalline phyllosilicates); bleach-inorganic (for example perhydrate salts such as perborate, percarbonate, perphosphate, persulfate and persilicate salts) and organic (for example organic peroxyacids including diacyl and tetraacylperoxides, especially diperoxydodecanedioc acid, diperoxytetradecanedioc acid, and diperoxyhexadecanedioc acid); bleach activators- organic peracid precursors in the range from about 0.1 to about 10% by weight; bleach catalysts (selected from manganese triazacyclononane and related complexes, Co, Cu, Mn and Fe bispyridylamine and related complexes, and pentamine acetate cobalt(III) and related complexes); metal care agents in the range from about 0.1 to 5% by weight (selected from benzatriazoles, metal salts and complexes, and/or silicates); enzymes in the range from about 0.01 to 5.0mg of active enzyme per gram of automatic dishwashing detergent composition (selected from a group of proteases; amylases; lipases; cellulases; choline oxidases; peroxidases/oxidases; pectate lyases; mannanases; cutinases; laccases; phospholipases; lysophospholipases; acyltransferase; perhydrolase; arylesterase and any mixture thereof); and enzyme stabilizer components (selected from oligosaccharides, polysaccharides and inorganic divalent metal salts).

Representative detergent formulations that beneficially include a serine protease polypeptide of the present invention include the detergent formulations found in WO2013063460, pages 78-152, and in particular the tables of pages 94-152 are hereby incorporated by reference. The serine proteases are normally incorporated into the detergent composition at a level of from 0.00001-10% of enzyme protein by weight of the composition. In some embodiments, the detergent composition comprises more than 0.0001%, 0.001%, 0.01%, or 0.1% of the serine protease by weight of the composition. In some embodiments, the detergent composition comprises less than 1%, 0.1%, 0.01%, or 0.001% of the serine protease by weight of the composition.

The cleaning compositions of the present invention are formulated into any suitable form and prepared by any process chosen by the formulator, non-limiting examples of which are described in USPNs 5,879,584, 5,691,297, 5,574,005, 5,569,645, 5,516,448, 5,489,392, and 5,486,303, all of which are incorporated herein by reference. In some embodiments in which a low pH cleaning composition is desired, the pH of such composition is adjusted via the addition of an acidic material such as HCl.

The cleaning compositions disclosed herein of find use in cleaning a *situs* (*e.g.,* a surface, item, dishware, or fabric). Typically, at least a portion of the situs is contacted with an embodiment of the present cleaning composition, in neat form or diluted in wash liquor, and then the situs is optionally washed and/or rinsed. For purposes of the present invention, "washing" includes but is not limited to, scrubbing, and mechanical agitation. In some embodiments, the cleaning compositions are typically employed at concentrations of from about 500 ppm to about 15,000 ppm in solution. When the wash solvent is water, the water temperature typically ranges from about 5°C to about 90°C and, when the situs comprises a fabric, the water to fabric mass ratio is typically from about 1:1 to about 30:1.

The cleaning compositions of the present invention are formulated into any suitable form and prepared by any suitable process chosen by the formulator, (*See e.g.,* USPNs 5,879,584, 5,691,297, 5,574,005, 5,569,645, 5,565,422, 5,516,448, 5,489,392, 5,486,303, 4,515,705, 4,537,706, 4,515,707, 4,550,862, 4,561,998, 4,597,898, 4,968,451, 5,565,145, 5,929,022, 6,294,514 and 6,376,445).

In some embodiments, the cleaning compositions of the present invention are provided in unit dose form, including tablets, capsules, sachets, pouches, and multi-compartment pouches. In some embodiments, the unit dose format is designed to provide controlled release of the ingredients within a multi-compartment pouch (or other unit dose format). Suitable unit dose and controlled release formats are known in the art *(See e.g.,* EP 2100949, WO02/102955, USPNs 4,765,916 and 4,972,017, and WO 04/111178 for materials suitable for use in unit dose and controlled release formats). In some embodiments, the unit dose form is provided by tablets wrapped with a water-soluble film or water-soluble pouches. Various formats for unit doses are provided in EP2100947, and are known in the art.

In some embodiments, the cleaning compositions of the present invention find use in cleaning surfaces (*e*.*g*., dishware), laundry, hard surfaces, contact lenses, etc. In some embodiments, at least a portion of the surface is contacted with at least one embodiment of the cleaning compositions of the present invention, in neat form or diluted in wash liquor, and then the surface is optionally washed and/or rinsed. For purposes of the present invention, "washing" includes, but is not limited to, scrubbing, and mechanical washing. In some embodiments, the cleaning compositions of the present invention are used at concentrations of from about 500 ppm to about 15,000 ppm in solution. In some embodiments in which the wash solvent is water, the water temperature typically ranges from about 5 °C to about 90 °C.

The present invention provides methods for cleaning or washing an item or surface (*e*.*g*., hard surface) in need of cleaning, including, but not limited to methods for cleaning or washing a dishware item, a tableware item, a fabric item, a laundry item, personal care item, etc., or the like, and methods for cleaning or washing a hard or soft surface (*e*.*g*., a hard surface of an item).

In some embodiments, the present invention provides a method for cleaning an item, object, or surface in need of cleaning, the method comprising contacting the item or surface (or a portion of the item or surface desired to be cleaned) with at least one variant LG12 protease of the present invention or a composition of the present invention for a sufficient time and/or under conditions suitable and/or effective to clean the item, object, or surface to a desired degree. Some such methods further comprise rinsing the item, object, or surface with water. For some such methods, the cleaning composition is a dishwashing detergent composition and the item or object to be cleaned is a dishware item or tableware item. As used herein, a "dishware item" is an item generally used in serving or eating food. A dishware item can be, but is not limited to for example, a dish, plate, cup, bowl, etc., and the like. As used herein, "tableware" is a broader term that includes, but is not limited to for example, dishes, cutlery, knives, forks, spoons, chopsticks, glassware, pitchers, sauce boats, drinking vessels, serving items, etc. It is intended that "tableware item" includes any of these or similar items for serving or eating food. For some such methods, the cleaning composition is an automatic dishwashing detergent composition or a hand dishwashing detergent composition and the item or object to be cleaned is a dishware or tableware item. For some such methods, the cleaning composition is a laundry detergent composition (*e*.*g*., a power laundry detergent composition or a liquid laundry detergent composition), and the item to be cleaned is a fabric item. In some other embodiments, the cleaning composition is a laundry pre-treatment composition.

In some embodiments, the present invention provides methods for cleaning or washing a fabric item optionally in need of cleaning or washing, respectively. In some embodiments, the methods comprise providing a composition comprising the variant protease, including but not limited to fabric or laundry cleaning composition, and a fabric item or laundry item in need of cleaning, and contacting the fabric item or laundry item (or a portion of the item desired to be cleaned) with the composition under conditions sufficient or effective to clean or wash the fabric or laundry item to a desired degree.

In some embodiments, the present invention provides a method for cleaning or washing an item or surface (*e*.*g*., hard surface) optionally in need of cleaning, the method comprising providing an item or surface to be cleaned or washed and contacting the item or surface (or a portion of the item or surface desired to be cleaned or washed) with at least one LG12 variant of the invention or a composition of the invention comprising at least one such LG12 variant for a sufficient time and/or under conditions sufficient or effective to clean or wash the item or surface to a desired degree. Such compositions include, but are not limited to for example, a cleaning composition or detergent composition of the invention (*e*.*g*., a hand dishwashing detergent composition, hand dishwashing cleaning composition, laundry detergent or fabric detergent or laundry or fabric cleaning composition, liquid laundry detergent, liquid laundry cleaning composition, powder laundry detergent composition, powder laundry cleaning composition, automatic dishwashing detergent composition, laundry booster cleaning or detergent composition, laundry cleaning additive, and laundry pre-spotter composition, etc.). In some embodiments, the method is repeated one or more times, particularly if additional cleaning or washing is desired. For example, in some instance, the method optionally further comprises allowing the item or surface to remain in contact with the at least one variant protease or composition for a period of time sufficient or effective to clean or wash the item or surface to the desired degree. In some embodiments, the methods further comprise rinsing the item or surface with water and/or another liquid. In some embodiments, the methods further comprise contacting the item or surface with at least one variant protease of the invention or a composition of the invention again and allowing the item or surface to remain in contact with the at least one variant protease or composition for a period of time sufficient to clean or wash the item or surface to the desired degree. In some embodiments, the cleaning composition is a dishwashing detergent composition and the item to be cleaned is a dishware or tableware item. In some embodiments of the present methods, the cleaning composition is an automatic dishwashing detergent composition or a hand dishwashing detergent composition and the item to be cleaned is a dishware or tableware item. In some embodiments of the methods, the cleaning composition is a laundry detergent composition and the item to be cleaned is a fabric item.

The present invention also provides methods of cleaning a tableware or dishware item in an automatic dishwashing machine, the method comprising providing an automatic dishwashing machine, placing an amount of an automatic dishwashing composition comprising at least one LG12 variant of the present invention or a composition of the invention sufficient to clean the tableware or dishware item in the machine (*e*.*g*., by placing the composition in an appropriate or provided detergent compartment or dispenser in the machine), putting a dishware or tableware item in the machine, and operating the machine so as to clean the tableware or dishware item (*e*.*g*., as per the manufacturer's instructions). In some embodiments, the methods include any automatic dishwashing composition described herein, which comprises, but is not limited to at least one LG12 variant provided herein. The amount of automatic dishwashing composition to be used can be readily determined according to the manufacturer's instructions or suggestions and any form of automatic dishwashing composition comprising at least one variant protease of the invention (*e*.*g*., liquid, powder, solid, gel, tablet, etc.), including any described herein, may be employed.

The present invention also provides methods for cleaning a surface, item or object optionally in need of cleaning, the method comprises contacting the item or surface (or a portion of the item or surface desired to be cleaned) with at least one variant LG12 of the present invention or a cleaning composition of the invention in neat form or diluted in a wash liquor for a sufficient time and/or under conditions sufficient or effective to clean or wash the item or surface to a desired degree. The surface, item, or object may then be (optionally) washed and/or rinsed if desired. For purposes of the present invention, "washing" includes, but is not limited to for example, scrubbing and mechanical agitation. In some embodiments, the cleaning compositions are employed at concentrations of from about 500 ppm to about 15,000 ppm in solution (*e*.*g*., aqueous solution). When the wash solvent is water, the water temperature typically ranges from about 5°C to about 90°C.

The present invention also provides methods of cleaning a laundry or fabric item in an washing machine, the method comprising providing an washing machine, placing an amount of a laundry detergent composition comprising at least one variant LG12 of the invention sufficient to clean the laundry or fabric item in the machine (*e*.*g*., by placing the composition in an appropriate or provided detergent compartment or dispenser in the machine), placing the laundry or fabric item in the machine, and operating the machine so as to clean the laundry or fabric item (*e*.*g*., as per the manufacturer's instructions). The methods of the present invention include any laundry washing detergent composition described herein, comprising but not limited to at least one of any variant LG12 provided herein. The amount of laundry detergent composition to be used can be readily determined according to manufacturer's instructions or suggestions and any form of laundry detergent composition comprising at least one variant protease of the invention (*e*.*g*., solid, powder, liquid, tablet, gel, etc.), including any described herein, may be employed.

In a further aspect of the invention, the LG-12 clade protease polypeptides of the present invention can be used as a component of an animal feed composition, animal feed additive and/or pet food comprising a LG-12 clade protease and variants thereof. The present invention further relates to a method for preparing such an animal feed composition, animal feed additive composition and/or pet food comprising mixing the LG-12 clade protease polypeptide with one or more animal feed ingredients and/or animal feed additive ingredients and/or pet food ingredients. Furthermore, the present invention relates to the use of the LG-12 clade protease polypeptide in the preparation of an animal feed composition and/or animal feed additive composition and/or pet food.

The term "animal" includes all non-ruminant and ruminant animals. In a particular embodiment, the animal is a non-ruminant animal, such as a horse and a mono-gastric animal. Examples of mono-gastric animals include, but are not limited to, pigs and swine, such as piglets, growing pigs, sows; poultry such as turkeys, ducks, chicken, broiler chicks, layers; fish such as salmon, trout, tilapia, catfish and carps; and crustaceans such as shrimps and prawns. In a further embodiment the animal is a ruminant animal including, but not limited to, cattle, young calves, goats, sheep, giraffes, bison, moose, elk, yaks, water buffalo, deer, camels, alpacas, llamas, antelope, pronghorn and nilgai.

In the present context, it is intended that the term "pet food" is understood to mean a food for a household animal such as, but not limited to, dogs, cats, gerbils, hamsters, chinchillas, fancy rats, guinea pigs; avian pets, such as canaries, parakeets, and parrots; reptile pets, such as turtles, lizards and snakes; and aquatic pets, such as tropical fish and frogs.

The terms "animal feed composition," "feedstuff" and "fodder" are used interchangeably and can comprise one or more feed materials selected from the group comprising a) cereals, such as small grains (e.g., wheat, barley, rye, oats and combinations thereof) and/or large grains such as maize or sorghum; b) by products from cereals, such as corn gluten meal, Distillers Dried Grain Solubles (DDGS) (particularly corn based Distillers Dried Grain Solubles (cDDGS), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone meal, potato protein, whey, copra, sesame; d) oils and fats obtained from vegetable and animal sources; e) minerals and vitamins.

Also contemplated are compositions and methods of treating fabrics (*e*.*g*., to desize a textile) using a LG-12 clade protease polypeptide of the present invention. Fabric-treating methods are well known in the art (*see*, *e.g.,* USPN 6,077,316). For example, the feel and appearance of a fabric can be improved by a method comprising contacting the fabric with a LG-12 clade protease in a solution. The fabric can be treated with the solution under pressure.

A LG-12 clade protease of the present invention can be applied during or after the weaving of a textile, or during the desizing stage, or one or more additional fabric processing steps. During the weaving of textiles, the threads are exposed to considerable mechanical strain. Prior to weaving on mechanical looms, warp yarns are often coated with sizing starch or starch derivatives to increase their tensile strength and to prevent breaking. A LG-12 clade protease of the present invention can be applied during or after the weaving to remove starch or starch derivatives. After weaving, the LG-12 clade protease can be used to remove the size coating before further processing the fabric to ensure a homogeneous and wash-proof result.

A LG-12 clade protease of the present invention can be used alone or with other desizing chemical reagents and/or desizing enzymes to desize fabrics, including cotton-containing fabrics, as detergent additives, e.g., in aqueous compositions. An amylase also can be used in compositions and methods for producing a stonewashed look on indigo-dyed denim fabric and garments. For the manufacture of clothes, the fabric can be cut and sewn into clothes or garments, which are afterwards finished. In particular, for the manufacture of denim jeans, different enzymatic finishing methods have been developed. The finishing of denim garment normally is initiated with an enzymatic desizing step, during which garments are subjected to the action of proteolytic enzymes to provide softness to the fabric and make the cotton more accessible to the subsequent enzymatic finishing steps. The LG-12 clade protease can be used in methods of finishing denim garments (*e*.*g*., a "bio-stoning process"), enzymatic desizing and providing softness to fabrics, and/or finishing process.

The LG-12 clade protease polypeptides described herein find further use in the enzyme aided bleaching of paper pulps such as chemical pulps, semi-chemical pulps, kraft pulps, mechanical pulps or pulps prepared by the sulfite method. In general terms, paper pulps are incubated with a LG-12 clade protease polypeptide of the present invention under conditions suitable for bleaching the paper pulp. In some embodiments, the pulps are chlorine free pulps bleached with oxygen, ozone, peroxide or peroxyacids. In some embodiments, the LG-12 clade protease polypeptides are used in enzyme aided bleaching of pulps produced by modified or continuous pulping methods that exhibit low lignin contents. In some other embodiments, the LG-12 clade protease polypeptides are applied alone or preferably in combination with xylanase and/or endoglucanase and/or alpha-galactosidase and/or cellobiohydrolase enzymes.

The LG-12 clade protease polypeptides described herein find further use in the enzyme aided removal of proteins from animals and their subsequent degradation or disposal, such as feathers, skin, hair, hide, and the like. In some instances, immersion of the animal carcass in a solution comprising a LG-12 clade protease polypeptide of the present invention can act to protect the skin from damage in comparison to the traditional immersion in scalding water or the defeathering process. In one embodiment, feathers can be sprayed with an isolated polypeptide of the present invention under conditions suitable for digesting or initiating degradation of the plumage. In some embodiments, a LG-12 clade protease of the present invention can be used, as above, in combination with an oxidizing agent.

In some embodiments, removal of the oil or fat associated with raw feathers is assisted by using a LG-12 clade protease polypeptide of the present invention. In some embodiments, the LG-12 clade protease polypeptides are used in compositions for cleaning the feathers as well as to sanitize and partially dehydrate the fibers. In some other embodiments, the LG-12 clade protease polypeptides are applied in a wash solution in combination with 95% ethanol or other polar organic solvent with or without a surfactant at about 0.5% (v/v).

In yet other embodiments, the disclosed LG-12 clade protease polypeptides find use in recovering protein from plumage. In some embodiments, the recovered protein can be subsequently used in animal or fish feed.

The LG-12 clade protease polypeptides described herein find further use in the enzyme aided debridement of tissue. This involves the removal of dead or damaged tissue, for example, removal from wounds to aid in healing.

The LG-12 clade protease polypeptides described herein find further use in tissue culture. In particular, LG-12 clade proteases of the present invention can be used to suspend or resuspend cells adherent to a cell culture wall, such as during the process of harvesting cells. LG-12 clade proteases of the present invention can be used to cleave protein bonds between cultured cells and the dish, allowing cells to become suspended in solution.

The LG-12 clade protease polypeptides described herein find further use as a food additive, a digestive aide or a food processing aid.

The LG-12 clade protease polypeptides described herein find further use in leather processing by removing hair from animal hides, soaking, degreasing, or bating, which is a process involving degradation of non-structural proteins during leather making.

### EXAMPLE 1

### Assays

### Performance Index

The performance index (PI) of an enzyme compares the performance of the variant (measured value) with the parent enzyme (theoretical value or measured value) at the same protein concentration. Theoretical concentrations for the parent enzyme can be calculated using the parameters extracted from a Langmuir fit of a standard curve of the parent enzyme. A PI that is greater than 1 (PI>1) indicates improved performance by a variant as compared to a parent or benchmark protease (defined for each analyzed data set), while a PI of 1 (PI=1) identifies a variant that performs the same as the parent, and a PI that is less than 1 (PI<1) identifies a variant that performs worse than the parent.

### Protein Purification

Samples were dialyzed overnight against 25 mM MES, pH 5.4, 1 mM CaCl₂ buffer, and loaded on a 30 mL Source 15S column (GE Healthcare) equilibrated with same buffer. A gradient of 0 to 150 mM NaCl was run, and fractions were collected. Fractions containing target protein were pooled, concentrated, and formulated with 40% Propylene Glycol, and sterile filtered.

### Protease Activity Assays

The protease activity of wild-type LG12 protease and variants thereof was tested by measuring hydrolysis of N-suc-AAPF-pNA, dimethyl casein (DMC), or skim milk substrates.

The reagent solutions used for the AAPF hydrolysis assay were: 100 mM Tris/HCl pH 8.6, containing 0.005% TWEEN®-80 (Tris dilution buffer); 100 mM Tris buffer pH 8.6, containing 10 mM CaCl₂ and 0.005% TWEEN®-80 (Tris/Ca buffer); and 160 mM suc-AAPF-pNA in DMSO (suc-AAPF-pNA stock solution) (Sigma: S-7388). To prepare a substrate working solution, 1 ml suc-AAPF-pNA stock solution was added to 100 ml Tris/Ca buffer and mixed well. An enzyme sample was added to a MTP plate (Greiner 781101) containing 1 mg/ suc-AAPF-pNA working solution and assayed for activity at 405 nm over 3 minutes using a SpectraMax plate reader in kinetic mode at RT. The protease activity was expressed as mOD·min⁻¹.

The reagent solutions used for the DMC assay were: 2.5% Dimethylcasein (DMC, Sigma) in 100 mM Sodium Carbonate pH 9.5, 0.075% TNBSA (2,4,6-trinitrobenzene sulfonic acid, Thermo Scientific) in Reagent A. Reagent A: 45.4 g Na₂B₄O₇.10H₂0 (Merck) in 15 mL 4N NaOH to reach a final volume of 1000 mL in MQ water, Dilution Solution: 10 mM NaCl, 0.1 mM CaCl₂, 0.005% Tween-80, 0.02% Na-azide. MTPs (Greiner PS-microwell 384) were filled with 47.5 uL DMC substrate following the addition of 2.5 uL of 20 ppm protease supernatant. 50 uL of TNBSA in Reagent A was then added with slow mixing. Activity was measured at 405 nm over 5 min using a SpectraMax plate reader in kinetic mode at RT. Activity was expressed as mOD^{∗}min⁻¹.

The reagent solutions used for the skim milk assay were: 40 mg/mL BD Difco skim milk powder in 100 mM Tris, 0.005% Tween-80, pH 8.6 with 10 gpg hardness. The solution was equilibrated for 1 h before use. Activity was measured at 650 nm over 3 min using a SpectraMax plate reader in kinetic mode at RT. Activity was expressed as mOD^{∗}min-1.

### Detergents

Proteases were tested for cleaning performance and/or stability in detergents set forth in Table 1. All commercially available HDL laundry detergents except Persil non-Bio and Blue Moon were inactivated to remove enzyme activity by heating to 95°C for 16 hours in a water bath. Protease activity was assayed following inactivation using the AAPF substrate to ensure complete inactivation. Commercially available HDD laundry detergents were inactivated to remove enzyme activity by preparing a 10X concentrated solution relative to what is used in the final cleaning assay and heating for 16 hours at 95°C. Protease activity was assayed following inactivation using the AAPF substrate. After heating the HDD and HDL detergents for 16 hours, protease activity was non-existent.

| **Table 1: Detergents** | | | |
|---|---|---|---|
| **NO.** | **NAME** | **TYPE** | **FORM** |
| 1 | Kirkland Ultra (Sun Products) ^{†} | Laundry | HDD |
| 2 | OMO Color (Unilever)^{†} | Laundry | HDD |
| 3 | Surf Excel (Henkel)^{†} | Laundry | HDD |
| 4 | OMO Klein & Krachtig (Unilever)^{†} | Laundry | HDL |
| 5 | Kirkland Ultraclean (Sun Products)^{†} | Laundry | HDL |
| 6 | Blue Moon (Guangzhou Blue Moon) ^{†,}* | Laundry | HDL |
| 7 | All 3 (Sun Products) ^{†,}* | Laundry | HDL |
| 8 | Purex Dirt Lift (Henkel)^{†},* | Laundry | HDL |
| 9 | Persil Non-Bio (Unilever)^{†,}* | Laundry | HDL |
| 10 | Test HDL* | Laundry | HDL |
| 11 | GSM-B | Dish | Powder |

| | | | |
|---|---|---|---|
| ^{†} Commercially available detergent; Detergent with ≤5mg/Kg boron | | | |

The composition of detergent number 10 is a custom-made in-house HDL detergent ("Test HDL") having the composition set forth in Table 2A.

| **Table 2A: Test HDL Detergent Composition** | | | | |
|---|---|---|---|---|
| **Component** | **Ingredient** | **Trade name** | **Wt.%** | **Amount(g)** |
| Solvent | Water (total) | - | 64.89 | 1297.8g |
| Surfactant | C12-C15 Pareth-7 | Empilan KCL 7 | 3 | 60g |
| | (Sodium) Dodecvlbenzenesulfonate | NANSA SSA F | 7.5 | 150g |
| | K-Cocoate | NANSA PC 38F | 3 | 60g |
| | Sodium Laureth Sulfate | Empicol ESB3/M6 | 9 | 180g |
| Builder | Sodium Citrate | Sodium Citrate Tribasic Dihydrate | 3 | 60g |
| Liquid properties/stability | Sorbitol | D-Sorbitol | 0.8 | 16 |
| | Propylene glycol | 1,2-Propanediol | 2.5 | 50g |
| | Glycerin | Glycerol | 0.8 | 16g |
| | Triethanolamine | Triethanolamine | 0.5 | 10g |
| | Methylisothiazolinone | 2-Methyl-4-isothiazolin-3-one | 0.01 | 0.2g |
| | Ethanol | | 1 | 20g |
| Neutralizer | NaOH (4M) | | | 103.3mL |

The composition of detergent number 11, GSM-B phosphate-free detergent, was purchased from WFK Testgewebe GmbH, Brüggen, Deutschland (www.testgewebe.de) and has the composition set forth in Table 2B.

| **Table 2B: GSM-B ADW Detergent Composition** | |
|---|---|
| **Component** | **Wt %** |
| Sodium citrate dehydrate | 30.0 |
| Maleic acid/ acrylic acid copolymer sodium Salt (SOKALAN® CP5; BASF) | 12.0 |
| Sodium perborate monohydrate | 5.0 |
| TAED | 2.0 |
| Sodium disilicate: Protil A (Cognis) | 25.0 |
| Linear fatty alcohol ethoxylate | 2.0 |
| Sodium carbonate anhydrous | add to 100 |

### Cleaning Performance in Detergent

Variants were tested for cleaning performance relative to wild-type LG12 or BPN'-Y217L protease on various microswatches: BMI (blood/milk/ink on cotton,- EMPA-116), egg yolk with pigment (aged by heat, CFT CS-38), and POM (pigment/oil/milk, CFT-C10), for laundry-based applications, and egg yolk (egg yolk on polyacryl fabric, aged and colored with carbon black dye, PAS-38) for dish-based applications. Pre-punched (to fit on MPT), rinsed, and filled swatch-containing plates (Corning 3641) were prepared by Center for Testmaterials BV, Vlaardingen, Netherlands. Detergent was added to the microswatch plates prior to enzyme addition. Prior to use, commercial detergents except Persil Non-Bio and Blue Moon were heat-inactivated to remove enzyme activity and then dosed as described in Table 3.

| **Table 3: List of Wash Conditions Used for Performance Assays** | | | | | |
|---|---|---|---|---|---|
| **Detergent** | **Final Wash Conc, (g/L)** | **Hardness Conc. (ppm)** | **Buffer** | **Set pH** | **Type** |
| Kirkland Ultra | 1.1 | 150 | 2 mM NaCO₃ | 10.6 | Laundry |
| OMO Color | 5.3 | 250 | 2 mM NaCO₃ | 10.6 | Laundry |
| Surf Excel | 4 | 400 | 2 mM NaCO₃ | 10.6 | Laundry |
| Kirkland Ultraclean | 0.17 | 150 | 5 mM sodium HEPES | 8.2 | Laundry |
| OMO Klein & Krachtig | 2.8 | 250 | 5 mM sodium HEPES | 8.2 | Laundry |
| Persil Non-Bio | 2.7 | 250 | 5 mM sodium HEPES | 8 | Laundry |
| Test HDL | 7.5 | 250 | 5 mM sodium HEPES | 8 | Laundry |
| Blue Moon | 13 | 250 | 5 mM sodium HEPES | 6.5 | Laundry |
| GSM-B | 3 | 374 | Not buffered | 10.5 | Dish |

Laundry cleaning assays with HDL or HDD formulas were carried out at 25°C for 15-30 min, while dish cleaning assay with GSM-B formula and PAS-38 swatches was carried out at 40°C for 30 min. Following incubation, 100 uL of supernatant was transferred to a fresh MTP (Costar 9017) and absorbance was measured at 600 nm for EMPA-116 swatches, or at 405 nm for CFT CS-38, CFT CS-10, or PAS-38 swatches using a SpectraMax plate reader. Absorbance results were obtained by subtracting the value for a blank control (no enzyme) from each sample value to determine relative performance.

### Stability Assays

Variants were tested for stability under various stress conditions of buffers or detergents as indicated in Table 4 below by measuring the residual activity following incubation at elevated temperature. The elevated temperature was set to obtain approximately 30% residual activity of the stressed sample compared to the unstressed sample. Diluted enzyme sample was mixed in stressor and unstressed protease activity was measured. Diluted enzyme sample in stressor was incubated at elevated temperature and stressed protease activity was measured either by AAPF, DMC, or skim milk hydrolysis.

The stability assay conditions are described in Table 4 below. The commercial detergents except Persil Non-Bio and Blue Moon were heated to inactivate enzymes prior to use in stability assays:

| **Table 4: Conditions Used for Protease Stability Assays** | | | |
|---|---|---|---|
| **Condition** | **Stress temp (°C)** | **Substrate** | **Type** |
| 0.02% LAS, 2.1mM EDTA in 50mM HEPES pH8, 0.005% Tween | 53.5 | AAPF/DMC | Laundry |
| 10% Kirkland Ultra | 56 | AAPF/DMC | Laundry |
| 10% OMO Klein & Krachtig | 51 | AAPF/DMC | Laundry |
| 10% Persil Non-Bio HDL | 60 | Skim milk | Laundry |
| 1.0% Test HDL | 60 | DMC | Laundry |
| 50mM Tris pH9; 1mM EDTA; 0.005% Tween | 51 | AAPF/DMC | Dish |
| 50mM Tris pH9; 2mM CaCl₂; 0.005% Tween | 72.5 | AAPF/DMC | Dish |

For the unstressed condition, enzyme was assayed immediately for activity on AAPF, DMC, or skim milk substrates (see above). For the stressed condition, the PCR plate was sealed and incubated at elevated temperature for 5 min using an Eppendorf 385 MasterCycler Pro Thermocycler, then assayed for activity. Stressed and unstressed activity was measured by either the hydrolysis of the synthetic substrate or by the DMC method, as described above. Percent (%) residual activities were calculated by calculating the ratio of the stressed over the unstressed activity and multiplying value by 100. Stability PIs were obtained by dividing the residual activity of a variant by that of the wild type.

### Aged Laundry Cleaning Assay

Each well of a 96 well MTP (Nunc 249946) was filled with a mini stir disc followed by addition of 270 uL 100% Persil Non-Bio detergent. 18.1 uL of enzyme supernatant diluted to ∼500 ppm was added to the detergent-filled plate and mixed using a magnetic tumbling mixing apparatus (V&P Scientific, VP710 series) for 5 min. For the initial time point (T=0), 25 uL of enzyme containing detergent was transferred to a MTP filled with 125 uL of buffer (5 mM HEPES, 250 ppm 3:1 Ca:Mg water hardness, pH 8). Five uL aliquots of enzyme diluted in detergent was then added to 185 uL buffer (5 mM HEPES, 250 ppm hardness, pH 8) previously added to a MTP containing an EMPA-116 BMI microswatch. At time point 0 (T=0), the cleaning assay was carried out in an analogous manner as described above for fresh cleaning. For the remaining time points, the detergent-enzyme plate was incubated at 55°C for either: 1.5 h, 5 h, or 24 h; aliquots were removed at the end of each time point; and the cleaning assay carried out in an analogous manner as described above for fresh cleaning. A residual cleaning value was obtained by comparing the cleaning at the various remaining time points to the initial cleaning at T=0.

### Long Term Stability in Boron-free Laundry Detergent

A subset of LG12-clade variants were tested for stability over a span of two weeks in concentrated boron-free HDL detergents: Persil Non-Bio, Blue Moon, All 3X, and Purex Dirt Lift. A boron-free detergent is a detergent to which borate stabilizers have not been added. The amount of boron present in the boron-free detergents of this Example was quantified by elemental analysis, and in all instances, only trace amounts (less than 5 mg) of boron per kg of detergent were detected.

The long term stability experiments were carried out as follows. Purified enzyme was added at 1% w/w to commercially purchased detergent and mixed by gentle pipetting in either a plate or tube-based set up. An initial aliquot of enzyme detergent mixture was removed from each detergent and stored at -20°C for use as a non-stressed reference. The remaining enzyme detergent samples were placed in a 37°C incubator to induce stress. Aliquots of the enzyme detergent mixture were removed at various time points from the incubator over the course of the experiment and stored at -20°C before measuring activity on AAPF substrate. The enzymatic activity of the detergent samples was measured by diluting then with 100 mM Tris pH 8.6, 0.005% Tween-80, 50 mM CaCl₂ buffer 70-150 fold and then assaying on the AAPF substrate as described above in the section on general set up for stability assays. The dilution factor for the detergent samples was adjusted to account for activity loss over the course of the study to remain within the standard curve of BPN'-Y217L subtilisin reference standard.

### EXAMPLE 2

### Generation of LG12-clade subtilisins

DNA manipulations to generate LG12-clade subtilisins were carried out via molecular biology techniques known in the art. An *E. coli* plasmid containing the genes of interest in its expression cassette and the chloramphenicol resistance marker from *Staphylococcus aureus* (CAT) was constructed. The expression cassette consisted of: *B. subtilis* aprE promoter, the *B. subtilis* aprE signal peptide sequence, the *B. lentus* protease pro-peptide, the region encoding the mature protease sequences for LG12-clade variants (SEQ ID NOs:1 and 3-8 for amino acid sequences) and a *B amyloliquefaciens* terminator.

Gibson assembly was performed to assemble the integration cassette containing sequences that target the *AprE* locus. A suitable *B. subtilis* strain was transformed with reaction products and the transformed cells were plated on Luria's Agar + 5 ppm chloramphenicol containing skim milk. Single colonies from the agar plates were picked into individual wells of a 96-well MTP that contained 150 uL of Luria's broth + 5 ppm chloramphenicol in each well. The cultures were grown overnight at 37°C while being shaken at 250 rpm. 75 uL of 50% glycerol (diluted in water) was added to each well and the MTPs (referred to as library stock plates) frozen at -80°C.

To produce the new proteases, the *B. subtilis* carrying each gene of interest was cultivated in an enriched semi-defined media for 2 days at 32°C. After centrifugation and filtration, culture supernatants with the novel subtilisins were used for assays and purification.

The mature LG12 wild-type (described as LG-12 SprC in Example 2, page 86, line 16 to page 87, line 14 of PCT Published Application WO2015/038792, filed September 11, 2014, which is hereby incorporated herein by reference) amino acid sequence is set forth in, for example, Figure 1A-1B as SEQ ID NO:10.

### EXAMPLE 3

### Evaluation of LG12-clade Protease Variants in Laundry Detergents

### Laundry Cleaning Performance Assay

LG12-clade protease variants listed in Table 5 were tested for cleaning performance relative to BPN'-Y217L protease on BMI (EMPA-116) microswatches. The BPN'-Y217L protease is commercially available and is set forth as SEQ ID NO:48: AQSVPYGVSQIKAPALHSQGYTGSNVKV AVIDSGIDSSHPDLKVAGGASMVPSETNPFQDNNSHGTHVAGTVAALNNSIGVLGVAPSASLYA VKVLGADGSGQYSWIINGIEWAIANNMDVINMSLGGPSGSAALKAAVDKAVASGVVVVAAAG NEGTSGSSSTVGYPGKYPSVIAVGAVDSSNQRASFSSVGPELDVMAPGVSIQSTLPGNKYGALN GTSMASPHVAGAAALILSKHPNWTNTQVRSSLENTTTKLGDSFYYGKGLINVQAAAQKSFPEVV GKTVDQAREYFTLHYPQYDVYFLPEGSPVTLDLRYNRYKVFYNFGTNVVNHVPHVG.

| **Table 5: LG12-clade Protease Variants** | | |
|---|---|---|
| **Sample ID** | **Sequence Substitutions Relative to LG12 WT** | **No. mutations vs LG12** |
| LG12-CP022 | A001T-N076D-T078S-T0791-Y086P-D089E-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-E248N | 33 |
| LG12-CP180 | S052A-N076D-T078S-T0791-Y086P-D089E-S097G-T103S-L104V-G106S-I111L-S114A-I115G-S116N-N120H-I122A-M124L-G128S-S129P-G131P-T133V-A134T-Q136E-C139V-N141S-Y143T-N144S-I147V-V148L-I150V-S158T-G166D-F217L-E248N | 34 |
| LG12-CP446 | A001T-N076D-T078S-T079I-Y086P-D089E-S097G-T103S-L104V-G106S-I111L-S114A-I1115G-S116N-N120H-I122A-M124L-G128S-S129P-G131P-T133V-A134T-Q136E-C139V-N141S-Y143T-N144S-I147V-V148L-I150V-S158T-G166D-T209Y-N212S-N213T-S216T-F217L-A224T-E248N-K251S-N252S-N256Y | 42 |
| LG12-CP455 | N076D-T078S-T079I-Y086P-D089E-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N | 38 |
| LG12-CP460 | N076D-T078S-T079I-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-F217L-E248N | 35 |
| LG12-CP462 | A001T-N076D-T078S-T079I-Y086P-D089E-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N | 39 |
| LG12-CP465 | A001T-N076D-T078S-T079I-Y086P-N087S-A088V-D089S-S097G-T103S-L104V-G106S-I111L-S114A-I115G-S116N-N120H-I122AM124L-G128S-S129P-G131P-T133A-A134T-Q136E-C139V-N141S-Y143T-N144S-I147V-V148L-I150V-S158T-G166D-T209Y-N212S-N213T-S216T-F217L-E248N | 40 |

The detergents used were commercially available Persil Non-Bio HDL (Unilever) at a final concentration of 2.7 g/L and Test HDL at a final concentration of 7.5 g/L. In both instances, the water hardness concentration was set at 250ppm, using 5 mM sodium HEPES buffer to achieve pH 8.

Aliquots of purified enzyme were added to the detergent-filled microswatch plates to reach a final volume of 200 uL with a final enzyme concentration of 0.62 ppm. Assays were carried out at 25°C for 30 min. Following incubation, 100 uL of supernatant was transferred to a fresh MTP (Costar 9017) and absorbance was read at 600 nm using the SpectraMax plate reader. Absorbance results were obtained by subtracting the value for a blank control (no enzyme) from each sample value. The cleaning PI for each assay condition was obtained by dividing the absorbance values for a given variant by that of the control (BPN'-Y217L).

**Table 6 shows the results obtained when the cleaning and stability of various LG12-clade protease variants were compared to the benchmark BPN'-Y217L in non-boron containing detergents.**

| **Table 6: Comparison of Cleaning and Stability of LG12-clade Protease Variants to BPN'-Y217L in Non-boron Containing Detergents** | | | | |
|---|---|---|---|---|
| **Variant** | **Microswatch Cleaning (PI vs BPN'-Y217L)** | | **Stability in 10% HDL, 60°C (% Residual protease activity)** | |
| | **Persil Non-Bio HDL** | **Test HDL** | **Persil Non-Bio HDL** | **Test HDL** |
| LG12-CP022 | 0.8 | 0.7 | 74.6 | 56.8 |
| LG12-CP180 | 0.9 | 0.7 | 69.2 | 44.0 |
| LG12-CP446 | 0.7 | 0.8 | 82.6 | 74.5 |
| LG12-CP455 | 0.9 | 0.8 | 81.2 | 66.4 |
| LG12-CP460 | 0.9 | 0.8 | 88.2 | 70.1 |
| LG12-CP462 | 0.9 | 0.8 | 86.0 | 64.2 |
| LG12-CP465 | 0.9 | 0.9 | 102.4 | 58.0 |
| BPN'-Y217L | 1.0 | 1.0 | 7.6 | 10.4 |

Table 6 data shows that following incubation in non-boron containing HDL detergents and heat stress, the variants: LG12-CP022, LG12-CP180, LG12-CP446, LG12-CP455, LG12-CP460, LG12-CP462, and LG12-CP465 all had significantly improved residual activity over BPN'-Y217L. These variants also retain a significant amount of cleaning efficiency while gaining on average 5-10 fold improvement in detergent stability.

### EXAMPLE 4

### Sequence Comparison of LG12-clade Protease Variants and LG12 Wild-type Protease

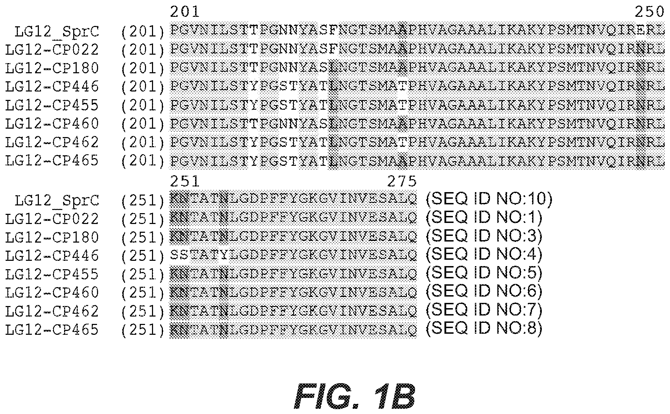

The sequences of the LG12-clade protease variants of Example 3 (i.e., LG12-CP022 (SEQ ID NO:1), LG12-CP180 (SEQ ID NO:3), LG12-CP446 (SEQ ID NO:4), LG12-CP455 (SEQ ID NO:5), LG12-CP460 (SEQ ID NO:6), LG12-CP462 (SEQ ID NO:7), and LG12-CP465 (SEQ ID NO:8)) were compared to the parent protease sequence of LG12 wild-type (SEQ ID NO: 10). Clustal W sequence alignment, shown in Figure 1, identifies regions of the amino acid sequence where changes occurred.

The LG12-CP022 (SEQ ID NO:1), LG12-CP180 (SEQ ID NO:3), LG12-CP446 (SEQ ID NO:4), LG12-CP455 (SEQ ID NO:5), LG12-CP460 (SEQ ID NO:6), LG12-CP462 (SEQ ID NO:7), and LG12-CP465 (SEQ ID NO:8) protease variants had superior stability and significant cleaning performance in laundry conditions. Most of these LG12-clade protease variants share 31 substitutions at the following positions: 76, 78, 79, 86, 97, 103, 104, 106, 111, 114, 115, 116, 120, 122, 124, 128, 129, 131, 134, 136, 139, 141, 143, 144, 147, 148, 150, 158, 166, 217 and 248, wherein the amino acid positions are numbered by correspondence with the linear LG12 wild-type (SEQ ID NO:10) amino acid sequence numbering. Furthermore, one or more LG12-clade protease variants share one or more of the following substitutions: N76D, T78S, T79I, Y86P, S97G, T103S, L104V, G106S, I111L, S114A, I115G, S116N, N120H, I122A, M124L, G128S, S129P, G131P, A134T, Q136E, C139V, N141S, Y143T, N144S, I147V, V148L, I150V, S158T, G166D, F217L and E248N.

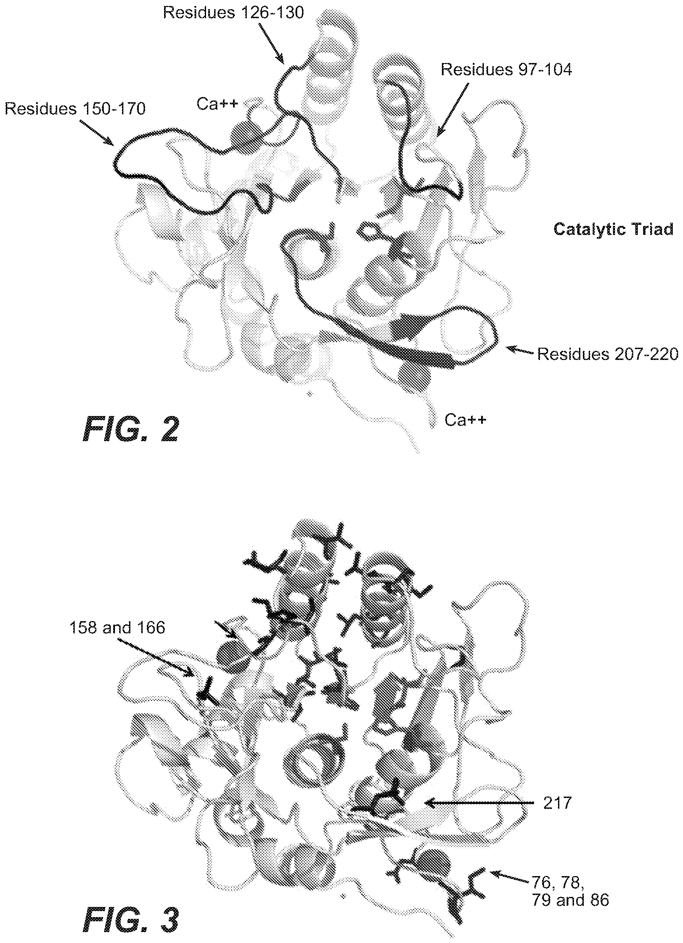

These sites are distributed primarily in three regions, two of which are structurally closely related. The overall protein fold observed on a crystallographic structure of LG12 wild-type protease is shown in Figure 2.

Figure 2 highlights key elements in the LG12 wild-type protease crystallographic model. The overall folding is represented as a schematic in light gray. The characteristic catalytic triad of the subtilisin proteases formed by Aspartic acid (D) 32, Histidine (H) 64 and Serine (S) 221 is shown as black stick figures. These 3 residues are found in a large canyon-like surface formed by segments comprising residues 97-104, 126-130, 150-170, and 207-220. The two calcium sites common to all commercial bacterial subtilisins are represented as dark gray spheres. The key loops forming the substrate binding region are highlighted as black lines and include residues 97-104, 126-130, 150-170 and 207-220. The 97-104 and 126-130 segments are followed by large helices comprising residues 105-117 and 131-145.

Figure 3 provides a diagram of the 3D structure of the LG12 wild-type protease depicting (i) side chains of substitutions common to the best LG12-Clade protease variants, which substitutions are primarily localized in two segments that form the walls of the substrate binding site and the following helical segments that serve to anchor the segment in structure residues 97-148, (ii) positions 158, 166 and 217, which line the substrate binding cavity, and (iii) positions 76, 78, 79 and 86, which are near one of the critical calcium binding sites.

As can be seen in the image shown in Figure 3, residues 97, 103, 104, 106, 111, 114, 115, 116, 120, 122, 124, 128, 129, 131, 134, 136, 139, 141, 143, 144, 147, 148, 150, and 158 are found either in the substrate binding region or in the helices following the highlighted segments. As a result, substitutions at these positions may contribute stabilizing inter-helical interactions and position these helices to act in concert with the substitutions in the substrate binding canyon to increase the overall stability and cleaning performance. Other beneficial mutations can be made at positions 76, 78, 79 and 86, which are localized near the tight calcium binding site common to all *Bacillus* subtilisins, as shown in Figure 3. In addition, including mutations at position 158, 166 and 217 is shown to impart significant benefits.

### EXAMPLE 5

### Generation of FurtherLG12-clade Protease Variants

Design of additional LG12-clade protease variants was performed using information from the site evaluation library screen that is described in PCT Published Application No. WO2015/038792, filed September 11, 2014. From 2 to 23 beneficial mutations were introduced into the LG12 wild-type protease resulting in LG12-Clade protease variants with improved cleaning performance and stability.

A suitable *B. subtilis* host strain was transformed with expression plasmids containing the variants. The variants were grown in 96-well MTPs in cultivation medium (enriched semi-defined media based on MOPs buffer, with urea as major nitrogen source, glucose as the main carbon source, and supplemented with 1% soytone for robust cell growth) containing 25 ppm tetracycline in square enzyscreen plates for 2 days at 32°C, 300rpm, with 80% humidity in shaking incubator.

### EXAMPLE 6

### Cleaning Performance and Stability of LG12-clade Variant Proteases

Additional LG12-clade protease variants generated as described in Example 5 above were screened in MTP assays (as described in Example 1) to compare their cleaning performance and stability to parent molecule LG12 wild-type protease. All variants exhibited expression (HPLC assay) and proteolytic activity on DMC substrate on par with LG12 wild-type protease (SEQ ID NO:10). The variants were grouped according to improved properties. Table 7 sets forth the LG12-clade protease variants constructed and tested.

| **Table 7: LG12-clade Variant Proteases** | | |
|---|---|---|
| **Sample ID** | **Sequence Substitutions Relative to LG12 WT** | **No. mutations vs LG12** |
| LG12-05502 | A001G-S024R-I030V-A048Q-S052Q-G053N-S114T-G128N-V149N-F217R-A228S-A236N-S272K | 13 |
| LG12-05503 | A001G-S052N-L090P-N117S-V149G-S159E-Y238F-S272N | 8 |
| LG12-05505 | T003V-P009V-A019R-S024T-I030V-A040L-A05713-N076D-S101TI107V-S116E-I147A-S156A-G166Y-T185Q-S194P-A215E-S240F-N256K-S272K | 20 |
| LG12-05517 | A001G-A048Q-S052T-T078G-Y091H-S114T-N117S-I147A-S156N-S159N-G166H-F217R-M222L-A236N-Y238F-Q245N-V267N | 17 |
| LG12-05521 | A001G-K027T-N038Q-A040L-N043S-S052Q-G053D-G100K-G128N-R145N-V149N-S159D-S181A-F217R-K251R-N256R-E271W-S272Q | 18 |
| LG12-05523 | A001G-P009T-A019R-N038A-A057P-V072A-N076D-S099L-T103E-S114T-R145W-V149S-S156N-G166H-T185Q-S194P-F217R-Y238F-K265S-S272K. | 20 |
| LG12-05524 | N123D-S159D-T209F-M222L-V267N | 5 |
| LG12-05525 | A001G-P009I-A019R-S024T-K027T-I030V-N038Q-N043H-A048Q-G053D-S101T-I107T-G128N-T133D-V149S-T185Q-A228S-T242S-K251R-S272K | 20 |
| LG12-05526 | K045Q-N123D-S156N-T209F-N256P | 5 |
| LG12-05527 | A001G-A048Q-S052T-T078G-Y091H-S114T-N117S-I147A-S156N-S159N-G166H-M222L-A236N-Y238F-Q245N-V267N | 16 |
| LG12-05534 | A001G-I030V-S052Q-G053N-S114T-G128N-V149N-S159N-F217R-A228S-A236N | 11 |
| LG12-05535 | A001G-A048Q-S052T-T078G-Y091H-S114T-N117S-I147A-S156N-S159N-G166H-M222L-A236N-Y238F-Q245N-N256E-V267N | 17 |
| LG12-05536 | A001G-I030V-A040E-N043D-A048Q-S052Q-G053N-S114T-G128N-V149N-I150V-SI56N-S159D-G166N-S181A-S216T-M222L-A228S-A236N | 19 |
| LG12-05537 | A001G-T003E-A040D-A048Q-S052T-G053E-T078G-Y091H-S114T-N117S-I147A-S156N-S159D-G166H-S182E-A215D-F217R-M222L-A236N-Y238F-Q245N-N256E-V267N | 23 |
| LG12-05540 | A001G-S024R-I030V-A048Q-S052Q-G053N-S114T-G128N-V149N-S159D-F217R-A228S-A236N-S272K | 14 |
| LG12-05541 | A001G-K027S-N038T-G053E-T078D-L090P-V093T-T103E-S159D-G166S-T185Q-F217R-A236N | 13 |
| LG12-05542 | A001G-K027T-A040L-N043S-S052Q-G053D-V149N-S159D-G166Y-S181A-F217R-M222L-N256R-E271W-S272Q | 15 |
| LG12-05543 | T003V-N043L-G053E-A057P-V072A-N076D-S101T-I107T-S116E-I147A-S159Q-G166A-S188Y-S194P-F217R-A236Q-S240F-I246V-S272Q | 19 |
| LG12-05546 | A001G-T003E-A040D-A048E-S052T-G053E-T078G-Y091H-S114T-N117S-I147A-S156N-S159D-G166H-S182E-A215D-M222L-A236N-Y238F-Q245N-N256E-V267N | 22 |
| LG12-05552 | A001G-K027T-N038Q-A040L-N043S-S052Q-G053D-N117E-R145N-V149N-S159D-G166Y-S181A-F217R-M222L-K251R-N256R-E271W-S272Q | 19 |
| LG12-05553 | A001G-A040D-S052T-T078G-Y091H-S114T-N117S-I147A-S156N-S159N-G166H-S182E-M222L-A236N-Y238F-Q245N-V267N | 17 |
| LG12-05557 | A001G-A048Q-S052Q-G053N-S114T-G128N-S159N-S216T-A228S | 9 |
| LG12-05559 | A001G-P009T-S024T-K027S-A057P-V072A-N076D-S101T-S116EN140Y-V149N-S159N-G166N-T185Q-S188Y-S194P-I234V-S240F-K251R-E271F | 20 |
| LG12-05561 | A001G-I030V-N043D-A048Q-S052Q-G053N-S114T-G128N-V149N-S159N-S216T-A228S-A236N | 13 |
| LG12-05563 | A001G-M30V-A040V-N076D-T078D-T103E-G166S-T185Q-F217R | 9 |
| LG12-05565 | A001G-K027S-A040V-G053N-T078D-L090P-T103E-S156N-S159D-G166S-F217R | 11 |
| LG12-05566 | N038Q-G100A-N117S-I147A-S158T-G166D-N256P-S272K | 8 |
| LG12-05567 | A001G-I030V-A048Q-S052Q-G053N-S114T-G128N-V149N-I150V-S156N-S159N-S181A-S216T-M222L-A228S-A236N | 16 |
| LG12-05569 | N038Q-G100A-N117S-I147A-S158T-N256P-S272K | 7 |
| LG12-05571 | A001G-I030V-N043D-A048E-S052Q-G053N-S114T-G128N-V149N-S159D-S216T-A228S-A236N | 13 |
| LG12-05576 | A001G-K027T-N038Q-A040L-N043S-S052Q-G053D-N117E-R145N-V149N-S159D-G166Y-S181A-F217R-M222L-K251R-N256R-E271W | 18 |
| LG12-05577 | S024R-I030V-A040E-N043D-A048E-S052Q-G053N-S114T-G128H-V149N-I150V-S156N-S159D-G166D-S181A-S216T-F217R-A228S-A236N-E271W-S272K | 21 |
| LG12-05626 | N076D-G118R-G128A-S129M-S130T-G166D-E248N | 7 |
| LG12-05652 | N076D-G118R-G128D-S129E-S130L-G166D-E248N | 7 |
| LG12-05657 | N076D-G118R-G128D-S129K-S130H-G166D-E248N | 7 |
| LG12-05659 | N076D-G118R-G128D-S129M-S130H-G166D-E248N | 7 |
| LG12-05661 | N076D-G118R-G128D-S129N-S130M-G166D-E248N | 7 |
| LG12-05662 | N076D-G118R-G128D-S129N-S130Y-G166D-E248N | 7 |
| LG12-05668 | N076D-G118R-G128D-S129R-S130I-G166D-E248N | 7 |
| LG12-05670 | N076D-GI118R-G128D-S129R-S130T-G166D-E249N | 7 |
| LG12-05671 | N076D-G118R-G128D-S130N-G166D-E248N-G160D | 7 |
| LG12-05672 | N076D-G118R-G128D-S129T-S130F-G166D-E248N | 7 |
| LG12-05673 | N076D-G118R-G128D-S129Y-S130R-G166D-E248N | 7 |
| LG12-05680 | N076D-G118R-G128E-S129G-S130Y-G166D-E248N | 7 |
| LG12-05681 | N076D-G118R-G128E-S129H-S130T-G166D-E248N | 7 |
| LG12-05687 | N076D-G118R-G128E-S129V-S130M-G166D-E248N | 7 |
| LG12-05707 | N076D-G118R-S129D-S130A-G166D-E248N | 6 |
| LG12-05710 | N076D-G118R-S129I-S130A-G166D-E248N | 6 |
| LG12-05720 | N076D-G118R-S129V-S130Q-G166D-E248N-Q275R | 7 |
| LG12-05731 | N076D-G118R-G128H-S129L-S130L-G166D-E248N | 7 |
| LG12-05781 | N076D-G118R-G128K-S129I-S130N-G166D-E248N | 7 |
| LG12-05829 | N076D-G118R-G128N-S129G-S130N-G166D-E248N | 7 |
| LG12-05831 | N076D-G118R-G128N-S129M-S130G-G166D-E248N | 7 |
| LG12-05832 | N076D-G118R-G128N-S129N-S130H-G166D-E248N | 7 |
| LG12-05837 | N076D-G118R-G128N-S129T-S130Q-G166D-E248N | 7 |
| LG12-05838 | N076D-G118R-G128N-S129V-S130V-G166D-E248N | 7 |
| LG12-05839 | N076D-G118R-G128N-S129Y-S130M-G166D-E248N | 7 |
| LG12-05863 | N076D-G118R-G128Q-S129M-S130H-G166D-E248N | 7 |
| LG12-05896 | N076D-G118R-G128S-S129E-S130W-G166D-E248N | 7 |
| LG12-05899 | N076D-G118R-G128S-S129H-S130Q-G166D-E248N | 7 |
| LG12-05901 | N076D-G118R-G128S-S129I-S130R-G166D-E248N | 7 |
| LG12-05902 | N076D-G118R-G128S-S129L-S130E-G166D-E248N | 7 |
| LG12-05904 | N076D-G118R-G128S-S129L-S130M-G166D-E248N | 7 |
| LG12-05908 | N076D-G118R-G128S-S129N-S130V-G166D-E248N | 7 |
| LG12-05911 | N076D-G118R-G128S-S129Q-G166D-E248N | 6 |
| LG12-05912 | N076D-G118R-G128S-S129V-S130L-G166D-E248N | 7 |
| LG12-06006 | N076D-G118R-G128Y-S129G-S130T-G166D-E248N | 7 |
| LG12-06029 | G118R-G128M-S129L-S130Q-G166D-E248N | 6 |
| LG12-06520 | G166H-S159D | 2 |
| LG12-06556 | N038T-N140D-G166Y-T185Q-S159D | 5 |
| LG12-06575 | N038T-S156H-G166Y-T185Q-S272Q-S159D | 6 |
| LG12-06582 | N038T-S156H-G166Y-S272Q-S159D | 5 |
| LG12-06593 | N140D-V149L-S156H-G166N-S272Q | 5 |
| LG12-06596 | N140D-V149L-G166Y-T185Q-S159D | 5 |
| LG12-06608 | N039T-N140D-V149L-S156H-G166Y-T185Q-S272Q-S159D | 8 |
| LG12-06612 | N140D-G166Y-T185Q-S159D | 4 |
| LG12-06801 | A040A-V149V-S156N-G166A-S181S-S182E-S194S-N256N | 8 |
| LG12-06924 | G053D-N076D-G166D-S272K | 4 |
| LG12-06925 | G053D-N076D-G118G-G166D-E248E-N256N-S272S | 7 |
| LG12-06929 | G053D-N076D-G166D-N256R-S272K | 5 |
| LG12-06948 | G053D-G166D-S272K | 3 |
| LG12-06949 | G053D-G166D | 2 |
| LG12-06954 | G053D-G118R-G166D | 3 |
| LG12-06960 | N076D-G166D-S272K | 3 |
| LG12-06961 | N076D-G166D-N256R | 3 |
| LG12-06964 | N076D-G166D-E248K | 3 |
| LG12-06969 | N076D-G118R-G166D | 3 |
| LG12-06978 | G166D-S272K | 2 |
| LG12-06992 | G118R-G166D-N256R-S272K | 4 |
| LG12-07003 | G053D-G118R-G166D-S272K | 4 |
| LG12-07004 | G053D-G118R-G166D-N256R-S272K | 5 |
| LG12-07016 | G053D-N076D-G166D-E248K-S272K | 5 |
| LG12-07024 | N076D-G166D | 2 |
| LG12-07027 | N076D-G118R-G166D-N256R-S272K | 5 |
| LG12-07133 | K027S-G053E-S159D-N256P | 4 |
| LG12-07143 | K045Q-G053E-S159D-N256P | 4 |
| LG12-07158 | P009T-K045R-A048E-T078A-G128N-N140D-S158V-S181N-S194P-T209V-Q245A-N256P-S272Q | 13 |

A set of LG12-clade protease variants with improved stability in 10% OMO HDL detergent over LG12 wild-type protease parent molecule had a PI >1.01 for stability in LAS/EDTA and 10% OMO HDL detergent, and a PI >0.75 for stability in 10% Kirkland HDL detergent. These LG12-clade variants are: LG12-07133, LG12-05524, LG12-05526, LG12-05575, LG12-05569, LG12-05503, LG12-05566, LG12-07075, LG12-05557, LG12-05563, LG12-05534, LG12-05565, LG12-05502, LG12-05541, LG12-05561, LG12-05571, LG12-07158, LG12-05540, LG12-05542, LG12-05527, LG12-05567, LG12-05517, LG12-05535, LG12-05553, LG12-05521, LG12-05576, LG12-05536, LG12-05543, LG12-05552, LG12-05505, LG12-05523, LG12-05525, LG12-05559, LG12-05577, LG12-05546, and LG12-05537.

A set of LG12-clade protease variants with improved laundry cleaning in HDD detergent over LG12 wild-type protease parent molecule had a PI >1.01 in Kirkland Ultra HDD detergent, OMO color HDD detergent, and in Surf Excel HDD for those variants marked with an asterisk(*). These LG12-clade variants are: LG12-05526*, LG12-05569*, LG12-05626, LG12-05652, LG12-05657, LG12-05659, LG12-05661, LG12-05662, LG12-05668, LG12-05670, LG12-05672, LG12-05673, LG12-05680, LG12-05681, LG12-05687, LG12-05707, LG12-05710, LG12-05731LG12-05781LG12-05829, LG12-05831, LG12-05832, LG12-05837, LG12-05838, LG12-05839, LG12-05863, LG12-05896, LG12-05899, LG12-05901, LG12-05902, LG12-05904, LG12-05908, LG12-05911, LG12-05912, LG12-06006, LG12-06029, LG12-06924, LG12-06929, LG12-06948, LG12-06949, LG12-06954, LG12-06960, LG12-06961, LG12-06964, LG12-06969, LG12-06978, LG12-06992, LG12-07003, LG12-07004, LG12-07016, LG12-07024, LG12-07027, LG12-05566*, LG12-05671, LG1205720, LG12-06593, LG12-07110*, LG12-05522*, LG12-06520, LG12-06556, LG12-06575, LG12-06582, LG12-06596, LG12-06608, LG12-06612, and LG12-05540*.

A set of LG12-clade protease variants with improved stability and laundry cleaning across all assays over LG12 wild-type protease parent molecule had a PI value > 1.01 in all laundry detergents, including Blue Moon HDL. These LG12-clade variants are: LG12-05566, LG12-07065, LG12-07110, LG12-05522, and LG12-05540.

A set of LG12-clade protease variants with improved ADW cleaning over LG12 wild-type protease parent molecule had a PI value >1.01 in GSM-B detergent. These LG12-clade variants are: LG12-05520, LG12-05566, LG12-05569, LG12-05626, LG12-05652, LG12-05657, LG12-05659, LG12-05661, LG12-05662, LG12-05668, LG12-05670, LG12-05671, LG12-05672, LG12-05673, LG12-05680, LG12-05681, LG12-05687, LG12-05707, LG12-05710, LG12-05720, LG12-05731, LG12-05781, LG12-05829, LG12-05831, LG12-05832, LG12-05837, LG12-05838, LG12-05839, LG12-05863, LG12-05896, LG12-05899, LG12-05901, LG12-05902, LG12-05904, LG12-05908, LG12-05911, LG12-05912, LG12-06006, LG12-06029, LG12-06924, LG12-06925, LG12-06929, LG12-06943, LG12-06948, LG12-06949, LG12-06954, LG12-06960, LG12-06961, LG12-06964, LG12-06969, LG12-06978, LG12-06992, LG12-07003, LG12-07004, LG12-07016, LG12-07024, LG12-07027, LG12-07051, LG12-07058, LG12-07098, LG12-07110, LG12-07115, LG12-07118, LG12-07143, LG12-07144, LG12-07158, LG12-07166, and LG12-07194.

A set of LG12-clade protease variants with improved stability and ADW cleaning over LG12-clade wild-type protease parent molecule had a stability PI >1.2 in buffer (50mM Tris pH9-2mM CaCl₂ and Tris pH9-1mM EDTA as described in Table 4) and an ADW cleaning PI > 1.0. These LG12-clade variants are: LG12-05566, LG12-05569, LG12-05657, LG12-05668, LG12-05670, LG12-06006, LG12-06029, LG12-06929, LG12-06948, LG12-06949, LG12-06954, LG12-06960, LG12-06961, LG12-06978, LG12-06992, LG12-07003, LG12-07004, LG12-07027, LG12-07143, LG12-07158, LG12-07051, LG12-07058, LG12-07098, LG12-07110, LG12-07115, LG12-07118, and LG12-07166. Substitutions that occurred frequently in the above improved LG12-clade protease variants include: S052, G053, G118, S129, S130, V149, S156, S159, G166, E248, N256 and S272, which can be observed in further combination with mutations N076D and/or F217R.

### EXAMPLE 7

### Further Evaluation of LG12-clade Variants

Additional LG12-clade protease variants listed in Table 8 were synthesized and expressed in *B*. *subtilis* as described in Example 2.

| **Table 8: LG12-clade Variants Purified for Further Analysis** | | |
|---|---|---|
| **Sample ID** | **Sequence Substitutions Relative to LG12 WT** | **No. mutations vs LG12** |
| LG12-05520 | G053D-N076D-G166D | 3 |
| LG12-05522 | N038Q-G053E-G100A-N117S-I147A-S158T-G166D-N256P-S272K | 9 |
| LG12-05547 | A001G-I030V-T078D-V093T-T103E-S156N-S159D-G166S-S194P-F217R-A236N | 11 |
| LG12-05575 | S024T-G053E-G166D-M222L-N256P | 5 |
| LG12-06491 | V149L-S156H-T185Q-S272Q-S159D | 5 |
| LG12-06943 | G053D-N076D-G118R-G166D-N256R | 5 |
| LG12-07051 | N038Q-G053E-G100A-N117S-I147A-S158T-G166D-S272K | 8 |
| LG12-07058 | S052T-V149G-S156N-G166H-F217R | 5 |
| LG12-07065 | A001G-S052Q-V149G-S156N-S159N-S182E-Y238F-S272Q | 8 |
| LG12-07075 | A001G-S052Q-S114T-S156N-S159N-S182E-Y238F-S272Q | 8 |
| LG12-07098 | S052T-V149G-S156N-S159D-G166D-F217R | 6 |
| LG12-07110 | N038Q-G053E-N117S-I147A-S158T-G166D-N256P-S272K | 8 |
| LG12-07115 | A040D-S114T-I147A-S159N-G166D-S182E | 6 |
| LG12-07118 | S052T-V149G-S156N-G166D-S182E-F217R | 6 |
| LG12-07166 | S052T-V149G-S156N-G166D-F217R | 5 |

For laundry cleaning assays, aliquots of purified enzyme were added to a detergent-filled microswatch plate to reach a final volume of 200 uL for laundry assays with a final enzyme concentration between 5-0.5 ppm and tested as described in Example 1. Samples of LG12 wild-type parent protease and BPN'-Y217L benchmark protease were also evaluated. The microswatch cleaning for purified LG12-clade protease variants in BMI laundry assays is set forth in Table 9A. PIs were calculated versus LG12 wild-type parent protease.

| **Table 9A: Comparison of Cleaning Performance of Various LG12-clade Protease Variants in HDD and HDL Laundry Detergents to LG12 WT and BPN'-Y217L, Reported as PI** | | | | | | |
|---|---|---|---|---|---|---|
| **Sample ID** | **Kirkland HDD** | **OMO HDD** | **Surf Excel** | **OMO HDL** | **Kirkland HDL** | **Blue Moon HDL** |
| LG12 WT | 1.0 | 1.0 | 1.0 | 1.0 | 1.0 | 1.0 |
| LG12-05520 | 1.1 | 0.9 | 1.0 | 1.4 | 1.1 | 5.4 |
| LG12-05547 | 0.7 | 0.4 | 0.5 | 1.0 | 0.7 | 3.3 |
| LG12-06491 | 1.6 | 1.4 | 1.1 | 1.9 | 1.7 | 2.4 |
| LG12-06943 | 2.5 | 1.6 | 1.8 | 1.8 | 2.2 | 3.7 |
| LG12-07051 | 2.2 | 0.6 | 0.9 | 2.5 | 2.2 | 4.8 |
| LG12-07058 | 1.2 | 1.1 | 0.9 | 0.9 | 1.0 | 1.0 |
| LG12-07098 | 1.6 | 0.7 | 0.9 | 1.2 | 1.1 | 2.8 |
| LG12-07115 | 1.0 | 0.8 | 0.9 | 1.0 | 1.0 | 4.1 |
| LG12-07118 | 1.3 | 0.8 | 0.8 | 1.1 | 0.9 | 2.5 |
| LG12-07166 | 1.7 | 1.3 | 1.1 | 1.6 | 1.5 | 1.7 |
| BPN'-Y217L | 0.5 | 0.1 | 0.2 | 1.25 | 1.4 | 1.45 |

The stability of several purified LG12-clade protease variants was measured as described in Example 1, Table 4, in LAS/EDTA and 10% HDL (Kirkland and OMO) and compared to LG12 wild-type parent protease and BPN'-Y217L benchmark protease. The results are shown in Table 9B below.

| **Table 9B: Comparison of Stability of Various LG12-clade Protease Variants in 10% HDL Laundry Detergents and in LAS/EDTA to LG12 WT and BPN'-Y217L, Reported as PI** | | | |
|---|---|---|---|
| **Sample ID** | **LAS/EDTA** | **10% OMO HDL** | **10% Kirkland HDL** |
| LG12 WT | 1.0 | 1.0 | 1.0 |
| LG12-05520 | 1.7 | 1.4 | 1.0 |
| LG12-05547 | 2.2 | 1.4 | 1.2 |
| LG12-06491 | 4.1 | 3.0 | 2.3 |
| LG12-06943 | 2.1 | 2.7 | 2.6 |
| LG12-07051 | 4.1 | 3.4 | 3.2 |
| LG12-07058 | 2.1 | 2.7 | 2.6 |
| LG12-07098 | 2.0 | 1.2 | 1.1 |
| LG12-07115 | 1.8 | 1.2 | 1.0 |
| LG12-07118 | 1.9 | 1.2 | 1.0 |
| LG12-07166 | 2.8 | 2.8 | 1.8 |
| BPN'-Y217L | 3.6 | 3.0 | 0.3 |

For ADW cleaning assays, aliquots of clarified culture supernatants were added to GSM-B detergent-filled microswatch (PAS-38) plate and tested as described in Example 1. The stability of these molecules was also tested in 50mM Tris pH9-2mM CaCl₂ and Tris pH9-1mM EDTA, as described in Example 1. Results for ADW cleaning and stability are shown in Table 9C below.

| **Table 9C: Comparison of ADW Cleaning Performance of Various LG12-clade Protease Variants vs LG12 WT in GSM-B Detergent and Stability Assays, Reported as PI** | | | |
|---|---|---|---|
| **Sample ID** | **ADW Cleaning** | **Stability TRIS-CA** | **Stability TRIS-EDTA** |
| LG12 WT | 1.0 | 1.0 | 1.0 |
| LG12-05668 | 2.2 | 2.2 | 1.9 |
| LG12-07051 | 1.1 | 2.1 | 2.1 |
| LG12-07058 | 1.3 | 1.6 | 2.3 |
| LG12-07098 | 1.5 | 1.7 | 2.2 |
| LG12-07110 | 1.6 | 2.0 | 2.1 |
| LG12-07115 | 1.3 | 1.7 | 2.0 |
| LG12-07118 | 1.5 | 1.8 | 2.6 |
| LG12-07166 | 1.6 | 2.0 | 2.6 |

The stability of several LG12-clade protease variants was also measured under long-term accelerated stability conditions in various boron-free detergents, and compared to LG12 wild-type parent protease and BPN'-Y217L benchmark protease. The results are shown in Table 10. These detergents represent a cross-section of geographies and formulations, and results indicate that stability improvements can be made for all the formulations examined.

The long term stability experiments were carried out as follows. Purified enzyme was added at 1% w/w to commercially purchased detergent and mixed by gentle pipetting in either a plate or tube-based set up. An initial aliquot of enzyme detergent mixture was removed from each detergent and stored at -20°C for use as a non-stressed reference. The remaining enzyme detergent samples were placed in a 37°C incubator to induce stress. Aliquots of the enzyme detergent mixture were removed at various time points from the incubator over the course of the experiment and stored at -20°C before measuring activity on AAPF. The enzymatic activity of the detergent samples was measured by diluting with 100 mM Tris pH 8.6, 0.005% Tween-80, 50 mM CaCl₂ buffer 70-150 fold before assaying on the AAPF substrate as described above in Example 1. The dilution factor for the detergent samples was adjusted to account for activity loss over the course of the study to remain within the standard curve of BPN'-Y217L.

The activity at each time point for a particular variant was measured and the residual activity was calculated by comparison to the initial unstressed activity for that variant (T=0). The loss in activity was fit to a one phase decay curve and the T₅₀ (half-life) parameter was extracted. The T₅₀ of each variant was compared to that of BPN'-Y217L to calculate the long term stability PI set forth in Table 10.

| **Table 10: Long term Stability of LG12-clade Protease Variants in Various Boron-free Detergents vs BPN'-Y217L Protease Benchmark, Reported as PI** | | | | |
|---|---|---|---|---|
| **Sample ID** | **Persil Non-Bio** | **All 3X** | **Purex Dirt Lift** | **Blue Moon** |
| LG12 WT | 0.7 | 0.8 | 1.7 | 0.7 |
| LG12-05520 | 0.5 | 1.2 | 1.7 | NA* |
| LG12-06491 | 0.5 | 0.4 | 0.9 | 1.4 |
| LG12-06943 | 0.6 | 1 | 8.7 | 16 |
| LG12-07051 | 0.6 | 1.3 | 6.7 | NA* |
| LG12-07058 | 0.7 | 1.8 | 12.7 | 0.70 |
| LG12-07166 | 1.03 | 1.5 | 2.0 | 5.3 |
| BPN'-Y217L | 1.0 | 1.0 | 1.0 | 1.0 |

| | | | | |
|---|---|---|---|---|
| *NA denotes Not Analyzed | | | | |

### EXAMPLE 8

### Laundry Cleaning in Boron-free Detergents

Several highly stable variants from the long term stability experiment were tested for cleaning in boron-free formulations as shown in Table 11, where PI values are reported versus LG12 wild-type protease parent.

The detergents used were the same as those used in the long term stability experiment (see above) and included Persil Non-Bio (Unilever), Blue Moon (Blue Moon), All 3X (Sun), and Purex Dirt Lift (Henkel). Superior cleaning was seen for LG12-06943 relative to LG12 wild-type in the two North American formulations (Purex and All 3X), the European formulation (Persil Non-Bio), and the Chinese formulation (Blue Moon).

| **Table 11: Cleaning of Select LG12-clade Protease Variants with Improved Performance vs LG12 WT in Boron-free Detergents, Reported as PI** | | | | |
|---|---|---|---|---|
| **Sample ID** | **All 3X** | **Purex Dirt Lift** | **Persil Non-Bio** | **Blue Moon** |
| LG12 WT | 1.0 | 1.0 | 1.0 | 1.0 |
| LG12-06943 | 1.8 | 1.6 | 1.5 | 1.3 |
| LG12-07051 | 1.5 | 2.0 | 2.0 | 1.4 |
| LG12-07166 | 0.7 | 1.2 | 1.2 | 1.0 |

### EXAMPLE 9

### Evaluation of amino acid deletions in the region of residues 53 to 58 of LG12 wild-type protease

One or more deletions at positions 53, 55, 56, 57, and/or 58 were made in the LG12 wild-type backbone. These variants were tested using the methods described in Example 1. Results for cleaning performance across multiple assays are reported in Table 12 as compared to the LG12 wild-type parent protease.

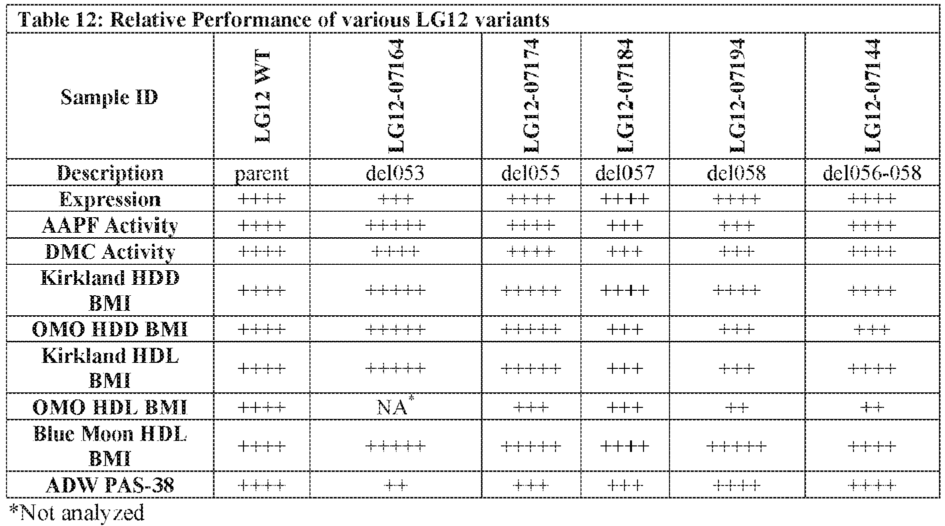

This data suggests that modifications to decrease the size of the 55 to 59 loop formed by these residues in the LG12 structure can be accommodated and may in some cases impart improved cleaning performance.

### EXAMPLE 10

### Sequence Comparison of LG12-clade Protease Variants

### Alignment of select LG12 variants with other subtilisins

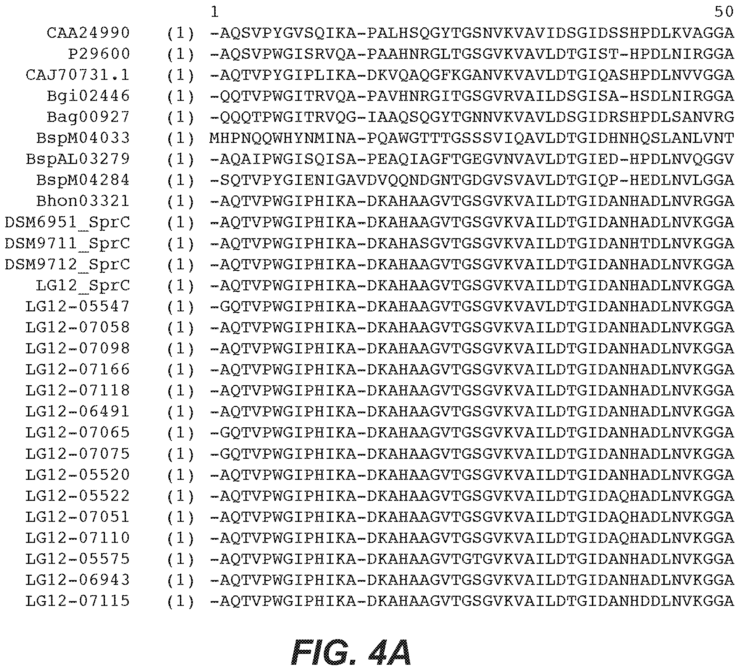

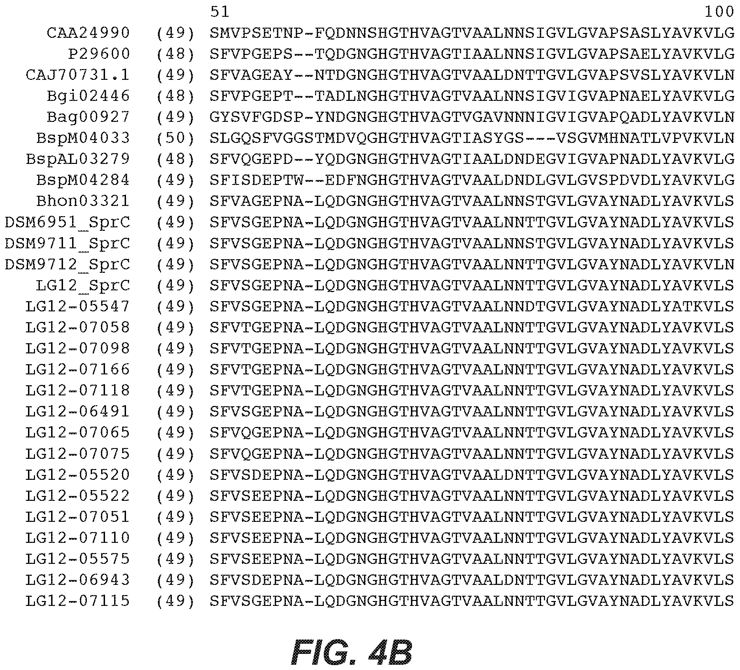

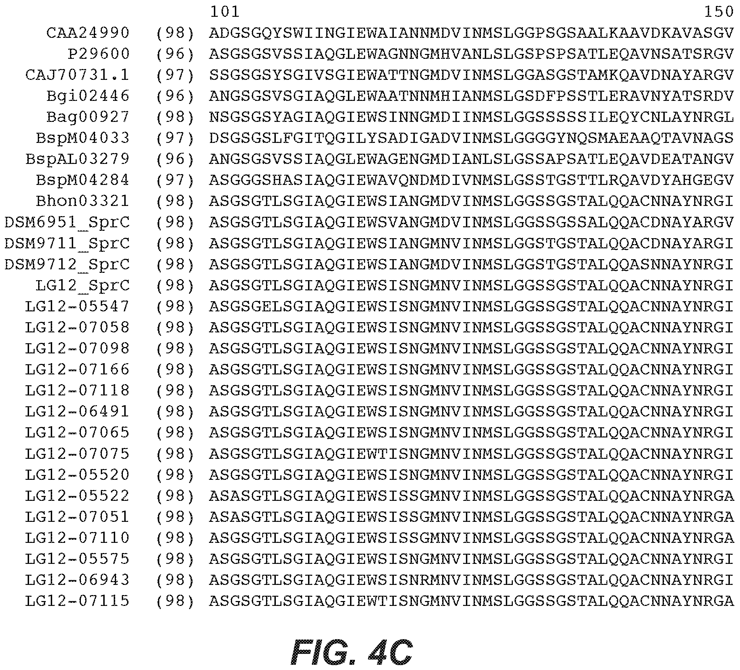

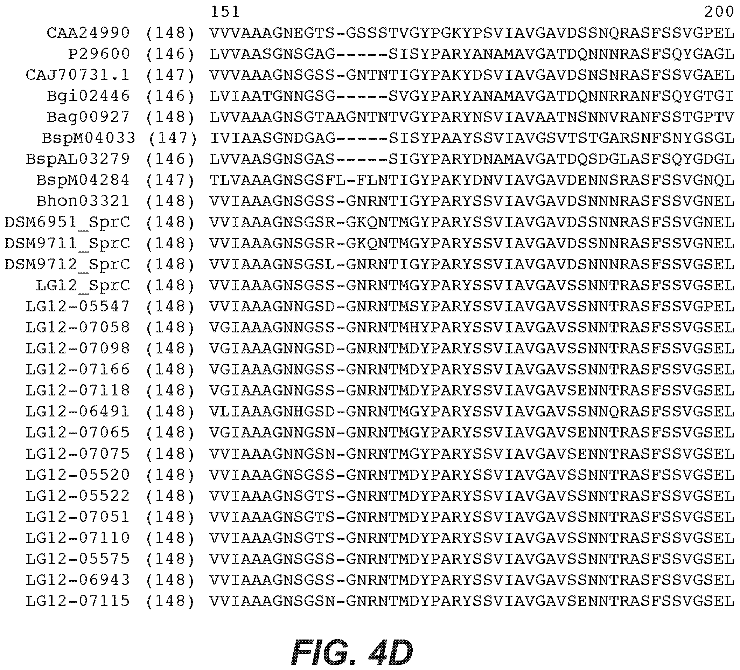

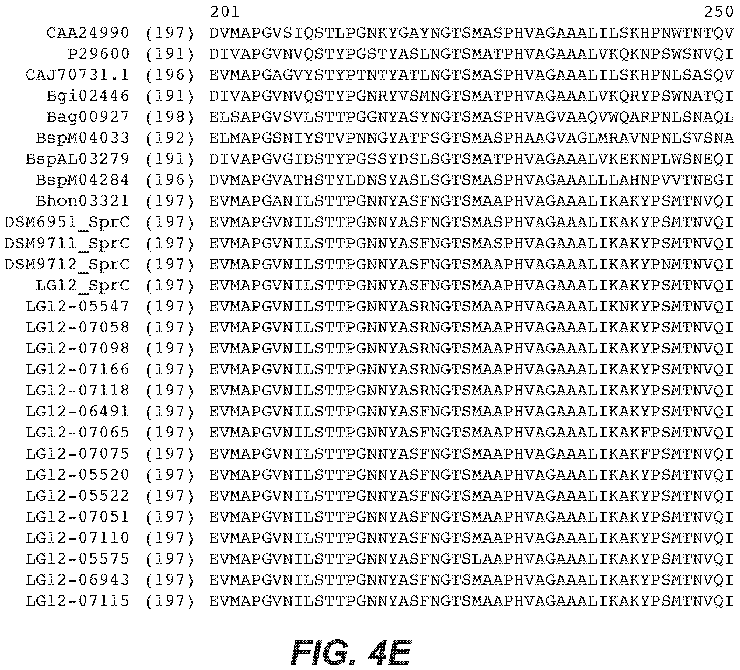

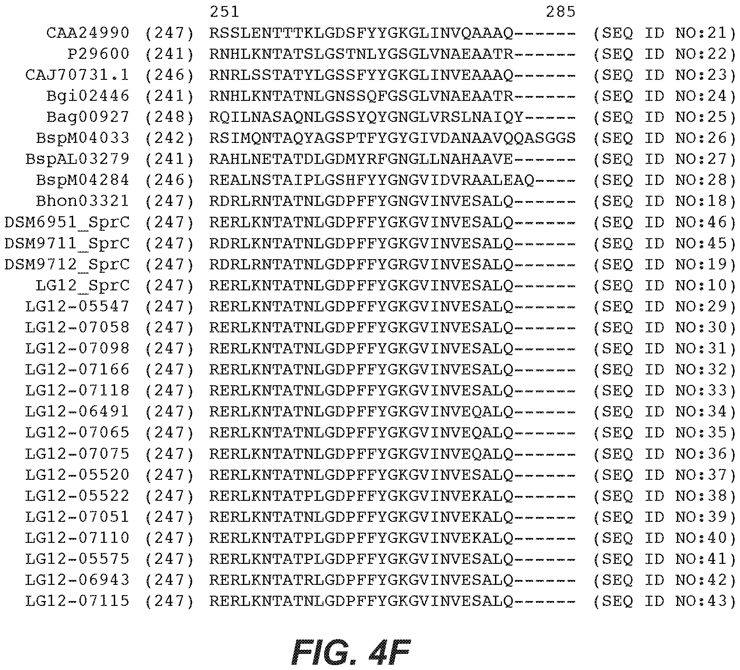

The amino acid sequences of LG12 wild-type (SEQ ID NO:10), LG12-05520 (SEQ ID NO:37), LG12-05522 (SEQ ID NO:38), LG12-05547 (SEQ ID NO:29), LG12-05575 (SEQ ID NO:41), LG12-06491 (SEQ ID NO:34), LG12-06943 (SEQ ID NO:42), LG12-07051 (SEQ ID NO:39), LG12-07058 (SEQ ID NO:30), LG12-07065 (SEQ ID NO:35), LG12-07075 (SEQ ID NO:36), LG12-07098 (SEQ ID NO:31), LG12-07110 (SEQ ID NO:40), LG12-07115 (SEQ ID NO:43), LG12-07118 (SEQ ID NO:33), and LG12-07166 (SEQ ID NO:32) were aligned with sequences of previously identified LG12-clade members: Bhon03321 (SEQ ID NO:18), DSM_9712 (SEQ ID NO:19), DSM_9711 (SEQ ID NO:45), and DSM_6951 (SEQ ID NO:46). The alignment also included sequences of the following additional subtilisin proteases: *B. amyloliquefacien*_CAA24990 (SEQ ID NO:21), *B_lents*_P29600 (SEQ ID NO:22), AprL(SEQ ID NO:23), Bag00927(SEQ ID NO:25), Bgi02446(SEQ ID NO:24), BspAL03279(SEQ ID NO:27), BspM04033(SEQ ID NO:26), and BspM04284 (SEQ ID NO:28) using CLUSTALW software (Thompson et al., Nucleic Acids Research, 22:4673-4680, 1994) with the default parameters. The alignment was performed using the following parameters: Kimura's correction for sequence distance and ignoring positions with gaps. The NJ method works on a matrix of distances between all pairs of sequences to be analyzed. These distances are related to the degree of divergence between the sequences. Figure 4 shows the alignment of these protease sequences. The position of the 4 amino acid delete that is observed in LG12 SprC when compared to *B. lentus* (P29600) sequence, located at positions encompassing G160DEL-N161DEL-R162DEL-N163DEL, was manually adjusted on this alignment to match details from the crystal structures.

### EXAMPLE 11

### Phylogenetic Tree for LG12-clade Protease Variants and Other Subtilisins

A phylogenetic tree for amino acid sequences of LG12 (SEQ ID NO:10), LG12-05520 (SEQ ID NO:37), LG12-05522 (SEQ ID NO:38), LG12-05547 (SEQ ID NO:29), LG12-05575 (SEQ ID NO:41), LG12-06491 (SEQ ID NO:34), LG12-06943 (SEQ ID NO:42), LG12-07051 (SEQ ID NO:39), LG12-07058 (SEQ ID NO:30), LG12-07065 (SEQ ID NO:35), LG12-07075 (SEQ ID NO:36), LG12-07098 (SEQ ID NO:31), LG12-07110 (SEQ ID NO:40), LG12-07115 (SEQ ID NO:43), LG12-07118 (SEQ ID NO:33), LG12-07166 (SEQ ID NO:32), LG12-CP022 (SEQ ID NO:1), LG12-CP180 (SEQ ID NO:3), LG12-CP446 (SEQ ID NO:4), LG12-CP455 (SEQ ID NO:5), LG12-CP460 (SEQ ID NO:6), LG12-CP462 (SEQ ID NO:7), and LG12-CP465 (SEQ ID NO:8) was built using sequences of previously identified LG12-clade members: Bhon03321 (SEQ ID NO: 18), DSM_9712 (SEQ ID NO:19, DSM_9711 (SEQ ID NO:45), DSM_6951 (SEQ ID NO:46), and sequences of additional subtilisin proteases: B. *amyloliquefaciens*_CAA24990 (SEQ ID NO:21), *B_lentus*_P29600(SEQ ID NO:22), AprL(SEQ ID NO:23), Bag00927(SEQ ID NO:25), Bgi02446(SEQ ID NO:24), BspAL03279(SEQ ID NO:27), BspM04033(SEQ ID NO:26), and BspM04284 (SEQ ID NO:28). The sequences were entered in the Vector NTI Advance suite and a Guide Tree was created using the Neighbor Joining (NJ) method (Saitou and Nei, Mol Biol Evol, 4:406-425, 1987). The tree construction was calculated using the following parameters: Kimura's correction for sequence distance and ignoring positions with gaps. The phylogenetic tree shown in Figure 5 was visualized using Geneious 8.1. (Biomatters Ltd.). The branch length is a measure of the amount of divergence between two nodes in the tree and is expressed in units of substitutions per site of the sequence alignment.

### EXAMPLE 12

### Sequence Motif and Structural Relationship of the LG12-clade Proteases

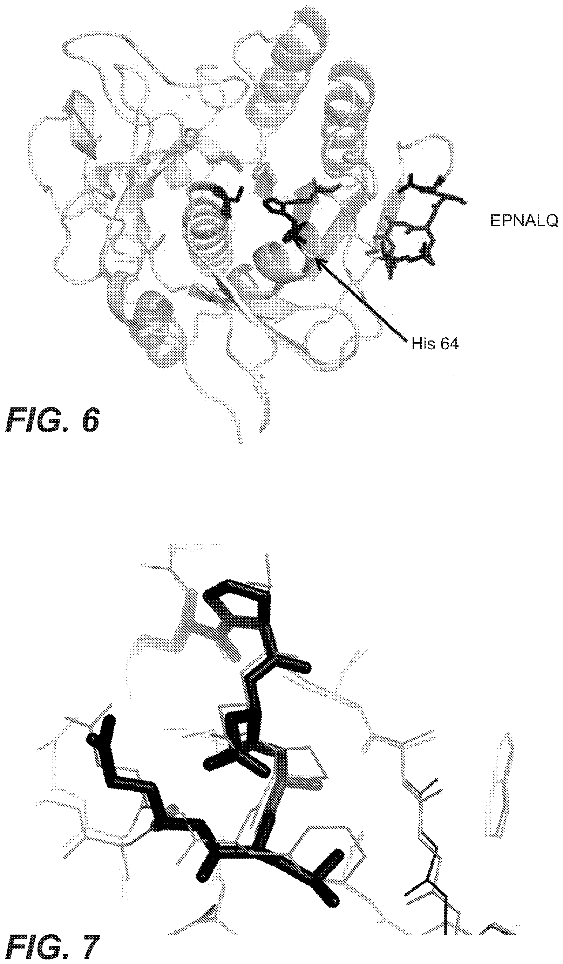

The LG12-clade motif E**PNAL**QDGNGHGTH includes the penta residue segment proline, asparagine, alanine, leucine, and glutamine at positions 54-59, which is a segment leading into the Histidine 64 of the catalytic triad. The relative juxtaposition of this segment with respect to the catalytic triad in the crystallographic model of LG12 is shown in Figure 6. Figure 6 shows a schematic of the overall fold of the main chain from the crystallographic model of LG12 wild-type protease in light gray. The side chains of the catalytic triad formed by Aspartic acid 32, Histidine 64 and Serine 221 are shown as black stick figures. Histidine 64 is indicated by the arrow. The region including residues 54-59 leading into His 64 of the triad are also shown as black slick figures.

This residue 54-59 segment of the molecule is seen to be in a loop leading into the helix that begins with Histidine 64. This residue 54-59 loop is critical in that it leads into and affects the juxtaposition of the following His64 and also that it forms part of the substrate binding surface.

Several subtilisin clades can be characterized by the sequences found between the Asp and Histidine that form the characteristic triad found in all classic serine hydrolases, both of the subtilisin and chymotrypsin type families. The conformation of the LG12-clade loop for the segment corresponding to residues 54-59 is distinctly different from that same loops found in the crystallographic models of sustilisin BPN', subtilisin Carlsberg and *Bacillus lentus* proteases, as shown in Figures 7, 8, and 9, respectively.

In Figure 7, the residue 54-59 sequence EPNALQ of the crystallographic structure of LG12 wild-type protease (shown as black sticks) is compared with the equivalent loop segment in subtilisin BPN' (pdb entry 2ST1) (shown as gray lines). The proline 55 of LG12 is found at the top. The main chain conformation of the loops in subtilisin LG12 and BPN' share the same overall fold while the sequences are quite different.

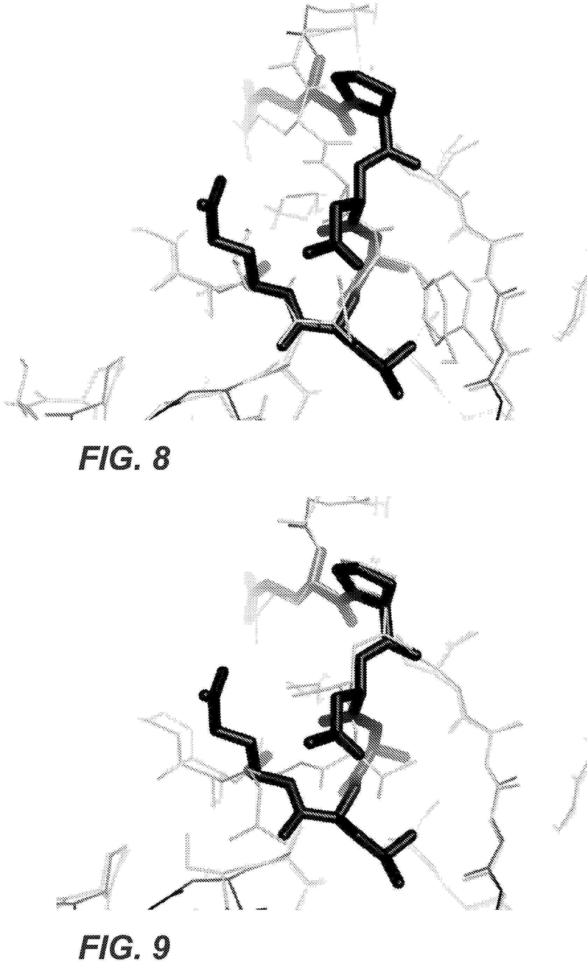

In Figure 8, the residue 54-59 sequence EPNALQ of the crystallographic structure of LG12 wild-type protease (shown as black sticks) is compared with the equivalent loop segment in subtilisin Carlsberg (pdb entry 3UNX) (shown as gray lines). In subtilisin Carlberg, there is a radical departure in the main chain conformation in this loop relative to LG12 in the vicinity of Pro55 of LG12. In the crystallographic model, this segment shows some disorder as modelled for the tyrosine side chain in the coordinates of pdb entry 3UNX.

In Figure 9, the residue 54-59 sequence EPNALQ of the crystallographic structure of LG12 wild-type protease (shown as black sticks) is compared with the equivalent loop segment in subtilisin from *B. lentus* (shown as gray lines). In the *B. lentus* subtilisin the main chain diverges (including a deletion of a residue in this loop in *B. lentus*) from that seen for LG12.

The LG12-clade sequence motif, extending from residue E54 to H67 in single letter code is EPNALQDGNGHGTH (SEQ ID NO:44) wherein the amino acid positions of the motif are numbered by correspondence with the amino acid sequence of LG12 wild-type set forth in SEQ ID NO:10.

### EXAMPLE 13

### Generation of Additional LG12-clade Subtilisins

Additional LG12-clade variant proteases were generated using methods described in Example 2, resulting in from 2 to 13 beneficial mutations being introduced into the LG12 wild-type protease as shown in Table 13A. These additional LG12-clade protease variants demonstrated improved ADW cleaning performance and/or stability over LG12 wild-type, which data is set forth in Table 13B.

| **Table 13A: LG12-clade Variant Proteases** | | |
|---|---|---|
| **Sample ID** | **Sequence Substitutions Relative to LG12 WT** | **No. Mutations vs LG12** |
| LG12-05653 | N076D-G118R-G128D-S129F-S130Y-G166D-E248N | 7 |
| LG12-05663 | N076D-G118R-G128D-S129P-S130F-G166D-E248N | 7 |
| LG12-05664 | N076D-G118R-G128D-S129P-S130Y-G166D-E248N | 7 |
| LG12-05685 | N076D-G118R-G128E-S130R-G166D-E248N | 6 |
| LG12-05729 | N076D-G118R-G128H-S129K-S130W-G166D-E248N | 7 |
| LG12-05734 | N076D-G118R-G128H-S129M-S130Y-G166D-E248N | 7 |
| LG12-05735 | N076D-G118R-G128H-S129N-S130V-G166D-E248N | 7 |
| LG12-05736 | N076D-G118R-G128H-S129P-S130L-G160D-E248N | 7 |
| LG12-05737 | N076D-G118R-G128H-S129P-S130N-G166D-E248N | 7 |
| LG12-05740 | N076D-G118R-G128H-S129P-G166D-E248N | 6 |
| LG12-05741 | N076D-G118R-G128H-S129Q-S130I-G166D-E248N | 7 |
| LG12-05743 | N076D-G118R-G128H-S129Q-S130W-G166D-E248N | 7 |
| LG12-05744 | N076D-G118R-G128H-S129R-S130H-G166D-E248N | 7 |
| LG12-05746 | N076D-G118R-G128H-S130I-G166D-E248N | 6 |
| LG12-05747 | N076D-G118R-G128H-S129T-S130H-G166D-E248N | 7 |
| LG12-05748 | N076D-G118R-G128H-S129T-S130T-G166D-E248N | 7 |
| LG12-05749 | N076D-G118R-G128H-S129V-S130H-G166D-E248N | 7 |
| LG12-05865 | N076D-G118R-G128Q-S129R-S130N-G166D-E248N | 7 |
| LG12-05890 | N076D-G118R-G128R-S129V-S130E-G166D-E248N | 7 |
| LG12-05909 | N076D-G118R-G128S-S129P-S130A-G166D-E248N | 7 |
| LG12-05913 | N076D-G118R-G128S-S129V-S130Q-G166D-E248N | 7 |
| LG12-05924 | N076D-G118R-G128T-S129K-S130L-G166D-E248N | 7 |
| LG12-05958 | N076D-G118R-G128V-S129H-S130E-G166D-E248N | 7 |
| LG12-05961 | N076D-G118R-G128V-S129M-S130H-G166D-E248N | 7 |
| LG12-05980 | N076D-G118R-G128W-S129H-S130D-G166D-E248N | 7 |
| LG12-05984 | N076D-G118R-G128W-S129L-G166D-E248N | 6 |
| LG12-05986 | N076D-G118R-G128W-S129Q-S130F-G166D-E248N | 7 |
| LG12-05993 | N076D-G118R-G128W-S129Y-S130D-G166D-E248N | 7 |
| LG12-06010 | N076D-G118R-G128Y-S129P-S130D-G166D-E248N | 7 |
| LG12-06017 | N076D-G118R-G128Y-S129V-S130M-G166D-E248N | 7 |
| LG12-06024 | G128T-S129H-S130D-G166D-E248N | 5 |
| LG12-06025 | G118R-G128L-S129M-S130N-G166D-E248N | 6 |
| LG12-06581 | N038T-S156H-G166Y-M222L | 4 |
| LG12-06945 | G053D-N076D-G118R-N256R-S272K | 5 |
| LG12-06971 | N076D-G118R-G166D-N236R | 4 |
| LG12-07057 | A001G-A040D-A048Q-S052T-G053E-Y091H-S114T-I147A-S156N-S159N-G166H-S182E-Y238F | 13 |
| LG12-07078 | A048E-S052T-V149G-S156N-G166D-F217R | 6 |
| LG12-07088 | S052T-V149G-S156N-S159D-G166H-F217R | 6 |
| LG12-07189 | S159D-G166D-S182E-F217R | 4 |
| LG12-07525 | G100A-G128H-S129F-S272K | 4 |
| LG12-07638 | N038Q-S052T-G100A-N1 17S-G118R-G128H | 6 |
| LG12-07890 | A040D-S052Q-G118R-G128M-S130Y-G166H-Y238F-N269K-V270C-E271G-S272K-A273V-275Y | 13 |
| LG12-07990 | S052T-N076D-G118R-G128M-S129V-S130F-G166H-Y238F | 8 |
| LG12-07997 | A040D-S052T-N076D-G128M-S129F-S130Y-G166H | 7 |
| LG12-08062 | S052T-N076D-G128S-S129F-S130F | 5 |
| LG12-08079 | S052T-S129F | 2 |
| LG12-08114 | N076D-G118R-G128M-S129F-S130F-Y238F | 6 |
| LG12-08137 | A040D-S052Q-N076D-G128S-S129F-S130Y-Y238F | 7 |
| LG12-08219 | K027M-G053Y-G128D-S129R-S130I-G166D-F217R-E248K-N256E-S272K | 10 |
| LG12-08225 | G007C-S052T-N076D-G128D-S129R-S130I-S156N-G166D-F217R-S272K | 10 |
| LG12-08281 | G128D-S129R-S130I-S156T-G166D-F217R-S272K | 7 |
| LG12-08308 | S032T-N076D-G118R-G128D-S129R-S130I-G166D-F217R-N256R | 9 |
| LG12-08355 | G128D-S129R-S130I-G166H-F217R-S272K | 6 |
| LG12-08419 | G053D-G118R-G128D-S129R-S130I-S156N-G166D-F217R-E248K | 9 |
| LG12-08478 | S052T-G053Y-N076D-G118R-G128D-S129F-S130Y-S156N-G166D-F217R-N256E-S272K | 12 |
| LG12-08598 | S052T-G053D-N076D-G128D-S129F-S130Y-S156N-F217R-N256E-S272K | 10 |
| LG12-08634 | S052F-N076D-G118R-G128D-S129F-S130Y-S156N-G166D-F217R-E248N-S272K | 11 |
| LG12-08636 | S052F-G053Y-N076D-G118R-G128D-S129F-S130Y-S156N-G166D-F217RE248N-N256E-S272K | 13 |
| LG12-08653 | S052F-N076D-G118R-G128D-S129F-S130Y-G166D-F217R-E248N | 9 |
| LG12-08677 | N076D-G118R-G128D-S129F-S130Y-G166D-F217R-E248N-N256E-S272K | 10 |
| LG12-08680 | N076D-G118R-G128D-S129F-S130Y-G166D-F217R-E248N-N256E | 9 |
| LG12-08684 | N076D-G118R-G128D-S129F-S130Y-S156N-G166D-F217R-E248N | 9 |
| LG12-08705 | S052T-N076D-G118R-G128D-S129F-S130Y-G166D-F217R-E248N-S272K | 10 |
| LG12-08706 | N076D-G118R-G128D-S129F-S130Y-S156N-G166D-F217R-E248N-S272K | 10 |
| LG12-08721 | S052F-G053Y-N076D-G118R-G128D-S129F-S130Y-G166D-F217RE248N-N256R-S272K | 12 |
| LG12-08727 | S052T-G053Y-N076D-G118R-G128D-S129F-S130Y-S156N-G166D-E248N | 10 |
| LG12-08731 | S052F-N076D-G118R-G128D-S129F-S130Y-G166D-F217R-E248N-N256E-S272K | 11 |
| LG12-08740 | S052F-N076D-G118R-G128D-S129F-S130Y-G166D-F217R-E248N-N256E | 10 |
| LG12-08755 | G007C-S052T-G053D-N076D-G118R-G128D-S129F-S130Y-G166D-F217R-E248N-N256E | 12 |
| LG12-08756 | S052F-G053Y-N076D-G118R-G128D-S129F-S130Y-G166D-E248N-N256E-S272K | 11 |
| LG12-08759 | N076D-G118R-G128D-S129F-S130Y-S156N-G166D-E248N-S272K | 9 |
| LG12-08764 | S052F-G053Y-G118R-G128D-S129F-S130Y-S156N-G166D-E248N-N256E-S272K | 11 |
| LG12-08780 | G053D-N076D-G118R-G128D-S129F-S130Y-G166D-E248N-S272K | 9 |
| LG12-08781 | S052F-N076D-G118R-G128D-S129F-S130Y-G166D-F217R-E248N-Q275R | 10 |
| LG12-08830 | S052F-N076D-G118R-G128D-S129F-S130Y-G166D-F217R-E248N-N256R | 10 |
| LG12-08841 | S052F-N076D-G118R-G128D-S129F-S130Y-G166D-F217R-E248N-N256R-S272K | 11 |
| LG12-08843 | S052F-N076D-G118R-G128D-S129F-S130Y-G166D-F217R-E248N-S272K | 10 |
| LG12-08863 | S052F-G053Y-N076D-G118R-G128D-S129F-S130Y-G166D-F217RE248N-S272K | 11 |
| LG12-08867 | S052F-N076D-G118R-G128D-S129F-S130Y-G166H-F217R-E248N-N256E-S272K | 11 |
| LG12-08870 | S052T-G053Y-N076D-G118R-G128D-S129F-S130Y-S156N-G166D-E248N-S272K | 11 |
| LG12-08875 | S052T-N076D-G118R-G128D-S129F-S130Y-S136N-G166D-E248N-N256E-S272K | 11 |
| LG12-08883 | N076D-G118R-G128D-S129F-S130Y-S156N-G166D-E248N-N256R | 9 |
| LG12-08888 | S052T-N076D-G118R-G128D-S129F-S130Y-S156N-G166H-F217R-E248N-N256R | 11 |
| LG12-08904 | S052F-N076D-G118R-G128D-S129F-S130Y-S156N-G166D-E248N-S272K | 10 |
| LG12-08913 | N076D-G118R-I122V-G128D-S129F-S130Y-G166D-F217R-E248N | 9 |
| LG12-08921 | S052T-N076D-G118R-G128D-S129F-S130Y-G166D-F217R-E248N-N256R | 10 |
| LG12-08933 | S052F-G053Y-N076D-G118R-G128D-S129F-S130Y-G166D-F217RE248N-N256E | 11 |
| LG12-08940 | S052F-N076D-G118R-G128D-S129F-S130Y-S156N-G166D-E248N | 9 |
| LG12-08957 | S052T-G128V-S129M-S130H-S156D-G166D-F217R-E248N-S272K | 9 |
| LG12-08960 | G128V-S129M-S130H-G166D-F217R-E248N-S272K | 7 |
| LG12-08968 | S052T-G053Y-G128V-S129M-S130H-S156D-G166H-F217R-E248N-N256E | 10 |
| LG12-08981 | G128V-S129M-S130H-G166D-E248N-S272K | 6 |
| LG12-08982 | S052T-N076D-G128V-S129M-S130H-G166D-F217R-E248N-N256E-S272K | 10 |
| LG12-08996 | S052T-N076D-G118R-G128V-S129M-S130H-G166D-F217R-N256E-S272K | 10 |
| LG12-09018 | N076D-G128V-S129M-S130H-S156D-G166D-F217R-E248N-S272K | 9 |
| LG12-09029 | S052T-G128V-S129M-S130H-G166D-F217R-E248N-N256E-S272K | 9 |
| LG12-09062 | S052T-N076D-G118R-G128V-S129M-S130H-G166D-F217R-S272K | 9 |
| LG12-09071 | S052T-G118R-G128V-S129M-S130H-G166D-F217R-S272K | 8 |
| LG12-09079 | G118R-G128V-S129M-S130H-S156D-G166D-F217R-E248N | 8 |
| LG12-09092 | S052T-N076D-G118R-G128V-S129M-S130H-G166D-F217R-E248N-N256E-S272K | 11 |
| LG12-09107 | N076D-G118R-G128V-S129M-S130H-S156D-G166D-F217R-E248N | 9 |
| LG12-09111 | S052T-G053Y-G128V-S129M-S130H-S156D-G166H-F217R-N256E-S272K | 10 |
| LG12-09112 | G128V-S129M-S130H-G166D-F217R-E248N | 6 |
| LG12-09119 | S052T-G053Y-G128V-S129M-S130H-G166D-E248N-S272K | 8 |
| LG12-09124 | S052T-G053Y-N076D-G118R-G128V-S129M-S130H-G166D-F217R-E248N | 10 |
| LG12-09142 | G053Y-G118R-G128V-S129M-S130H-G166D-E248N-N256E-S272K | 9 |
| LG12-09147 | G128V-S129M-S130H-G166D-F217R-E248D-S272K | 7 |
| LG12-09156 | G128V-S129M-S130H-G166D-E248N | 5 |
| LG12-09158 | N076D-G118R-G128V-S129M-S130H-G166D-E248N-N256E-S272K | 9 |
| LG12-09167 | S052T-G053Y-N076D-G118R-G128V-S129M-S130H-S156D-G166D-F217R-E248N-S272K | 12 |
| LG12-09176 | S052T-N076D-G118R-G128V-S129M-S130H-G166D-E248N-N256E-S272K | 10 |
| LG12-09210 | N076D-G118R-G128V-S129M-S130H-G166D-F217R | 7 |
| LG12-09245 | S052T-N076D-G118R-G128V-S129M-S130H-S156D-G166D-F217RE248N-N256E-S272K | 12 |
| LG12-09260 | G128V-S129M-S130H-F217R-E248N | 5 |

| **Table13B. Cleaning performance of Additional LG12-clade Variants, Reported as PI** | | | |
|---|---|---|---|
| **Sample ID** | **PAS-38 in GSM-B** | **Stability TRIS-CA** | **Stability TRIS-EDTA** |
| LG12 WT | 1.0 | 1.0 | 1.0 |
| LG12-05653 | 2.6 | 0.4 | 0.5 |
| LG12-05663 | 2.6 | 2.2 | 1.8 |
| LG12-05664 | 2.8 | 2.5 | 2.0 |
| LG12-05685 | 2.6 | 1.6 | 1.2 |
| LG12-05729 | 4.0 | 2.5 | 1.8 |
| LG12-05734 | 2.9 | 1.9 | 1.4 |
| LG12-05735 | 2.3 | 1.9 | 1.6 |
| LG12-05736 | 3.4 | 2.6 | 1.7 |
| LG12-05737 | 3.1 | 2.7 | 1.8 |
| LG12-05740 | 4.1 | 2.5 | 1.7 |
| LG12-05741 | 2.5 | 2.1 | 1.3 |
| LG12-05743 | 3.7 | 1.5 | 1.3 |
| LG12-05744 | 2.7 | 2.4 | 1.4 |
| LG12-05746 | 2.4 | 2.3 | 1.7 |
| LG12-05747 | 2.8 | 2.2 | 1.4 |
| LG12-05748 | 3.3 | 2.3 | 1.7 |
| LG12-05749 | 2.9 | 1.7 | 1.3 |
| LG12-05865 | 2.4 | 2.0 | 1.3 |
| LG12-05890 | 2.1 | 1.5 | 1.5 |
| LG12-05909 | 2.2 | 2.2 | 1.2 |
| LG12-05913 | 5.1 | 1.8 | 1.4 |
| LG12-05924 | 2.1 | 1.8 | 1.4 |
| LG12-05958 | 1.6 | 2.0 | 1.3 |
| LG12-05961 | 6.9 | 1.1 | 1.1 |
| LG12-05980 | 1.5 | 1.7 | 1.5 |
| LG12-05984 | 1.8 | 1.6 | 1.8 |
| LG12-05986 | 2.2 | 2.4 | 2.2 |
| LG12-05993 | 2.2 | 1.9 | 1.4 |
| LG12-06010 | 1.3 | 2.1 | 1.6 |
| LG12-06017 | 2.0 | 1.5 | 1.3 |
| LG12-06024 | 1.5 | 1.6 | 1.4 |
| LG12-06025 | 1.9 | 1.6 | 1.4 |
| LG12-06581 | 2.7 | 1.3 | 1.4 |
| LG12-06945 | 1.7 | 2.3 | 1.2 |
| LG12-06971 | 1.8 | 2.0 | 1.4 |
| LG12-07057 | 1.4 | 1.8 | 2.2 |
| LG12-07078 | 1.5 | 2.0 | 2.3 |
| LG12-07088 | 1.6 | 1.7 | 2.1 |
| LG12-07189 | 1.3 | 2.1 | 2.7 |
| LG12-07525 | 1.6 | 1.3 | 1.3 |
| LG12-07638 | 2.0 | 1.4 | 1.3 |
| LG12-07890 | 1.3 | 2.4 | 1.2 |
| LG12-07990 | 4.2 | 1.8 | 1.4 |
| LG12-07997 | 1.3 | 1.7 | 1.3 |
| LG12-08062 | 1.6 | 1.2 | 1.4 |
| LG12-08079 | 1.4 | 1.7 | 1.2 |
| LG12-08114 | 2.9 | 1.3 | 1.7 |
| LG12-08137 | 2.2 | 1.3 | 1.7 |
| LG12-08219 | 2.3 | 4.1 | 2.0 |
| LG12-08225 | 1.9 | 1.4 | 1.3 |
| LG12-08281 | 1.8 | 2.9 | 2.9 |
| LG12-08308 | 1.2 | 3.6 | 3.0 |
| LG12-08355 | 1.4 | 2.5 | 2.4 |
| LG12-08419 | 3.7 | 2.3 | 3.0 |
| LG12-08478 | 1.4 | 9.8 | 3.9 |
| LG12-08598 | 1.7 | 3.5 | 2.6 |
| LG12-08634 | 1.2 | 2.2 | 2.2 |
| LG12-08636 | 2.0 | 3.0 | 3.7 |
| LG12-08653 | 1.5 | 2.2 | 2.1 |
| LG12-08677 | 1.4 | 2.3 | 2.3 |
| LG12-08680 | 1.3 | 2.4 | 2.1 |
| LG12-08684 | 1.4 | 3.3 | 2.6 |
| LG12-08705 | 1.3 | 1.4 | 1.9 |
| LG12-08706 | 1.3 | 2.7 | 2.7 |
| LG12-08721 | 5.7 | 1.6 | 3.2 |
| LG12-08727 | 1.3 | 2.0 | 1.8 |
| LG12-08731 | 1.4 | 1.7 | 2.3 |
| LG12-08740 | 1.4 | 2.1 | 2.2 |
| LG12-08755 | 1.4 | 1.5 | 1.5 |
| LG12-08756 | 3.5 | 1.4 | 2.0 |
| LG12-08759 | 1.3 | 3.2 | 2.2 |
| LG12-08764 | 2.3 | 2.6 | 2.2 |
| LG12-08780 | 1.2 | 1.3 | 1.6 |
| LG12-08781 | 2.0 | 2.2 | 2.3 |
| LG12-08830 | 1.4 | 2.0 | 1.8 |
| LG12-08841 | 1.3 | 1.4 | 2.4 |
| LG12-08843 | 1.3 | 1.5 | 2.2 |
| LG12-08863 | 2.2 | 2.2 | 2.6 |
| LG12-08867 | 1.3 | 1.3 | 2.0 |
| LG12-08870 | 1.3 | 2.4 | 2.1 |
| LG12-08875 | 1.3 | 2.0 | 2.2 |
| LG12-08883 | 1.2 | 1.8 | 2.3 |
| LG12-08888 | 1.4 | 2.0 | 2.4 |
| LG12-08904 | 1.4 | 2.4 | 2.1 |
| LG12-08913 | 1.6 | 1.5 | 2.4 |
| LG12-08921 | 1.4 | 1.5 | 2.3 |
| LG12-08933 | 2.9 | 1.7 | 3.0 |
| LG12-08940 | 1.5 | 3.0 | 2.4 |
| LG12-08957 | 1.6 | 1.2 | 1.6 |
| LG12-08960 | 1.4 | 2.7 | 2.6 |
| LG12-08968 | 1.6 | 2.3 | 1.9 |
| LG12-08981 | 2.9 | 2.3 | 2.1 |
| LG12-08982 | 1.2 | 2.5 | 2.3 |
| LG12-08996 | 1.3 | 3.2 | 3.2 |
| LG12-09018 | 1.2 | 2.3 | 1.4 |
| LG12-09029 | 1.4 | 3.0 | 2.1 |
| LG12-09062 | 1.5 | 3.1 | 2.3 |
| LG12-09071 | 1.3 | 2.7 | 2.5 |
| LG12-09079 | 1.3 | 1.3 | 1.5 |
| LG12-09092 | 1.3 | 3.1 | 2.6 |
| LG12-09107 | 1.3 | 1.5 | 1.3 |
| LG12-09111 | 1.4 | 2.3 | 2.8 |
| LG12-09112 | 1.3 | 2.0 | 2.4 |
| LG12-09119 | 2.3 | 2.5 | 2.0 |
| LG12-09124 | 1.5 | 1.8 | 2.0 |
| LG12-09142 | 1.8 | 1.9 | 2.3 |
| LG12-09147 | 2.2 | 2.1 | 1.9 |
| LG12-09156 | 3.6 | 1.8 | 2.7 |
| LG12-09158 | 2.0 | 2.6 | 2.4 |
| LG12-09167 | 1.2 | 1.5 | 1.7 |
| LG12-09176 | 1.9 | 2.5 | 2.4 |
| LG12-09210 | 1.2 | 2.7 | 2.4 |
| LG12-09245 | 1.3 | 1.8 | 1.7 |
| LG12-09260 | 1.3 | 3.2 | 2.4 |

### EXAMPLE 14

### Generation of Additional LG12-clade Subtilisins

Additional LG12-clade variant proteases were generated using methods described in Example 2, resulting in from 36 to 41 beneficial mutations being introduced into the LG12 wild-type protease as set forth in Table 14.

| **Table 14: LG12-clade Variant Proteases** | | |
|---|---|---|
| **Sample ID** | **Sequence Substitutions Relative to LG12 WT** | **No. mutations vs LG12** |
| LG12-CP00172 | S052A-N076D-T078S-T0791-Y086P-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-F217L-A224T-E248N | 36 |
| LG12-CP00195 | S052A-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144SI147V-V148L-I150V-S158T-G166D-T209Y-N212S-N213T-S216T-F217L-A224T-E248N | 39 |
| LG12-CP00227 | A001T-N076D-T078S-T079I-Y086P-D089E-S097G-T103S-L104V-G106S-I1 11L-S114A-I115G-S116N-N120H-I122A-M124L-G128S-S129P-G131P-T133A-A134T-Q136E-C139V-N141S-Y143T-N144SI147V-V148L-I150V-S158T-G166D-T209Y-N212S-N213T-S216T-F217L-E248N | 38 |
| LG12-CP00234 | A001T-S052A-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-A153S-S158T-G166D-T209Y-N212S-N213T-S216T-F217L-A224T-E248N | 41 |
| LG12-CP00376 | A001T-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144SI147V-V148L-I150V-A153S-S158T-G166D-T209Y-N212S-N213T-S216T-F217L-A224T-E248N | 40 |
| LG12-CP00415 | A001T-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144SI147V-V148L-I150V-S158T-G166D-T209Y-N212S-N213T-S216T-F217L-A224T-E248N-Q275H | 40 |
| LG12-CP00466 | A001T-N076D-T078S-T079I-Y086P-N087S-A088V-D089S-S097G-T103S-L104V-G106S-I111L-S114A-T115G-S116N-N120H-I122AM124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N | 41 |

### EXAMPLE 15

### Cleaning Performance and Stability of LG12-clade Variant Proteases

LG12-clade protease variants generated as described in Example 14 above and LG12 wild-type protease (SEQ ID NO: 10) were screened in MTP assays, as described in Example 1 to compare their cleaning performance and/or stability to LG12-CP462 protease (SEQ ID NO:7). The results of these comparisons are set forth in Table 15.

| **Table 15: Cleaning and Stability of LG12-clade Protease Variants Compared to LG12-CP462, reported as PI** | | | | | | | |
|---|---|---|---|---|---|---|---|
| | **EMPA-116** | | **CFT CS-38** | | **CFT C-10** | | **Stability** |
| **Sample ID** | **Persil Non-Bio** | **Test HDL** | **Persil Non-Bio** | **Test HDL** | **Persil Non-Bio** | **Test HDL** | **Persil Non-Bio** |
| LG12-CP462 | 1.0 | 1.0 | 1.0 | 1.0 | 1.0 | 1.0 | 1.0 |
| LG12-CP00466 | 1.0 | 0.9 | 1.1 | 1.0 | 1.2 | 1.1 | 0.9 |
| LG12-CP00195 | 0.9 | 0.9 | 1.2 | 1.3 | 1.2 | 1.2 | 1.0 |
| LG12-CP00234 | 0.9 | 0.9 | 1.2 | 1.1 | 1.2 | 1.1 | 1.0 |
| LG12-CP00172 | 0.9 | 0.9 | 1.7 | 2.1 | 1.4 | 1.2 | 1.6 |
| LG12-CP00376 | 0.9 | 0.9 | 1.2 | 1.3 | 0.7 | 1.1 | 1.0 |
| LG12-CP00227 | 0.9 | 0.9 | 1.0 | 0.8 | 0.6 | 1.0 | 1.0 |
| LG12-CP00415 | 0.9 | 0.9 | 1.6 | 1.5 | 1.7 | 1.3 | 0.9 |
| LG12 WT | 0.7 | ND | 0.6 | ND | 1.2 | ND | ND |

### EXAMPLE 16

### Comparison of LG12-clade Protease Variants to Related Molecules

### Identification of Homologous Proteases

Homologs were identified by a BLAST search (Altschul et al., Nucleic Acids Res, 25:3389-402, 1997) against the NCBI non-redundant protein database and the Genome Quest (GQ) Patent database with search parameters set to default values using the mature protein amino acid sequence for LG12-CP462 protease (SEQ ID NO:7) as the query sequence. Percent identity (PID) is defined as the number of identical residues divided by the number of aligned residues in the pairwise alignment. Value labeled "sequence length" on tables corresponds to the length (in amino acids) for the proteins referenced with the listed Accession numbers, while "aligned length" refers to sequence used for alignment and PID calculation. Table 16A provides a list of sequences with the PID to LG12-CP462 from the NCBI non-redundant protein database and Table 16B provides a list of sequences from the GQ patent database.

| **Table 16A: NCBI Non-redundant Protein Database Results for LG12-CP462** | | | | |
|---|---|---|---|---|
| **Accession #** | **PID** | **Organism** | **Sequence Length** | **Alignment Length** |
| AAC43580 | 86.1 | *Bacillus sp.* | 378 | 274 |
| WP_047970940 | 82.8 | *Bacillus sp. LL01* | 377 | 274 |
| KPB03414 | 80.3 | *Bacillus sp. CHD6a* | 377 | 274 |
| WP_010192403 | 80.3 | *Bacillus sp. m3-13* | 381 | 274 |
| AAA22212.1 | 75.4 | *B. alcalophilus* | 380 | 272 |
| BAA02442.1 | 75.0 | *B. alcalophilus* | 380 | 272 |
| AFR78140 | 74.3 | *synthetic construct* | 269 | 272 |
| BAD11988 | 71.9 | *Bacillus sp. KSM-LD1 SA* | 376 | 274 |
| BAD21128 | 71.0 | *Bacillus sp. KSM-LD1 SB* | 377 | 276 |
| KJD53753 | 70.7 | *B. amyloliquefaciens* | 333 | 229 |
| WP_026080796 | 70.4 | *B. licheniformis* | 378 | 274 |
| AAG00492 | 70.4 | *B. licheniformis* | 379 | 274 |
| ADK11044 | 70.4 | *B. licheniformis* | 379 | 274 |
| WP_031314535 | 70.1 | *B. licheniformis* | 378 | 274 |
| EQM28932 | 70.1 | *B. licheniformis CG-B52* | 379 | 274 |
| AAG00493 | 70.1 | *B. licheniformis* | 310 | 274 |

| **Table 16B: GQ Patent Database Results for LG12-CP462** | | | | |
|---|---|---|---|---|
| **Patent ID #** | **PID** | **Organism** | **Sequence Length** | **Alignment Length** |
| WO2015038792-multiple sequences | 86.5-85.8 | *Bacillus sp.; LG12 Synthetic* | 275 | 274-273 |
| WO2015038792-0004 | 86.1 | *Bacillus sp.; LG12* | 381 | 274 |
| WO2015038792-0019 | 83.6 | *B. horikoshii; DSM 69511* | 275 | 274 |
| WO2015038792-0016 | 83.2 | *B. horikoshii DSM 97121* | 275 | 274 |
| WO2015038792-0010 | 83.2 | *B. horikoshii DSM 87192* | 275 | 274 |
| WO2015038792-0027 | 82.1 | *Synthetic Construct* | 268 | 274 |
| WO2015038792-0007 | 80.3 | *Bacillus sp. m3-134* | 275 | 274 |
| WO2015038792-0006 | 80.3 | *Bacillus sp. m3-132* | 383 | 274 |
| EP1160327-multiple sequences | 76.1-75.7 | *Bacillus sp. Synthetic* | 269 | 272 |
| WO9402618 | 75.7 | *Bacillus novalis* | 269 | 272 |
| US20130123162-multiple sequences | 75.74 | *B. lentus Synthetic* | 269 | 272 |
| WO2011140364-multiple sequences | 75.7-75.4 | *B. lentus Synthetic* | 380-377 | 272 |
| US20140045268-0040 | 75.4 | *Synthetic Construct* | 353 | 272 |
| WO2015144932-0006 | 75.4 | *Artificial Sequence, B. lentus* | 269 | 272 |

### EXAMPLE 17

### Sequence comparison of LG12-clade protease variants

### Alignment of LG12-clade protease variants with other subtilisins

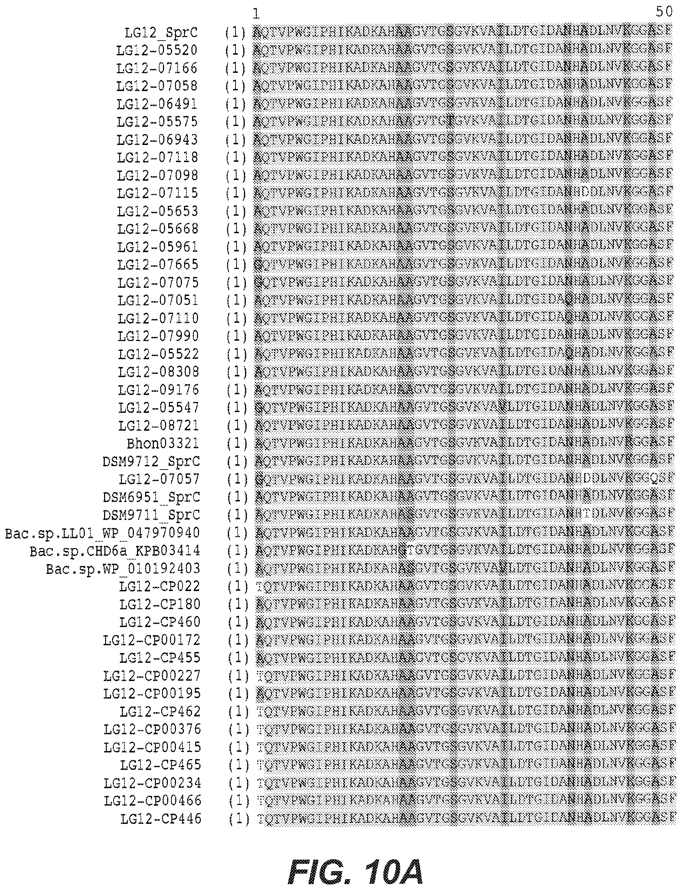

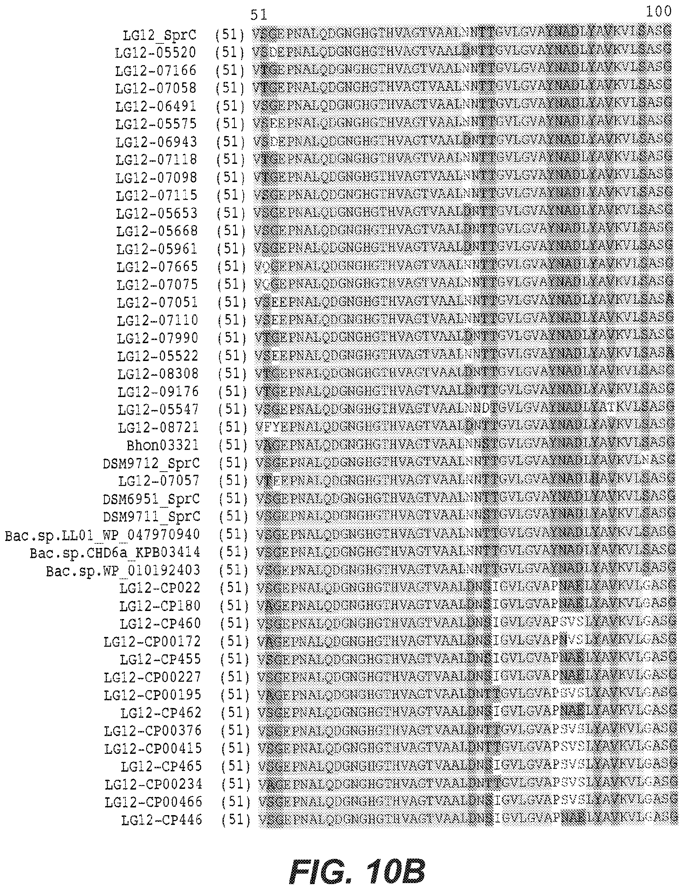

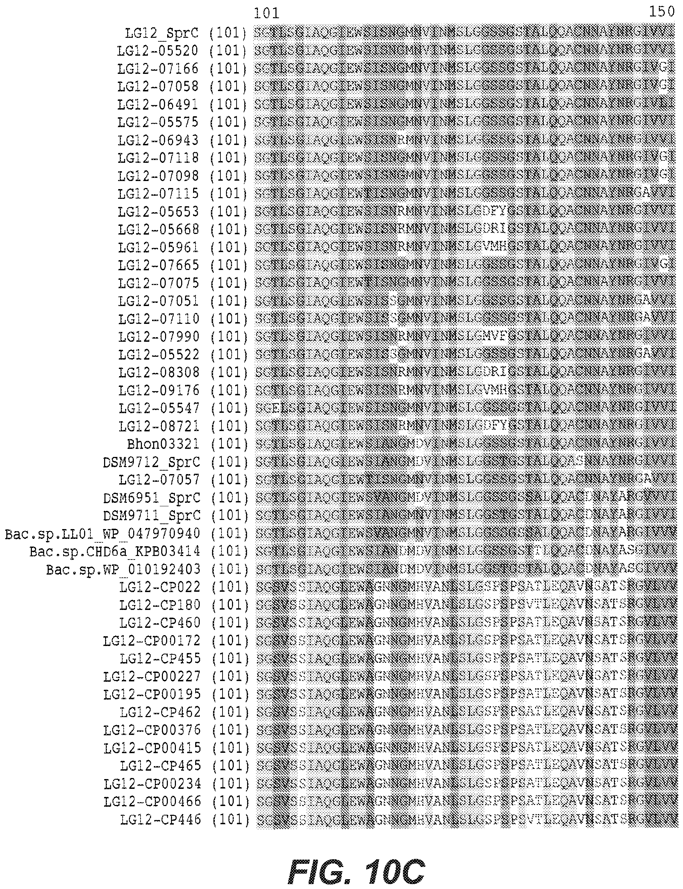

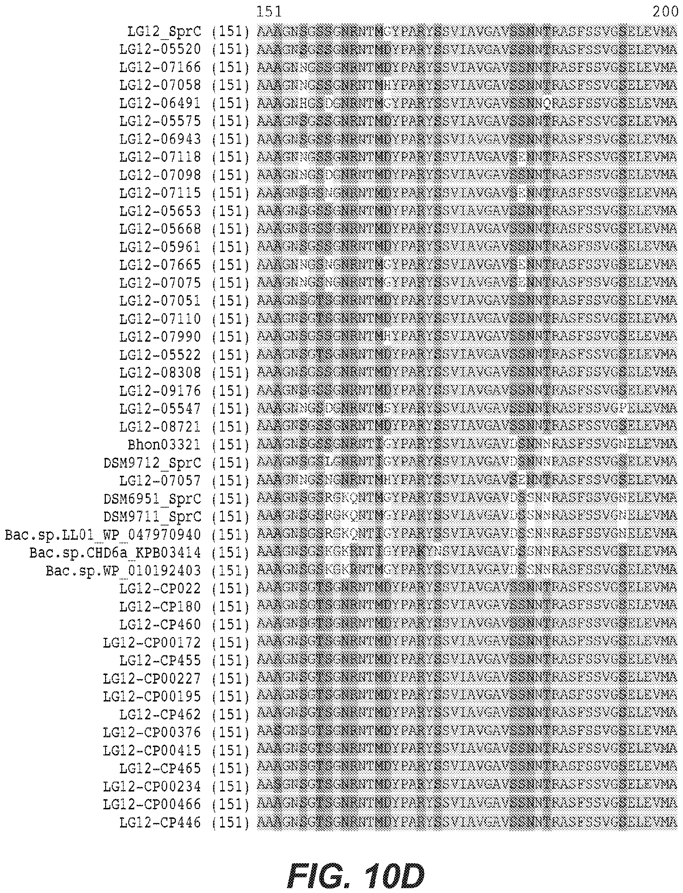

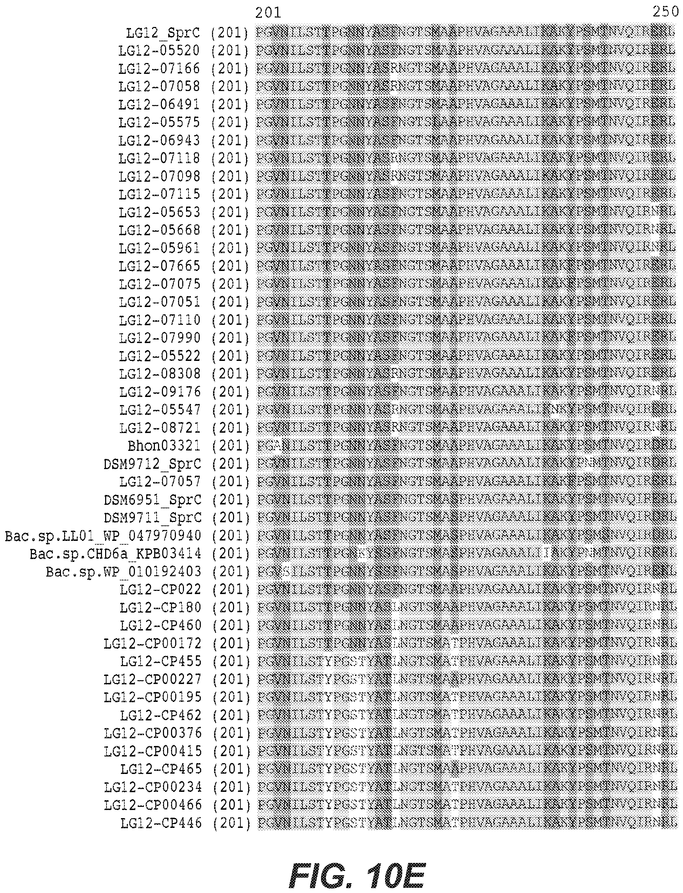

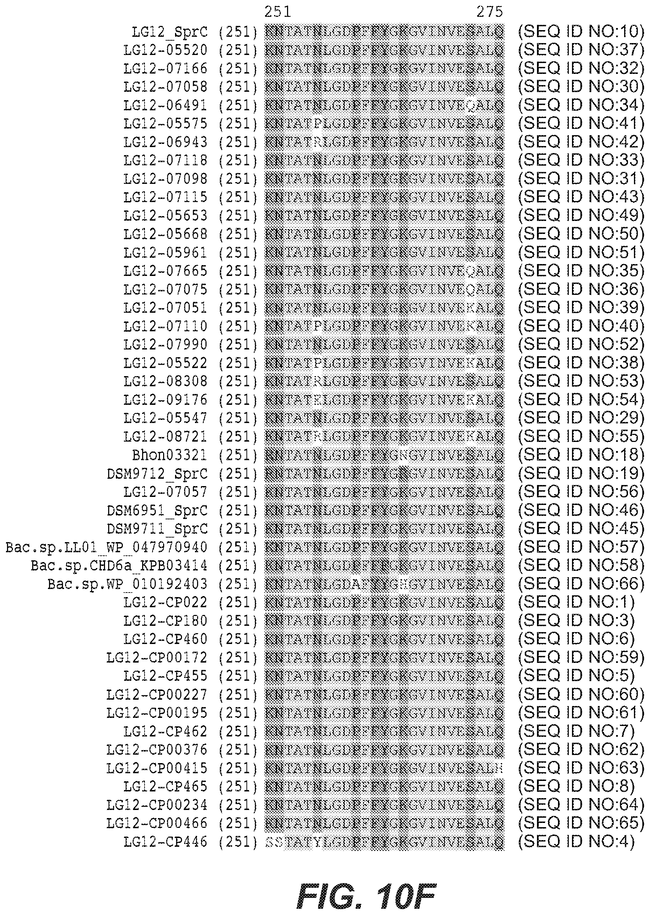

The amino acid sequences of LG12 wild-type (SEQ ID NO:10); variants: LG12-CP022 (SEQ ID NO:1), LG12-CP180 (SEQ ID NO:3), LG12-CP446 (SEQ ID NO:4), LG12-CP455 (SEQ ID NO:5), LG12-CP460 (SEQ ID NO:6), LG12-CP462 (SEQ ID NO:7), LG12-CP465 (SEQ ID NO:8), LG12-05520 (SEQ ID NO:37), LG12-05522 (SEQ ID NO:38), LG12-05547 (SEQ ID NO:29), LG12-05575 (SEQ ID NO:41), LG12-06491 (SEQ ID NO:34), LG12-06943 (SEQ ID NO:42), LG12-07051 (SEQ ID NO:39), LG12-07058 (SEQ 1D NO:30), LG12-07065 (SEQ ID NO:35), LG12-07075 (SEQ ID NO:36), LG12-07098 (SEQ ID NO:31), LG12-07110 (SEQ ID NO:40), LG12-07115 (SEQ ID NO:43), LG12-07118 (SEQ ID NO:33), LG12-07166 (SEQ ID NO:32), LG12-CP00172 (SEQ ID NO:59), LG12-CP00195 (SEQ ID NO:61), LG12-CP00227 (SEQ ID NO:60), LG12-CP00234 (SEQ ID NO:64), LG12-CP00376 (SEQ ID NO:62), LG12-CP00415 (SEQ ID NO:63), LG12-CP00466 (SEQ ID NO:65), LG12-05653 (SEQ ID NO:49), LG12-05668 (SEQ ID NO:50), LG12-05961 (SEQ ID NO:51), LG12-08308 (SEQ ID NO:53), LG12-08721 (SEQ ID NO:55), LG12-09176 (SEQ ID NO:54), LG12-07990 (SEQ ID NO:52), and LG12-07057 (SEQ ID NO:56); previously identified LG12-clade members: Bhon03321 (SEQ ID NO:18), DSM_9712 (SEQ ID NO:19), DSM_9711 (SEQ ID NO:45), DSM_6951 (SEQ ID NO:46), Bac.sp.WP_010192403 (SEQ ID NO:66); and more recently identified LG12-clade members: Bac.sp.LL01_WP_047970940 (SEQ ID NO:57) and Bac.sp.CHD6a_KPB03414 (SEQ ID NO:58), were aligned using CLUSTALW software (Thompson et al., Nucleic Acids Research, 22:4673-4680, 1994) with the default parameters. The alignment was performed using the following parameters: Kimura's correction for sequence distance and ignoring positions with gaps. The NJ method works on a matrix of distances between all pairs of sequences to be analyzed. These distances are related to the degree of divergence between the sequences. Figure 10A-F shows the alignment of these protease sequences. The sequences were entered in the Vector NTI Advance suite and a Guide Tree was created using the Neighbor Joining (NJ) method (Saitou and Nei, Mol Biol Evol, 4:406-425, 1987). The tree construction was calculated using the following parameters: Kimura's correction for sequence distance and ignoring positions with gaps. AlignX displays the calculated distance values in parenthesis following the molecule name displayed on the phylogenetic tree shown in Figure 11.

The following numbered paragraphs (paras.) contain statements of broad combinations of the inventive technical features herein disclosed:-
1. A protease variant, or a recombinant polypeptide or an active fragment thereof comprising an amino acid sequence comprising two or more substitutions at one or more positions selected from :
   (i) 1, 3, 7, 9, 19, 24, 27, 30, 38, 40, 43, 45, 48, 52, 53, 57, 72, 76, 78, 79, 86, 87, 88, 89, 90, 91, 93, 97, 99, 100, 101, 103, 104, 106, 107, 111, 114, 115, 116, 117, 118, 120, 122, 123, 124, 128, 129, 130, 131, 133, 134, 136, 139, 140, 141, 143, 144, 145, 147, 148, 149, 150, 153, 156, 158, 159, 160, 166, 181, 182, 185, 188, 194, 209, 212, 213, 215, 216, 217, 222, 224, 228, 234, 236, 238, 240, 242, 245, 246, 248, 251, 252, 256, 265, 267, 269, 270, 271, 272, 273, and 275;
   (ii) 1, 3, 9, 19, 24, 27, 30, 38, 40, 43, 45, 48, 52, 53, 57, 72, 76, 78, 79, 86, 87, 88, 89, 90, 91, 93, 97, 99, 100, 101, 103, 104, 106, 107, 111, 114, 115, 116, 117, 118, 120, 122, 123, 124, 128, 129, 130, 131, 133, 134, 136, 139, 140, 141, 143, 144, 145, 147, 148, 149, 150, 153, 156, 158, 159, 160, 166, 181, 182, 185, 188, 194, 209, 212, 213, 215, 216, 217, 222, 224, 228, 234, 236, 238, 240, 242, 245, 246, 248, 251, 252, 256, 265, 267, 271, 272, and 275;
   (iii) 1, 7, 9, 27, 38, 40, 45, 48, 52, 53, 76, 78, 91, 100, 114, 117, 118, 122, 128, 129, 130, 140, 147, 149, 156, 158, 159, 160, 166, 181, 182, 194, 209, 217, 222, 228, 234, 236, 238, 245, 248, 256, 269, 270, 271, 272, 273, and 275;
   (iv) 1, 24, 30, 38, 40, 48, 52, 53, 76, 78, 79, 86, 87, 88, 89, 91, 93, 97, 100, 103, 104, 106, 111, 114, 115, 116, 117, 118, 120, 122, 124, 128, 129, 130, 131, 133, 134, 136, 139, 141, 143, 144, 147, 148, 149, 150, 153, 156, 158, 159, 166, 182, 185, 194, 209, 212, 213, 216, 217, 222, 224, 236, 238, 248, 251, 252, 256, 272, and 275;
   (v) 1,24,30,38,40,52,53, 76, 78, 79, 86, 87, 88, 89, 93, 97, 100, 103, 104, 106, 111, 114, 115, 116, 117, 118, 120, 122, 124, 128, 129, 131, 133, 134, 136, 139, 141, 143, 144, 147, 148, 149, 150, 153, 156, 158, 159, 166, 182, 185, 194, 209, 212, 213, 216, 217, 222, 224, 236, 238, 248, 251, 252, 256, 272, and 275; or
   (vi) 1, 38, 40, 48, 52, 53, 76, 91, 114, 117, 118, 128, 129, 130, 147, 149, 156, 158, 159, 166, 182, 217, 238, 248, 256, and 272;
   wherein the amino acid positions of the variant or recombinant polypeptide or active fragment thereof are numbered by correspondence with the amino acid sequence of SEQ ID NO:10.
2. The protease variant, or recombinant polypeptide or active fragment thereof of any preceding para. , wherein said variant or recombinant polypeptide or active fragment thereof comprises an amino acid sequence comprising two or more substitutions selected from:
   (i) A1X, T3X, G7X, P9X, A19X, S24X, K27X, I30X, N38X, A40X, N43X, K45X, A48X, S52X, G53X, A57X, V72X, N76X, T78X, T79X, Y86X, N87X, A88X, D89X, L90X, Y91X, V93X, S97X, S99X, G100X, S101X, T103X, L104X, G106X, I107X, I111X, S114X, I115X, S116X, N117X, G118X, N120X, I122X, N123X, M124X, G128X, S129X, S130X, G131X, T133X, A134X, Q136X, C139X, N140X, N141X, Y143X, N144X, R145X, I147X, V148X, V149X, 1150X, A153X, S156X, S158X, S159X, G160X, G166X, S181X, S182X, T185X, S188X, S194X, T209X, N212X, N213X, A215X, S216X, F217X, M222X, A224X, A228X, I234X, A236X, Y238X, S240X, T242X, Q245X, I246X, E248X, K251X, N252X, N256X, K265X, V267X, N269X, V270X, E271X, S272X, A273X, and Q275X;
   (ii) A1X, T3X, P9X, A19X, S24X, K27X, I30X, N38X, A40X, N43X, K45X, A48X, S52X, G53X, A57X, V72X, N76X, T78X, T79X, Y86X, N87X, A88X, D89X, L90X, Y91X, V93X, S97X, S99X, G100X, S101X, T103X, L104X, G106X, I107X, I111X, S114X, I115X, S116X, N117X, G118X, N120X, 1122X, N123X, M124X, G128X, S129X, S130X, G131X, T133X, A134X, Q136X, C139X, N140X, N141X, Y143X, N144X, R145X, I147X, V148X, V149X, I150X, A153X, S156X, S158X, S159X, G160X, G166X, S181X, S182X, T185X, S188X, S194X, T209X, N212X, N213X, A215X, S216X, F217X, M222X, A224X, A228X, I234X, A236X, Y238X, S240X, T242X, Q245X, I246X, E248X, K251X, N252X, N256X, K265X, V267X, E271X, S272X, and Q275X;
   (iii) A1X, G7X, P9X, K27X, N38X, A40X, K45X, A48X, S52X, G53X, N76X, T78X, Y91X, G100X, S114X, N117X, G118X, I122X, G128X, S129X, S130X, N140X, I147X, V149X, S156X, S158X, S159X, G160X, G166X, S181X, S182X, S194X, T209X, F217X, M222X, A228X, I234X, A236X, Y238X, Q245X, E248X, N256X, N269X, V270X, E271X, S272X, A273X, and Q275X;
   (iv) A1X, S24X, I30X, N38X, A40X, A48X, S52X, G53X, N76X, T78X, T79X, Y86X, N87X, A88X, D89X, Y91X, V93X, S97X, G100X, T103X, L104X, G106X, I111X, S114X, I115X, S116X, N117X, G118X, N120X, I122X, M124X, G128X, S129X, S130X, G131X, T133X, A134X, Q136X, C139X, N141X, Y143X, N144X, I147X, V148X, V149X, I150X, A153X, S156X, S158X, S159X, G166X, S182X, T185X, S194X, T209X, N212X, N213X, S216X, F217X, M222X, A224X, A236X, Y238X, E248X, K251X, N252X, N256X, S272X, and Q275X;
   (v) A1X, S24X, I30X, N38X, A40X, S52X, G53X, N76X, T78X, T79X, Y86X, N87X, A88X, D89X, V93X, S97X, G100X, T103X, L104X, G106X, I111X, S114X, I115X, S116X, N117X, G118X, N120X, 1122X, M124X, G128X, S129X, G131X, T133X, A134X, Q136X, C139X, N141X, Y143X, N144X, I147X, V148X, V149X, I150X, A153X, S156X, S158X, S159X, G166X, S182X, T185X, S194X, T209X, N212X, N213X, S216X, F217X, M222X, A224X, A236X, Y238X, E248X, K251X, N252X, N256X, S272X, and Q275X; or
   (vi) A1X, N38X, A40X, A48X, S52X, G53X, N76X, Y91X, S114X, N117X, G118X, G128X, S129X, S130X, I147X, V149X, S156X, S158X, S159X, G166X, S182X, F217X, Y238X, E248X, N256X, and S272X;
   wherein X is any amino acid and the amino acid positions of the variant or recombinant polypeptide or active fragment thereof are numbered by correspondence with the amino acid sequence of SEQ ID NO:10.
3. The protease variant, or recombinant polypeptide or active fragment thereof of any preceding para., wherein said variant or recombinant polypeptide or active fragment thereof comprises an amino acid sequence comprising two or more substitutions selected from:
   (i) X1G, X1T, X3E, X3V, X7C, X9I, X9T, X9V, X19R, X24R, X24T, X27S, X27T, X30V, X38A, X38Q, X38T, X40D, X40E, X40L, X40V, X43D, X43H, X43L, X43S, X45Q, X45R, X48E, X48Q, X52A, X52N, X52Q, X52T, X53D, X53E, X53N, X57P, X72A, X76D, X78A, X78D, X78G, X78S, X79I, X86P, X87S, X88V, X89E, X89S, X90P, X91H, X93T, X97G, X99L, X100A, X100K, X101T, X103E, X103S, X104V, X106S, X107T, X107V, X111L, X114A, X114T, X115G, X116E, X116N, X117E, X117S, X118R, X120H, X122A, X123D, X124L, X128A, X128D, X128E, X128H, X128K, X128M, X128N, X128Q, X128S, X128Y, X129D, X129E, X129G, X129H, X129I, X129K, X129L, X129M, X129N, X129P, X129Q, X129R, X129T, X129V, X129Y, X130A, X130E, X130F, X130G, X130H, X130I, X130L, X130M, X130N, X130Q, X130R, X130T, X130V, X130W, X130Y, X131P, X133A, X133D, X133V, X134T, X136E, X139V, X140D, X140Y, X141S, X143T, X144S, X145N, X145W, X147A, X147V, X148L, X149G, X149L, X149N, X149S, X150V, X153S, X156A, X156H, X156N, X158T, X158V, X159D, X159E, X159N, X159Q, X160D, X166A, X166D, X166H, X166N, X166S, X166Y, X181A, X181N, X182E, X185Q, X188Y, X194P, X209F, X209V, X209Y, X212S, X213T, X215D, X215E, X216T, X217L, X217R, X222L, X224T, X228S, X234V, X236N, X236Q, X238F, X240F, X242S, X245A, X245N, X246V, X248K, X248N, X251R, X251S, X252S, X256E, X256K, X256P, X256R, X256Y, X265S, X267N, X269K, X270C,X271F, X271W, X272K, X272N, X272Q, X273V, X275H, and X275R;
   (ii) X1G, X1T, X3E, X3V, X9I, X9T, X9V, X19R, X24R, X24T, X27S, X27T, X30V, X38A, X38Q, X38T, X40D, X40E, X40L, X40V, X43D, X43H, X43L, X43S, X45Q, X45R, X48E, X48Q, X52A, X52N, X52Q, X52T, X53D, X53E, X53N, X57P, X72A, X76D, X78A, X78D, X78G, X78S, X79I, X86P, X87S, X88V, X89E, X89S, X90P, X91H, X93T, X97G, X99L, X100A, X100K, X101T, X103E, X103S, X104V, X106S, X107T, X107V, X111L, X114A, X114T, X115G, X116E, X116N, X117E, X117S, X118R, X120H, X122A, X123D, X124L, X128A, X128D, X128E, X128H, X128K, X128M, X128N, X128Q, X128S, X128Y, X129D, X129E, X129G, X129H, X129I, X129K, X129L, X129M, X129N, X129P, X129Q, X129R, X129T, X129V, X129Y, X130A, X130E, X130F, X130G, X130H, X130I, X130L, X130M, X130N, X130Q, X130R, X130T, X130V, X130W, X130Y, X131P, X133A, X133D, X133V, X134T, X136E, X139V, X140D, X140Y, X141S, X143T, X144S, X145N, X145W, X147A, X147V, X148L, X149G, X149L, X149N, X149S, X150V, X153S, X156A, X156H, X156N, X158T, X158V, X159D, X159E, X159N, X159Q, X160D, X166A, X166D, X166H, X166N, X166S, X166Y, X181A, X181N, X182E, X185Q, X188Y, X194P, X209F, X209V, X209Y, X212S, X213T, X215D, X215E, X216T, X217L, X217R, X222L, X224T, X228S, X234V, X236N, X236Q, X238F, X240F, X242S, X245A, X245N, X246V, X248K, X248N, X251R, X251S, X252S, X256E, X256K, X256P, X256R, X256Y, X265S, X267N, X271F, X271W, X272K, X272N, X272Q, X275H, and X275R;
   (iii) X1G, X7C, X9T, X27M, X38Q, X38T, X40D, X45Q, X45R, X48E, X48Q, X52F, X52Q, X52T, X53D, X53E, X53Y, X76D, X78A, X91H, X100A, X114T, X117S, X118R, X122V, X128A, X128D, X128E, X128H, X128K, X128L, X128M, X128N, X128Q, X128R, X128S, X128T, X128V, X128W, X128Y, X129D, X129E, X129F, X129G, X129H, X129I, X129K, X129L, X129M, X129N, X129P, X129Q, X129R, X129T, X129V, X129Y, X130A, X130D, X130E, X130F, X130G, X130H, X130I, X130L, X130M, X130N, X130Q, X130R, X130T, X130V, X130W, X130Y, X140D, X147A, X149G, X156D, X156H, X156N, X156T, X158T, X158V, X159D, X159N, X160D, X166D, X166H, X166Y, X181N, X182E, X194P, X209V, X217R, X222L, X228S, X234V, X236N, X236Q, X238F, X245A, X248D, X248K, X248N, X256E, X256P, X256R, X269K, X270C, X271G, X272K, X272Q, X273V, X275R, and X275Y;
   (iv) X1G, X1T, X24R, X24T, X30V, X38A, X38Q, X38T, X40D, X40E, X40L, X40V, X48Q, X52A, X52N, X52Q, X52T, X53D, X53E, X53N, X76D, X78A, X78D, X78G, X78S, X79I, X86P, X87S, X88V, X89E, X91H, X93T, X97G, X100A, X100K, X103E, X103S, X104V, X106S, X111L, X114A, X114T, X115G, X116E, X116N, X117E, X117S, X118R, X120H, X122A, X124L, X128A, X128D, X128E, X128H, X128K, X128M, X128N, X128Q, X128S, X128Y, X129D, X129E, X129G, X129H, X129I, X129K, X129L, X129M, X129N, X129P, X129Q, X129R, X129T, X129V, X129Y, X130F, X130H, X130I, X130Y, X131P, X133A, X133D, X133V, X134T, X136E, X139V, X141S, X143T, X144S, X147A, X147V, X148L, X149G, X149L, X149N, X149S, X150V, X153S, X156A, X156H, X156N, X158T, X158V, X159D, X159E, X159N, X159Q, X166A, X166D, X166H, X166N, X166S, X166Y, X182E, X185Q, X194P, X209F, X209V, X209Y, X212S, X213T, X216T, X217L, X217R, X222L, X224T, X236N, X236Q, X238F, X248K, X248N, X251R, X251S, X252S, X256E, X256K, X256P, X256R, X256Y, X272K, X272N, X272Q, X275H, and X275R;
   (v) X1G, X1T, X24R, X24T, X30V, X38A, X38Q, X38T, X40D, X40E, X40L, X40V, X52A, X52N, X52Q, X52T, X53D, X53E, X53N, X76D, X78A, X78D, X78G, X78S, X79I, X86P, X87S, X88V, X89E, X93T, X97G, X100A, X100K, X103E, X103S, X104V, X106S, X111L, X114A, X114T, X115G, X116E, X116N, X117E, X117S, X118R, X120H, X122A, X124L, X128A, X128D, X128E, X128H, X128K, X128M, X128N, X128Q, X128S, X128Y, X129D, X129E, X129G, X129H, X129I, X129K, X129L, X129M, X129N, X129P, X129Q, X129R, X129T, X129V, X129Y, X131P, X133A, X133D, X133V, X134T, X136E, X139V, X141S, X143T, X144S, X147A, X147V, X148L, X149G, X149L, X149N, X149S, X150V, X153S, X156A, X156H, X156N, X158T, X158V, X159D, X159E, X159N, X159Q, X166A, X166D, X166H, X166N, X166S, X166Y, X182E, X185Q, X194P, X209F, X209V, X209Y, X212S, X213T, X216T, X217L, X217R, X222L, X224T, X236N, X236Q, X238F, X248K, X248N, X251R, X251S, X252S, X256E, X256K, X256P, X256R, X256Y, X272K, X272N, X272Q, X275H, and X275R; or
   (vi) X1G, X38Q, X40D, X48Q, X52F, X52T, X53E, X53Y, X76D, X91H, X114T, X117S, X118R, X128D, X128M, X128V, X129F, X129M, X129R, X129V, X130F, X130H, X130I, X130Y, X147A, X149G, X156N, X158T, X159N, X166D, X166H, X182E, X217R, X238F, X248N, X256E, X256P, X256R, and X272K;
   wherein X is any amino acid and the amino acid positions of the variant or recombinant polypeptide or active fragment thereof are numbered by correspondence with the amino acid sequence of SEQ ID NO:10.
4. The protease variant, or recombinant polypeptide or active fragment thereof of any preceding para. , wherein said variant or recombinant polypeptide or active fragment thereof comprises an amino acid sequence comprising two or more substitutions selected from:
   (i) A001G, A001T, T003E, T003V, G007C, P009I, P009T, P009V, A019R, S024R, S024T, K027S, K027T, I030V, N038A, N038Q, N038T, A040D, A040E, A040L, A040V, N043D, N043H, N043L, N043S, K045Q, K045R, A048E, A048Q, S052A, S052N, S052Q, S052T, G053D, G053E, G053N, A057P, V072A, N076D, T078A, T078D, T078G, T078S, T079I, Y086P, N087S, A088V, D089E, D089S, L090P, Y091H, V093T, S097G, S099L, G100A, G100K, S101T, T103E, T103S, L104V, G106S, I107T, I107V, I111L, S114A, S114T, I115G, S116E, S116N, N117E, N117S, G118R, N120H, I122A, N123D, M124L, G128A, G128D, G128E, G128H, G128K, G128M, G128N, G128Q, G128S, G128Y, S129D, S129E, S129G, S129H, S1291, S129K, S129L, S129M, S129N, S129P, S129Q, S129R, S129T, S129V, S129Y, S130A, S130E, S130F, S130G, S130H, S130I, S130L, S130M, S130N, S130Q, S130R, S130T, S130V, S130W, S130Y, G131P, T133A, T133D, T133V, A134T, Q136E, C139V, N140D, N140Y, N141S, Y143T, N144S, R145N, R145W, I147A, 1147V, V148L, V149G, V149L, V149N, V149S, 1150V, A153S, S156A, S156H, S156N, S158T, S158V, S159D, S159E, S159N, S159Q, G160D, G166A, G166D, G166H, G166N, G166S, G166Y, S181A, S181N, S182E, T185Q, S188Y, S194P, T209F, T209V, T209Y, N212S, N213T, A215D, A215E, S216T, F217L, F217R, M222L, A224T, A228S, I234V, A236N, A236Q, Y238F, S240F, T242S, Q245A, Q245N, I246V, E248K, E248N, K251R, K251S, N252S, N256E, N256K, N256P, N256R, N256Y, K265S, V267N, N269K, V270C, E271F, E271W, S272K, S272N, S272Q, A273V, Q275H, and Q275R;
   (ii) A001G, A001T, T003E, T003V, P009I, P009T, P009V, A019R, S024R, S024T, K027S, K027T, I030V, N038A, N038Q, N038T, A040D, A040E, A040L, A040V, N043D, N043H, N043L, N043S, K045Q, K045R, A048E, A048Q, S052A, S052N, S052Q, S052T, G053D, G053E, G053N, A057P, V072A, N076D, T078A, T078D, T078G, T078S, T0791, Y086P, N087S, A088V, D089E, D089S, L090P, Y091H, V093T, S097G, S099L, G100A, G100K, S101T, T103E, T103S, L104V, G106S, I107T, I107V, I111L, S114A, S114T, 1115G, S116E, S116N, N117E, N117S, G118R, N120H, I122A, N123D, M124L, G128A, G128D, G128E, G128H, G128K, G128M, G128N, G128Q, G128S, G128Y, S129D, S129E, S129G, S129H, S1291, S129K, S129L, S129M, S129N, S129P, S129Q, S129R, S129T, S129V, S129Y, S130A, S130E, S130F, S130G, S130H, S130I, S130L, S130M, S130N, S130Q, S130R, S130T, S130V, S130W, S130Y, G131P, T133A, T133D, T133V, A134T, Q136E, C139V, N140D, N140Y, N141S, Y143T, N144S, R145N, R145W, I147A, I147V, V148L, V149G, V149L, V149N, V149S, I150V, A153S, S156A, S156H, S156N, S158T, S158V, S159D, S159E, S159N, S159Q, G160D, G166A, G166D, G166H, G166N, G166S, G166Y, S181A, S181N, S182E, T185Q, S188Y, S194P, T209F, T209V, T209Y, N212S, N213T, A215D, A215E, S216T, F217L, F217R, M222L, A224T, A228S, I234V, A236N, A236Q, Y238F, S240F, T242S, Q245A, Q245N, I246V, E248K, E248N, K251R, K251S, N252S, N256E, N256K, N256P, N256R, N256Y, K265S, V267N, E271F, E271W, S272K, S272N, S272Q, Q275H, and Q275R;
   (iii) A001G, G007C, P009T, K027M, N038Q, N038T, A040D, K045Q, K045R, A048E, A048Q, S052F, S052Q, S052T, G053D, G053E, G053Y, N076D, T078A, Y091H, G100A, S114T, N117S, G118R, I122V, G128A, G128D, G128E, G128H, G128K, G128L, G128M, G128N, G128Q, G128R, G128S, G128T, G128V, G128W, G128Y, S129D, S129E, S129F, S129G, S129H, S129I, S129K, S129L, S129M, S129N, S129P, S129Q, S129R, S129T, S129V, S129Y, S130A, S130D, S130E, S130F, S130G, S130H, S130I, S130L, S130M, S130N, S130Q, S130R, S130T, S130V, S130W, S130Y, N140D, I147A, V149G, S156D, S156H, S156N, S156T, S158T, S158V, S159D, S159N, G160D, G166D, G166H, G166Y, S181N, S182E, S194P, T209V, F217R, M222L, Y238F, Q245A, E248D, E248K, E248N, N256E, N256P, N256R, N269K, V270C, E271G, S272K, S272Q, A273V, Q275R, and Q275Y;
   (iv) A001G, A001T, S024T, I030V, N038Q, A040D, A048Q, S052A, S052Q, S052T, G053D, G053E, N076D, T078D, T078S, T079I, Y086P, N087S, A088V, D089E, D089S, Y091H,V093T, S097G, G100A, T103E, T103S, L104V, G106S, I111L, S114A, S114T, I115G, S116N, N117S, G118R, N120H, I122A, M124L, G128S, S129P, S130F, S130H, S130I, S130Y, G131P, T133A, T133V, A134T, Q136E, C139V, N141S, Y143T, N144S, I147A, I147V, V148L, V149G, V149L, I150V, A153S, S156H, S156N, S158T, S159D, S159N, G166D, G166H, G166S, S182E, T185Q, S194P, T209Y, N212S, N213T, S216T, F217L, F217R, M222L, A224T, A236N, Y238F, E248N, K251S, N252S, N256P, N256R, N256Y, S272K, S272Q, and Q275H;
   (v) A001G, A001T, S024T, I030V, N038Q, A040D, S052A, S052Q, S052T, G053D, G053E, N076D, T078D, T078S, T079I, Y086P, N087S, A088V, D089E, D089S, V093T, S097G, G100A, T103E, T103S, L104V, G106S, I111L, S114A, S114T, I115G, S116N, N117S, G118R, N120H, I122A, M124L, G128S, S129P, G131P, T133A, T133V, A134T, Q136E, C139V, N141S, Y143T, N144S, I147A, I147V, V148L, V149G, V149L, I150V, A153S, S156H, S156N, S158T, S159D, S159N, G166D, G166H, G166S, S182E, T185Q, S194P, T209Y, N212S, N213T, S216T, F217L, F217R, M222L, A224T, A236N, Y238F, E248N, K251S, N252S, N256P, N256R, N256Y, S272K, S272Q, and Q275H; or
   (vi) A001G, N038Q, A040D, A048Q, S052F, S052T, G053E, G053Y, N076D, Y091H, S114T, N117S, G118R, G128D, G128M, G128V, S129F, S129M, S129R, S129V, S130F, S130H, S130I, S130Y, I147A, V149G, S156N, S158T, S159N, G166D, G166H, S182E, F217R, Y238F, E248N, N256E, N256P, N256R, and S272K;
   wherein the amino acid positions of the variant or recombinant polypeptide or active fragment thereof are numbered by correspondence with the amino acid sequence of SEQ ID NO:10.
5. The protease variant, or recombinant polypeptide or active fragment thereof of any preceding para. , wherein said variant or recombinant polypeptide or active fragment thereof comprises an amino acid sequence comprising two or more substitutions at one or more position selected from:
   (i) 52, 53, 76, 86, 97, 103, 104, 106, 111, 114, 115, 116, 118, 120, 122, 124, 128, 129, 130, 131, 134, 136, 139, 141, 143, 144, 147, 148, 150, 156, 158, 159, 166, 217, 248, and 272;
   (ii) 53, 76, 106, 128, 131, 134, 139, 158, 159, 166, 217, 248, and 272;
   (iii) 52, 53, 76, 106, 118, 128, 129, 130, 131, 134, 139, 156, 158, 166, 217, 248, and 272;
   (iv) 52, 53, 76, 128, 118, 156, 158, 166, 217, and 248;
   (v) 53, 76, 128, 158, 166, and 248;
   (vi) 52, 53, 76, 118, 128, 156, 158, 166, 217, and 248; or
   (vii) 52-129, 53-166, 76-166, 166-159, 166-27, 53-76-166, 53-118-166, 53-166-272, 76-118-166, 76-166-248, 76-166-256, and 76-166-272;
   wherein the amino acid positions of the variant or recombinant polypeptide or active fragment thereof are numbered by correspondence with the amino acid sequence of SEQ ID NO:10.
6. The protease variant, or recombinant polypeptide or active fragment thereof of any preceding para., wherein said variant or recombinant polypeptide or active fragment thereof comprises an amino acid sequence comprising two or more substitutions selected from:
   (i) S52X, G53X, N76X, Y86X, S97X, T103X, L104X, G106X, I111X, S114X, I115X, S116X, G118X, N120X, I122X, M124X, G128X, S129X, S130X, G131X, A134X, Q136X, C139X, N141X, Y143X, N144X, I147X, V148X, I150X, S156X, S158X, S159X, G166X, F217X, E248X, and S272X;
   (ii) G53X, N76X, G106X, G128X, G131X, A134X, C139X, S158X, S159X, G166X, F217X, E248X, and S272X;
   (iii) S52X, G53X, N76X, G106X, G118X, G128X, S129X, S130X, G131X, A134X, C139X, S156X, G166X, F217X, E248X, and S272X;
   (iv) S52X, G53X, N76X, G118X, G128X, S156X, S158X, G166X, F217X, and E248X;
   (v) G53X, N76X, G128X, S158X, G166X, and E248X;
   (vi) S52X, G53X, N76X, G118X, G128X, S156X, S158X, G166X, F217X, and E248X; or
   (vii) S52X-S129X, G53X-G166X, N76X-G166X, G166X-S159X, G166X-S272X, G53X-N76X-G166X, G53X-G118X-G166X, G53X-G166X-S272X, N76X-G118X-G166X, N76X-G166X-E248X, N76X-G166X-N256X, and N76X-G166X-S272X;
   wherein X is any amino acid and the amino acid positions of the variant or recombinant polypeptide or active fragment thereof are numbered by correspondence with the amino acid sequence of SEQ ID NO:10.
7. The protease variant, or recombinant polypeptide or active fragment thereof of any preceding para. , wherein said variant or recombinant polypeptide or active fragment thereof comprises an amino acid sequence comprising two or more substitutions selected from:
   (i) X52T, X53D, X76D, X86P, X97G, X103S, X104V, X106S, X111L, X114A, X115G, X116N, X118R, X120H, X122A, X124L, X128D, X128S, X129F, X130Y, X131P, X134T, X136E, X139V, X141S, X143T, X144S, X147V, X148L, X150V, X156N, X158T, X159D, X166D, X217R, X248N, and X272K;
   (ii) X53D, X76D, X106S, X128S, X131P, X134T, X139V, X158T, X159D, X166D, X217R, X248N, and X272K;
   (iii) X52T, X53D, X76D, X106S, X118R, X128D, X129F, X130Y, X131P, X134T, X139V, X156N, X166D, X217R, X248N, and X272K;
   (iv) X52T, X53D, X76D, X118R, X128S, X156N, X158T, X166D, X217R, and X248N;
   (v) X53D, X76D, X128S, X158T, X166D, and X248N;
   (vi) X52T, X53D, X76D, X118R, X128S, X156N, X158T, X166D, X217R, and X248N; or
   (vii) X52T-X129F, X53D-X166D, X76D-X166D, X166H-X159D, X166D-X272K, X53D-X76D-X166D, X53D-X118R-X166D, X53D-X166D-X272K, X76D-X118R-X166D, X76D-X166D-X248K, X76D-X166D-X256R, and X76D-X166D-X272K;
   wherein X is any amino acid and the amino acid positions of the variant or recombinant polypeptide or active fragment thereof are numbered by correspondence with the amino acid sequence of SEQ ID NO:10.
8. The protease variant, or recombinant polypeptide or active fragment thereof of any preceding para., wherein said variant or recombinant polypeptide or active fragment thereof comprises an amino acid sequence comprising two or more substitutions selected from:
   (i) S52T, G53D, N76D, Y086P, S097G, T103S, L104V, G106S, I111L, S114A, I115G, S116N, G118R, N120H, I122A, M124L, G128D, G128S, S129F, S129P, S130Y, G131P, A134T, Q136E, C139V, N141S, Y143T, N144S, I147V, V148L, I150V, S156N, S158T, S159D, G166D, F217R, E248N, and S272K;
   (ii) G53D, N76D, G106S, G128S, G131P, A134T, C139V, S158T, S159D, G166D, F217R, E248N, and S272K;
   (iii) S52T, G53D, N76D, G106S, G118R, G128D, S129F, S130Y, G131P, A134T, C139V, S156N, G166D, F217R, E248N, and S272K;
   (iv) S52T, G53D, N76D, G118R, G128S, S156N, S158T, G166D, F217R, and E248N;
   (v) G53D, N76D, G128S, S158T, G166D, and E248N;
   (vi) S52T, G53D, N76D, G118R, S156N, S158T, G166D, F217R, and E248N; or
   (vii) S52T-S129F, G53D-G166D, N76D-G166D, G166H-S159D, G166D-S272K, G53D-N76D-G166D, G53D-G118R-G166D, G53D-G166D-S272K, N76D-G118R-G166D, N76D-G166D-E248K, N76D-G166D-N256R, and N76D-G166D-S272K;
   wherein the amino acid positions of the variant or recombinant polypeptide or active fragment thereof are numbered by correspondence with the amino acid sequence of SEQ ID NO:10.
9. The protease variant, or recombinant polypeptide or active fragment thereof of any preceding para., wherein said two or more substitutions is selected from S052T-S129F; G053D-G166D; N076D-G166D; G166H-S159D; G166D-S272K; G053D-N076D-G166D; G053D-G118R-G166D; G053D-G166D-S272K; N076D-G118R-G166D; N076D-G166D-E248K; N076D-G166D-N256R; N076D-G166D-S272K; S024T-G053E-G166D-M222L-N256P; V149L-S156H-T185Q-S272Q-S159D; G053D-N076D-G118R-G166D-N256R; S052T-V149G-S156N-G166H-F217R; S052T-V149G-S156N-G166D-F217R; S052T-V149G-S156N-S159D-G166D-F217R; S052T-V149G-S156N-G166D-S182E-F217R; A040D-S114T-1147A-S159N-G166D-S182E; N038Q-G053E-G100A-N117S-I147A-S158T-G166D-S272K; N038Q-G053E-N117S-I147A-S158T-G166D-N256P-S272K; A001G-S052Q-V149G-S156N-S159N-S182E-Y238F-S272Q; A001G-S052Q-S114T-S156N-S159N-S182E-Y238F-S272Q; N038Q-G053E-G100A-N117S-I147A-S158T-G166D-N256P-S272K; A001G-I030V-T078D-V093T-T103E-S156N-S159D-G166S-S194P-F217R-A236N; A001T-N076D-T078S-T079I-Y086P-D089E-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-E248N; S052A-N076D-T078S-T079I-Y086P-D089E-S097G-T103S-L104V-G106S-I111L-S114A-I115G-S116N-N120H-I122A-M124L-G128S-S129P-G131P-T133V-A134T-Q136E-C139V-N141S-Y143T-N144S-I147V-V148L-I150V-S158T-G166D-F217L-E248N; A001T-N076D-T078S-T079I-Y086P-D089E-S097G-T103S-L104V-G106S-I111L-S114A-I115G-S116N-N120H-I122A-M124L-G128S-S129P-G131P-T133V-A134T-Q136E-C139V-N141S-Y143T-N144S-I147V-V148L-I150V-S158T-G166D-T209Y-N212S-N213T-S216T-F217L-A224T-E248N-K251S-N252S-N236Y; N076D-T078S-T079I-Y086P-D089E-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N; N076D-T078S-T079I-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-1147V-V148L-I150V-S158T-G166D-F217L-E248N; A001T-N076D-T078S-T079I-Y086P-D089E-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G160D-T209Y-N212S-N213T-S216T-F217L-A224T-E248N; A001T-N076D-T078S-T079I-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-E248N;S052A-N076D-T078S-T0791-Y086P-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-F217L-A224T-E248N; S052A-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N; A001T-N076D-T078S-T079I-Y086P-D089E-S097G-T103S-L104V-G106S-II11L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-E248N; A001T-S052A-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-A153S-S158T-G166D-T209Y-N212S-N213T-S216T-F217L-A224T-E248N; A001T-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-A153S-S158T-G166D-T209Y-N212S-N213T-S216T-F217L-A224T-E248N; A001T-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N-Q275H;A001T-N076D-T078S-T0791-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N; N076D-G118R-G128D-S129R-S1301-G166D-E248N; N076D-G118R-G128D-S129F-S130Y-G166D-E248N; N076D-G118R-G128V-S129M-S130H-G166D-E248N; S052T-N076D-G118R-G128M-S129V-S130F-G166H-Y238F; S052T-N076D-G118R-G128D-S129R-S130I-G166D-F217R-N256R; S052T-N076D-G118R-G128V-S129M-S130H-G166D-E248N-N256E-S272K; S052F-G053Y-N076D-G118R-G128D-S129F-S130Y-G166D-F217R-E248N-N256R-S272K; and A001G-A040D-A048Q-S052T-G053E-Y091H-S114T-I147A-S156N-S159N-G166H-S182E-Y238F; wherein the amino acid positions of the variant or recombinant polypeptide or active fragment thereof are numbered by correspondence with the amino acid sequence of SEQ ID NO:10.
10. A protease variant, or a recombinant polypeptide or an active fragment thereof comprising an amino acid sequence comprising one or more deletions at one or more positions selected from 53, 55, 56, 57, and 58 or G053, P055, N056, A057 and L058; wherein the amino acid positions of the variant or recombinant polypeptide or active fragment thereof are numbered by correspondence with the amino acid sequence of SEQ ID NO:10.
11. The protease variant, or a recombinant polypeptide or an active fragment thereof of any one of paras. 1-9, wherein said variant or recombinant polypeptide or active fragment thereof further comprises an EPNALQDGNGHGTH (SEQ ID NO:44) motif.
12. The protease variant, or recombinant polypeptide or active fragment thereof of para. 11, wherein said motif extends from residue E54 to H67, wherein the amino acid positions are numbered by correspondence with the amino acid sequence of SEQ ID NO:10.
13. The protease variant, or recombinant polypeptide or active fragment thereof of para. 11 or 12, with the proviso that the variant or recombinant polypeptide or active fragment thereof does not comprise WP_047970940, WP_010192403, KPB_03414, BAD11988, BAD21128, Bhon03321, DSM9712_SprC, DSM6951_SprC, or DSM9711_SprC.
14. The protease variant, or recombinant polypeptide or active fragment thereof of any one of paras. 1-12, wherein said variant or recombinant polypeptide or active fragment thereof further comprises an amino acid sequence having at least 75% amino acid sequence identity to SEQ ID NO:10.
15. The protease variant, or recombinant polypeptide or active fragment thereof of para. 14, with the proviso that the variant or recombinant polypeptide or active fragment thereof does not comprise WP_047970940, WP_010192403, KPB_03414, BAD11988, BAD21128, Bhon03321, DSM9712_SprC, DSM6951_SprC, or DSM9711_SprC.
16. The protease variant, or recombinant polypeptide or active fragment thereof of any preceding para., wherein said protease variant, or recombinant polypeptide or active fragment thereof has protease activity in the presence of a surfactant, cleaning activity in a detergent composition, or a combination thereof.
17. The protease variant, or recombinant polypeptide or active fragment thereof of para. 16, wherein the detergent composition is an automatic dish washing detergent or a laundry detergent and/or the cleaning activity comprises hydrolysis of a substrate on a protein stain.
18. The protease variant, or recombinant polypeptide or active fragment thereof of any preceding para., wherein said protease variant, or recombinant polypeptide or active fragment thereof has one or more improved property selected from improved cleaning performance, improved stability, and improved residual protease activity when compared to SEQ ID NO:10 and/or 48.
19. The protease variant, or recombinant polypeptide or active fragment thereof of para. 18, wherein said improved property is
   (i) improved residual protease activity and said protease variant, or recombinant polypeptide or active fragment thereof has 15-100%, 20-100%, 25-100%, 30-100%, 35-100%, or 40-100% residual protease activity when measured in accordance with the Stability Assay of Example 1;
   (ii) improved cleaning performance and said protease variant, or recombinant polypeptide or active fragment thereof has a BMI cleaning PI of ≥0.7 in comparison to SEQ ID NO:48 when measured in accordance with the Cleaning Performance in Detergent assay of Example 1;
   (iii) improved stability and said protease variant, or recombinant polypeptide or active fragment thereof has a stability PI ≥1.01 or 1.1 or 1.2 in comparison to SEQ ID NO:10 when measured in accordance with the Stability Assay of Example 1; and/or
   (iv) improved cleaning performance and said protease variant, or recombinant polypeptide or active fragment thereof has a cleaning PI ≥1.01 or 1.1 or 1.2 in comparison to SEQ ID NO:10 when measured in accordance with the Cleaning Performance in Detergent Assay of Example 1.
20. The protease variant, or recombinant polypeptide or active fragment thereof of any preceding para., wherein said protease variant, or recombinant polypeptide or active fragment thereof is derived from a reference polypeptide selected from SEQ ID NOs: 10, 18, 19, 45, 46, 57, 58, and 66.
21. The protease variant, or recombinant polypeptide or active fragment thereof of para. 20, wherein the reference polypeptide is SEQ ID NO:10 and said protease variant, or recombinant polypeptide or active fragment thereof has one or more substitutions in one or more molecular segments selected from Segment 1 comprising positions 76-79 and 86, Segment 2 comprising positions 97-148, Segment 3 comprising positions 150-170, and Segment 4 comprising positions 207-220.
22. An LG12-Clade of proteases comprising a protease variant or a recombinant polypeptide or an active fragment thereof of any one of para. 1-9 and 11-21.
23. A cleaning composition comprising the protease variant, or recombinant polypeptide or active fragment thereof of any one of para. 1-21.
24. The cleaning composition of para. 23, wherein said composition further comprises a surfactant; at least one calcium ion and/or zinc ion; at least one stabilizer; from about 0.001% to about 1.0 weight % of said recombinant polypeptide; at least one bleaching agent; at least one adjunct ingredient; one or more additional enzymes or enzyme derivatives selected from the group consisting of acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, xylosidases, metalloproteases, additional serine proteases, or a combination thereof.
25. The cleaning composition of para. 23 or 24, wherein said composition contains phosphate, is phosphate-free, contains borate, is boron-free, or combinations thereof.
26. A method of cleaning comprising contacting a surface or an item in need of cleaning with the protease variant, or recombinant polypeptide or active fragment thereof of any one of paras. 1-21 or the cleaning composition of any one of para. 23-25; and optionally further comprising the step of rinsing said surface or item after contacting said surface or item with said variant or composition.
27. A polynucleotide comprising a nucleic acid sequence that encodes the protease variant, or recombinant polypeptide or active fragment thereof of any one of para. 1-21.

## Claims

1. A protease variant, or recombinant polypeptide or active fragment thereof comprising an amino acid sequence comprising two or more substitutions including X129P and at least one of:
(i) X1G, X1T, X3E, X3V, X7C, X9I, X9T, X9V, X19R, X24R, X24T, X27S, X27T, X30V, X38A, X38Q, X38T, X40D, X40E, X40L, X40V, X43D, X43H, X43L, X43S, X45Q, X45R, X48E, X48Q, X52A, X52N, X52Q, X52T, X53D, X53E, X53N, X57P, X72A, X76D, X78A, X78D, X78G, X78S, X79I, X86P, X87S, X88V, X89E, X89S, X90P, X91H, X93T, X97G, X99L, X100A, X100K, X101T, X103E, X103S, X104V, X106S, X107T, X107V, X111L, X114A, X114T, X115G, X116E, X116N, X117E, X117S, X118R, X120H, X122A, X123D, X124L, X128A, X128D, X128E, X128H, X128K, X128M, X128N, X128Q, X128S, X128Y, X130A, X130E, X130F, X130G, X130H, X130I, X130L, X130M, X130N, X130Q, X130R, X130T, X130V, X130W, X130Y, X131P, X133A, X133D, X133V, X134T, X136E, X139V, X140D, X140Y, X141S, X143T, X144S, X145N, X145W, X147A, X147V, X148L, X149G, X149L, X149N, X149S, X150V, X153S, X156A, X156H, X156N, X158T, X158V, X159D, X159E, X159N, X159Q, X160D, X166A, X166D, X166H, X166N, X166S, X166Y, X181A, X181N, X182E, X185Q, X188Y, X194P, X209F, X209V, X209Y, X212S, X213T, X215D, X215E, X216T, X217L, X217R, X222L, X224T, X228S, X234V, X236N, X236Q, X238F, X240F, X242S, X245A, X245N, X246V, X248K, X248N, X251R, X251S, X252S, X256E, X256K, X256P, X256R, X256Y, X265S, X267N, X269K, X270C,X271F, X271W, X272K, X272N, X272Q, X273V, X275H, and X275R;
(ii) X1G, X1T, X3E, X3V, X9I, X9T, X9V, X19R, X24R, X24T, X27S, X27T, X30V, X38A, X38Q, X38T, X40D, X40E, X40L, X40V, X43D, X43H, X43L, X43S, X45Q, X45R, X48E, X48Q, X52A, X52N, X52Q, X52T, X53D, X53E, X53N, X57P, X72A, X76D, X78A, X78D, X78G, X78S, X79I, X86P, X87S, X88V, X89E, X89S, X90P, X91H, X93T, X97G, X99L, X100A, X100K, X101T, X103E, X103S, X104V, X106S, X107T, X107V, X111L, X114A, X114T, X115G, X116E, X116N, X117E, X117S, X118R, X120H, X122A, X123D, X124L, X128A, X128D, X128E, X128H, X128K, X128M, X128N, X128Q, X128S, X128Y, X130A, X130E, X130F, X130G, X130H, X130I, X130L, X130M, X130N, X130Q, X130R, X130T, X130V, X130W, X130Y, X131P, X133A, X133D, X133V, X134T, X136E, X139V, X140D, X140Y, X141S, X143T, X144S, X145N, X145W, X147A, X147V, X148L, X149G, X149L, X149N, X149S, X150V, X153S, X156A, X156H, X156N, X158T, X158V, X159D, X159E, X159N, X159Q, X160D, X166A, X166D, X166H, X166N, X166S, X166Y, X181A, X181N, X182E, X185Q, X188Y, X194P, X209F, X209V, X209Y, X212S, X213T, X215D, X215E, X216T, X217L, X217R, X222L, X224T, X228S, X234V, X236N, X236Q, X238F, X240F, X242S, X245A, X245N, X246V, X248K, X248N, X251R, X251S, X252S, X256E, X256K, X256P, X256R, X256Y, X265S, X267N, X271F, X271W, X272K, X272N, X272Q, X275H, and X275R;
(iii) X1G, X7C, X9T, X27M, X38Q, X38T, X40D, X45Q, X45R, X48E, X48Q, X52F, X52Q, X52T, X53D, X53E, X53Y, X76D, X78A, X91H, X100A, X114T, X117S, X118R, X122V, X128A, X128D, X128E, X128H, X128K, X128L, X128M, X128N, X128Q, X128R, X128S, X128T, X128V, X128W, X128Y, X130A, X130D, X130E, X130F, X130G, X130H, X130I, X130L, X130M, X130N, X130Q, X130R, X130T, X130V, X130W, X130Y, X140D, X147A, X149G, X156D, X156H, X156N, X156T, X158T, X158V, X159D, X159N, X160D, X166D, X166H, X166Y, X181N, X182E, X194P, X209V, X217R, X222L, X228S, X234V, X236N, X236Q, X238F, X245A, X248D, X248K, X248N, X256E, X256P, X256R, X269K, X270C, X271G, X272K, X272Q, X273V, X275R, and X275Y;
(iv) X1G, X1T, X24R, X24T, X30V, X38A, X38Q, X38T, X40D, X40E, X40L, X40V, X48Q, X52A, X52N, X52Q, X52T, X53D, X53E, X53N, X76D, X78A, X78D, X78G, X78S, X79I, X86P, X87S, X88V, X89E, X91H, X93T, X97G, X100A, X100K, X103E, X103S, X104V, X106S, X111L, X114A, X114T, X115G, X116E, X116N, X117E, X117S, X118R, X120H, X122A, X124L, X128A, X128D, X128E, X128H, X128K, X128M, X128N, X128Q, X128S, X128Y, X130F, X130H, X130I, X130Y, X131P, X133A, X133D, X133V, X134T, X136E, X139V, X141S, X143T, X144S, X147A, X147V, X148L, X149G, X149L, X149N, X149S, X150V, X153S, X156A, X156H, X156N, X158T, X158V, X159D, X159E, X159N, X159Q, X166A, X166D, X166H, X166N, X166S, X166Y, X182E, X185Q, X194P, X209F, X209V, X209Y, X212S, X213T, X216T, X217L, X217R, X222L, X224T, X236N, X236Q, X238F, X248K, X248N, X251R, X251S, X252S, X256E, X256K, X256P, X256R, X256Y, X272K, X272N, X272Q, X275H, and X275R; or
(v) X1G, X1T, X24R, X24T, X30V, X38A, X38Q, X38T, X40D, X40E, X40L, X40V, X52A, X52N, X52Q, X52T, X53D, X53E, X53N, X76D, X78A, X78D, X78G, X78S, X79I, X86P, X87S, X88V, X89E, X93T, X97G, X100A, X100K, X103E, X103S, X104V, X106S, X111L, X114A, X114T, X115G, X116E, X116N, X117E, X117S, X118R, X120H, X122A, X124L, X128A, X128D, X128E, X128H, X128K, X128M, X128N, X128Q, X128S, X128Y, X131P, X133A, X133D, X133V, X134T, X136E, X139V, X141S, X143T, X144S, X147A, X147V, X148L, X149G, X149L, X149N, X149S, X150V, X153S, X156A, X156H, X156N, X158T, X158V, X159D, X159E, X159N, X159Q, X166A, X166D, X166H, X166N, X166S, X166Y, X182E, X185Q, X194P, X209F, X209V, X209Y, X212S, X213T, X216T, X217L, X217R, X222L, X224T, X236N, X236Q, X238F, X248K, X248N, X251R, X251S, X252S, X256E, X256K, X256P, X256R, X256Y, X272K, X272N, X272Q, X275H, and X275R;
wherein X is any amino acid and the amino acid positions of the variant or recombinant polypeptide or active fragment thereof are numbered by correspondence with the amino acid sequence of SEQ ID NO:10 and wherein said variant or recombinant polypeptide or active fragment thereof further comprises an amino acid sequence having at least 75% amino acid sequence identity to SEQ ID NO:10.

2. The protease variant, or recombinant polypeptide or active fragment thereof of claim 1, wherein said variant or recombinant polypeptide or active fragment thereof comprises an amino acid sequence comprising two or more substitutions including S129P and optionally at least one of:
(i) A001G, A001T, T003E, T003V, G007C, P009I, P009T, P009V, A019R, S024R, S024T, K027S, K027T, I030V, N038A, N038Q, N038T, A040D, A040E, A040L, A040V, N043D, N043H, N043L, N043S, K045Q, K045R, A048E, A048Q, S052A, S052N, S052Q, S052T, G053D, G053E, G053N, A057P, V072A, N076D, T078A, T078D, T078G, T078S, T079I, Y086P, N087S, A088V, D089E, D089S, L090P, Y091H, V093T, S097G, S099L, G100A, G100K, S101T, T103E, T103S, L104V, G106S, I107T, I107V, I111L, S114A, S114T, I115G, S116E, S116N, N117E, N117S, G118R, N120H, I122A, N123D, M124L, G128A, G128D, G128E, G128H, G128K, G128M, G128N, G128Q, G128S, G128Y, S130A, S130E, S130F, S130G, S130H, S130I, S130L, S130M, S130N, S130Q, S130R, S130T, S130V, S130W, S130Y, G131P, T133A, T133D, T133V, A134T, Q136E, C139V, N140D, N140Y, N141S, Y143T, N144S, R145N, R145W, I147A, I147V, V148L, V149G, V149L, V149N, V149S, I150V, A153S, S156A, S156H, S156N, S158T, S158V, S159D, S159E, S159N, S159Q, G160D, G166A, G166D, G166H, G166N, G166S, G166Y, S181A, S181N, S182E, T185Q, S188Y, S194P, T209F, T209V, T209Y, N212S, N213T, A215D, A215E, S216T, F217L, F217R, M222L, A224T, A228S, I234V, A236N, A236Q, Y238F, S240F, T242S, Q245A, Q245N, I246V, E248K, E248N, K251R, K251S, N252S, N256E, N256K, N256P, N256R, N256Y, K265S, V267N, N269K, V270C, E271F, E271W, S272K, S272N, S272Q, A273V, Q275H, and Q275R;
(ii) A001G, A001T, T003E, T003V, P009I, P009T, P009V, A019R, S024R, S024T, K027S, K027T, I030V, N038A, N038Q, N038T, A040D, A040E, A040L, A040V, N043D, N043H, N043L, N043S, K045Q, K045R, A048E, A048Q, S052A, S052N, S052Q, S052T, G053D, G053E, G053N, A057P, V072A, N076D, T078A, T078D, T078G, T078S, T079I, Y086P, N087S, A088V, D089E, D089S, L090P, Y091H, V093T, S097G, S099L, G100A, G100K, S101T, T103E, T103S, L104V, G106S, I107T, I107V, I111L, S114A, S114T, I115G, S116E, S116N, N117E, N117S, G118R, N120H, I122A, N123D, M124L, G128A, G128D, G128E, G128H, G128K, G128M, G128N, G128Q, G128S, G128Y, S130A, S130E, S130F, S130G, S130H, S130I, S130L, S130M, S130N, S130Q, S130R, S130T, S130V, S130W, S130Y, G131P, T133A, T133D, T133V, A134T, Q136E, C139V, N140D, N140Y, N141S, Y143T, N144S, R145N, R145W, I147A, I147V, V148L, V149G, V149L, V149N, V149S, I150V, A153S, S156A, S156H, S156N, S158T, S158V, S159D, S159E, S159N, S159Q, G160D, G166A, G166D, G166H, G166N, G166S, G166Y, S181A, S181N, S182E, T185Q, S188Y, S194P, T209F, T209V, T209Y, N212S, N213T, A215D, A215E, S216T, F217L, F217R, M222L, A224T, A228S, I234V, A236N, A236Q, Y238F, S240F, T242S, Q245A, Q245N, I246V, E248K, E248N, K251R, K251S, N252S, N256E, N256K, N256P, N256R, N256Y, K265S, V267N, E271F, E271W, S272K, S272N, S272Q, Q275H, and Q275R;
(iii) A001G, G007C, P009T, K027M, N038Q, N038T, A040D, K045Q, K045R, A048E, A048Q, S052F, S052Q, S052T, G053D, G053E, G053Y, N076D, T078A, Y091H, G100A, S114T, N117S, G118R, I122V, G128A, G128D, G128E, G128H, G128K, G128L, G128M, G128N, G128Q, G128R, G128S, G128T, G128V, G128W, G128Y, S130A, S130D, S130E, S130F, S130G, S130H, S130I, S130L, S130M, S130N, S130Q, S130R, S130T, S130V, S130W, S130Y, N140D, I147A, V149G, S156D, S156H, S156N, S156T, S158T, S158V, S159D, S159N, G160D, G166D, G166H, G166Y, S181N, S182E, S194P, T209V, F217R, M222L, Y238F, Q245A, E248D, E248K, E248N, N256E, N256P, N256R, N269K, V270C, E271G, S272K, S272Q, A273V, Q275R, and Q275Y;
(iv) A001G, A001T, S024T, I030V, N038Q, A040D, A048Q, S052A, S052Q, S052T, G053D, G053E, N076D, T078D, T078S, T079I, Y086P, N087S, A088V, D089E, D089S, Y091H,V093T, S097G, G100A, T103E, T103S, L104V, G106S, I111L, S114A, S114T, I115G, S116N, N117S, G118R, N120H, I122A, M124L, G128S, S130F, S130H, S130I, S130Y, G131P, T133A, T133V, A134T, Q136E, C139V, N141S, Y143T, N144S, I147A, I147V, V148L, V149G, V149L, I150V, A153S, S156H, S156N, S158T, S159D, S159N, G166D, G166H, G166S, S182E, T185Q, S194P, T209Y, N212S, N213T, S216T, F217L, F217R, M222L, A224T, A236N, Y238F, E248N, K251S, N252S, N256P, N256R, N256Y, S272K, S272Q, and Q275H; or
(v) A001G, A001T, S024T, I030V, N038Q, A040D, S052A, S052Q, S052T, G053D, G053E, N076D, T078D, T078S, T079I, Y086P, N087S, A088V, D089E, D089S, V093T, S097G, G100A, T103E, T103S, L104V, G106S, I111L, S114A, S114T, I115G, S116N, N117S, G118R, N120H, I122A, M124L, G128S, G131P, T133A, T133V, A134T, Q136E, C139V, N141S, Y143T, N144S, I147A, 1147V, V148L, V149G, V149L, I150V, A153S, S156H, S156N, S158T, S159D, S159N, G166D, G166H, G166S, S182E, T185Q, S194P, T209Y, N212S, N213T, S216T, F217L, F217R, M222L, A224T, A236N, Y238F, E248N, K251S, N252S, N256P, N256R, N256Y, S272K, S272Q, and Q275H;
wherein the amino acid positions of the variant or recombinant polypeptide or active fragment thereof are numbered by correspondence with the amino acid sequence of SEQ ID NO:10.

3. The protease variant, or recombinant polypeptide or active fragment thereof of any preceding claim, wherein said variant or recombinant polypeptide or active fragment thereof comprises an amino acid sequence comprising two or more substitutions at one or more position selected from:
(i) 52, 53, 76, 86, 97, 103, 104, 106, 111, 114, 115, 116, 118, 120, 122, 124, 128, 129, 130, 131, 134, 136, 139, 141, 143, 144, 147, 148, 150, 156, 158, 159, 166, 217, 248, and 272;
(ii) 52, 53,76, 106, 118, 128, 129, 130, 131, 134, 139, 156, 158, 166, 217, 248, and 272; or
(iii) 52-129;
wherein the amino acid positions of the variant or recombinant polypeptide or active fragment thereof are numbered by correspondence with the amino acid sequence of SEQ ID NO:10.

4. The protease variant, or recombinant polypeptide or active fragment thereof of any preceding claim, wherein said variant or recombinant polypeptide or active fragment thereof comprises an amino acid sequence comprising two or more substitutions selected from:
S52T, G53D, N76D, Y086P, S097G, T103S, L104V, G106S, I111L, S114A, I115G, S116N, G118R, N120H, I122A, M124L, G128D, G128S, S129F, S129P, S130Y, G131P, A134T, Q136E, C139V, N141S, Y143T, N144S, I147V, V148L, I150V, S156N, S158T, S159D, G166D, F217R, E248N, and S272K;
wherein the amino acid positions of the variant or recombinant polypeptide or active fragment thereof are numbered by correspondence with the amino acid sequence of SEQ ID NO:10.

5. The protease variant, or recombinant polypeptide or active fragment thereof of any preceding claim, wherein said two or more substitutions is selected from A001T-N076D-T078S-T079I-Y086P-D089E-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-E248N; S052A-N076D-T078S-T079I-Y086P-D089E-S097G-T103S-L104V-G106S-I111L-S114A-I115G-S116N-N120H-I122A-M124L-G128S-S129P-G131P-T133V-A134T-Q136E-C139V-N141S-Y143T-N144S-I147V-V148L-I150V-S158T-G166D-F217L-E248N; A001T-N076D-T078S-T079I-Y086P-D089E-S097G-T103S-L104V-G106S-I111L-S114A-I115G-S116N-N120H-I122A-M124L-G128S-S129P-G131P-T133V-A134T-Q136E-C139V-N141S-Y143T-N144S-I147V-V148L-1150V-S158T-G166D-T209Y-N212S-N213T-S216T-F217L-A224T-E248N-K251S-N252S-N256Y; N076D-T078S-T079I-Y086P-D089E-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N; N076D-T078S-T079I-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-F217L-E248N; A001T-N076D-T078S-T079I-Y086P-D089E-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N; A001T-N076D-T078S-T079I-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-E248N;S052A-N076D-T078S-T0791-Y086P-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-F217L-A224T-E248N; S052A-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N; A001T-N076D-T078S-T079I-Y086P-D089E-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-E248N; A001T-S052A-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-A153S-S158T-G166D-T209Y-N212S-N213T-S216T-F217L-A224T-E248N; A001T-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-A153S-S158T-G166D-T209Y-N212S-N213T-S216T-F217L-A224T-E248N; A001T-N076D-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N-Q275H; A001T-N076D-T078S-T079I-Y086P-N087S-A088V-D089S-S097G-T103S-L104V-G106S-I111L-S114A-I115G-S116N-N120H-I122A-M124L-G128S-S129P-G131P-T133A-A134T-Q136E-C139V-N141S-Y143T-N144S-I147V-V148L-I150V-S158T-G166D-T209Y-N212S-N213T-S216T-F217L-A224T-E248N; N076D-G118R-G128D-S129P-S130F-G166D-E248N; N076D-G118R-G128D-S129P-S130Y-G166D-E248N; N076D-G118R-G128H-S129P-S130L-G166D-E248N; N076D-G118R-G128H-S129P-S130N-G166D-E248N; N076D-G118R-G128H-S129P-G166D-E248N; N076D-G118R-G128S-S129P-S130A-C166D-E248N; N076D-G118R-G128Y-S129P-S130D-G166D-E248N; wherein the amino acid positions of the variant or recombinant polypeptide or active fragment thereof are numbered by correspondence with the amino acid sequence of SEQ ID NO:10.

6. The protease variant, or a recombinant polypeptide or an active fragment thereof of any preceding claim, wherein said variant or recombinant polypeptide or active fragment thereof further comprises an EPNALQDGNGHGTH (SEQ ID NO:44) motif.

7. The protease variant, or recombinant polypeptide or active fragment thereof of Claim 6, wherein said motif extends from residue E54 to H67, wherein the amino acid positions are numbered by correspondence with the amino acid sequence of SEQ ID NO:10.

8. The protease variant, or recombinant polypeptide or active fragment thereof of any preceding claim, wherein said protease variant, or recombinant polypeptide or active fragment thereof has protease activity in the presence of a surfactant, cleaning activity in a detergent composition, or a combination thereof, optionally wherein the detergent composition is an automatic dish washing detergent or a laundry detergent and/or the cleaning activity comprises hydrolysis of a substrate on a protein stain.

9. The protease variant, or recombinant polypeptide or active fragment thereof of any preceding claim, wherein said protease variant, or recombinant polypeptide or active fragment thereof has one or more improved property selected from improved cleaning performance, improved stability, and improved residual protease activity when compared to SEQ ID NO:10 and/or 48, optionally wherein said improved property is
(i) improved residual protease activity and said protease variant, or recombinant polypeptide or active fragment thereof has 15-100%, 20-100%, 25-100%, 30-100%, 35-100%, or 40-100% residual protease activity when measured in accordance with the Stability Assay of Example 1;
(ii) improved cleaning performance and said protease variant, or recombinant polypeptide or active fragment thereof has a BMI cleaning PI of ≥0.7 in comparison to SEQ ID NO:48 when measured in accordance with the Cleaning Performance in Detergent assay of Example 1;
(iii) improved stability and said protease variant, or recombinant polypeptide or active fragment thereof has a stability PI ≥1.01 or 1.1 or 1.2 in comparison to SEQ ID NO:10 when measured in accordance with the Stability Assay of Example 1; and/or
(iv) improved cleaning performance and said protease variant, or recombinant polypeptide or active fragment thereof has a cleaning PI ≥1.01 or 1.1 or 1.2 in comparison to SEQ ID NO:10 when measured in accordance with the Cleaning Performance in Detergent Assay of Example 1.

10. The protease variant, or recombinant polypeptide or active fragment thereof of any preceding claim, wherein said protease variant, or recombinant polypeptide or active fragment thereof is derived from a reference polypeptide selected from SEQ ID NOs:10, 18, 19, 45, 46, 57, 58, and 66, optionally wherein the reference polypeptide is SEQ ID NO:10 and said protease variant, or recombinant polypeptide or active fragment thereof has one or more substitutions in one or more molecular segments selected from Segment 1 comprising positions 76-79 and 86, Segment 2 comprising positions 97-148, Segment 3 comprising positions 150-170, and Segment 4 comprising positions 207-220.

11. An LG12-Clade protease comprising a protease variant or a recombinant polypeptide or an active fragment thereof of any one of claims 1-10.

12. A cleaning composition comprising the protease variant, or recombinant polypeptide or active fragment thereof of any one of claims 1-10.

13. The cleaning composition of claim 12, wherein:
(a) said composition further comprises a surfactant; at least one calcium ion and/or zinc ion; at least one stabilizer; from about 0.001% to about 1.0 weight % of said recombinant polypeptide; at least one bleaching agent; at least one adjunct ingredient; one or more additional enzymes or enzyme derivatives selected from the group consisting of acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, xylosidases, metalloproteases, additional serine proteases, or a combination thereof; and/or
(b) said composition contains phosphate, is phosphate-free, contains borate, is boron-free, or combinations thereof.

14. A method of cleaning comprising contacting a surface or an item in need of cleaning with the protease variant, or recombinant polypeptide or active fragment thereof of any one of claims 1-10 or the cleaning composition of any one of claims 12-13; and optionally further comprising the step of rinsing said surface or item after contacting said surface or item with said variant or composition.

15. A polynucleotide comprising a nucleic acid sequence that encodes the protease variant, or recombinant polypeptide or active fragment thereof of any one of claims 1-10.
